# EUROPEAN PATENT SPECIFICATION

(11) **EP 3 724 232 B1**
(45) Date of publication and mention of the grant of the patent: **04.12.2024**
(21) Application number: 18888333.4
(22) Date of filing: 14.12.2018
(51) Int. Cl.: C07K 19/00, A61K 47/64, A61K 47/66, A61K 47/68, A61K 9/50, A61P 25/28, A61P 9/10, C07K 14/47, C07K 16/00, C12N 15/62, C12N 15/85, C12N 15/86, C12N 5/10, C12N 7/01, C12P 21/02, C07K 14/705, C07K 16/18, C12N 9/64

(54) **EXOSOME PACKAGING AND TARGETED AUTOPHAGY**
EXOSOM-VERPACKUNG UND ZIELGERICHTETE AUTOPHAGIE
ENCAPSULATION D'EXOSOMES ET AUTOPHAGIE CIBLÉE

(30) Priority: 14.12.2017 US 201762598622 P
(43) Date of publication of application: 21.10.2020
(73) Proprietor: The University of Ottawa, Ottawa, Ontario K1N 6N5 (CA)
(72) Inventor: GIBBINGS, Derrick, Ottawa, Ontario K1C 1E2 (CA)
(74) Representative: D Young & Co LLP
(86) International application number: PCT/CA2018/051605
(87) International publication number: WO 2019/113711

(56) References cited:
- WO-A2-2007/098060
- WO-A2-2007/098060
- CA-A1- 2 997 811
- US-A1- 2017 145 064
- TAMAKI Y ET AL: "Objective SHIFT 5 -MOTOR NEURON DISEASE Degradative intrabody for selective elimination of pathogenic TDP-43 aggregates in vitro and in murine embryos' cerebrum", 15 October 2017 (2017-10-15), XP093020371, Retrieved from the Internet <URL:https://www.jns-journal.com/action/showPdf?pii=S0022-510X(17)32511-X> [retrieved on 20230202], DOI: https://doi.org/10.1016/j.jns.2017.08.2014
- WU HAIJIAN ET AL: "Crosstalk Between Macroautophagy and Chaperone-Mediated Autophagy: Implications for the Treatment of Neurological Diseases", MOLECULAR NEUROBIOLOGY, SPRINGER US, NEW YORK, vol. 52, no. 3, 21 October 2014 (2014-10-21), pages 1284 - 1296, XP036181665, ISSN: 0893-7648, [retrieved on 20141021], DOI: 10.1007/S12035-014-8933-0
- CHEN CLAIRE C ET AL: "Elucidation of Exosome Migration Across the Blood-Brain Barrier ModelIn Vitro", CELLULAR AND MOLECULAR BIOENGINEERING, SPRINGER INTERNATIONAL PUBLISHING, CHAM, vol. 9, no. 4, 7 July 2016 (2016-07-07), pages 509 - 529, XP036100191, ISSN: 1865-5025, [retrieved on 20160707], DOI: 10.1007/S12195-016-0458-3
- P. M. LONG ET AL: "Cell Biology and Signaling", NEURO-ONCOLOGY, vol. 12, no. Supplement 4, 29 November 2010 (2010-11-29), pages iv7 - iv25, XP055126556, ISSN: 1522-8517, DOI: 10.1093/neuonc/noq116.s2
- ARYANI ARIAN ET AL: "Exosomes as a Nanodelivery System: a Key to the Future of Neuromedicine?", MOLECULAR NEUROBIOLOGY, HUMANA PRESS, US, vol. 53, no. 2, 15 December 2014 (2014-12-15), pages 818 - 834, XP035953030, ISSN: 0893-7648, [retrieved on 20141215], DOI: 10.1007/S12035-014-9054-5
- GUO, H. ET AL.: "Atg5 Disassociates the V1 V0-ATPase to Promote Exosome Production and Tumor Metastasis Independent of Canonical Macroautophagy", DEVELOPMENTAL CELL, vol. 43, 18 December 2017 (2017-12-18), pages 716 - 730, XP085314270
- CHEN, C. C. ET AL.: "Elucidation of Exosome Migration across the Blood-Brain Barrier Model In Vitro", CELL MOL BIOENG, vol. 9, no. 4, December 2016 (2016-12-01), pages 509 - 529, XP036100191, doi:10.1007/s12195-016-0458-3
- ARYANI, A.: "Exosomes as alpha Nanodelivery System: alpha Key to the Future of Neuromedicine?", MOL NEUROBIOL, vol. 53, no. 2, March 2016 (2016-03-01), pages 818 - 834, XP035953030

## Description

### FIELD OF INVENTION

The present invention relates generally to exosome packaging. More specifically, the present invention relates to exosome packaging of protein-containing agents and uses of the packaged exosomes for cellular delivery and/or targeted autophagy.

### BACKGROUND

Misfolded proteins, protein inclusions, and/or protein aggregates are believed to cause neurodegenerative pathology in a spectrum of diseases including Alzheimer's, Parkinson's, Frontal Temporal Dementia and/or Amyotrophic Lateral Sclerosis (ALS). Recombinant antibodies are an emerging class of therapeutics with potential for treating central nervous system (CNS) diseases by specifically recognizing disease-associated misfolded proteins/inclusions/aggregates.

However, treating neurodegenerative diseases with antibodies has so far presented multiple challenges. In particular, it is believed that only a small percentage of antibodies from the blood actually enter the brain, and even less actually enters cells of the brain. A principal limitation is likely poor blood-brain barrier (BBB) permeability. Typically, less than about 1% of antibody administered by intravenous injection is believed to enter the brain parenchyma, which is where targeting of misfolded proteins involved in neurodegenerative diseases would occur. Current strategies to increase antibody delivery to the brain typically undesirably reduce the bivalency and affinity of antibodies for their ligands, which may be detrimental for differentiating misfolded proteins from native protein in neurodegenerative diseases, for example. Antibodies are not the only protein-based active agents for which delivery to the brain (and/or other particular tissues of the body) has proven difficult and/or ineffective, indeed this is a challenging facing may protein-based active agents.

A substantial portion of misfolded proteins in neurodegenerative disease, such as TDP-43, Tau, or alpha-Synuclein, are believed to be located inside cells and believed to form inclusions in the cytoplasm where they are largely inaccessible to antibodies. Antibodies typically cannot readily traverse the cell's plasma membrane in such circumstances. Several strategies aiming to express antibodies, or antibody variants like nanobodies, in the cytoplasm of cells have been developed. For example, antibodies introduced in the genome of adeno-associated viruses (AAV) may be expressed in many cells after infection of patients with AAV. However, AAV treatments are believed to be suboptimum given expression difficulties and/or pre-existing immunity that may prevent AAV from being used in many patients. Accordingly, strategies to increase antibody entry into the brain and/or antibody entry into cells in the brain are highly desirable, particularly in developing antibody therapeutics for neurodegenerative diseases, for example. Furthermore, the ability of traditional antibodies to enter cells and clear certain targets, such as large aggregates of misfolded proteins, may be limited.

WO 2007/098060 A2 is directed to the targeting of proteins or peptides of interest to an autophagosome. Jiang et al., "Cell Biology And Signaling", Abstract CB-69, Neuro-Oncology, Vol. 12, No. Supplement 4, 2010 discloses an autophagy flux study with double-labelled EGFP-mRFP-LC3 fusion protein. US 2017/145064 A1 discloses a fusion protein including a LC3 protein which includes a first tag and a protein which includes a second tag. Tamaki et al., Abstract 1984; WCN17-1445, Journal of the Neurological Sciences, 381, (2017), page 715 relates to a degradative intrabody for selective elimination of TDP-43 aggregates in vitro and in murine embryos' cerebrum. Chen et al., Cellular and Molecular Bioengineering, Vol. 9, No. 4, 2016, p509-529 relates to the elucidation of exosome migration across the blood-brain barrier model in vitro.

Alternative, additional, and/or improved methods for exosome packaging, packaged exosome compositions, fusion constructs, and uses thereof, is desirable.

### SUMMARY OF INVENTION

Delivery of protein-containing active agents such as, but not limited to, antibodies and antibody-like proteins to the certain tissues/cells such as those of the brain has proven challenging. Poor cellular delivery, combined with only limited ability of traditional antibodies to enter cells and clear certain targets such as large aggregates of misfolded proteins, has traditionally hindered treatment of neurodegenerative diseases such as Alzheimer's, Parkinson's, Frontal Temporal Dementia and/or Amyotrophic Lateral Sclerosis (ALS), for example.

Described herein are methods for packaging exosomes with one or more active agents of interest such as, but not limited to, antibodies, antigen-binding fragments thereof, and/or antibody derivatives such as scFVs and/or nanobodies, for example. Also described herein are protein-containing constructs designed for exosome packaging, and exosomes packaged with such cargo interest which are designed for enhancing cellular delivery of their cargo into cells such as cells of the brain. In certain embodiments, constructs and active agents described herein may be designed for targeting one or more undesirable or disease-associated cellular targets (such as, but not limited to, misfolded proteins, protein inclusions, and/or protein aggregates), and in certain embodiments may further be designed for triggering targeted autophagy/degradation of the undesirable or disease-associated cellular target(s).

In an embodiment, there is provided herein a fusion construct comprising a biologically active agent functionally linked with a functional moiety comprising an LC3 or GABARAP protein, wherein the biologically active agent comprises an antibody, an antigen-binding fragment thereof, a single-chain variable fragment (scFv), or a nanobody which specifically binds a cellular target comprising misfolded proteins, protein inclusions, protein aggregates, lipid droplets, or mitochondria.

In another embodiment of the above fusion construct or fusion constructs, the biologically active agent may comprise an antibody, an antigen-binding portion thereof, or an antibody derivative or mimic, wherein the antibody derivative or mimic is a single-chain variable fragment (scFv) or a nanobody from a camel, llama, shark, or other animal.

In still another embodiment of the above fusion construct or fusion constructs, the cellular target may be associated with a neurodegenerative disease, non-alcoholic steatohepatitis, inflammatory disease, autoimmune disease, or another disease or disorder relating to or treatable by autophagy.

In yet another embodiment of the above fusion construct or fusion constructs, the misfolded proteins, altered protein, protein inclusions, and/or protein aggregates may comprise TDP-43 inclusions found in ALS patients; Tau fibrils found in Alzheimer's, CTE, Corticobasal Degeneration, Progressive Supranuclear Palsy, and/or other Tauopathies patients; synuclein or Alpha-synuclein or Lewy bodies and/or damaged mitochondria found in Parkinson's patients; Htt repeats in Huntington's disease; dipeptide repeats produced by C9ORF72 intronic repeats; misfolded SOD1; and/or hyperphosphorylated or fibrillary Tau.

In another embodiment of the above fusion construct or fusion constructs, the biologically active agent may be functionally linked with the functional moiety directly, or indirectly via one or more linkers and/or intervening groups.

In still another embodiment of the above fusion construct or fusion constructs, the biologically active agent may be functionally linked to an N-terminal portion or end of the functional moiety.

In yet another embodiment of the above fusion construct or fusion constructs, the biologically active agent may be functionally linked to a C-terminal portion or end of the functional moiety.

In still another embodiment of the above fusion construct or fusion constructs, the functional moiety may comprise LC3 protein.

In yet another embodiment of the above fusion construct or fusion constructs, the LC3 protein may comprise an LC3A, LC3B, LC3C, GABARAP, GABARAPL1, GABARAPL2, or GABARAPL3 homologue.

In another embodiment of the above fusion construct or fusion constructs, the LC3 protein may comprise LC3A.

In still another embodiment of the above fusion construct or fusion constructs, the LC3 protein may comprise LC3B.

In yet another embodiment of the above fusion construct or fusion constructs, the biologically active agent may comprise anti-TDP43 scFv clone VH7; anti-TDP-43 scFv 4aD1; anti-Tau scFv 4A3; anti-Tau scFv 4E4; adipophilin; perilipin2; PINK1; or Parkin.

In still another embodiment, there is provided herein a nucleic acid encoding a fusion construct as defined herein.

In yet another embodiment, there is provided herein an expression vector for expressing the fusion construct as defined herein.

In another embodiment, there is provided herein a host cell comprising a nucleic acid as described herein or an expression vector as described herein, the host cell expressing the fusion construct as described herein.

In another embodiment of the above host cell, the host cell may produce exosomes comprising a fusion construct as described herein.

In yet another embodiment of the above host cell or host cells, the host cell may have low or reduced levels of Atg7, or another protein required for autophagy such as Atg4, Atg14, Atg3, Atg14, or Atg12.

In still another embodiment of the above host cell or host cells, the host cell may be Atg7^{-/-}.

In yet another embodiment of the above host cell or host cells, the host cell may be a 293 cell; a human neonatal fibroblast; an NSC-34 cell; an SH5Y cell; or a BV2 cell.

In still another embodiment, there is provided herein an exosome comprising the fusion construct as described herein.

In yet another embodiment, there is provided herein a composition comprising one or more of a fusion construct as described herein; a nucleic acid as described herein; an expression vector as described herein; a host cell as described herein; and/or an exosome as described herein, further comprising an autophagy-activating agent, an siRNA or other gene silencing agent, or both; and, optionally, a pharmaceutically acceptable excipient, carrier, or diluent.

In yet another embodiment of the above composition or compositions, the autophagy-activating agent may comprise an mTOR inhibitor.

In still embodiment of the above composition or compositions, the autophagy-activating agent may comprise one or more of rapamycin, sirolimus, eversolimus, tacrolimus, INK128, pp242, starvation, or other mTORC1 and/or mTORC inhibitor.

In still another embodiment of the above composition or compositions, the autophagy-activating agent may comprise Trehalose and/or Beclin1 peptide.

In another embodiment, there is provided herein a method for packaging a fusion construct as described herein into an exosome, said method comprising:
expressing the fusion construct in an exosome-producing cell or otherwise introducing the fusion construct into the exosome-producing cell; and
culturing the exosome-producing cell in a cell media under conditions in which the exosome-producing cell generates and secretes exosomes comprising the fusion construct into the cell media.

In another embodiment of the above method, the method may further comprise a step of obtaining, isolating, or purifying the secreted exosomes comprising the fusion construct from the cell media.

In still another embodiment of the above method or methods, the exosome-producing cell may have low or reduced levels of Atg7.

In still another embodiment of the above method or methods, the exosome-producing cell may be Atg7^{-/-}.

In yet another embodiment of the above method or methods, the exosome-producing cell may be a 293 cell; a human neonatal fibroblast; an NSC-34 cell; an SH5Y cell; or a BV2 cell.

In still another embodiment of the above method or methods, the exosome-producing cell may be a host cell as described herein.

In yet another embodiment of the above method or methods, the secreted exosomes may further comprise an autophagy-activating agent, a gene silencing nucleic acid, or both.

In yet another embodiment, there is provided herein an exosome produced by any of the method or methods described herein.

In another embodiment, there is provided herein an in vitro method for delivering a biologically active agent into a cell, said method comprising:
expressing or introducing a fusion construct as described herein into an exosome-producing cell; and
culturing the exosome-producing cell in a cell media under conditions in which the exosome-producing cell generates and secretes exosomes comprising the fusion construct into the cell media;
obtaining, isolating, or purifying the secreted exosomes comprising the fusion construct from the cell media; and
contacting the cell with the secreted exosomes.

In still another embodiment of the above method or methods, the exosome-producing cell may have low or reduced levels of Atg7.

In yet another embodiment of the above method or methods, the exosome-producing cell may be Atg7^{-/-}.

In another embodiment of the above method or methods, the exosome-producing cell may be a 293 cell; a human neonatal fibroblast; an NSC-34 cell; an SH5Y cell; or a BV2 cell.

In still another embodiment of the above method or methods, the exosome-producing cell may be a 293 cell and the cell is a liver cell, a fibroblast cell, or a motor neuron.

In still another embodiment of the above method or methods, the exosome-producing cell may be a human neonatal fibroblast, and the cell is a liver cell, a brain cell, a spinal cord cell, or a kidney cell.

In another embodiment of the above method or methods, wherein the exosome-producing cell may be an NSC-34 cell, and the cell is a liver cell, a small intestine cell, a brain cell, or a spinal cord cell.

In yet another embodiment of the above method or methods, the exosome-producing cell may be a SH5Y cell, and the cell is a liver cell, a kidney cell, or a brain cell.

In another embodiment of the above method or methods, the exosome-producing cell may be a host cell as described herein.

In still another embodiment of the above method or methods, the secreted exosomes comprising the fusion construct may further comprise an autophagy-activating agent, a gene silencing nucleic acid, or both.

In another embodiment, there is provided herein a fusion construct as described herein, a nucleic acid as described herein; an expression vector as described herein; a host cell as described herein; an exosome as described herein; a composition as described herein; or any combination thereof; for use in treating or preventing a disease or disorder in a subject in need thereof.

In another embodiment of the above use or uses, the disease or disorder may be caused by, or associated with, any one or more of misfolded proteins, altered proteins, protein inclusions, protein aggregates, lipid droplets, bacteria, cytoplasmic DNA, or mitochondria.

In another embodiment of the above use or uses, the disease or disorder may be a neurodegenerative disease or atherosclerosis.

In another embodiment of the above use or uses, the disease or disorder may be Alzheimer's, CTE, and/or Tauopathy-related disease; Parkinson's; Frontal Temporal Dementia; and/or Amyotrophic Lateral Sclerosis (ALS).

In yet another embodiment, the disease or disorder may be a neurodegenerative disease or atherosclerosis or NASH or a viral infection.

In another embodiment of the above use or uses, a fusion construct as described herein; a nucleic acid a fusion construct as described herein; an expression vector a fusion construct as described herein; a host cell a fusion construct as described herein; an exosome a fusion construct as described herein; a composition a fusion construct as described herein; an exosome a fusion construct as described herein; or any combination thereof; may be for use in combination with at least one autophagy-activating agent, a gene silencing agent, or both.

In another embodiment of the above use or uses, the autophagy-activating agent may comprise an mTOR inhibitor.

In another embodiment of the above use or uses, the autophagy-activating agent may comrpise one or more of rapamycin, sirolimus, eversolimus, tacrolimus, INK128, pp242, starvation, or other mTORC1 and/or mTORC inhibitor.

In another embodiment of the above use or uses, the autophagy-activating agent may comprise Trehalose and/or Beclin1 peptide.

In another embodiment of the above use or uses, the gene silencing agent may target a cellular target which is also targeted by the biologically active agent of the fusion construct.

### BRIEF DESCRIPTION OF DRAWINGS

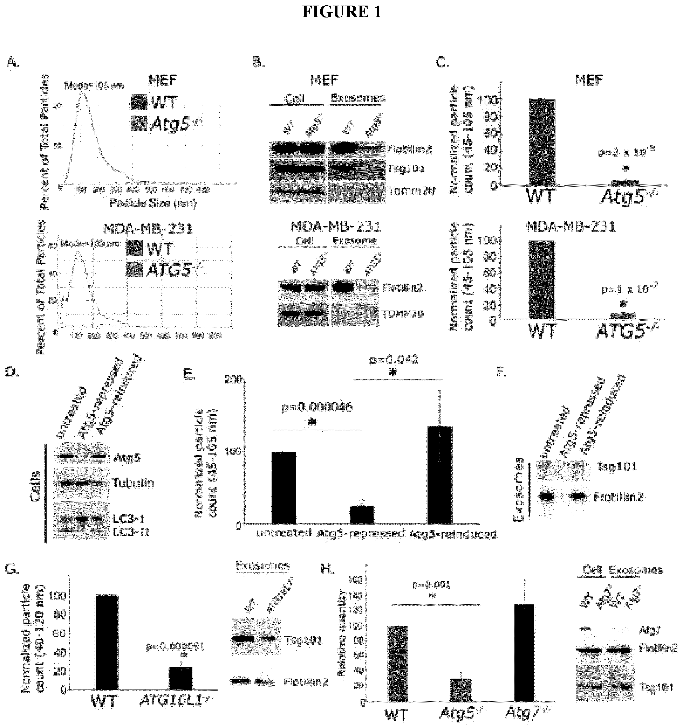

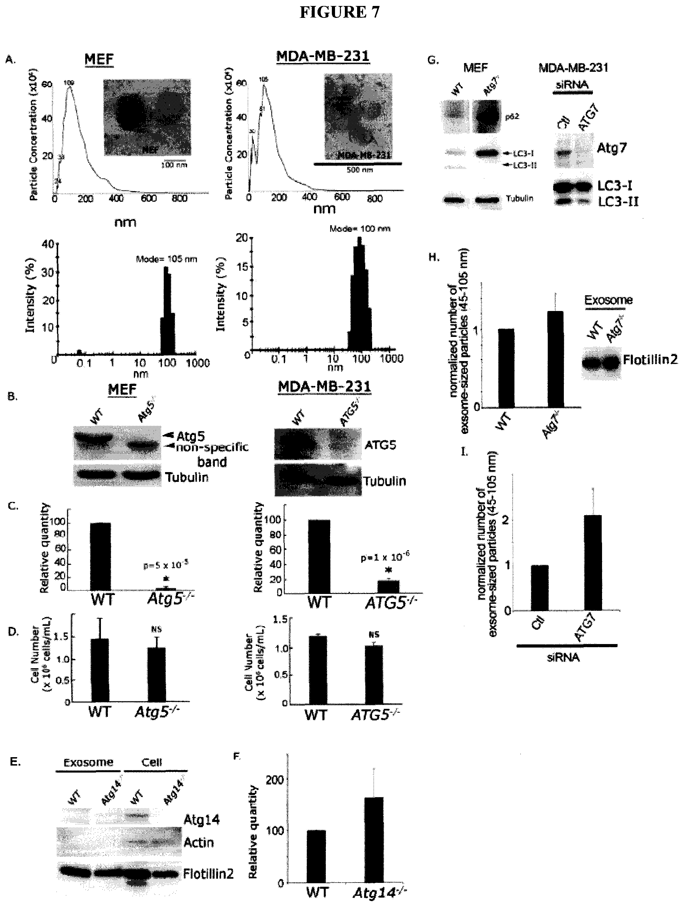

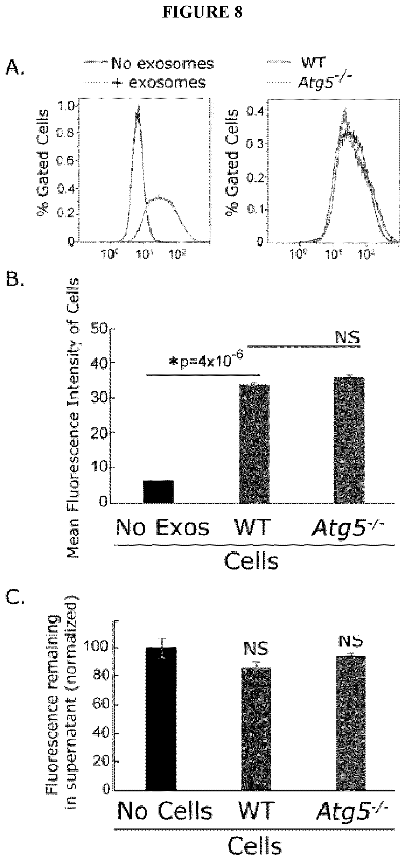

Figure 1. Exosome abundance is reduced in supernatants of Atg^{5-/-} (note, "-/-"is intended to identify a "double knockout") and Atgl6L1^{-/-} cells. (A) Representative histograms of a Nanosight Nanoparticle tracking analysis of exosomes produced by MEF and MDA-MB-231 wild-type and Atg5^{-/-} cells showing percent of total particles (y-axis) vs. size (nm, x-axis). (B) Western blot of Flotillin2, Tsg101, and Tomm20 (negative control) in equal amounts of total cell lysates and exosome preparations from equal amounts of wild-type and Atg5^{-/-} MEF and MDA-MB-231 cells. Irrelevant lanes between cell and exosome samples were removed. (C) Normalized quantification of exosome-sized particles (45-105 nm) released by wild-type and Atg5^{-/-} MEF and MDA-MB-231 cells using Nanoparticle tracking analysis (n=3). (D) Western blot of Atg5 and LC3 in MEF cells with doxycycline-repressed Atg5 cultured in parallel either in DMEM (untreated), DMEM containing doxycycline (AtgS-repressed), or DMEM containing doxycycline that was subsequently removed for 2 days (Atg5 re-induced). (E) Quantification of particles in exosome preparations from MEF with doxycycline-repressed Atg5 treated as in (D) using Nanoparticle tracking (n=3). (F) Western blot of Tsg101 and Flotillin2 in exosome preparations from equivalent amounts of MEF cells with doxycycline-repressible Atg5. (G) Quantification of particles in exosome preparations from wild-type and ATG16L1^{-/-} HCT-116 using Nanoparticle tracking (n=3) and western blot for exosome markers Tsg101 and Flotillin2 in exosome preparations from equal amounts of cells. (H) Quantification of particles in exosome preparations from wild-type, Atg5^{-/-} and Atg7^{-/-} MEF using dynamic light scattering intensity (n=3). Western blots quantify exosome markers in exosome preparations (Tsg101, Flotillin2) produced by equal amounts of cells and confirm Atg7 knockout in cells. At times, western blots were run on the same samples on separate gels in parallel. Data are represented as mean +/-SEM. See also Fig.7 and 8;

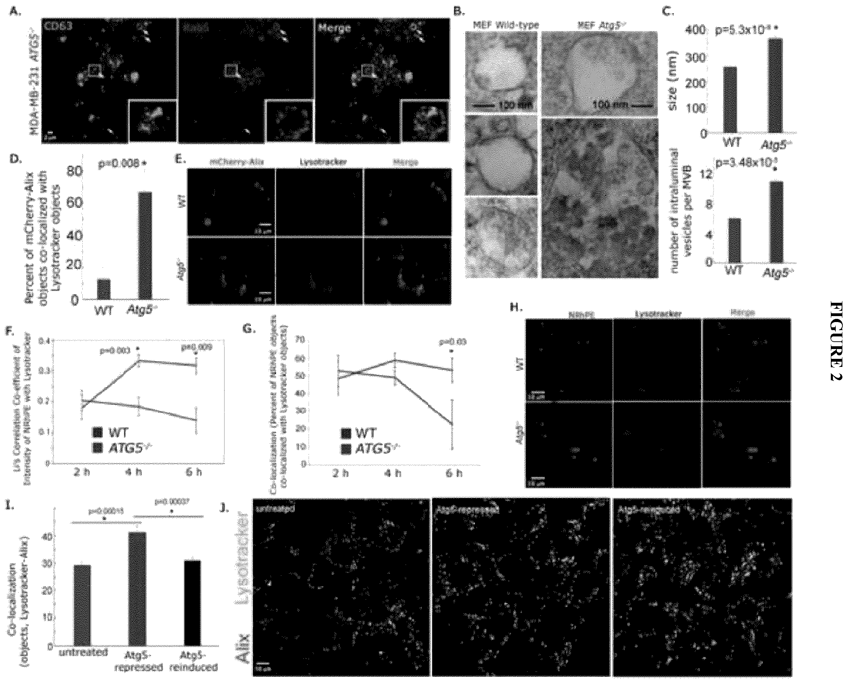

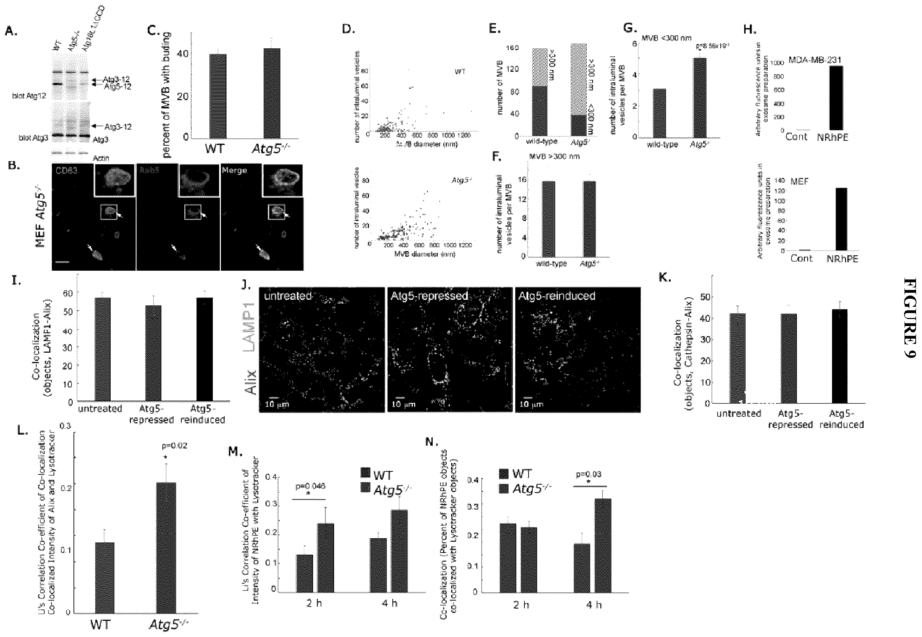

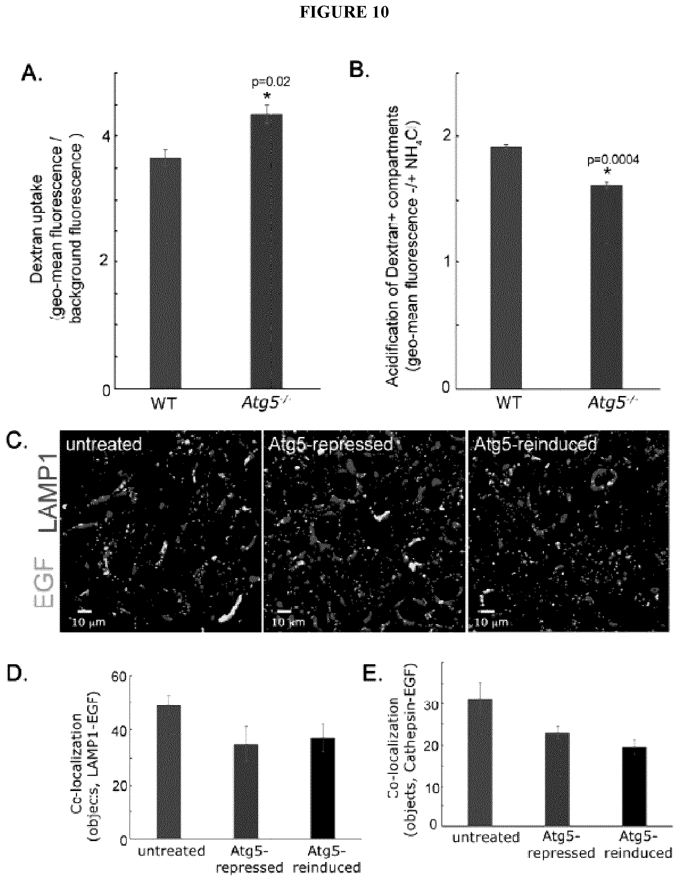

Figure 2. Loss of Atg5 increases MVB size, intraluminal vesicle number and acidification. (A) STED microscopy of the exosome marker CD63 and the early endosome/MVB marker Rab5 in ATG5^{-/-} MDA-MB-231 cells demonstrating punctate CD63 staining within Rab5+ endosomes. Scale bar = 2µm. (B) Representative electron microscopy images of MVB in wild-type and Atg5^{-/-} MEF cells. Scale of all images in wild-type or Atg5^{-/-} group are identical, scale bar represents 100 nm. (C) Average size of MVB (upper) and number of intraluminal vesicles per MVB (lower) in wild-type and Atg5^{-/-} MEF, Wild-type 167 MVB, Atg5^{-/-} MEF 159, Wild-type 996 intraluminal vesicles, Atg5^{-/-} MEF 1726 intraluminal vesicles. Electron microscopy data derived from two independent biological replicates. (D) Quantification of the co-localization of mCherry-Alix with Lysotracker in wild-type and Atg5^{-/-} MEF using automated segmentation and quantification of objects. A total of 75 wild-type MEF and 51 Atg5^{-/-} MEF cells were quantified. (E) Representative images of co-localization of mCherry-Alix with Lysotracker in wild-type and Atg5^{-/-} MEF. Scale bars represent 10 µm. (F,G) Quantification of the co-localization of the MVB marker NRhPE with Lysotracker using Li's correlation co-efficient (F) and automated segmentation and quantification of objects (G) in MDA-MB-231 wild-type and ATG5^{-/-} cells. A total of 30-70 cells were quantified in each condition. (H) Representative images of NRhPE with Lysotracker in MDA-MB-231 cells at 4 h. Scale bars represent 10 um. (I) Quantification of the co-localization of mCherry-Alix with Lysotracker using automated segmentation and quantification of objects in MEF cells either untreated, with Atg5 repressed, or Atg5 re-induced as in Fig.1D. A total of 30-70 cells were quantified in each condition. (J) Representative images of mCherry-Alix with Lysotracker in MEF cells in (I). Scale bars represent 10 um. All data are represented as mean +/- SEM. See also Fig.9 and 10;

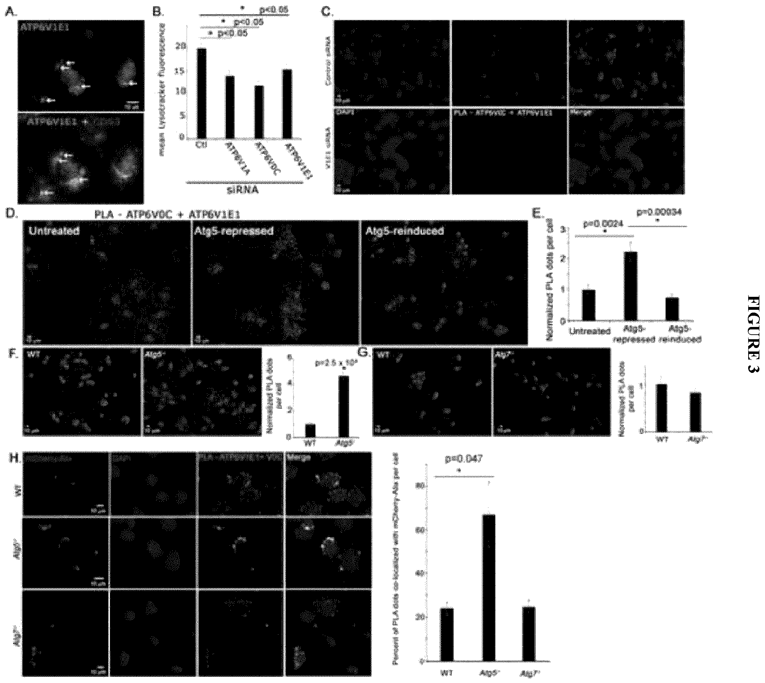

Figure 3. Atg5 disassociates ATP6V1E1 from the V₁V₀-ATPase. (A) Immunofluorescence microscopy of ATP6V1E1 showing its co-localization with the MVB and exosome marker Tsg101. Scale bars represent 10 um. (B) Mean Lysotracker fluorescence measured in cells treated with siRNA targeting ATP6V1E1, ATP6V1A, ATP6V0C or control non-specific RNA. (C) Representative fluorescent microscopy image of proximity ligation assay signals demonstrating association of ATP6V1E1 with the core V₁V₀-ATPase component ATP6V0C that is lost in cells treated with siRNA targeting ATP6V1E1. Scale bars represent 10 um. (D) Representative proximity ligation assay analyses of ATP6V1E1 with the core V₁V₀-ATPase component ATP6VOC in MEF cells that were untreated, Atg5-repressed (+Doxycycline), or AtgS-reinduced. Scale bars represent 10 µm. (E) Quantification of proximity ligations assays of ATP6V1E1 with ATP6VOC as in (D) in MEF cells that were untreated, Atg5-repressed (+Doxycycline), or AtgS-reinduced. (F) Representative proximity ligation assay analyses of ATP6V1E1 with ATP6V0C in MEF wild-type and Atg5-/- cells and their quantification. Scale bars represent 10 um. (G) Representative proximity ligation assay analyses of ATP6V1E1 with ATP6VOC in MEF wild-type and Atg7^{-/-} cells and their quantification. Scale bars represent 10 um. (H) Representative proximity ligation assay analyses of ATP6V1E1 with ATP6VOC in MEF wild-type, Atg5^{-/-} and Atg7^{-/-} cells expressing mCherry-Alix. Graph of percent of mCherry-Alix punctae (MVB) that co-localize with proximity ligation assay signals of ATP6V1E1 with ATP6V0C. Scale bars represent 10 um. All data are represented as mean +/- SEM;

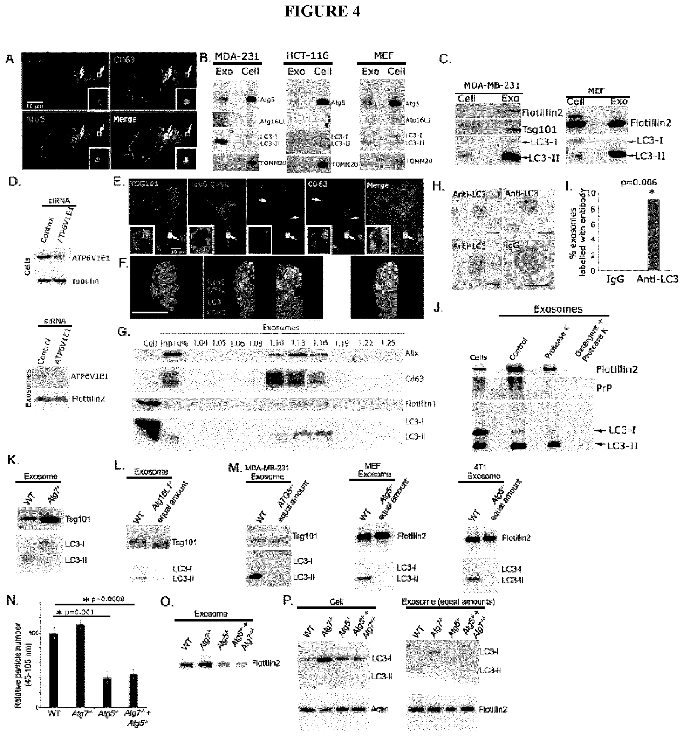

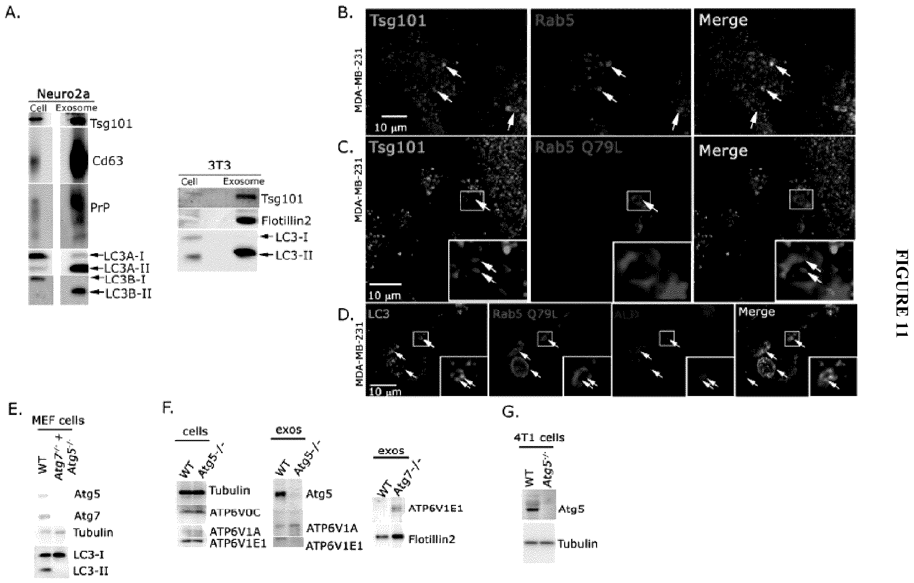

Figure 4. Atg5 recruits LC3 into exosomes. (A) Immunofluorescent microscopy highlighting Atg5 co-localization with punctae that are marked by both Rab5 and CD63 (white arrows). Scale bars represent 10 um. (B) Western blot of Atg5, Atg16L1 and LC3 in equivalent amounts of exosomes or cells of several cell types. (C) Western blot of LC3 and exosome markers in (Tsg101, Flotillin2) in equivalent amounts of exosomes or cells (MDA-MB-231 and MEF) (D) Western blot of ATP6V1El in cells treated with siRNA targeting ATP6V1E1 and the exosomes they produce. Equal amounts of exosomes were used to show reduced levels of ATP6V1E1-specific band in exosomes. (E) Confocal microscopy of Tsg101, CD63, LC3 and constitutively active dsRed-Rab5 Q79L in MDA-MB-231 cells. Scale bar = 10 µm (F) 3-D reconstruction of LC3 and CD63 inside endosomes labeled with constitutively active mutant dsRed-Rab5 Q79L in MDA-MB-231 cells. Scale bar = 1 µm (G) Western blot analysis of sucrose density gradient fractions of exosome preparations from Neuro2a cells for LC3 and exosome markers (Alix, CD63 and Flotillinl) showing partitioning of LC3 with exosomes at densities characteristic of exosomes. Inp=input. (H) lmmunogold electron microscopy with anti-LC3 antibody in exosome preparations of Neuro2a cells. Scale bars = 100 nm. (I) Quantification of percent of exosomes labeled with LC3 antibody by electron microscopy (647 exosomes total in 3 replicates, no labeling was observed in samples labeled with control antibody). (J) Western blot of equivalent amounts of exosomes from Neuro2a cells mock treated, treated with protease K or treated with detergent and protease K. Blots for the proteins on the surface of exosomes (PrP) or inside exosomes (Flotillin2) are controls for the protease and detergent treatments. (K) Western blot of LC3 and exosome marker Tsg101 in equivalent amounts of exosomes produced by wild-type and Atg7^{-/-} MEF to show alterations in specific cargoes in exosomes. (L) Western blot of LC3 and exosome marker Tsg101 in equivalent amounts of exosomes from wild-type and Atg16L1^{-/-} HCT-116 to show alterations in specific cargoes in exosomes. (M) Western blot of LC3 and exosome marker Tsg101 in equal amounts of exosomes produced by wild-type and Atg5^{-/-} MDA-MB-231, MEF or 4T1 cells to show alterations in specific cargoes in exosomes. (N) Quantification of particles in exosome preparations from equal numbers of wild-type and Atg7^{-/-}, Atg5^{-/-} and Atg5^{-/-} & Atg7^{-/-} MEF cells using Nanoparticle tracking (n=3) and (O) western blot for exosome markers Flotillin2 in exosome preparations from equal amounts of cells. (P) Western blot of cells for LC3 and Beta-actin and of LC3 and exosome marker Flotillin2 in equivalent amounts of exosomes from wild-type and Atg7^{-/-}, Atg5^{-/-} and Atg5^{-/-} & Atg7^{-/-} MEF to show alterations in specific cargoes in exosomes. Data are represented as mean +/- SEM. See also Fig.11;

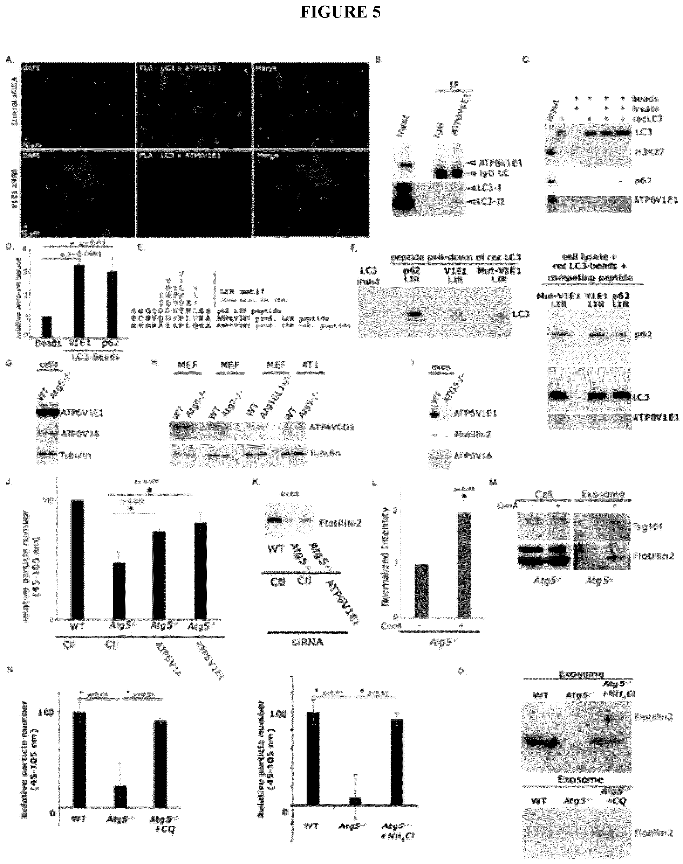

Figure 5. LC3 binds ATP6V1E1 and selectively removes it into exosomes to modulate exosome production. (A) Representative fluorescent microscopy of proximity ligation assay of ATP6V1E1 with LC3 in MEF. Signals are lost in cells treated with siRNA targeting ATP6V1E1 validating the specificity of the assay. Scale bars represent 10 µm. (B) Western blot of LC3 in immunoprecipitates of ATP6V1E1 compared to negative control IgG. IgG LC indicates the IgG light chain band found in both ATP6V1E1 and control IgG immunoprecipitates. (C) Western blot of known LC3-interacting protein p62 (positive control), non-LC3 interacting protein H3K27 (negative control) and ATP6V1E1 in pull-downs of recombinant LC3. (D) Quantification of the LC3 pulldown experiments in (C) over 3 independent experiments. (E) Top, Canonical LIR motif is color-coded for acidic (red), aromatic (blue) or hydrophobic (green) residues with preferential LIR-motif amino acids stacked vertically at each position. Bottom, alignment of canonical LIR motif peptide with established p62 LIR peptide and predicted LIR peptide in ATP6V1E1 or a mutated version thereof. (F) Left, Western blot of LC3 after pulldown of recombinant LC3 with biotinylated peptides from p62 LIR motif, ATP6V1E1 predicted LIR motif, and a mutated version thereof (negative control). Right, Western blot of p62 and ATP6V1E1 in pulldowns from cell lysates with recombinant LC3 in the presence of a competitive excess of the indicated peptides. (G) Western blot of ATP6V1E1 and other components of the V₁V₀-ATPase in wild-type and Atg5^{-/-} MEF cells (H) Western blot of ATP6V1E1 and other components of the V₁V₀-ATPase in wild-type, Atg5^{-/-}, Atg7^{-/-}, Atgl6L1^{-/-} MEF cells and Atg5^{-/-} 4T1 cells. (I) Western blot of ATP6V1E1 and other components of the V₁V₀-ATPase in equivalent amounts of exosomes produced by wild-type and ATG5^{-/-} MDA-MB-231 to show alterations in levels of specific cargoes in exosomes. (J) Quantification of exosome production by nanoparticle tracking analysis of particles (45-105 nm) in wild-type and Atg5^{-/-} MEF treated with control non-specific siRNA or siRNA targeting ATP6V1E1 or ATP6V1A. (K) Western blot of exosome marker Flotillin2 in exosome preparations produced by equivalent amounts of wild-type and Atg5^{-/-} MEF treated with control non-specific siRNA or siRNA targeting ATP6V1E1. (L) Quantification of exosome production using dynamic light scattering in Atg5^{-/-} MEF treated with Concanamycin A or mock treated. (M) Western blot of exosome markers Tsg101 and Flotillin2 in cells and exosome preparations from equivalent amounts of Atg5^{-/-} MEF treated with Concanamycin or mock treated. (N) Quantification of exosome production by nanoparticle tracking analysis of particles (45-105 nm) in wild-type and Atg5^{-/-} MEF either untreated or treated with Chloroquine (CQ) for 16 h (left) or with NH₄Cl for 30 min (right). (O) Quantification of exosome production by western blot for Flotillin2 in exosome preparations produced by wild-type and Atg5^{-/-} MEF either untreated or treated with 100 µM Chloroquine (CQ) for 16 h (bottom) or with 20 mM NH₄Cl for 30 min (top). Data are represented as mean +/- SEM;

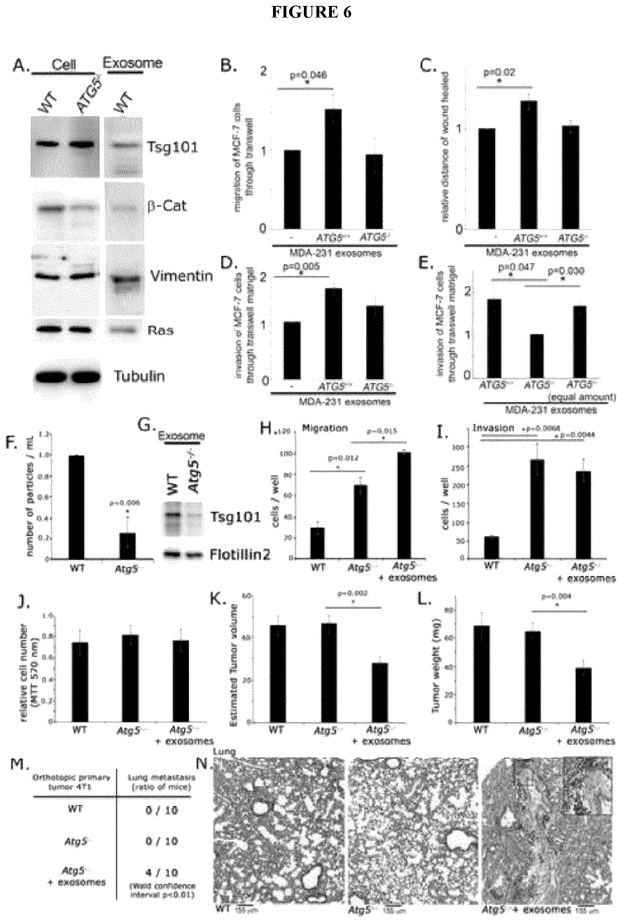

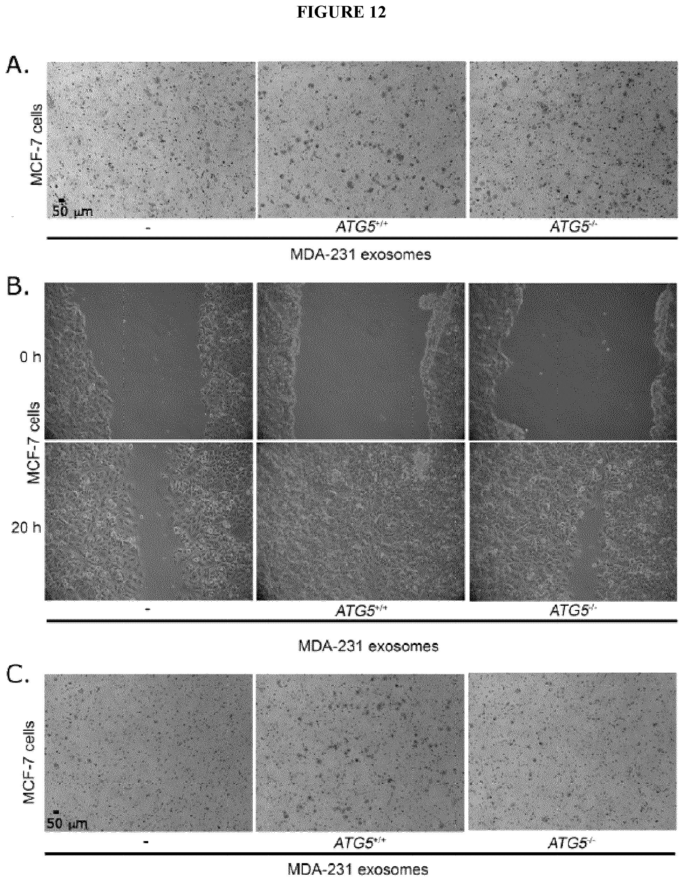

Figure 6. Atg5 promotes migration and metastasis of breast cancer cells by increasing exosome production. (A) Western blot of wild-type and ATG5^{-/-} MDA-MB-231 cells or wild-type exosomes for exosome markers (Tsg101) and proteins with roles in migration and metastasis (Vimentin, Ras, β-catenin). (B) Quantification of migration of MCF-7 cells through a transwell chamber upon incubation with no exosomes or exosomes from equivalent amounts of wild-type or ATG5^{-/-} MDA-MB-231 cells. (C) Quantification of wound closure by MCF-7 cells incubated with no exosomes or exosomes from equivalent amounts of wild-type or ATG5^{-/-} MDA-MB-231 cells. (D) Quantification of invasion of MCF-7 cells through a transwell chamber coated with matrigel upon incubation with no exosomes or exosomes from equivalent amounts of wild-type or ATG5^{-/-} MDA-MB-231 cells. (E) Quantification of invasion of MCF-7 cells through a transwell chamber coated with matrigel upon incubation with exosomes from wild-type MDA-MB-231 cells, exosomes from an equivalent number of MDA-MB-231 ATG5^{-/-} cells, or an equivalent amount of exosomes from ATG5^{-/-} cells as produced by wild-type cells (equal amount exosome). (F) Quantification of exosome production by nanoparticle tracking analysis of particles (45-105 nm) in wild-type and Atg5^{-/-} 4T1. (G) Western blot of exosome markers Tsg101 and Flotillin2 in exosome preparations produced by equivalent amounts of wild-type and Atg5^{-/-} 4T1 cells. (H) Quantification of migration through a transwell chamber of wild-type, Atg5^{-/-} or Atg5^{-/-} 4T1 cells supplemented with exosomes from wild-type 4T1 cells. (I) Quantification of invasion through a matrigel coated transwell chamber of wild-type, Atg5^{-/-} or Atg5^{-/-} 4T1 cells pretreated with exosomes from wild-type 4T1 cells. (J) Quantification of cell quantity by MTT assay of wild-type, Atg5^{-/-} or Atg5^{-/-} 4T1 cells pretreated with exosomes from wild-type 4T1 cells after 48 h. (K) Estimated volume of primary tumor extracted from the mammary pad of mice implanted 2 weeks prior with wild-type, Atg5^{-/-} or Atg5^{-/-} 4T1 cells pre-treated with wild-type exosomes. Volume = [width(2) x length]/2. (L) Tumor mass of primary tumor extracted from the mammary pad of mice implanted 2 weeks prior with wild-type, Atg5^{-/-} or Atg5^{-/-} 4T1 cells pre-treated with wild-type exosomes. (M) Table of number of mice with metastatic cancer nodes in at least one of 4 sections of the left lung lobe analyzed. (N) Images of haemotoxylin-eosin stained left lower lung lobe sections from mice implanted with wild-type, Atg5^{-/-} or Atg5^{-/-} 4T1 cells pre-treated with wild-type exosomes in the mammary pad three weeks after removal of the primary tumor. Scale bars represent 155 µm. Data are represented as mean +/- SEM. Western blots were run on separate gels in parallel using samples prepared in parallel. See also Fig. 12;
Figure 7. Atg5, but not Atg7 is required for exosome production. Also see Figure 1. (A) Representative histograms of Nanoparticle tracking analysis measuring percent total particles (y-axis) vs. size (nm, x-axis) of exosome preparations from MEF and MDA-MB-231. Inset, electron microscopy images of exosome preparations from MEF and MDA-MB-231 including scale bars (100 nm left, 500 nm right). Below, Dynamic light scattering histograms of exosome preparations representing intensity (relative number of particles) and size of particles. (B) Western blot of Atg5 and Tubulin (loading control) in equivalent amounts of wild-type and Atg5^{-/-} MEF and MDA-MB-231 cells. (C) Quantification by dynamic light scattering of particles in exosome preparations from wild-type and Atg5^{-/-} MEF and MDA-MB-231 cells. (D) Quantification of cell number by trypan blue exclusion (n=3) after culture of wild-type and Atg5^{-/-} MEF and MDA-MB-231 cells for exosome production in (C). (E) Western blot of Atg14, Flotillin2 (exosome marker), and Actin (loading control for cell lysates) in lysates from equal amounts of cells and exosomes produced by equal amounts of cells (to assess changes in exosome production). (F) Quantification by dynamic light scattering of particles in exosome preparations from wild-type and Atg14^{-/-} MEF cells (n=3). (G) Western blot of equivalent amounts of cell lysates from wild-type and Atg7^{-/-} MEF for LC3, autophagy substrate p62 and Tubulin (loading control). As expected, Atg7^{-/-} MEF do not convert LC3-I to LC3-1I and accumulate p62. Right, western blot of Atg7 and LC3 in equivalent amounts of MDA-MB-231 cells treated with control siRNA or siRNA targeting Atg7. Samples were run on the same gel and irrelevant lanes were excised in the Figure. (H) Nanoparticle tracking analysis quantification of exosome-sized particles (45-105 nm) released by wild-type and Atg7⁻¹⁻ MEF (n=3). Western blot of exosome marker Flotillin2 in exosome preparations produced by equal amounts of wild type and Atg7^{-/-} MEF cells. (I) Nanoparticle tracking analysis quantification of exosome-sized particles (45-105 nm) released by MDA-MB-231 cells treated with control or Atg7 targeting siRNA (n=3). Data are represented as mean +1- SEM;
Figure 8. Uptake of exosomes from the extracellular space in Atg5^{-/-} MEF is similar to wild-type MEF. See also Figure 1. (A) Representative flow cytometry histograms of cells incubated for 1 h with exosomes fluorescently labeled with Di0 dye. (B) Fluorescence of wild-type and Atg5^{-/-} MEF cells incubated with DiO-labeled exosome preparations from wild-type MEF cells for 1 h (n=3). Fluorescence was measured by flow cytometry (median). (C) Fluorescence remaining in supernatant after incubation of DiO-labeled exosome preparations with wild-type or Atg5^{-/-} MEF cells (n=3). Data are represented as mean +/- SEM;
Figure 9. Multivesicular bodies exhibit increased acidification in the absence of Atg5. See also Figure 2. (A) Western blot for Atg12, Atg3 and Actin (control) in equivalent amounts of wild-type, Atg5-/-, or Atg16l1^{ΔCCD} MEF. (B) Confocal microscopy of CD63 in Rab5+ organelles of Atg5^{-/-} MEF. Scale bar = 1µm. (C) Percent of MVB exhibiting budding of their membrane in wild-type and Atg5^{-/-} MEF. Wild-type 167 MVB, Atg5^{-/-} MEF 159 MVB. (D) Dot plot of number of intraluminal vesicles per MVB (y-axis) vs. MVB diameter (x-axis) in wild-type (WT) and Atg5^{-/-} MEF. A shift toward larger MVB with more intraluminal vesicles can be seen in Atg5^{-/-} MEF. Wild-type 996 intraluminal vesicles, Atg5^{-/-} MEF 1726 intraluminal vesicles. WT 167 MVB, Atg5^{-/-} MEF 159 MVB. (E) Number of MVB in wild-type and Atg5^{-/-} MEF stratified by size (<300 nm solid bars and >300 nm lined bars). 167 MVB, Atg5^{-/-} MEF 159 MVB. (F) Number of intraluminal vesicles per MVB of (>300 nm) in wild-type and Atg5^{-/-} MEF. Wild-type 38 MVB, 598 intraluminal vesicles; Atg5^{-/-} 90 MVB, 1382 intraluminal vesicles. (G) Number of intraluminal vesicles per MVB of (<300 nm) in wild-type and Atg5^{-/-} MEF. Wild-type 129 MVB, 298 intraluminal vesicles; Atg5^{-/-} 69 MVB, 347 intraluminal vesicles. All error bars represent SEM. (H) Quantification of NRhPE fluorescence in exosome preparations prepared by differential centrifugation from MDA-MB-231 and MEF treated with NRhPE or vehicle. (I-K) Co-localization of Alix with lysosomal markers (LAMP1, CathepsinB). Experiments were performed in MEF untreated, treated with 1 ng/mL doxycycline (2 d) to repress Atg5 (Atg-repressed) or treated with doxycycline and then left untreated for 2 d (Atg5-reinduced). (I) Quantification of co-localization of LAMP1 with Alix using automated segmentation and quantification of objects. (J) Representative images of mCherry-Alix co-localization with LAMP1 in MEF that were untreated, Atg5-repressed or AtgS-reinduced. LAMP1-tagBFP is pseudocolored green. Scale bar = 10 µm. (K) Quantification of co-localization of CathepsinB with Alix using object-based methods by SQASSH. (L) Quantification of the co-localization of mCherry-Alix with Lysotracker in wild-type or Atg5^{-/-} MEF using Li's correlation co-efficient of fluorescent intensity. A total of 75 wild-type MEF and 51 Atg5^{-/-} MEF cells were quantified. (M) Quantification of the co-localization of NRhPE with Lysotracker using Li's correlation co-efficient of fluorescent intensity in MEF wild-type and Atg5^{-/-} cells. (N) Quantification of the co-localization of NRhPE with Lysotracker using automated segmentation and quantification of objects in MEF wild-type and Atg5^{-/-} cells. Data are represented as mean +/- SEM;
Figure 10. Atg5 does not increase acidification of dextran-containing compartments or localization of EGF to lysosomes. See also Figure 2. (A) Uptake of pHrodo labeled dextran by wild-type and Atg5^{-/-} MEF. Geometric mean of fluorescent intensity was measured by flow cytometry. (B) Acidification of compartments containing pHrodo labeled dextran in wild-type and Atg5^{-/-} MEF after 20 minutes. Ratio of the geometric mean of fluorescent intensity measured by flow cytometry before vs. after addition of NH₄Cl to neutralize pH-dependent increase in fluorescence of pH-sensitive pHrodo dye. (C) Representative images of co-localization of Alexa488-EGF with LAMP1-tagBFP pseudocolored red. Scale bar = 10 µm. (D) Quantification of co-localization of LAMP1 with Alexa488-EGF using automated segmentation and quantification of objects. (E) Quantification of co-localization of Cathepsin with Alexa488-EGF automated segmentation and quantification of objects. Data are represented as mean +/- SEM;
Figure 11. LC3 is found in exosomes and Atg5, but not Atg7 controls sorting of ATP6V1El into exosomes. See also Figures 4 and 5. (A) Western blot analysis of equivalent amounts (µg protein) of exosome preparations and total cell lysate from 3T3 and Neuro2a cells for exosome markers (Tsg101, CD63, PrP, Flotillin2) and LC3. Neuro2A cells and exosomes were blotted for both LC3A and LC3B. Neuro2a cell and exosome samples were run on the same gel and irrelevant lanes were excised in the Figure. (B) Confocal microscopy of Tsg101 with endogenous Rab5 in MDA-MB-231 cells. Scale bar = 10 µm. (C) Confocal microscopy of Tsg101 with constitutively active mutant dsRed-Rab5 Q79L in MDA-MB-231 cells. Scale bar = 10 µm. (D) Confocal microscopy of LC3 and the exosome marker Alix with constitutively active mutant dsRed-Rab5 Q79L in MDA-MB-231 cells. Scale bar = 10 µm. (E) Western blot of Atg5, Atg7, LC3 and Tubulin in MEF cells genetically deleted of both Atg5 and Atg7. (F) Western blot of components of the V₁V₀ ATPase in equivalent amounts of exosomes produced by Atg5^{-/-} or Atg7^{-/-} MEF cells. (G) Western blot of Atg5 in equivalent amounts of wild-type and Atg5^{-/-} 4T1 cells compared to loading control (Tubulin);
Figure 12. Atg5 is required for exosome production that promotes wound healing, migration and invasion of MDA-MB-231 breast cancer cells. See also Figure 6. (A-C) Representative images of MCF-7 cells incubated without exosomes, or with exosomes from equivalent amounts of wild-type or ATG5^{-/-} MDA-MB-231 cells in (A) transwell migration assay (Scale bar = 50 µm) (B) wound healing assay (Images were acquired with a 4x objective lens) and (C) and transwell invasion assay (C). Cells in A and C were stained with crystal violet (Scale bar = 50 µm);

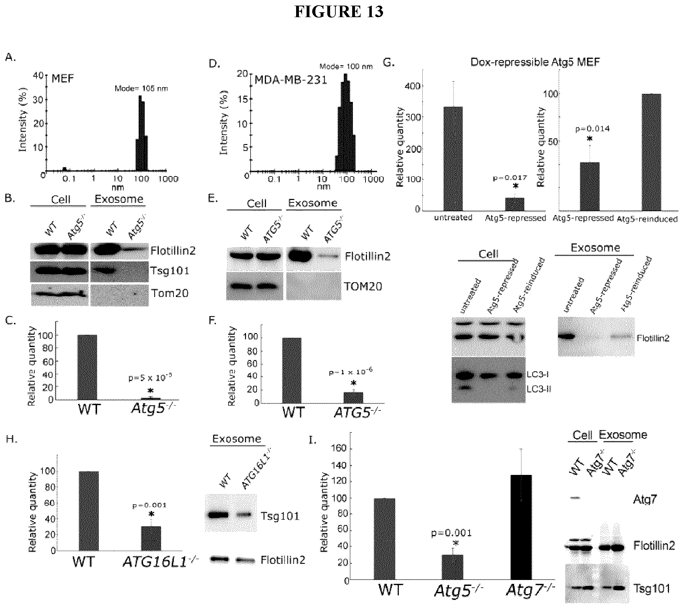

Figure 13. Exosome abundance is reduced in supernatants of Atg5^{-/-} and Atg16L1^{-/-} cells. (A) Representative histograms of dynamic light scattering intensities (y-axis) vs. binned size (nm, x-axis) of exosome preparations from MEF. (B) Western blot of Flotillin2, Tsg101, and Tom20 (negative control) in equal amounts of total cell lysates and exosome preparations from wild-type and Atg5^{-/-} MEF cells. (C) Quantification of particles in exosome preparations from wild-type and Atg5^{-/-} MEF cells using dynamic light scattering intensity (n=3). (D-F) As in (A-C) except with exosomes from MDA-MB-231 cells. (G) Quantification of particles in exosome preparations from MEF with doxycycline-repressed Atg5 using dynamic light scattering intensity (n=3) and western blot for exosome marker Flotillin2. (H) Quantification of particles in exosome preparations from wild-type and ATG16L1^{-/-} HCT-116 using dynamic light scattering intensity (n=3) and western blot for exosome markers. (I) Quantification of particles in exosome preparations from wild-type, Atg5^{-/-} and Atg7^{-/-} MEF using dynamic light scattering intensity (n=3) and western blot for exosome markers. Error bars represent SEM. Western blots were run on separate gels in parallel using samples prepared in parallel;

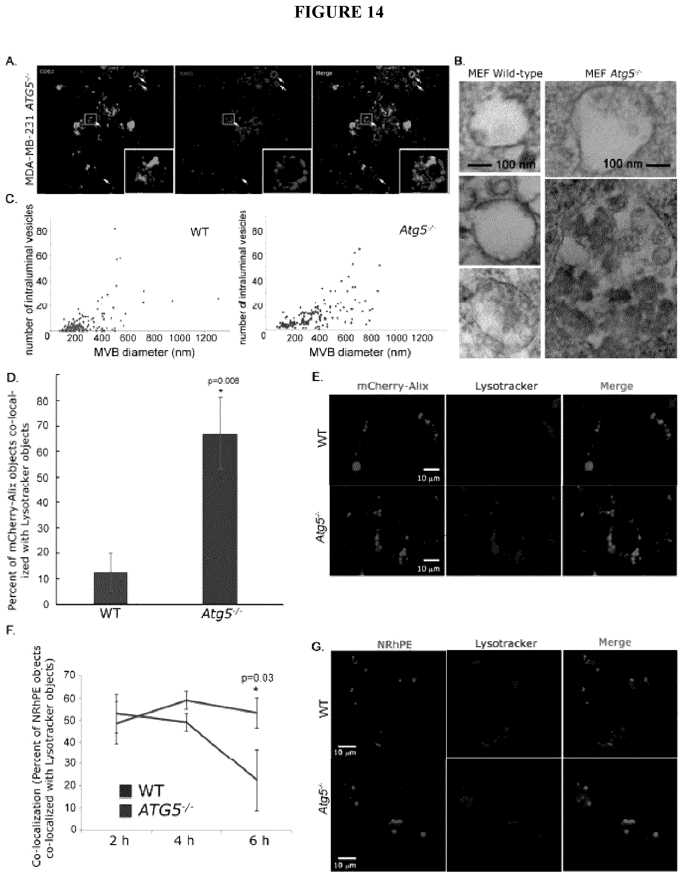

Figure 14. Loss of Atg5 increases MVB size, intraluminal vesicle number and acidification. (A) STED microscopy of CD63 and Rab5 in ATG5^{-/-} MDA-MB-231 cells. (B) Representative electron microscopy images of MVB in wild-type and Atg5^{-/-} MEF cells. Scale of all images are identical, scale bar represents 100 nm. (C) Scatter plot of diameter of MVB (x-axis) and number of intraluminal vesicles in MVB in wild-type and Atg5^{-/-} MEF. Wild-type 167 MVB, Atg5^{-/-} MEF 159, Wild-type 996 intraluminal vesicles, Atg5^{-/-} MEF 1726 intraluminal vesicles. Electron microscopy data derived from two independent biological replicates. (D) Quantification of the colocalization of mCherry-Alix with Lysotracker in wild-type and Atg5^{-/-} MEF using automated segmentation and quantification of objects. A total of 75 wild-type MEF and 51 Atg5^{-/-} MEF cells were quantified. (E) Representative images of co-localization of mCherry-Alix with Lysotracker in wild-type and Atg5^{-/-} MEF. (F) Quantification of the co-localization of NRhPE with Lysotracker using automated segmentation and quantification of objects in MDA-MB-231 wild-type and ATG5-/- cells. A total of 30-70 cells were quantified in each condition. (G) Representative images of NRhPE with Lysotracker in MDA-MB-231 cells at 4 h;

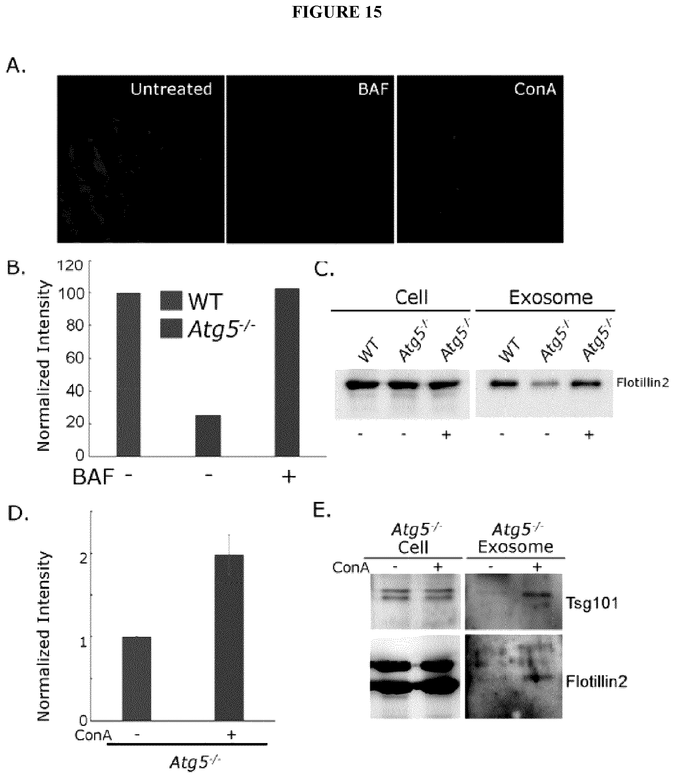

Figure 15. Exosome production is increased when MVB acidification is reversed with inhibitors of V₀ ATPase. (A) Labeling of MEF with pH-sensitive Lysotracker after 12 h treatment with Bafilomycin Al (BAF) or Concanamycin A (ConA). (B) Representative quantification of particles in exosome preparations using dynamic light scattering intensity from wild-type, Atg5^{-/-} , or BAF-treated Atg5^{-/-} MEF (n=3). (C) Western blot of Flotillin2 in wild-type, *Atg5*^{*-*/*-*}, or BAF-treated Atg5^{-/-} MEF and exosomes. (D) Quantification by dynamic light scattering of particles the size of exosomes released by Atg5^{-/-} MEF treated with vehicle or ConA (12 h). (E) Western blot of Flotillin2 and Tsg101 in Atg5^{-/-} and ConA-treated Atg5^{-/-} MEF and exosomes;

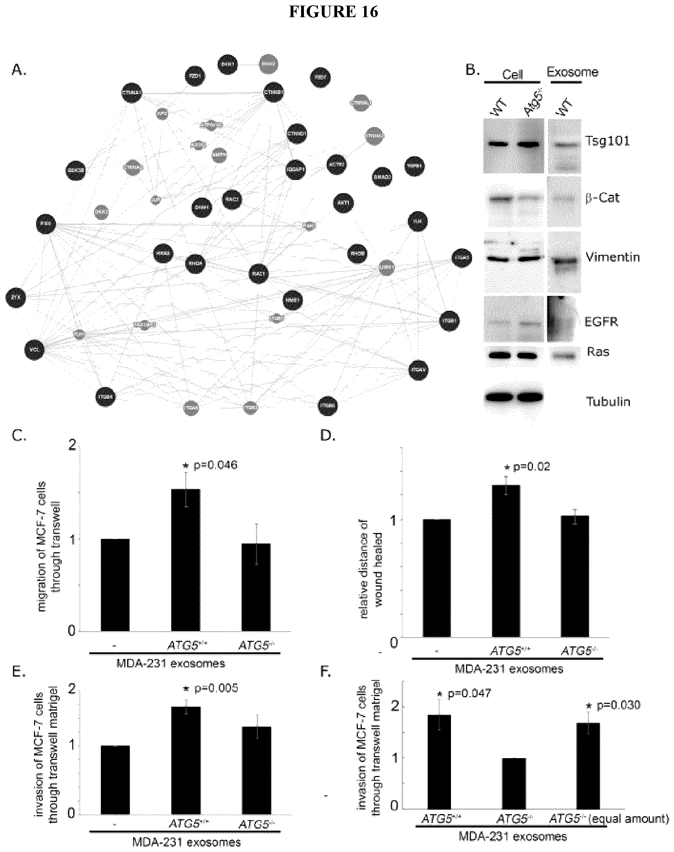

Figure 16. Atg5 controls spread of intercellular phenotype by controlling exosome production. (A) Network pathway analysis of proteins in exosomes released by MDA-MB-231. Proteins with an established role in cancer cell invasion that are present in exosomes are included (black circles). Proteins in grey circles were not detected in exosomes but form part of pathways enriched in exosomes. (B) Western blot of wild-type and ATG5^{-/-} MDA-MB-231 cells or wild-type exosomes. (C) Quantification of migration of MCF-7 cells through a transwell chamber upon incubation with no exosomes or exosomes from wild-type or ATG5^{-/-} MDA-MB-231 cells. (D) Quantification of wound closure by MCF-7 cells incubated with no exosomes or exosomes from wild-type or ATG5^{-/-} MDA-MB-231 cells. (E) Quantification of invasion of MCF-7 cells through a transwell chamber coated with matrigel upon incubation with no exosomes or exosomes from wild-type or ATG5^{-/-} MDA-MB-231 cells. (G) Quantification of invasion of MCF-7 cells through a transwell chamber coated with matrigel upon incubation with an equivalent quantity of exosomes from wild-type or *ATG5*^{*-*/*-*} MDA-MB-231 cells (equal amount). Error bars represent SEM. Western blots were run on separate gels in parallel using samples prepared in parallel.

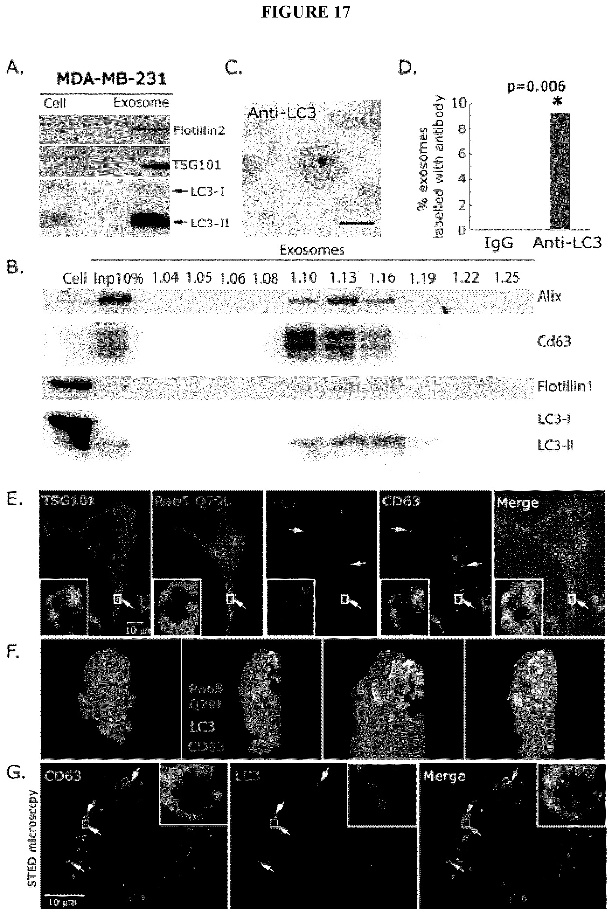

Figure 17. LC3-II is enriched in exosomes compared to LC3-I. (A) Western blot analysis of equivalent amounts (µg protein) of total cell lysate and exosome preparations from MDA-MB-231 for exosome markers (Flotillin2, Tsg101) and LC3. Arrows indicate bands corresponding to unmodified LC3-I and phosphatidylethanolamine-linked LC3-II. (B) Western blot analysis of sucrose density gradient fractions of exosome preparations from Neuro2a cells. (C) Immunogold electron microscopy with anti-LC3 antibody in exosome preparations of Neuro2a cells. Scale bar = 100 nm. (D) Quantification of percent of exosomes labeled with LC3 antibody by electron microscopy (647 exosomes total in 3 replicates, no labeling was observed in samples labeled with control antibody). (E) Confocal microscopy of Tsg101, CD63, LC3 and constitutively active dsRed-Rab5 Q79L in MDA-MB-231 cells. (F) 3-D reconstruction of LC3 and CD63 inside endosomes labeled with constitutively active mutant dsRed-Rab5 Q79L in MDA-MB-231 cells. (G) STED microscopy of CD63 and LC3 in MDA-MB231 cells.

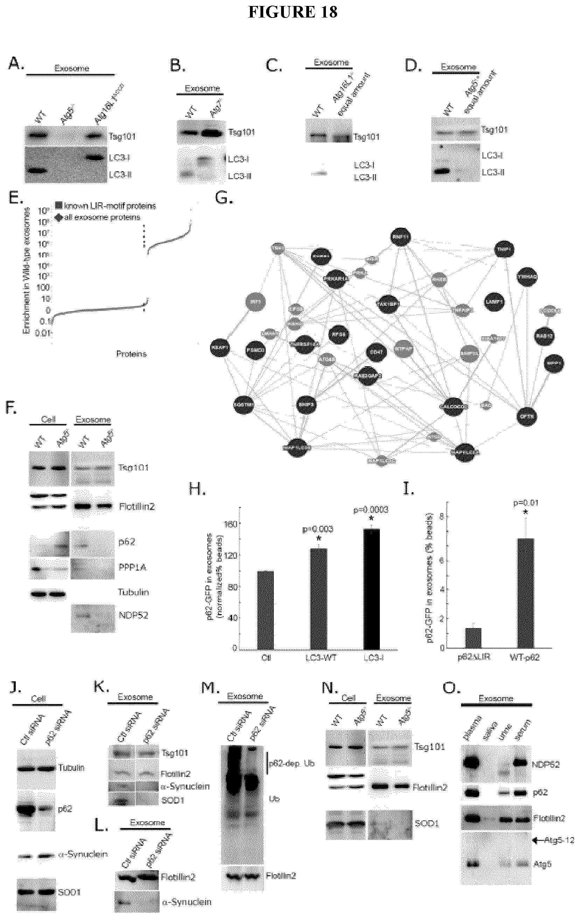

Figure 18. Atg5 controls packaging of LC3 and p62 into exosomes. (A) Western blot of exosomes from wild-type, Atg5^{-/-} and Atg16L1^{ΔCCD} MEF. (B) Western blot of exosomes from wild-type and Atg7^{-/-} MEF. (C) Western blot of exosomes from equivalent amounts of exosomes from wild-type and Atg16L1^{-/-} HCT-116. (D) Western blot of equivalent amounts of exosomes from wild-type and ATG5-/- MDA-MB-231. (E) Plot of the log(10)-fold enrichment of proteins in wild-type vs ATG5^{-/-} MDA-MB-231 in two independent exosome preparations. Proteins with experimentally-defined LIR motifs (Birgisdottir et al., 2013) that were detected in exosomes are illustrated with red squares. (F) Western blot of equivalent amounts of exosomes from wild-type and ATG5^{-/-} MDA-MB-231. (G) Protein interaction network of proteins found in exosomes. LC3A, LC3B, autophagy receptors and proteins with experimentally-defined LIR motifs are included. Proteins detected in exosomes are denoted by black circles. Proteins not detected in exosomes are denoted with grey circles. (H) Percent anti-CD63 beads positive for GFP-p62 in HCT-116 cells transfected with control or plasmid expressing wild-type LC3 or LC3ΔGly221. Normalized to percent beads labeled with exosome marker PrP. (I) Percent anti-CD63 beads positive for GFP in HCT-116 cells transfected with wild-type GFP-p62 or GFP-p62ΔLIR. Normalized to percent beads labeled with exosome marker PrP. Error bars represent SEM. (J) Western blot of lysates of Neuro2A cells transfected with p62 siRNA or control siRNA. (K) Western blot of exosomes derived from Neuro2A cells transfected with p62 siRNA or control siRNA. (L) Western blot of exosomes derived from Neuro2A cells transfected with plasmid expressing α-synuclein and p62 siRNA or control siRNA. (M) Western blot of equivalent amounts of exosomes from wild-type and ATG5^{-/-} MDA-MB-231. (N) Western blot of lysates of Neuro2A cells transfected with p62 siRNA or control siRNA. (O) Western blot of exosomes purified from human plasma, saliva, urine or serum (obtained from Hansa Biomed). Western blots were run on separate gels in parallel using samples prepared in parallel;

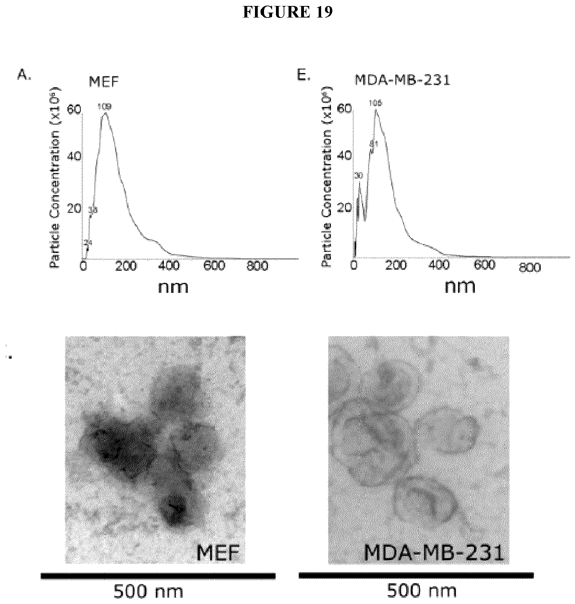

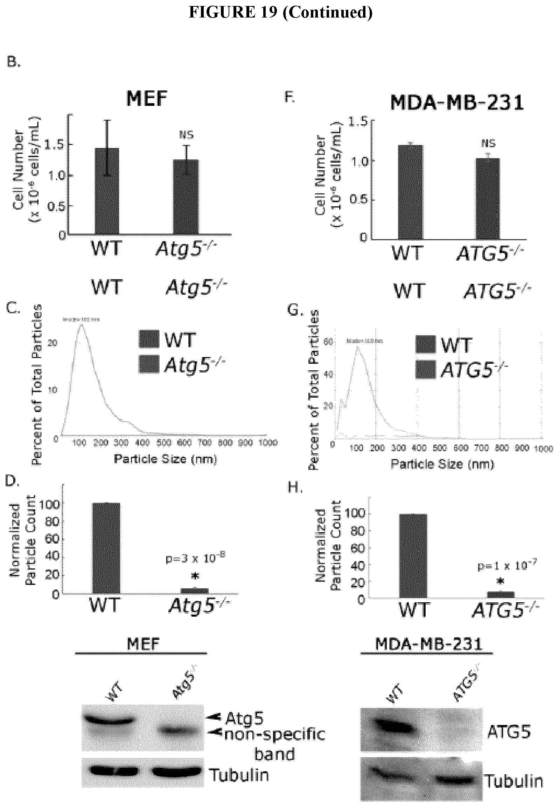

Figure 19. (A) Representative histograms of Nanosight particle tracking measuring percent total particles (y-axis) vs. size (nm, x-axis) of exosome preparations from MEF. Below, electron microscopy images of exosome preparations from MEF. (B) Quantification of cell number by trypan blue exclusion (n=3) after culture of wild-type and Atg5^{-/-} MEF cells for exosome production in Fig. 13. (C) Representative Nanosight particle tracking size histograms of exosomes from wildtype and Atg5^{-/-} MEF cells. (D) Quantification of particles in exosome preparations from wildtype and Atg5^{-/-} MEF cells using Nanosight particle tracking (n=3). Below, western blot of Atg5 in wild-type and Atg5^{-/-} MEF cells. (E-H) As in (A-D) except that MDA-MB-231 and their exosomes are analyzed;

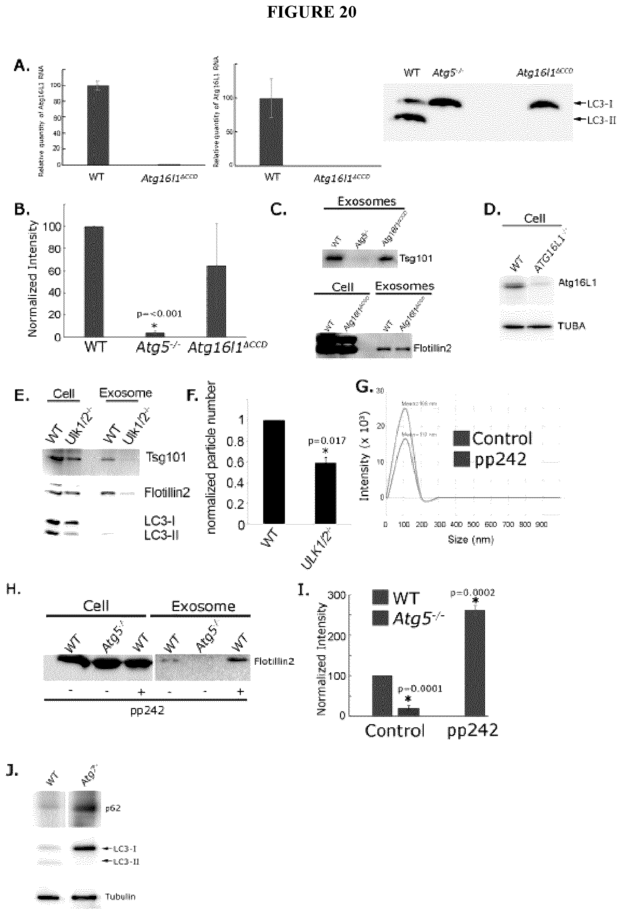

Figure 20. (A) RT-qPCR analysis using primers overlapping the deleted coiled-coil (CCD) region of Atg16l1 in wild-type and Atg16l1^{ΔCCD} MEF. Right, western blot analysis of LC3A in wild-type, Atg5^{-/-} and Atg16l1^{ΔCCD} MEF. Arrows indicate bands corresponding to unmodified LC3-I and phosphatidylethanolamine-linked LC3-II. (B) Quantification of particles in exosome preparations from wild-type, Atg5^{-/-} and Atg16l1^{ΔCCD} MEF using dynamic light scattering intensity (n=3). (C) Quantification of exosome preparations from wild-type, Atg5^{-/-} and Atg16l1^{ΔCCD} MEF using western blot for exosome markers. (D) Western blot for ATG16L1 in wild-type and Atg16l1^{-/-} HCT-116. (E,F) Quantification of particles in exosome preparations from wild-type and ULK1/2^{-/-} MEF using western blot for exosome markers and (E) and dynamic light scattering intensity (F, n=3). (G) Representative histogram of quantification and sizing of particles in exosome preparations using dynamic light scattering intensity from wild-type MEF treated with pp242 (1 µM) or vehicle. X-axis represents intensity. Y-axis represents binned size (nm). (H) Western blot of Flotillin2 in equal amounts of total cell lysates and exosome preparations from wild-type or Atg5^{-/-} MEF cells treated with pp242 or vehicle. (I) Quantification of particles in exosome preparations using dynamic light scattering intensity from wild-type or Atg5^{-/-} MEF cells treated with pp242 or vehicle (n=4). (J) Western blot of p62 and LC3 in wild-type and Atg7^{-/-} MEF;

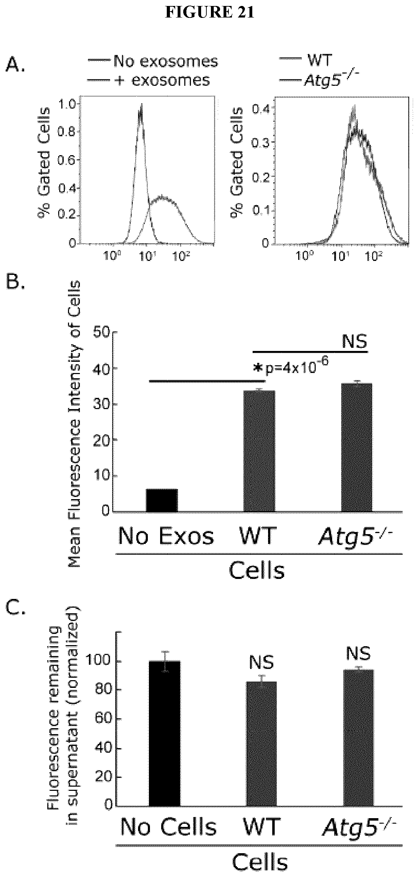

Figure 21. Uptake of exosomes from the extracellular space in Atg5^{-/-} MEF is similar to wild-type MEF. (A) Representative flow cytometry histograms of cells incubated for 1 h with exosomes fluorescently labeled with DiO dye. (B) Fluorescence of wild-type and Atg5^{-/-} MEF cells incubated with DiO-labeled exosome preparations from wild-type MEF cells for 1 h (n=3). Fluorescence was measured by flow cytometry (median). (C) Fluorescence remaining in supernatant after incubation of DiO-labeled exosome preparations with wild-type or Atg5^{-/-} MEF cells (n=3).

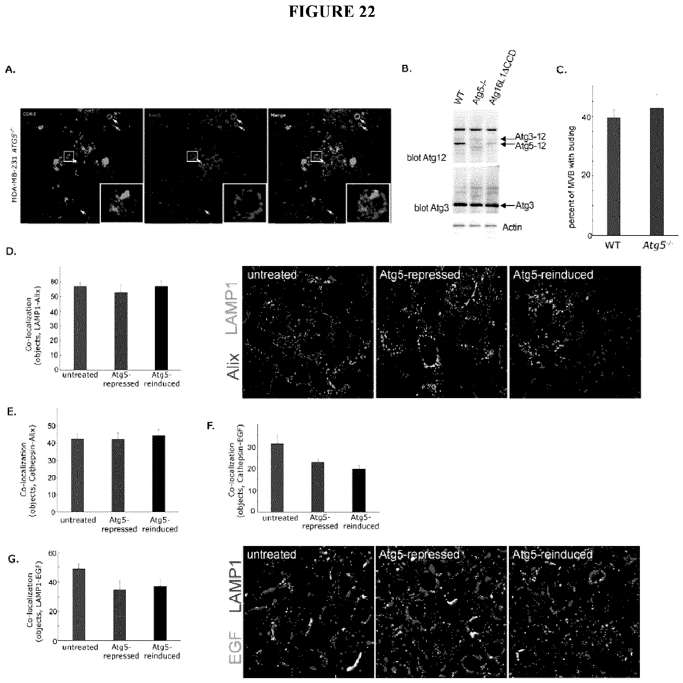

Figure 22. (A) Confocal microscopy of CD63 in Rab5+ organelles of ATG5^{-/-} MDA-MB-231. (B) Western blot for Atg12, Atg3 and Actin (control) in wild-type, Atg5-/-, or Atg16l1^{ΔCCD} MEF. (C) Percent of MVB exhibiting budding of their membrane in wild-type and Atg5^{-/-} MEF. Wild-type 167 MVB, Atg5^{-/-} MEF 159 MVB. (D-G) Co-localization of Alix and EGF with lysosomal markers (LAMP1, CathepsinB). Experiments were performed in MEF untreated, treated with 1 ng/mL doxycycline (2 d) to repress Atg5 (Atg-repressed) or treated with doxycycline and then left untreated for 2 d (AtgS-redinduced). (D) Quantification of co-localization of LAMP1 with Alix and representative images. LAMP1-tagBFP is pseudocolored green. (E) Quantification of co-localization of CathepsinB with Alix (F) Quantification of co-localization of CathepsinB with Alexa488-EGF (G) Quantification of co-localization of LAMP1 with Alexa488-EGF and representative images. LAMP1-tagBFP is pseudocolored red;

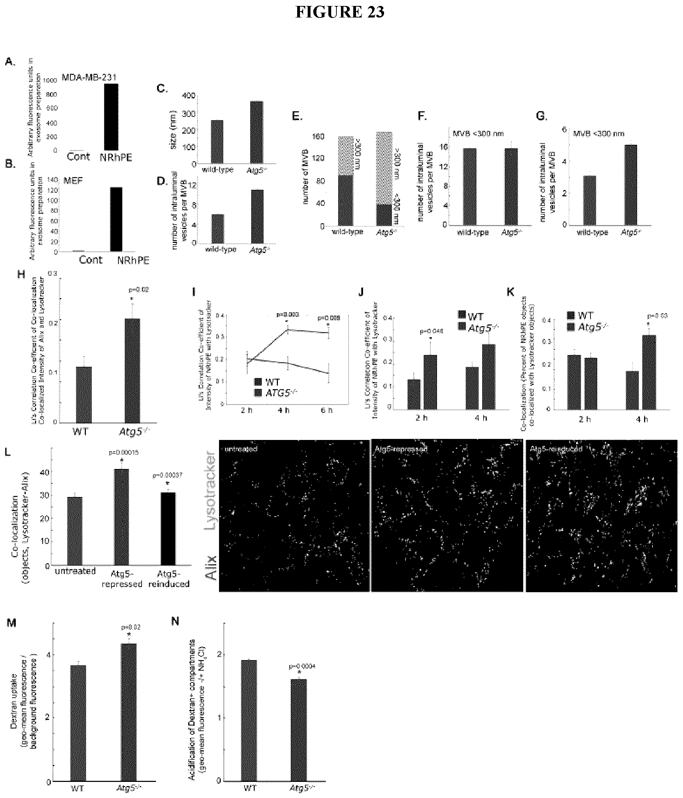

Figure 23. (A,B) Quantification of NRhPE fluorescence in exosome preparations prepared by differential centrifugation from MDA-MB-231 (A) and MEF (B) treated with NRhPE or vehicle. (C) Diameter of MVB in wild-type and Atg5^{-/-} MEF. Wild-type 167 MVB, Atg5^{-/-} MEF 159 MVB. (D) Number of intraluminal vesicles per MVB in wild-type and Atg5^{-/-} MEF. Wild-type 996 intraluminal vesicles, Atg5^{-/-} MEF 1726 intraluminal vesicles. (E) Number of MVB in wild-type and Atg5^{-/-} MEF stratified by size (<300 nm solid bars and >300 nm lined bars). (F) Number of intraluminal vesicles per MVB of (>300 nm) in wild-type and Atg5^{-/-} MEF. Wild-type 38 MVB, 598 intraluminal vesicles; Atg5^{-/-} 90 MVB, 1382 intraluminal vesicles. (G) Number of intraluminal vesicles per MVB of (<300 nm) in wild-type and Atg5-/- MEF. Wild-type 129 MVB, 298 intraluminal vesicles; Atg5^{-/-} 69 MVB, 347 intraluminal vesicles. All error bars represent SEM. Electron microscopy data derived from two independent biological replicates. (H) Quantification of the co-localization of mCherry-Alix with Lysotracker in wild-type or Atg5^{-/-} MEF using Li's correlation co-efficient of fluorescent intensity. A total of 75 wild-type MEF and 51 Atg5^{-/-} MEF cells were quantified. (I) Quantification of the co-localization of NRhPE with Lysotracker using Li's correlation co-efficient of fluorescent intensity in MDA-MB-231 wild-type and ATG5^{-/-} cells. A total of 30-70 cells were quantified in each condition. (J) Quantification of the co-localization of NRhPE with Lysotracker using Li's correlation co-efficient of fluorescent intensity in MEF wild-type and Atg5^{-/-} cells. (K) Quantification of the co-localization of NRhPE with Lysotracker using automated segmentation and quantification of objects in MEF wild-type and Atg5^{-/-} cells. (L) Quantification of colocalization of Lysotracker with Alix using automated segmentation and quantification of objects and representative images. Experiments were performed in MEF untreated, treated with 1 ng/mL doxycycline (2 d) to repress Atg5 (Atg-repressed) or treated with doxycycline and then left untreated for 2 d (Atg5-redinduced). (M) Uptake of pHrodo labeled dextran by wild-type and Atg5^{-/-} MEF. Geometric mean of fluorescent intensity was measured by flow cytometry with NH₄Cl to neutralize pH-dependent increase in pH-sensitive pHrodo dye. (N) Acidification of compartments containing pHrodo labeled dextran in wild-type and Atg5^{-/-} MEF after 20 minutes. Ratio of the geometric mean of fluorescent intensity measured by flow cytometry before vs. after addition of NH₄Cl.

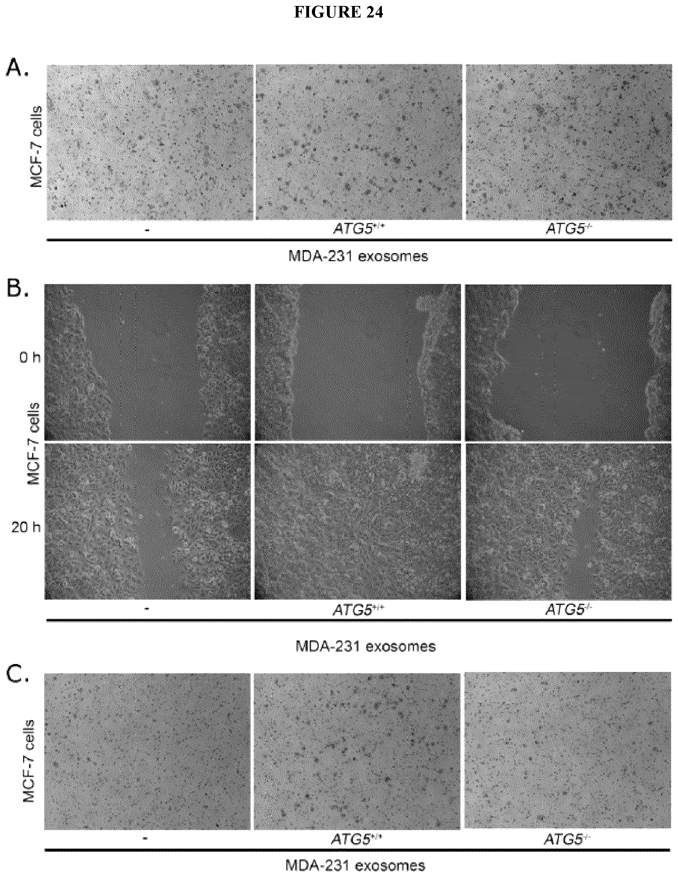

Figure 24. Representative images of MCF-7 cells incubated without exosomes, or with exosomes from equivalent amounts of wild-type or ATG5^{-/-} MDA-MB-231 cells in transwell migration assay (A), wound healing assay (B), and transwell invasion assay (C). Cells in A and C were stained with crystal violet;

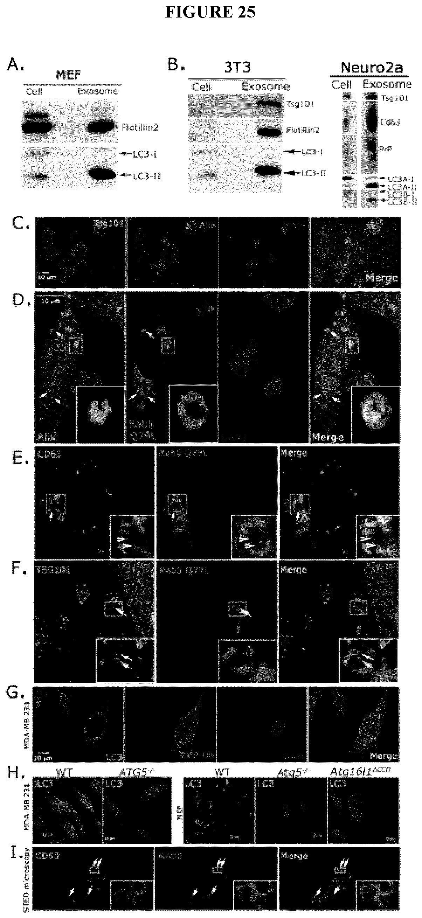

Figure 25. (A-B) Western blot analysis of equivalent amounts (µg protein) of exosome preparations and total cell lysate from MEF (A), 3T3 and Neuro2a cells for exosome markers and LC3. Neuro2A cells and exosomes were blotted for both LC3A and LC3B. (C) Fluorescent microscopy of Tsg101 and Alix in wild-type MDA-MB-231 cells. Tsg101 and Alix co-localize as expected. (D) Confocal microscopy of Alix with constitutively active mutant dsRed-Rab5 Q79L in MEF cells. (E) Confocal microscopy of Cd63 with constitutively active dsRed-Rab5 Q79L in MEF cells. (F) Confocal microscopy of Tsg101 and constitutively active dsRed-Rab5 Q79L in MDA-MB-231 cells. (G) Immunofluorescent detection of LC3 in cells transfected with RFP-ubiquitin. (H) Immunofluorescent detection of LC3 in wild-type MEF or MDA-MB-231 cells or identical cells with genetic deletion of Atg5 or coiled-coil region of Atg16L1. (I) STED microscopy of CD63 and Rab5 in MDA-MB-231 cells.

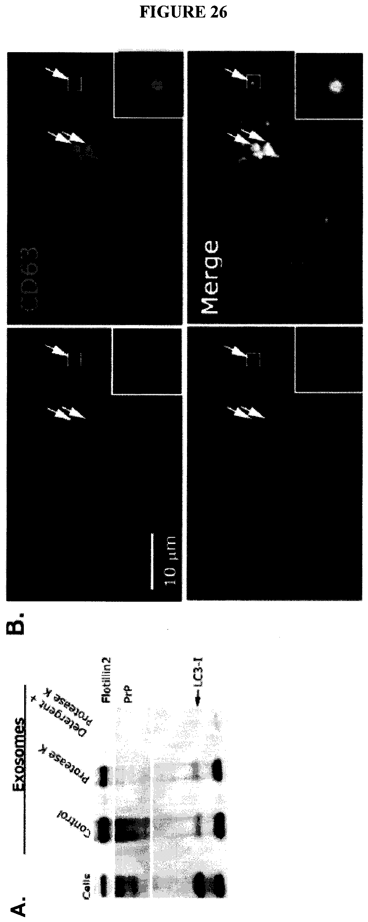

Figure 26. (A) Western blot analysis of equivalent amounts (µg protein) of exosome preparations from Neuro2a cells after pre-treatment with control buffer, protease K, or protease K and detergent (1% Triton-X 100 and 0.1% SDS). LC3, proteins on the external surface (PrP) and proteins in the lumen (Flotillin2) of exosomes were blotted. (B) Fluorescent microscopy of mCherry-Atg5, Rab5 and CD63 in MDA-MB-231 cells;

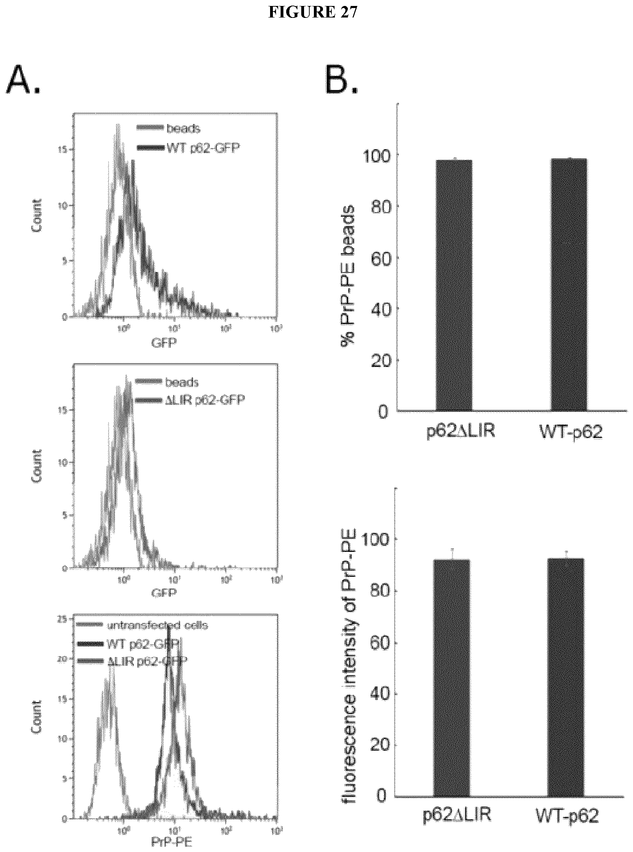

Figure 27. (A) Overlayed flow cytometry histograms of GFP staining detected on anti-CD63 coated beads incubated with 0.22 µM filtered media from cells untransfected, or transfected with GFP-p62 or GFP-p62ΔLIR. (B) Quantification of PrP-PE label (total exosomes captured) of anti-CD63 coated beads incubated with 0.22 µM filtered media from cells untransfected, or transfected with GFP-p62 or GFP-p62ΔLIR. Top, percent PrP-PE+ beads. Bottom, PrP-PE fluorescence intensity of beads;

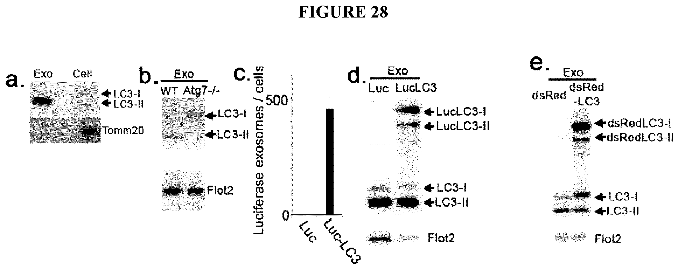

Figure 28 shows that the autophagy protein LC3 is highly enriched in exosomes, independent of autophagy (Atg7^{-/-}). (A) shows that LC3-II was enriched in exosomes, as indicated by Western blot of equal amounts of lysate from exosomes (Exo) or cells. (B) shows that LC3-I is packaged into exosomes made by autophagy-deficient Atg7^{-/-} cells, like LC3-II in exosomes made by WT cells. Data indicated that fusing diverse proteins like luciferase, dsRed to LC3 may induce ~400-fold more packaging of the proteins into exosomes versus simple over-expression approaches (see (C)-(E)). (C) shows luciferase light measured in exosomes compared to its level in cells (fusion to LC3 packages about 500x more cellular LC3 into exosomes). (D) and (E) show Western blots of exosomes transfected with Luciferase or Luciferase-LC3 fusion (D), or dsRed or dsRed-LC3 fusion (E);

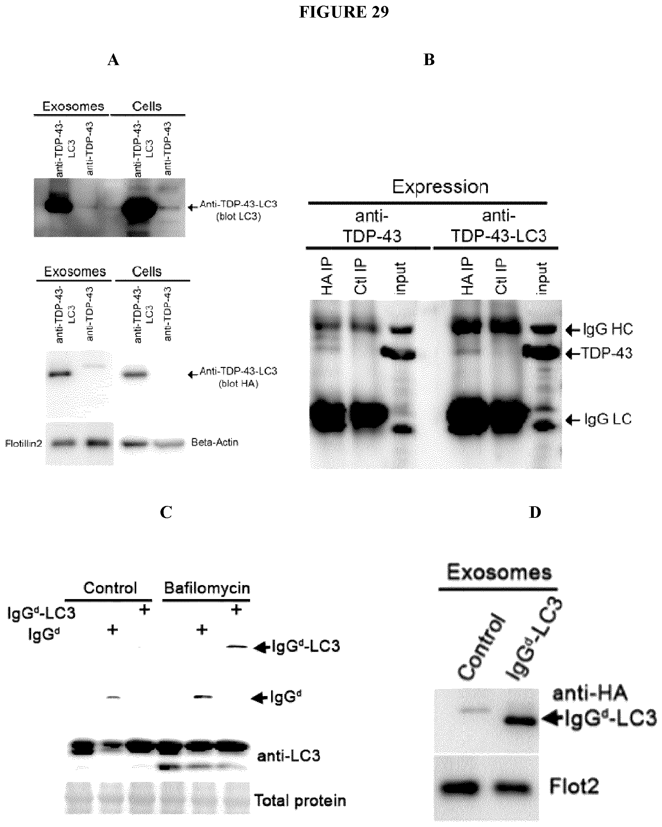

Figure 29 shows that fusion of LC3 to intrabodies, antibodies engineered for intracellular expression (scFv, nanobodies etc.), also resulted in packaging of intrabodies into exosomes. In (A), anti-TDP-43 scFv antibodies were fused to LC3, and the Antibody-LC3 construct was packaged into exosomes efficiently. (A) shows expression of scFv-LC3 fusion constructs and packaging of the constructs into exosomes. The scFv in this experiment was anti-TDP-43 VH7 antibody scFv. Detection of the fusion was by Western blot with anti-LC3 antibody or antibody vs. HA tag incorporated into the linker region in the fusion. (B) shows that the Antibody-LC3 construct binds to TDP-43 antigen target. (C) shows that the antibody-LC3 construct was degraded by autophagy, while antibody alone was not (bafilomycin used in this test inhibits autophagy). As shown in (C), IgG^{d}-LC3 fusion versus TDP-432, but not IgG^{d}, accumulates when autophagy is inhibited with Bafilomycin. In (C), expression of scFv-LC3 construct in cells was achieved using lentivirus. In (C), cells were expressing anti-TDP-43 antibody scFv clone Vh7. (D) shows that IgG^{d}-LC3 vs TDP-43 is packaged in exosomes. Intrabody-LC3 fusions may be targeted for degradation by autophagy, while intrabody alone is not (see (C)), indicating the potential for intrabody-LC3 to degrade targets by autophagy;

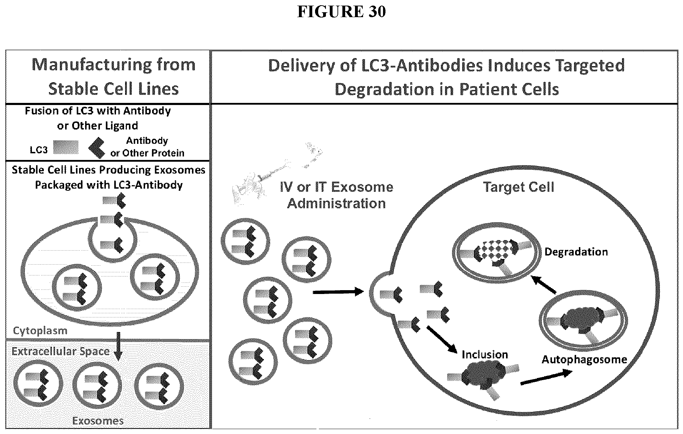

Figure 30 shows a proposed model for targeted autophagy, in which an active agent, such as an antibody or derivate thereof (such as an antigen-binding fragment or scFV, for example), is fused with an LC3 protein sequence (or portion thereof), and packaged into exosomes by expression in stable cell lines producing exosomes. As shown in the Figure, exosomes may be generated by inward budding of late endosomes to create vesicles inside late endosomes, which may then be released to the extracellular space when these late endosomes (or multivesicular bodies) fuse with the plasma membrane. This may result in exosomes with cytoplasmic contents in their interior and plasma membrane receptors on their surface. The so-produced exosomes are obtained/isolated/purified from the cells, and then administered to a cell or subject in need thereof. In the depicted model, the packaged exosomes are administered to a mouse by IV or IT administration as shown. The administered exosomes may fuse with cells in the mouse (for example, brain cells) to deliver their interior contents (i.e. the LC3-antibody fusion), which may further include RNA and/or protein, into the cytoplasm, where the antibody portion of the fusion construct binds the cellular target (such as TDP-43 inclusion in this model). Binding to the cellular target results in the LC3 portion of the fusion construct triggering degradation of the cellular target via autophagy through recruitment of LC3 to autophagosomes;

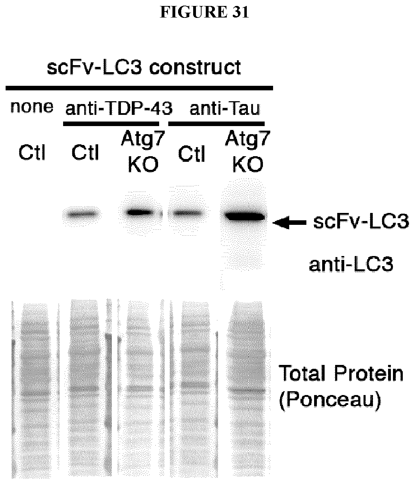

Figure 31 shows expression of scFv-LC3 fusion constructs in cells using lentivirus. These results demonstrate that if these constructs are expressed by virus (e.g. lentivirus, adeno-associated virus, or oncolytic virus), they are degraded by autophagy, since they accumulate in Atg7 knockout cells. Shown in this Figure are anti-Tau clone 4E4 antibody scFv and anti-TDP-43 clone VH3 antibody scFv constructs;

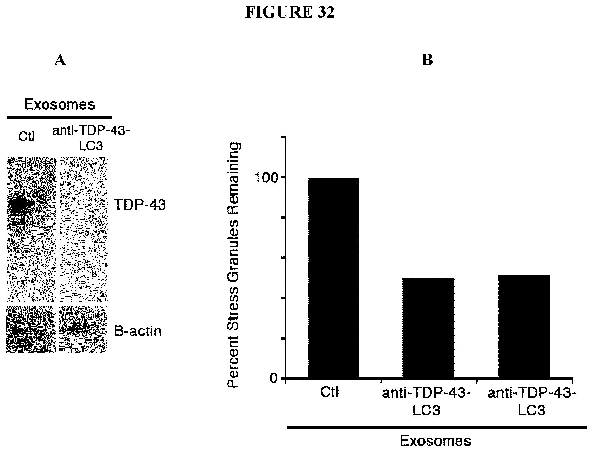

Figure 32 shows experiments in which scFv-LC3 constructs targeting TDP-43 (clone VH7) were stably expressed in cells using lentiviral transduction. Exosomes were purified from the media of these cells and added onto primary mouse mixed motor neurons. Two or six hours later the levels of cytoplasmic TDP43 was measured by Western blot, or the number of stress granules remaining in cells was quantified. These results demonstrate that exosomes packaged with scFV-LC3 conjugates can deliver the constructs into target cells and decrease levels of target proteins and large substrates (i.e. about 200 nm - 10um stress granules in this example) which contain those targets. In (A), a Western blot of primary mixed motor neurons is shown, where scFv-anti-TDP-43-LC3 fusion delivered by exosomes reduced levels of TDP-43 in cells. In (B), quantification by microscopy of stress granules in primary mixed motor neurons treated with arsenite for 1 hour is shown. Cells were fixed 30 minutes after removal or arsenite. scFv-anti-TDP-43-LC3 fusion delivered by exosomes reduced G3BP1+ stress granules in cells (stress granules also contain TDP-43);

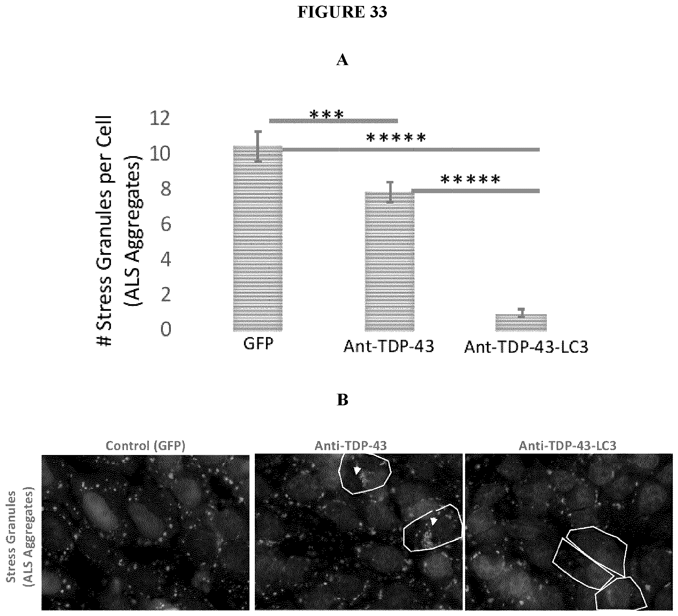

Figure 33 shows that the Antibody-LC3 fusion construct eliminated cellular aggregates linked to ALS much better than antibody alone. Stress granules are TDP-43+ precursors of aggregates/inclusions in ALS (see Figures (A), (B)). As shown, the number of stress granules per cell was much lower following treatment with anti-TDP-43/LC3 fusion versus anti-TDP-43 and GFP control. In this Figure, scFv-LC3 fusion targeted TDP-43 (clone VH7) was expressed in HeLa cells treated with arsenite for 30 min to induce stress granules and left to begin eliminating stress granules by removing arsenite for 30 min. Stress granules contain TDP-43 and are a cellular model or homologue of aggregates in the neurodegenerative disease Amyotrophic Lateral Sclerosis (ALS) which contain TDP-43 in about 95% of patients. Anti-TDP-43-LC3 fusion exhibited increased elimination of stress granules (G3BP1) in cells versus those transfected with GFP (control) or anti-TDP-43 scFv alone. These results demonstrate that the LC3 tag of the fusion increased the elimination of substrates like stress granules;

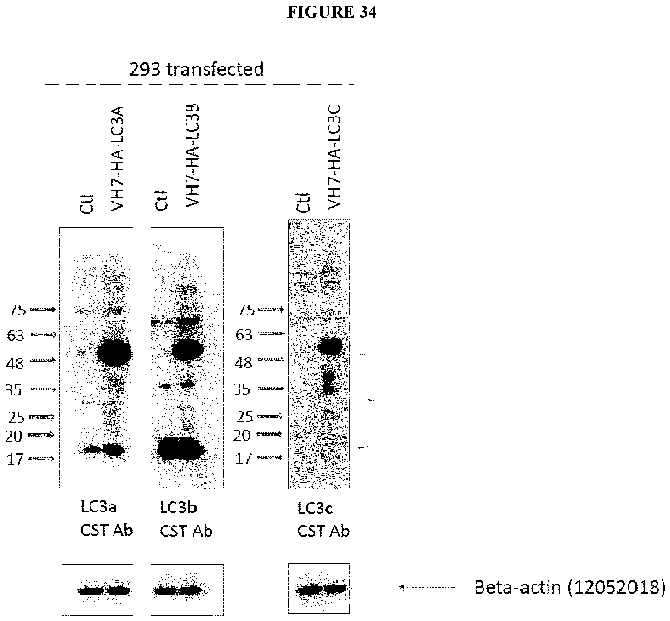

Figure 34 shows results indicating that scFv may be conjugated to any of LC3A, LC3B, or LC3C and be expressed in cells. Anti-TDP-43 clone VH7 is shown;

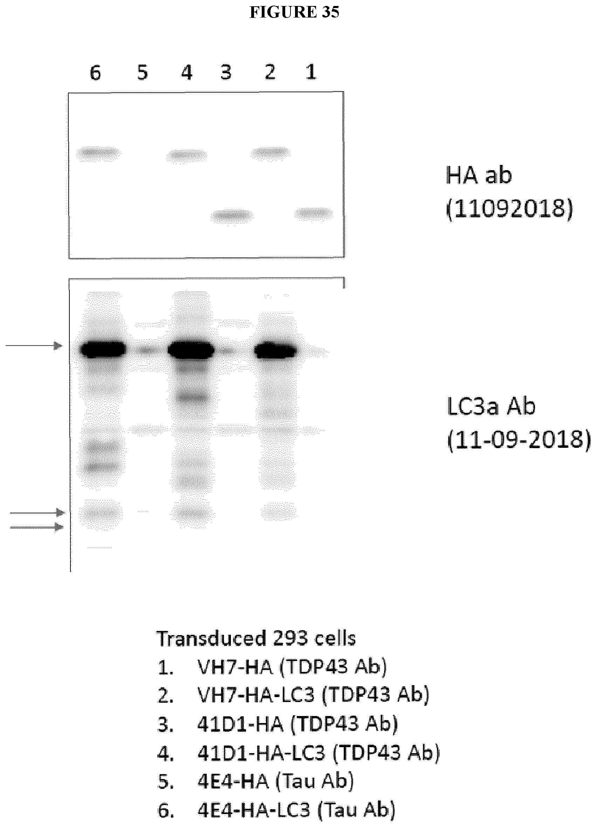

Figure 35 shows results in which scFv conjugated to LC3 are expressed at similar levels in cells as scFv which are unconjugated. The data are for 3 scFvs fused to LC3 derived from anti-TDP-43 VH7, anti-TDP-43, 41D1, and anti-Tau 4E4;

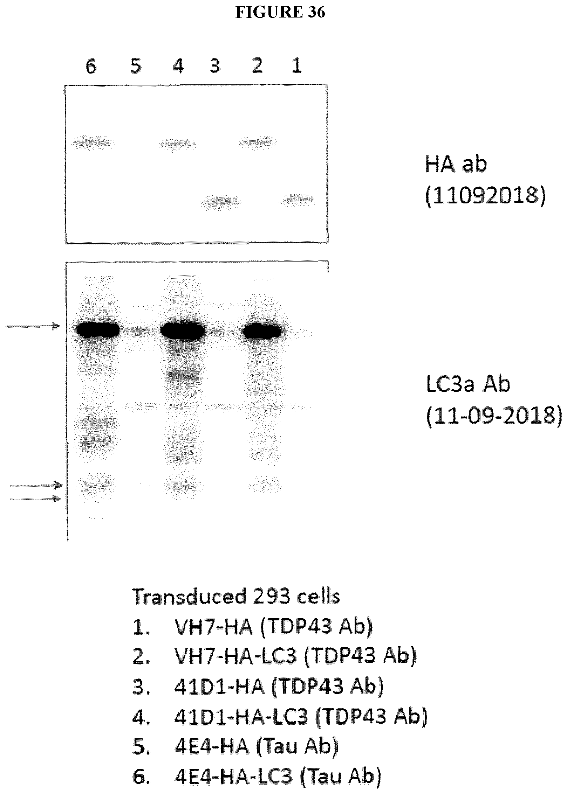

Figure 36 shows results indicating that fusion constructs of scFv conjugated to LC3 were expressed at similar levels in cells as scFv that were unconjugated. The data are for 3 scFv fused to LC3 derived from anti-TDP-43 VH7, anti-TDP-43 41D1, and anti-Tau 4E4;

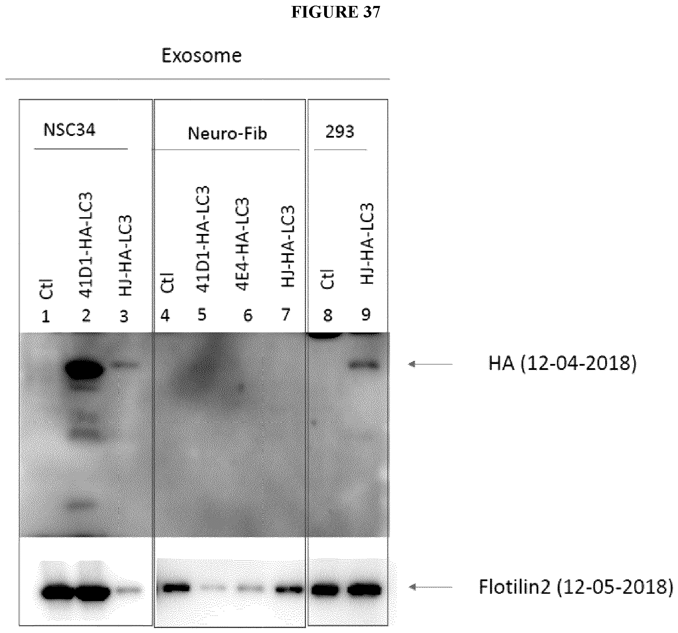

Figure 37 shows results in which various fusion constructs of scFv conjugated to LC3 were packaged into exosomes produced by multiple cell types. Cells were transduced with scFv-LC3 conjugates, and exosomes were purified from these and analyzed by Western blot. The data are for 3 scFv fused to LC3 derived from anti-TDP-43 41D1, anti-Tau HJ, and anti-Tau 4E4. Flotillin2 is a marker of exosomes;

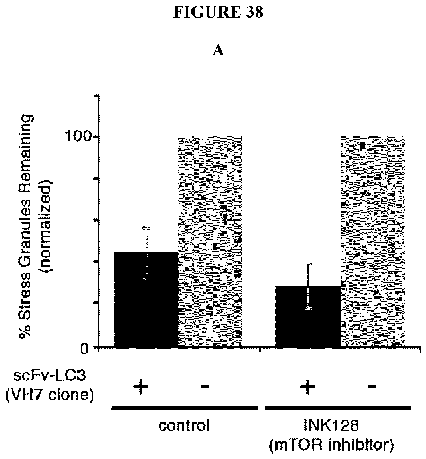

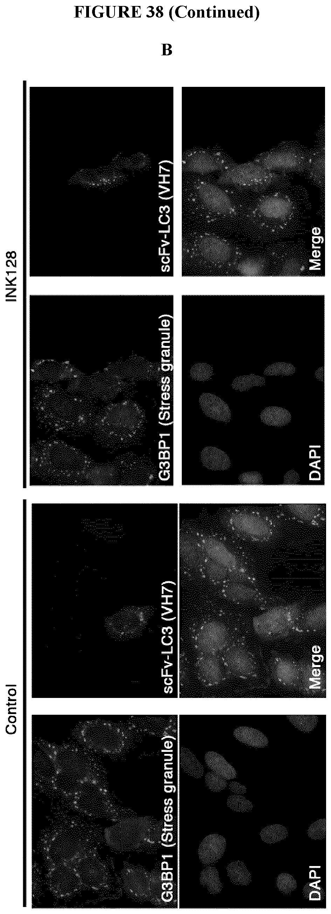

Figure 38 shows results of experiments in which cells were transfected with scFv versus TDP-43 fused to LC3, and treated or not treated with the mTOR inhibitor INK128 to assess autophagy activation. Stress granules were induced by arsenite treatment for 1 h and then removed to allow cells to recover for 30 min before fixation and staining for stress granules (G3BP1+). Results are shown in Figure 38 A. As indicated, the % of stress granules remaining in the scFv-LC3 fusion construct (VH7 clone) treatment condition was beneficially improved by treatment with INK128 inhibitor. INK128 inhibitor alone did not have a measureable effect in the % stress granules remaining, suggesting that the effect observed with the scFv-LC3 fusion construct + INK128 condition was more than an additive effect. Representative images are shown in Figure 38 B; and

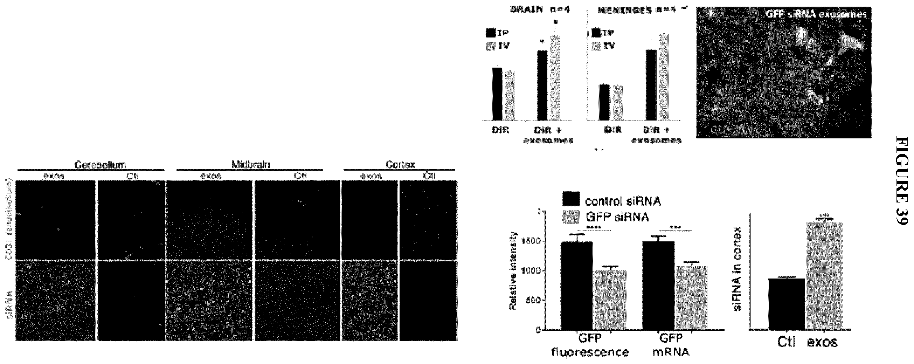

Figure 39 shows (Top left) exosomes labeled with DiR and injected IV accumulate in the brain and meninges as measured by IVIS preclinical imaging of harvested tissues. (Top Right) After IV injection exosomes labeled with PKH67 fluorescence dye and carrying GFP siRNA are detected in brain parenchyma beyond the CD31+ endothelium by confocal microscopy. (Bottom left) Detection of siRNA specific to exosomes by FISH in brain parenchyma (beyond CD31+ endothelium after IV injection of exosomes. Bottom right (Quantification of siRNA in brain parenchyma by FISH after IV injection. (Bottom middle) Quantification of loss of GFP fluorescence and GFP mRNA in cortex after IV injection of exosomes packaged with GFp siRNA or control siRNA.

### DETAILED DESCRIPTION

Described herein are methods for packaging exosomes with one or more active agents of interest such as, but not limited to, antibodies, antigen-binding fragments thereof, and/or antibody derivatives such as scFVs and/or nanobodies, for example. Also described herein are protein-containing constructs designed for exosome packaging, and exosomes packaged with such cargo of interest which are designed for enhancing cellular delivery of their cargo into cells such as cells of the brain. In certain embodiments, constructs and active agents described herein may be designed for targeting one or more undesirable or disease-associated cellular targets (such as, but not limited to, misfolded proteins, protein inclusions, and/or protein aggregates), and in certain embodiments may further be designed for triggering targeted autophagy/degradation of the undesirable or disease-associated cellular target(s). It will be appreciated that embodiments and examples are provided for illustrative purposes intended for those skilled in the art, and are not meant to be limiting in any way.

There is provided herein a fusion construct comprising a biologically active agent functionally linked with a functional moiety. The biologically active agent is a targeting agent, acting as an agent which targets or binds a therapeutically relevant target within a cell. In certain embodiments where targeted autophagy is desired, the role of the biologically active agent may be to bind the target for which autophagic destruction is desired. In certain embodiments, the biologically active agent may specifically and/or tightly bind to a therapeutically relevant target found in the cell. As for the functional moiety, in certain embodiments the functional moiety has one or both of [1] a property of being directed into exosomes during exosome production and secretion from exosome-producing cells; and/or [2] a property of marking a cellular target (typically bound by the biologically active agent of the fusion construct) for autophagic destruction by triggering degradation of the cellular target via autophagy through recruitment of the functional moiety (and groups associated therewith including the cellular target) to autophagosomes. As will be understood, in certain embodiments the biologically active agent may be functionally linked to the functional moiety in generally any suitable manner, either covalently or non-covalently. In certain embodiments, the biologically active agent may be covalently bound to the functional moiety, either directly or through one or more intervening linker groups. For amino acid-based fusion constructs, for example, the biologically active agent peptide or protein may be positioned N- or C-terminal to the functional moiety peptide or protein along a single amino acid chain, either adjoining or separated by one or more peptide (or non-peptide) spacers, intervening groups, and/or linkers, for example. In certain embodiments, the biologically active agent may be non-covalently bound to the functional moiety, and instead may be associated together by electrostatic interactions and/or hydrogen-bonding, for example. In certain embodiments, it is contemplated that the biologically active agent may be modified with a first binding partner and the functional moiety may be modified with a second binding partner, where the first binding partner and the second binding partner have a high affinity for binding together (for example, an antibody-antigen pair, or a streptavidin-biotin pair). Accordingly, in certain embodiments, the fusion construct may comprise a single unitary structure or molecule, or may comprise two or more distinct structures or molecules which are associated with one another.

In certain embodiments, the biologically active agent and the functional moiety may be functionally linked by any number of linkers, or no linker. In data described in the Examples section below, HcRed was fused to LC3B with a linker of five amino acids derived from the multiple cloning site (Ser Gly Leu Arg Ser (SEQ ID NO: 10)). A luciferase-LC3 fusion was also prepared, and had no linker peptide. The scFv fusions used in the Examples section below have a linker sequence separating the scFv from LC3A as follows: GSAGSAAGSGEF (SEQ ID NO: 11), which is based on Waldo et al., Nature Biotechnology, Rapid protein-folding assay using green fluorescent protein, 1999, 17:691-695.

The biologically active agent may be functionally linked to an N-terminal portion or end of the functional moiety, or to a C-terminal portion or end of the functional moiety. N-terminus versus C-terminus functional linkage may be selected based on the particular functional moiety and/or biologically active agent being used, and/or based on the intended application of the fusion construct. Where, for example, the functional moiety comprises LC3 (as described in further detail below), selection of N-terminus versus C-terminus functional linkage to the biologically active agent may provide for different effects. In both cases, packaging of the fusion construct into exosomes by exosome-producing cells may be achieved (although in cases where C-terminal linkage is used, Atg4 deficient exosome-producing cells may be used for the reasons described below under certain conditions), and the packaged exosomes may be used to deliver the fusion construct into the cytoplasm of targeted cells. Because the C-terminus of LC3 is typically cleaved by Atg4 before the lipid phosphatidylethanolamine is added to it, a biologically active agent linked to the C-terminus may be released from the LC3 portion once inside the target cells. Such release may be desirable where, for example, cellular delivery of an intact, substantially unmodified biologically active agent into target cells is desired such that the biologically active agent may perform it's *in vivo* activity. Such release may be desirable where, for example, a protein replacement therapy is desired (for example, an enzyme could be delivered to a cell in need thereof), and/or where a prodrug-type delivery is desired where cleavage within the target cell converts the prodrug to a drug form. In contrast, a biologically active agent linked to the N-terminus of LC3, or linked to an LC3 from which the Atg4 cleavage site has been removed and/or mutated such that it is no longer cleaved, may remain joined with LC3 inside the target cells, which may be desirable where, for example, the fusion construct is intended to bind a cellular target via the biologically active agent and direct autophagic destruction of the cellular target via recruitment to autophagosomes via the LC3 portion of the fusion construct.

There are four Atg4 proteases (Atg4A, Atg4B, Atg4C, Atg4D) that preferentially cleave different LC3 or GABARAP proteins. The target sites where these enzymes cleave may be quite variable. In human LC3 proteins, these sites are: LC3A: VYASQETFGF (SEQ ID NO: 27), LC3B: VYASQETFGF (SEQ ID NO: 28), LC3C: TYASQETFGCLESA (SEQ ID NO: 29), GABARAP: AYSDESVYGL (SEQ ID NO: 30), GABARAPL1: AYSDESVYGK (SEQ ID NO: 31), GABARAPL2: AYSGENTFGF (SEQ ID NO: 32), GABARAPL3: AYSNESVYGK (SEQ ID NO: 33). In each of these sequences cleavage occurs at the C-terminal glycine. Preferred motifs include a large amino acid containing a phenol group (Phenylalanine, Tyrosine) N-terminal of the cleavage site, and at least one acidic amino acid (D,E) not directly next to it but 3-5 amino acids N-terminal of the cleavage site. It is contemplated that in certain embodiments, any of these sites, or a site resembling or functionally mimicking these motifs, may be used to create an Atg4 cleavage site as desired (for example, where a prodrug-type approach is desired). For example, the sequence GTFG serves as a substrate for Atg4 cleavage (see Li et al., Measurement of the activity of the Atg4 Cysteine Proteases, Methods Enzymol, 2017, 587:207-225).

In certain embodiments, such cleavage sites, for example, may be included or inserted at or near the N or C-terminus of a protein of interest to accommodate the structure and function of the protein of interest.

Indeed, it is believed that the C-terminus of LC3 is cleaved by Atg4 before the lipid phosphatidylethanolamine is added to it. Atg4 can cleave in the middle of the protein, and therefore may cleave sequence off the C-terminal end of LC3. Accordingly, in certain embodiments, it is contemplated that if a protein for which delivery into cells is desired is fused to the C-terminal end of LC3 and expressed in cells lacking Atg4 (so the protein is not cleaved off LC3), then the LC3-protein fusion construct may be packaged into exosomes. Atg4 is a ubiquitously expressed enzyme, therefore when the LC3-protein fusion construct is delivered into target cells by the exosomes, Atg4 may cleave the protein free from LC3 for it to be free and active in the target cell. Some strategies to package proteins into exosomes use binding to a transmembrane or membrane-bound protein, or another protein with its own localization. In certain embodiments, it is contemplated that by releasing the protein free from LC3 in target cells, the present system may allow a more functional, unhindered protein normalized to its regular, endogenous location in cells, for example.

The biologically active agent will be a protein or peptide (i.e. a substantially amino acid-based agent) of interest, although it is contemplated that non-amino acid-based agents of interest may also be used. As will be understood, in embodiments where for example the fusion construct is to be expressed within a cell for packaging into exosomes, an amino acid-based biologically active agent may be preferred to allow for expression in the cell from an expression vector, for example. As will be understood, however, where a pre-prepared fusion construct is to be introduced into the cell for packing into exosomes, the biologically active agent may be amino acid-based or non-amino acid based as desired.

The biologically active agent comprises a targeting moiety for binding a cellular target. The biologically active agent comprises an antibody, an antigen-binding fragment thereof, or an antibody derivative or mimic, which is a single-chain variable fragment (scFv) or a nanobody. In certain embodiments, the antibody derivative or mimic may comprise a nanobody from an organism such as, but not limited to, a camel, llama, shark, or other animal. The person of skill in the art having regard to the teachings herein will be aware of many different antibodies, antibody derivatives, and/or antibody mimics which may be used.

In certain embodiments, the biologically active agent may comprise, for example, anti-TDP43 scFv clone VH7; anti-TDP-43 scFv 4aD1; anti-Tau scFv 4A3; anti-Tau scFv 4E4; adipophilin; perilipin2; PINK1; or Parkin, for example.

The biologically active agent comprises an antibody, antigen-binding fragment thereof, an antibody derivative or mimic, or a protein or peptide which specifically binds a cellular target comprising misfolded proteins, protein inclusions, protein aggregates, lipid droplets, or mitochondria. In certain embodiments, the cellular target may be a cellular target associated with or causative of a neurodegenerative disease, or another disease such as, but not limited to, non-alcoholic steatohepatitis for lipid droplets, inflammatory and/or autoimmune disease, or a disease relating to, or treatable by, autophagy, for example. By way of example, in certain embodiments the cellular target may comprise one or more misfolded proteins, protein inclusions, and/or protein aggregates such as, but not limited to, TDP-43 inclusions found in ALS patients; Tau fibrils found in Alzheimer's, CTE, Corticobasal Degeneration, Progressive Supranuclear Palsy, and/or other Tauopathies patients; synuclein or Alpha-synuclein or Lewy bodies and/or damaged mitochondria found in Parkinson's patients; Htt repeats in Huntington's diseases; dipeptide repeats produced by C9ORF72 intronic repeats; misfolded SOD1; and/or hyperphosphorylated or fibrillar Tau.

In certain embodiments, cellular targets of the biologically active agent may include any suitable target of interest. In most embodiments, the cellular target will be an intracellular target believed to be associated with or a causative factor of a disease or disorder for which treatment is desirable. In certain embodiments, the cellular target may comprise one or more of a misfolded protein, protein inclusion, and/or protein aggregate associated with a neurodegenerative disease or disorder such as, but not limited to, Alzheimer's, Parkinson's, Frontal Temporal Dementia and/or Amyotrophic Lateral Sclerosis (ALS). In certain embodiments, the cellular target may be TDP-43 inclusions found in most ALS patients; Tau fibrils found in Alzheimer's, CTE, Tauopathies; and/or Alpha-synuclein (Lewy bodies) and/or damaged mitochondria found in Parkinson's, for example. In certain embodiments, the cellular target may be a cellular target unrelated to the brain and/or neurodegenerative diseases/disorders. For example, in certain embodiments, the cellular target may be lipid droplets in fatty liver and/or atherosclerosis, for example.

The functional moiety of the fusion construct comprises an LC3 or GABARAP protein. In certain embodiments, the functional moiety may comprise two or more copies of any of these, or may comprise a combination of any two or more of these, or any combination thereof, for example. The functional moiety may have one or both of [1] a property of being directed into exosomes during exosome production and secretion from exosome-producing cells; and/or [2] a property of marking a cellular target (typically bound by the biologically active agent of the fusion construct) for autophagic destruction by triggering degradation of the cellular target via autophagy through recruitment of the functional moiety (and groups associated therewith including the cellular target) to autophagosomes.

In humans, there are 7 LC3 homologues (LC3A, LC3B, LC3C, GABARAP, GABARAPL1, GABARAPL2, GABARAPL3). The present inventors have demonstrated that exosomes contain at least LC3A and LC3B, and have tested in the Examples section below fusions of both LC3A and LC3B for packaging into exosomes. The scFv fusions described in the Examples section below were fused to LC3A. HcRed and Luciferease fusions described in the Examples section below were fused to LC3B. These were fused to the N-terminus of LC3, because the C-terminus is cleaved off by Atg4 (and this would remove the fused protein), as described above. The present inventors have also shown that proteins may be fused to LC3C, and it is contemplated that fusions of proteins to other LC3 homologues are expected to perform similarly, as N-terminal fusions to these proteins (e.g. GABARAPs) have been used to examine their localization and degradation by autophagy.

In certain embodiments, the functional moiety may comprise an LC3 protein. As will be understood, LC3 has various homologues and/or isoforms, and is found in various different mammals, and it is contemplated that in certain embodiments these homologues and/or isoforms and/or species variants may used as a functional moiety in fusion constructs as described herein. Given the sequence variations found among closely related LC3 homologues (see Example 4), it is contemplated that LC3 derivatives and/or mimics having sequence variation from natural LC3 sequence may also be used. The present inventors have identified that human LC3A, LC3B, LC3C, mouse LC3A, and mouse LC3B homologs can all be found in exosomes, for example. The lipid PE is added to LC3-I to make LC3-II. Either of these are packaged into exosomes. In most cells, LC3-II is packaged into exosomes, but in Atg7 knockout cells and other cells, LC3-I may also be packaged into exosomes. Thus, both forms of LC3 homologues are contemplated herein.

In certain embodiments, therefore, it is contemplated that the functional moiety may comprise LC3 protein, or a sequence having at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity therewith.

In certain embodiments, it is contemplated that the functional moiety may comprise human LC3A, human LC3B, human LC3C, mouse LC3A, mouse LC3B, human GABARAP, human GABARAPL1, human GABARAPL2, human GABARAPL3, mouse GABARAP, mouse GABARAPL1, or mouse GABARAPL2, or a sequence having at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity therewith.

In another embodiment, the functional moiety may comprise:
SEQ ID NO: 1 (human LC3A) 
MPSDRPFKQRRSFADRCKEVQQIRDQHPSKIPVIIERYKGEKQLPVLDKTKFLVPDHVNM
SELVKIIRRRLQLNPTQAFFLLVNQHSMVSVSTPIADIYEQEKDEDGFLYMVYASQETFG
F;
SEQ ID NO: 2 (human LC3B) 
MPSEKTFKQRRTFEQRVEDVRLIREQHPTKIPVIIERYKGEKQLPVLDKTKFLVPDHVNM
SELIKIIRRRLQLNANQAFFLLVNGHSMVSVSTPISEVYESEKDEDGFLYMVYASQETFG
MKLSV;
SEQ ID NO: 3 (human LC3C) 
MPPPQKIPSVRPFKQRKSLAIRQEEVAGIRAKFPNKIPVVVERYPRETFLPPLDKTKFLVP
QELTMTQFLSIIRSRMVLRATEAFYLLVNNKSLVSMSATMAEIYRDYKDEDGFVYMTY
ASQETFGCLESAAPRDGSSLEDRPCNPL;
SEQ ID NO: 4 (mouse LC3A) 
MPSDRPFKQRRSFADRCKEVQQIRDQHPSKIPVIIERYKGEKQLPVLDKTKFLVPDHVNM
SELVKIIRRRLQLNPTQAFFLLVNQHSMVSVSTPIADIYEQEKDEDGFLYMVYASQETFG
F;
SEQ ID NO: 5 (mouse LC3B) 
MPSEKTFKQRRSFEQRVEDVRLIREQHPTKIPVIIERYKGEKQLPVLDKTKFLVPDHVNM
SELIKIIRRRLQLNANQAFFLLVNGHSMVSVSTPISEVYESERDEDGFLYMVYASQETFGT
AMAV;
SEQ ID NO: 34: (human GABARAP) 
MKFVYKEEHPFEKRRSEGEKIRKKYPDRVPVIVEKAPKARIGDLDKKKYLVPSDLTVGQ
FYFLIRKRIHLRAEDALFFFVNNVIPPTSATMGQLYQEHHEEDFFLYIAYSDESVYGL
SEQ ID NO: 6 (human GABARAPL1) 
MKFQYKEDHPFEYRKKEGEKIRKKYPDRVPVIVEKAPKARVPDLDKRKYLVPSDLTVG
QFYFLIRKRIHLRPEDALFFFVNNTIPPTSATMGQLYEVMVLVAQYWMPSSAVWHPLAL
VLDALITHLRSGAEGVIYPDPLTYGSVRL;
SEQ ID NO: 7 (human GABARAPL2) 
MKWMFKEDHSLEHRCVESAKIRAKYPDRVPVIVEKVSGSQIVDIDKRKYLVPSDITVAQ
FMWIIRKRIQLPSEKAIFLFVDKTVPQSSLTMGQLYEKEKDEDGFLYVAYSGENTFGF;
SEQ ID NO: 35 (human GABARAPL3) 
        10         20         30         40         50
MKFQYKEVHP FEYRKKEGEK IRKKYPDRVP LIVEKAPKAR VPDLDRRKYL
        60         70         80         90        100
VPSDLTDGQF YLLIRKRIHL RPEDALFFFV NNTIPPTSAT MGQLYEDSHE
       110
EDDFLYVAYS NESVYGK
SEQ ID NO: 36 (mouse GABARAP) 
MKFVYKEEHPFEKRRSEGEKIRKKYPDRVPVIVEKAPKARIGDLDKKKYLVPSDLTVGQ
FYFLIRKRIHLRAEDALFFFVNNVIPPTSATMGQLYQEHHEEDFFLYIAYSDESVYGL
SEQ ID NO: 8 (mouse GABARAPL1) 
MKFQYKEDHPFEYRKKEGEKIRKKYPDRVPVIVEKAPKARVPDLDKRKYLVPSDLTVG
QFYFLIRKRIHLRPEDALFFFVNNTIPPTSATMGQLYEDNHEEDYFLYVAYSDESVYGK;
 or
SEQ ID NO: 9 (mouse GABARAPL2) 
MKWMFKEDHSLEHRCVESAKIRAKYPDRVPVIVEKVSGSQIVDIDKRKYLVPSDITVAQ
FMWIIRKRIQLPSEKAIFLFVDKTVPQSSLTMGQLYEKEKDEDGFLYVAYSGENTFGF;
or a sequence having at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity with any of these sequences.

In certain embodiments, the functional moiety may comprise an amino acid sequence of any one of SEQ ID NOs: 1-9, or a sequence having at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity therewith. In certain embodiments, these may be expressed with the C-terminal portion already removed. As will be understood, the cleavage sites will typically be at the C-terminal glycine residue in each sequence.

In certain embodiments, the functional moiety of the fusion construct may comprise an LC3 protein which is (or comprises) an LC3A, LC3B, LC3C, GABARAP, GABARAPL1, GABARAPL2, or GABARAPL3 homologue.

As referenced herein, percent (%) identity or % sequence identity with respect to a particular sequence, or a specified portion thereof, may be defined as the percentage of nucleotides or amino acids in the candidate sequence identical with the nucleotides or amino acids in the subject sequence (or specified portion thereof), after aligning the sequences and introducing gaps, if necessary, to achieve the maximum percent sequence identity, as generated by the program WU-BLAST-2.0 with search parameters set to default values (Altschul et al., J. Mol. Biol. (1990) 215:403-410; website at blast.wustl.edu/blast/README.html). By way of example, a % identity value may be determined by the number of matching identical nucleotides or amino acids divided by the sequence length for which the percent identity is being reported. Percent (%) amino acid sequence similarity may be determined by the same calculation as used for determining % amino acid sequence identity, but may, for example, include conservative amino acid substitutions in addition to identical amino acids in the computation. Oligonucleotide alignment algorithms such as, for example, BLAST (GenBank; using default parameters) may be used to calculate sequence identity %.

In certain embodiments, and without wishing to be bound by theory, it is contemplated that the fusion construct may be designed such that the fusion construct is packaged into exosomes by exosome-packaging cells (by function of the functional moiety), and upon introduction into a target cell, the fusion construct may bind a disease-related target (by function of the biologically active agent) and trigger autophagic degradation of the disease-related target (by recruitment of the functional moiety to autophagosomes). The present inventors have demonstrated that LC3, for example, is strongly enriched in exosomes. LC3 is a central player in autophagy, which can engulf and degrade a wide variety of targets in the cytoplasm from aggregates in neurodegeneration, to lipid droplets, bacteria, cytoplasmic DNA or mitochondria, for example. Data in the Examples below shows that by fusing proteins to LC3, they may be packaged efficiently into exosomes, up to 400-fold more than without an LC3 fusion. Once exosomes deliver such LC3-fusions into target cells, LC3 may promote the degradation by autophagy of a molecule or structure which the fused protein binds to. In this model, the biologically active agent fused to LC3 provides the target recognition to induce autophagic degradation of specific targets in the cytoplasm. In certain embodiments, the biologically active agent of the fusion construct may comprise an antibody derivative such as scFv or nanobodies, which may be designed for intracellular expression fused to LC3 or another such functional moiety. After exosome delivery into cells, the antibody may recognize its target, such as aggregates containing misfolded or altered proteins such as misfolded or cytoplasmic TDP-43, dipeptide repeats produced by C9ORF72 intronic repeats, misfolded SOD1, hyperphosphorylated or fibrillar Tau, synuclein or Lewy bodies, etc..., and LC3 may induce the degradation of these targets by autophagy.

In certain embodiments, the functional moiety of the fusion construct may comprise LC3. The functional moiety may comprise LC3-interacting motifs (LIM) (also known as LC3-interacting regions (LIR)). These motifs/regions may bind to LC3, allowing fusion construct packaging into exosomes. The present inventors have demonstrated this by testing the packaging into exosomes of either wild-type p62 or p62 deleted of its LIR motif, and found that much less p62 was packaged into exosomes. Similarly, proteomic analysis of exosomes by the present inventors demonstrated that exosomes lacking LC3 (Atg5 knockout exosomes) are depleted of proteins containing LIR motifs, again supporting that LIR motifs promote packaging of proteins into exosomes (see Figure 18, where data (E) shows enrichment of LIR motif proteins in exosomes from Wild-type cells vs. Atg5 knockout cells; (G) shows a protein-interaction network of LC3 binding proteins enriched in exosomes from wild-type but not Atg5 knockout cells; (F) shows western blot showing that many proteins with LIR motifs (p62, PPP1A, NDP52) are decreased in exosomes from Atg5 knockout cells; (H) shows GFP-p62 is packaged into exosomes but packaging into exosomes is decreased with the LIR motif is deleted; where this assay was performed measuring GFP tag on p62 by flow cytometry). Once delivered by exosomes, these proteins may then bind LC3 in cells to promote degradation of their interacting substrates by autophagy. In a similar way, sequences or domains of other proteins which bind LC3 may be used as functional moieties in the fusion constructs to similar effect. Other proteins known to interact with LC3 may include, for example, Atg4, Atg7, Atg10 among others. Fusion of a whole protein, or domains thereof, to a biologically active agent, such as a target recognition protein, may promote packaging into exosomes. It is further contemplated that the functional moiety may comprise Atg5 or Atg16L1, among other autophagy proteins, which may also be used to similar effect. These proteins are also abundant in exosomes. As these proteins also are recruited to the autophagosome, it is contemplated that fusion of biologically active agents to Atg5, Atg16L1, or other autophagy-related proteins, may also induce their packaging into exosomes and/or the degradation of targets of the biologically active agent of the fusion construct by autophagy.

In another embodiment, there is provided herein a nucleic acid encoding a fusion construct as described herein, or a nucleic acid partially or fully complementary thereto. In yet another embodiment, there is provided herein an expression vector, plasmid, or cassette for expressing a fusion construct as described herein.

In still another embodiment, there is provided herein a host cell comprising any of the nucleic acids or expression vectors as described herein, the host cell expressing a fusion construct as described herein. In certain embodiments, the host cell may produce and/or secrete exosomes comprising a fusion construct as described herein. In certain embodiments, the host cell may be a host cell having low, reduced, knockout, or knockdown levels of Atg7, so as to enhance production of exosomes containing the fusion construct. In certain embodiments, the host cell may be Atg7^{-/-}. By way of non-limiting example, in certain embodiments, the host cell may be a 293 cell; a human neonatal fibroblast; an NSC-34 cell; an SH5Y cell; a BV2 cell; or another exosome-producing cell.

As will be understood, in certain embodiments, the host cell may comprise any suitable exosome-producing cell selected to suit the particular application. Examples of exosome-producing host cells are described in, for example, WO2017/054085, By way of non-limiting example, in certain embodiments, the host cell or exosome-producing cell may include exosome-producing mesenchymal stem cells, primary dendritic cells, MDA-MB-231 cells, plasma cells, serum cells, mast cells, glioblastoma cells, B cells, cardiac progenitor cells, or MSC cells. In still further embodiments, a host cell or exosome-producing cell may be, but is not limited to, an embryonic stem cell, a mesenchymal stem cell, or a differentiated version of the two former stem cells, a dendritic cell, a macrophage, a monocyte, a T or B cell, a fibroblast, or a cell line such as but not limited to a HeLa, 293T, or MDA-MB-231 cell line. In certain embodiments, a host cell or exosome-producing cell may be a cell which naturally produces exosomes. In certain further embodiments, the cell may be a primary human mesenchymal stem cell, a primary mouse macrophage, a human breast cancer cell line such as MDA-MB-231, a mouse or human neuronal cell line such as Neuro2a or SHSY, a mouse astrocyte cell line such as C8Da or SIM, a mouse microglia cell line such as BV2, a mouse motor neuron cell line such as NSC-34 or MN-1, a HeLa, mouse embryonic fibroblast, or a mouse dendritic cell such as JAWS II. By way of example, the cell may be an MEF or JAWSII cell. Examples of exosome-producing cells, in approximate order of exosome production from least to most in standard cell culture conditions, may include (but are not limited to): glioblastoma cell line U251-MG; epithelial and fibroblast cells like HeLa, MDA-MB-231, and HCT-116 cells (produce moderate amounts of exosomes); and neurons, immune and blood cells (including dendritic cells, macrophages, T cells, B cells, reticulocytes), mesenchymal stem cells, and embryonic stem cells (produce abundant exosomes). In certain non-limiting embodiments, the exosome-producing cells may be human cells. Exosomes produced by human cells may have reduced immunogenicity as compared to exosomes from mouse cells when introduced into human patients, which may be due to decreased differences in histocompatibility complexes (Bach, 1987, N Engl J Med, 317:489). In another non-limiting embodiment, an exosome-producing cell may be an induced pluripotent stem cell, such as an induced pluripotent stem cell derived from a patient to be treated. In certain embodiments, the host cell or exosome-producing cell may comprise a human primary fibroblast from either adult or neonatal, 293 cell, HeLa cell, or SH5Y cell.

Normally, LC3 and proteins bound to it or fused to it are degraded by autophagy in cells. This may limit the amount of fusion construct that can be packaged into exosomes in certain embodiments. Accordingly, in certain embodiments, it is contemplated that the host cells and/or exosome-producing cells may be cells lacking or depleted of proteins such as Atg7. These cells may still package LC3 (and/or other such functional moieties) into exosomes, even though they are incapable of (or deficient at) autophagy. Accordingly, in certain embodiments, by producing exosomes from cells depleted of Atg7, it is contemplated that a larger amount of fusion construct may be available for packaging into exosomes. This may increase the therapeutic activity of the so-produced exosomes in certain embodiments.

In certain embodiments, the host cell or exosome-producing cell may be a cell which does not produce LC3-II, such that resulting exosomes contain LC3-I, and/or fusion constructs comprising LC3-I as functional moiety. As LC3-II is constitutively degraded by autophagy, proteins fused to LC3 may be degraded by autophagy, at least in part, when this is converted to LC3-II, which may reduce the amount that is packaged into exosomes. LC3-I is not degraded by autophagy effectively, so by producing exosomes from cells that cannot make LC3-II (or which make reduced amounts of LC3-II), more fusion-LC3 may be present in exosome-producing cells and packaged into exosomes. Examples of cells that will not convert LC3-I to LC3-II include cells that lack Atg7, Atg4, Atg12, Atg10, Atg16, Atg14, Atg3 and/or other proteins known to those skilled in the art. Once LC3-I fusion proteins enter target cells, which contain functional autophagy machinery, then it may be converted to LC3-II and cause degradation by autophagy where the fusion construct is so-configured, for example.

As will be understood, exosomes produced by different cell types may not deliver to the same tissues (i.e. exosomes from different cell types may have different target cell specificities in terms of delivery). In certain embodiments, it is contemplated herein that the exosome-producing host cell may be selected based on the intended application and/or delivery target of the exosome. By way of non-limiting example, in certain embodiments the host cell may be selected from a variety of cell types producing exosomes that deliver cargoes to specific tissues, which may include, but are not limited to:
(a) 293 cells, which may deliver to, for example, liver cells, fibroblasts, and/or mouse (or mammal) motor neurons;
(b) Human neonatal fibroblasts, which may deliver to, for example, liver, brain, spinal cord, and/or kidney;
(c) NSC-34 cells, which may deliver to, for example, liver, small intestine, brain, and/or spinal cord (and may cross the blood brain barrier); or
(d) SHSY cells, which may deliver to, for example, liver, kidney, and/or brain.

In certain embodiments, the exosomes may be produced from an exosome-producing cell type which is appropriate for the target cell to which targeted cargo delivery by the exosome is desired. In certain embodiments, the exosome-producing cell type may be selected such that resulting exosomes carry one or more surface markers making the exosomes particularly suited for delivery to a particular target cell, such as a cell involved in a disease or disorder for which treatment is desired. Considerations for selection of exosome-producing cell type and/or exosome modification to tailor exosomes for delivery to cells of interest are described, for example, in WO 2017/054085.

In another embodiment, there is provided herein an exosome containing a fusion construct as described herein as cargo.

In still another embodiment, there is provided herein a composition comprising one or more of a fusion construct as described herein; a nucleic acid as described herein; an expression vector as described herein; a host cell as described herein; and/or an exosome as described herein; and, optionally, a pharmaceutically acceptable excipient, carrier, or diluent.

Fusion constructs, compounds, and/or compositions as described herein may include one or more pharmaceutically acceptable excipients, diluents, buffers, and/or carriers. A pharmaceutically acceptable carrier, diluent, buffer, or excipient may include any suitable carrier, diluent, buffer or excipient known to the person of skill in the art. Examples of pharmaceutically acceptable excipients may include, but are not limited to, cellulose derivatives, sucrose, and starch. The person of skill in the art will recognize that pharmaceutically acceptable excipients may include suitable fillers, binders, lubricants, buffers, glidants, and disentegrants known in the art (see, for example, Remington: The Science and Practice of Pharmacy (2006)). Examples of pharmaceutically acceptable carriers, diluents, and excipients may be found in, for example, Remington's Pharmaceutical Sciences (2000 - 20th edition) and in the United States Pharmacopeia: The National Formulary (USP 24 NF19) published in 1999.

In certain embodiments, the composition may further comprise at least one autophagy-activating agent. In certain embodiments, a fusion construct may be for use with at least one autophagy-activating agent. In certain embodiments, a fusion construct may be for administration in combination with, or separately from, at least one autophagy-activating agent. In certain embodiments, the fusion construct may be administered simultaneously, sequentially, or in combination with, at least one autophagy-enhancing agent. In certain embodiments, the autophagy-activating agent may comprise an mTOR inhibitor. In certain embodiments, the autophagy-activating agent may comprise one or more of rapamycin, sirolimus, eversolimus, tacrolimus, INK128, pp242, starvation, or other mTORC1 and/or mTORC inhibitor. In certain embodiments, the autophagy-activating agent may comprise Trehalose and/or Beclin1 peptide.

There are an increasing number of drugs which activate autophagy and autophagic degradation. As a complex pathway involving over 35 proteins and regulated by many metabolic pathways like starvation or depletion of amino acids, ATP or other key metabolites, there are many potential ways to activate autophagy. In certain embodiments, the autophagy-activating agent may comprise a drug that directly or indirectly inhibits PI3K and/or mTOR. Several such activating agents are already FDA-approved. As well, there are many variants of such drugs that have been developed. These may include, for example, Rapamycin, Temsirolimus, Everolimus, Ridaforolimus, PI1-3, PF-04691502, BEZ-235, GNE-493GDC-0980, pp242, pp30, INK128, XL765, GSK2126458, Torin1, WAY-600, WYE-687, WYE354, KU0063794, AZD8055, AZD2014, metformin, resveratrol, and/or latrepirdine. In certain embodiments, the autophagy-activating agent may comprise Beclin1 inhibitor peptides such as TNVFNATFHIWHSGQFGT, that may be conjugated to cell penetrating peptides or delivered or expressed in cells using other mechanisms. These may include other BH3 mimetics like Venetoclax, for example. In another embodiment, the autophagy-activating agent may comprise TFEB, which is a transcription factor that activates expression of many proteins involved in autophagy, and/or TFEB activators that enhance transcription promotion of TFEB such as curcumin, its analogues or drugs such as those described in Wang, et al., Nature Communications, Small-molecule TFEB pathway agonists that ameliorate metabolic syndrome in mice and extend C. elegans lifespan, 9:2270; PMID:29784984.

In another embodiment, the autophagy-activating agent may comprise mTOR independent autophagy activators such as trehalose, SMER10, SMER18, SMER28, or SMER analogs; BRD5631 as described in Kuo et al., PNAS, Small-molecule enhancers of autophagy modulate cellular disease phenotypes suggested by human genetics, 2015, 112(31):E4281-7.

In another embodiment, the autophagy-activating agent may comprise flubendazole, or another compound as described in Chauhan, et al., Nat Commun, Pharmaceutical screen identifies novel target processes for activation of autophagy with a broad translational potential, 2015, 6:8620 (herein incorporated by reference in its entirety). In another embodiment, the autophagy-activating agent may comprise an 5-HT6R inhibitor such as AVN-211, LuAE58054, or SB-742457, for example. In yet another embodiment, the autophagy-activating agent may comprise a compound as listed in Table 1 of Vakifahmetoglu-Norberg, et al., J Clin Invest, Pharmacologic agents targeting autophagy, 2015, 125(1):5-13; Table 1 of Renna et al., J Biol Chem, Chemical Inducers of Autophagy that Enhance the Clearance of Mutant Proteins in Neurodegenerative Diseases, 2010, 285(15):11061-7; and/or Table 4 of Levine et al., J Clin Invest, Development of Autophagy Inducers in Clinical Medicine, 2015, 125(1)14-24.

In certain embodiments, the composition may further comprise at least one siRNA, antisense oligonucleotide (AON), shRNA, miRNA, pre-miRNA, or other gene silencing agent or precursor thereof. In certain embodiments, the gene silencing agent may be a gene silencing agent causing targeted gene silencing of a disease-related target, such as a cellular target which is being targeted by the biologically active agent of the fusion construct, or a cellular target which is associated with or upstream or downstream of the cellular target which is being targeted by the biologically active agent of the fusion construct. In certain embodiments, it is contemplated that an exosome may be provided comprising both a fusion construct as described herein, and a gene silencing agent as described herein, or a mixture of fusion construct- and gene silencing agent-containing exosomes may be provided. Examples of methods for generating exosomes containing gene silencing agents have been described in detail in WO2017/054085, entitled "Exosome Packaging of Nucleic Acids".

In certain embodiments, it is contemplated that the cellular target of the biologically active agent of the fusion construct and the cellular target of the gene silencing agent may be the same or related, such that the fusion construct is able to trigger autophagic degradation of existing cellular target in the cell, while the gene silencing agent prevents accumulation of new cellular target in the cell, thus acting in concert with one another.

In still another embodiment, there is provided herein a method for packaging a fusion construct as described herein into an exosome, said method comprising:
expressing the fusion construct in an exosome-producing cell, or otherwise introducing the fusion construct into the exosome-producing cell; and
culturing the exosome-producing cell in a cell media under conditions in which the exosome-producing cell generates and secretes exosomes comprising the fusion construct into the cell media.

In another embodiment, the methods above may further comprise a step of obtaining, isolating, or purifying the secreted exosomes comprising the fusion construct from the cell media.

As an example, in certain embodiments, exosome-producing cells may be made to constitutively produce a fusion construct as described herein using, for example, a retroviral or lentiviral transduction (or other viral or non-viral transduction) with nucleic acid (for example, DNA) encoding these, or by insertion of similar nucleic acid (i.e. DNA) into the genome using CRISPR or Zinc Finger technology or another suitable tehcnology. In one embodiment, the fusion constructs may be inserted in a pGIPZ lentivirus and stably transduced cells may be selected. Cell types used may include, for example, 293, human fibroblasts, human mesenchymal stem cells, HeLa, ShSY, or any other exosome-producing cell type preferably of human origin (particularly where the subject to be treated is human, for example). In certain embodiments, cells may be preferably grown in media without FBS, or variants of serum-free media such as Lonza Ultraculture, to avoid contamination with exosomes that are abundant in fetal bovine serum used in many cell culture systems. Media may be harvested from cells preferentially after about 24 to 48 h, or another suitable duration appropriate for the particular application. Exosomes may, in certain embodiments, be purified by any suitable method(s) known to those skilled in the art having regard to the teachings herein. By way of non-limiting example, in certain embodiments exosomes may be concentrated from media by Tangential Flow Filtration using a 750 kDa filter, and then dialyzed using a 750 kDa to replace the cell media with a buffer of choice compatible for injection into animals, mammals, or patients, which may in certain embodiments comprise, for example, a sterile saline solution such as PlasmaLyte, for example.

As will be understood, in embodiments where the fusion construct is amino acid-based (i.e. a protein or peptide), the fusion construct may be expressed in the exosome-producing cell from an expression vector or other nucleic acid introduced into the cell, which may be configured with a coding sequence encoding the fusion construct, and a promoter sequence and/or other suitable sequences such that the fusion protein will be expressed in the cell (i.e. the cellular machinery will produce the fusion construct. In certain embodiments, the promoter sequences may include, for example, CMV or CAG promoters, EF1A, UBB, H1, or Tet-inducible promoters, or another suitable promoter that may induce a sufficient and/or high level of expression.

In certain embodiments, the fusion construct may be introduced into the exosome-producing cell, rather than expressed in the exosome-producing cell. By way of example, in certain embodiments, the fusion construct may be transfected or otherwise delivered into the exosome-producing cell using a suitable delivery vehicle selected based on the nature of the fusion construct, and/or the nature of the exosome-producing cell. In certain embodiments, delivery approaches and/or delivery vehicles may comprise using, for example, one or more cell penetrating peptides, lipid nanoparticles, gold nanoparticles, electroporation, Calcium phosphate, attachment to magnetic beads and penetration into cells using magnets, or any combination thereof, or another delivery approach and/or delivery vehicle known to the person of skill in the art having regard to the teachings herein. In certain embodiments, it is contemplated that exosomes may be squeezed under pressure, for example as done by SQZ to introduce things into cells or directly into exosomes.

In certain embodiments, the step of culturing the exosome-producing cell may include culturing or incubating the cell under conditions in which the exosome-producing cell generates and secretes exosomes (loaded with the fusion construct) into the cell media in which the exosome-producing cell is incubated. In certain embodiments, serum-free media or exosome-depleted media may be used with otherwise standard and/or suitable cell culture conditions.

In certain non-limiting embodiments, the exosome-producing cells may be cultured in serum-free media, or in serum media which has been previously treated or processed to remove or reduce exosomal content (i.e. exosome-depleted serum media), while producing exosomes, so as to prevent or reduce contamination of produced exosomes with exosomes typically present in typical serum-containing media. In certain non-limiting embodiments involving cells which require serum-containing media for growth, it may also be possible to remove the serum media and culture the cells temporarily in serum-free media during production/harvest of produced exosomes being released into the serum-free media. In certain cases, however, abrupt removal of serum media may decrease exosome production in certain cells.

Generally speaking, exosomes are typically 40-150 nm vesicles released by a variety of cell types. Exosomes may be composed of a lipid bilayer and a luminal space containing a variety of proteins, RNAs and other molecules derived from the cytoplasm of the exosome-producing cell. Both the membrane and lumen contents of exosomes may be selectively enriched in subpopulations of lipids, proteins and RNA from the exosome-producing cell. The exosome membrane is frequently, but not necessarily, enriched in lipids including cholesterol and sphingomyelin and contain less phosphatidycholine. The membrane of exosomes may be enriched in particular proteins derived from the plasma membrane of cells such as tetraspanins (e.g. CD63, CD81 CD9), PrP and MHC class I, II. The exosome lumen may be enriched in proteins such as Flotillinl and 2, annexin 1 and 2, heat shock proteins, Alix and Tsg101. It will be understood that exosomes as described herein may, in certain non-limiting embodiments, also encompass exosome-like vesicles. The person of skill in the art will recognize that references to exosomes herein may include other suitable exosome-like vesicles which may vary somewhat from typical exosomes, but are still functionally and/or structurally similar or related.

In certain embodiments, the step of obtaining, isolating, or purifying the secreted exosomes comprising the fusion construct from the cell media may comprise one or more of ultracentrifugation, precipitation, and/or size selection such as by gel filtration, tangential flow filtration, and/or other suitable methods using size selection.

Introduction of gene or a transcribed sequence into a cell may be accomplished using any of several methods known in the art. By way of example, a vector (either viral, plasmid, or other) comprising one or more copies of the particular gene each driven by a suitable promoter sequence (for example, a constitutive or inducible promoter), may be introduced into cells via transfection, electroporation, or viral infection, or another suitable method know in the art. Suitable expression vector techniques for overexpressing or introducing a particular gene into a cell are known in the art (see, for example, Molecular Cloning: A Laboratory Manual (4th Ed.), 2012, Cold Spring Harbor Laboratory Press). As will be known to one of skill in the art, nucleotide sequences for expressing a particular gene or transcribed sequence/region may encode or include features as described in "Genes VII", Lewin, B. Oxford University Press (2000) or "Molecular Cloning: A Laboratory Manual", Sambrook et al., Cold Spring Harbor Laboratory, 3rd edition (2001). A nucleotide sequence encoding a particular nucleic acid construct may be incorporated into a suitable vector, such as a commercially available vector. Vectors may also be individually constructed or modified using standard molecular biology techniques, as outlined, for example, in Sambrook et al. (Cold Spring Harbor Laboratory, 3rd edition (2001)). The person of skill in the art will recognize that a vector may include nucleotide sequences encoding desired elements that may be operably linked to a nucleotide sequence encoding a nucleic acid construct. Such nucleotide sequences encoding desired elements may include transcriptional promoters, transcriptional enhancers, transcriptional terminators, and/or an origin of replication. Selection of a suitable vector may depend upon several factors, including, without limitation, the size of the nucleic acid to be incorporated into the vector, the type of transcriptional and translational control elements desired, the level of expression desired, copy number desired, whether chromosomal integration is desired, the type of selection process that is desired, or the host cell or the host range that is intended to be transformed.

In certain embodiments, the above method may, optionally, include a step of collecting or enriching the produced exosomes. As will be understood, exosomes may be purified by any of several suitable methods known to those skilled in the art having regard to the teachings provided herein. For example, exosomes may be purified by differential centrifugation in which cells and larger vesicles or debris are eliminated in preliminary centrifugation steps of up to 10 to 20 000 g, and exosomes may be subsequently enriched from the resulting supernatant by centrifugation at or above 70 000 g for 1 h in a SW28 or SW32 rotor (or an equivalent in other rotor types). Exosomes may also be purified by precipitation using reagents such as Systems Biosciences Exoquick, Exiqon miRCURY exosome isolation kit, or Total exosome isolation kit from Thermofisher, or similar techniques. Exosomes may also be enriched using size based filtration using vacuum pumps, tangential flow filtration, or centrifugal filtration. In such methods cells and larger vesicles or debris may be eliminated by filtering through a filter with pores larger than 100 nm and typically of 0.22 um. Exosomes may then be concentrated using filters with pores smaller than 100 nm by tangential flow filtration or other filtering methods. Exosomes may also be purified by affinity purification. In such methods, antibodies or other ligands which bind to exosomes may be coupled to beads or other fixed supports to allow capture, purification and concentration of exosomes from liquids. The person of skill in the art having regard to the teachings herein will be able to select a suitable collection or enriching technique suitable for a particular application. By way of non-limiting example for illustrative purposes, exosomes may be purified by a number of methods including those detailed in Thery et al. Isolation and characterization of exosomes from cell culture supernatants and biological fluids. Curr Protoc. Cell Biol. 2006 Alternative methods to purify exosomes may include precipitation methods, such as those used in the Systems Biosciences Exoquick kit or similar kits sold by companies such as Life Technologies or Qiagen. Exosomes may also be purified using affinity-purification, such as beads coated with antibodies recognizing elements of exosomes. Exosomes may also be purified using density gradients (e.g. sucrose density gradients) based on their unusual density (Thery et al. above, Lamparski et al. J. Immunological Methods 2002).

As well, Exosomes may be purified by chromatography, such as by size exclusion chromatography or field-flow fractionation or tangential flow filtration, as will be known to those of skill in the art.

In certain embodiments of the above-described methods, the exosome-producing cell may have low or reduced levels of Atg7. In certain embodiments, the exosome-producing cell may be a cell having low, reduced, knockout, or knockdown levels of Atg7, so as to enhance production of exosomes containing the fusion construct. In certain embodiments, the exosome-producing cell may be Atg7^{-/-}. By way of non-limiting example, in certain embodiments, the exosome-producing cell may be a 293 cell; a human neonatal fibroblast; an NSC-34 cell; an SH5Y cell; a BV2 cell; or another exosome-producing cell. Examples of exosome-producing cells, also referred to as host cells herein, have already been described in detail hereinabove.

In certain embodiments of methods as described herein, the methods may further comprise a step of treating the exosome-producing cell with a lysosomal or autophagy inhibitor. By way of example, exosome-producing cells may be treated with inhibitors of lysosomal acidification or VIVO ATPase for 2 to 72 hours to increase production of exosomes. Examples of such inhibitors may include Bafilomycin Al, concanamycin, NH₄Cl, and/or chloroquine. It is contemplated that other compounds having similar effects on lysosomes (i.e. affecting pH or Ca²⁺ balance) may have similar effects, such as NAADP, for example.

In certain embodiments if the above methods, the secreted exosomes may further comprise, in addition to the fusion construct, an autophagy-activating agent, a gene silencing nucleic acid, or both. In certain embodiments, the methods described herein may be adapted such that the produced exosomes contain not only the fusion construct, but also one or both of an autophagy-enhancing agent, a gene silencing nucleic agent, or both. In certain embodiments, a fusion construct as described herein may be provided in combination with a gene silencing nucleic acid (for example, siRNA), and/or another nucleic acid or RNA, which one or both may (or may not) be packaged into exosomes (either the same or different). In certain embodiments, there is provided herein a composition comprising a fusion construct as described herein, and a gene silencing nucleic acid, and/or another nucleic acid of interest. In certain embodiments, the fusion construct and the gene silencing nucleic acid (or other nucleic acid of interest) may both be packaged into the same exosome. In certain embodiments, the biological function of the biologically active agent of the fusion construct and the biological function of the gene silencing nucleic acid (or other nucleic acid of interest) may be selected so as to be complementary, such that an improved therapeutic or other biological effect is provided as compared to the effect of either component alone. By way of example, in certain embodiments, it is contemplated that both the biologically active agent and gene silencing agent may be selected to target viral infection (for example, by targeting the viral genome or other viral component, and/or may degrade viral genome and/or eliminate sites of viral replication, for example). As well, it is contemplated that the fusion construct may be designed to cause elimination of existing inclusions, and the gene silencing agent may be designed to prevent formation of new inclusions, in a neurodegenerative disease, for example. Examples of methods for generating exosomes containing gene silencing agents have been described in detail in WO2017/054085, entitled "Exosome Packaging of Nucleic Acids".

It is contemplated that in certain embodiments, nucleic acid constructs described in this reference may be adapted for introducing gene silencing nucleic acids into exosomes containing the fusion constructs as described herein, for example.

In certain embodiments, it is contemplated that an exosome packaged with a gene silencing nucleic acid may be for administration to a cell or subject in need thereof either before, concurrently with, or after administration of a fusion construct as described herein (which may, or may not, be packaged in an exosome) to the cell or subject in need thereof, for example.

In yet another embodiment, there is provided herein an exosome produced by any of the method or methods described herein. In certain embodiments, the exosome may be packaged with one or more fusion constructs as described herein. In certain embodiments, the exosome may be provided in a cell media, or may be provided in purified or isolated form, or may be provided in a pharmaceutically acceptable carrier, diluent, or buffer. In certain embodiments, the exosomes may be provided in a pharmaceutically acceptable carrier, diluent, or buffer, which is suitable for maintaining the exosomes substantially intact prior to use thereof. In certain embodiments, the exosomes may be stored with an osmotic buffering agent, such as sucrose, so as to improve their ability to be frozen and thawed substantially intact. In certain embodiments, it is contemplated that the exosomes might be dried and reconstituted. In certain embodiments, the exosomes may be mixed with, or provided with, an additional autophagy-activating agent, examples of which have already been described in detail hereinabove. In certain embodiments, the exosomes as described herein may further comprise an autophagy-activating agent, a gene silencing nucleic acid, or both, as already described in detail herein.

In still another embodiment, there is provided herein an in vitro method for delivering a biologically active agent into a cell, said method comprising:
expressing or introducing a fusion construct as described herein (comprising the biologically active agent) into an exosome-producing cell; and
administering the exosome-producing cell such that exosomes produced by the exosome-producing cell contact the cell; or
culturing the exosome-producing cell in a cell media under conditions in which the exosome-producing cell generates and secretes exosomes comprising the fusion construct into the cell media;
obtaining, isolating, or purifying the secreted exosomes comprising the fusion construct from the cell media; and
contacting the cell with the secreted exosomes.

In certain embodiments, the exosomes may further comprise an autophagy-activating agent, a gene silencing nucleic acid, or both, as already described in detail herein.

As will be understood, the step of contacting the cell with the secreted exosomes may comprise generally any suitable technique for administering the exosomes to a cell in need thereof such that the exosomes contact the cell, and are able to deliver their cargo into the cell. Where the cell is cultured *in vitro,* the exosome may be directly added to the cell media in which the cell is cultured, for example. In certain embodiments, as described hereinabove, the exosomes may, by virtue of the cell-type of the exosome-producing cells from which they are obtained, feature one or more surface proteins or markers targeting the exosomes to a particular cell type or tissue. Accordingly, in certain embodiments, it is contemplated that the exosomes may be selected so as to provide targeted delivery to the particular type of disease cell to be treated. Examples of exosome-producing cell types, and exosomes produced therefrom, favoring targeted delivery to certain cells or tissues are described herein for illustrative purposes.

It is contemplated that nucleic acids and/or expression vectors as described herein may be administered to a subject or cell in need thereof such that the cell is made to express a fusion construct as described herein. It is contemplated that a target cell, such as a cell of a subject in need of treatment, may be made to express a fusion construct as described herein. Where the cell of the subject is an exosome-producing cell, the cell may then produce exosomes packaged with the fusion construct *in vivo,* thereby providing a therapeutic benefit to the subject, for example. It is contemplated that a host cell and/or an exosome-producing cell as described herein, expressing a fusion construct as described herein, which may produce exosomes packaged with the fusion construct, may be administered to a subject in need thereof such that exosomes from the host cell or exosome-producing cell may then provide a therapeutic benefit to the subject. Accordingly, while administration of exosomes to the subject in need of treatment is described herein, it is also described herein that host cells/exosome-producing cells, and/or nucleic acids encoding/expression fusion constructs, as described herein may be administered to the subject in need of treatment. For example, it is contemplated that cells producing exosomes packaged with a fusion construct as described herein may be introduced into, or created in, a subject in need of treatment, for example by using a viral-based gene delivery approach such as an adeno-associated virus or lentivirus to express a fusion construct as described herein in cells of a patient, which may then produce exosomes and delivery the fusion construct. As well, it is contemplated that exosome-producing cells as described herein expressing a fusion construct as described herein may be administered to a subject in need thereof, which may then produce exosomes in the subject. It is contemplated that such approaches may be of particular interest where, for example, cases of bone marrow or organ transplant are involved, and/or in treatments with cells such as mesenchymal stem cells or monocytic/microglia cells that may or may not integrate into tissue for the long term (but may act as a source of exosomes in the subject), for example. The subject may be an animal, a mammal, or a human, for example.

A nucleic acid or expression vector as described herein, or an AAV, lentivirus, or oncolytic virus comprising such nucleic acid or expression vector, or another construct, composition, or exosome as described herein, for example, may be used to treat a patient directly as described above, or may be used to treat a CAR T cell or transplanted cell or organ *ex vivo,* for example.

The exosome-producing cell may have low or reduced levels of Atg7. The exosome-producing cell may have low or reduced levels of another protein required for autophagy, such as Atg4, Atg14, Atg3, Atg14, Atg12 (but typically not Atg5 or Atg16L1), for example. The exosome-producing cell may be a cell having low, reduced, knockout, or knockdown levels of Atg7, so as to enhance production of exosomes containing the fusion construct. The exosome-producing cell may be Atg7^{-/-}. By way of non-limiting example, the exosome-producing cell may be a 293 cell; a human neonatal fibroblast; an NSC-34 cell; an SH5Y cell; a BV2 cell; or another exosome-producing cell. Examples of exosome-producing cells, also referred to as host cells herein, have already been described in detail hereinabove.

The exosome-producing cell may be a 293 cell, and the cell to be treated may be a liver cell, a fibroblast cell, or a motor neuron.

The exosome-producing cell may be a human neonatal fibroblast, and the cell to be treated may be a liver cell, a brain cell, a spinal cord cell, or a kidney cell.

The exosome-producing cell may be an NSC-34 cell, and the cell may be a liver cell, a small intestine cell, a brain cell, or a spinal cord cell.

The exosome-producing cell may be a SH5Y cell, and the cell to be treated may be a liver cell, a kidney cell, or a brain cell.

In certain embodiments of the above-described methods, the exosome-producing cell may be an exosome-producing cell or a host cell as already described in detail hereinabove.

WO2017/054085, entitled "Exosome Packaging of Nucleic Acids", describes considerations for packaging of gene silencing nucleic acids of interest into exosomes (which may be relevant where, for example, a gene silencing nucleic acid agent is to be included in the exosome along with a fusion construct as described herein), as well as relevant methods and/or considerations for selecting exosome-producing cells appropriate for a particular application and/or delivery to a particular target cell; for culturing cells for producing exosomes (as well as relevant steps/conditions therefor); for obtaining/isolating/purifying produced exosomes; and/or for administering the exosomes to a subject in need thereof, for example.

As will be understood, in certain embodiments, the fusion construct may comprise any suitable protein or peptide sequence of interest for which exosomal packaging and/or delivery into cells (via exosomes) is desired, wherein the protein or peptide sequence of interest is fused with a functional moiety as described herein. Depending on the particular application, the protein or peptide sequence may be fused with the N- or C-terminus of the functional moiety, depending on the nature of the functional moiety and/or whether release of the protein or peptide from the functional moiety once inside the target cell is desired (see discussion of N- or C-terminus fusion to LC3 discussed hereinabove for further detail). Accordingly, in certain embodiments, methods as described herein may be considered as methods for packaging a protein of interest into an exosome, and/or as methods for delivering a protein of interest into a target cell via an exosome. Furthermore, in certain embodiments such as those where the protein or peptide of interest remains fused with the functional moiety in the target cell, and the protein or peptide of interest has an ability to bind a cellular or cytoplasmic target in the target cell, then methods as described herein may be considered as methods for triggering targeted degradation of a cellular target by autophagy, for example (see, for example, Figure 30).

In still another embodiment, there is provided herein a fusion construct as described herein; a nucleic acid as described herein; an expression vector as described herein; a host cell as described herein; an exosome as described herein; a composition as described herein; or any combination thereof; for use in treating or preventing a disease or disorder in a subject in need thereof. In certain embodiments of the above uses, the disease or disorder may be any suitable disease or disorder for which the biologically active agent of the fusion construct may provide a benefit. In embodiments where the fusion construct is designed to trigger autophagy and/or targeted autophagy, the disease or disorder may be any suitable disease or disorder related to, or treatable by, autophagy. As will be known to the person of skill in the art, a wide variety of diseases and disorders may involve autophagy, and/or may be treatable by agents triggering autophagy and/or targeted autophagy. Diseases or disorders which may be associated with and/or treatable by autophagy-modulation are described, for example, in Galluzzi et al., Pharmacological modulation of autophagy: therapeutic potential and persisting obstacles, Nature Reviews Drug Discovery, 2017, 16:487-511; Rubinsztein et al., Autophagy modulation as a potential therapeutic target for diverse diseases, Nat Rev Drug Discov, 2012, 11(9):709-730; and Dikic et al., Mechanism and medical implications of mammalian autophagy, Nature Reviews Molecular Cell Biology, 2018, 19 349-364.

As will be understood, fusion constructs as described herein may be designed to target generally any suitable target, such as but not limited to disease-related targets ranging from very small in size all the way to very large in size (for example, it is contemplated that targets of even up to about 10 um, or up to about 20 µm, or perhaps larger, in size may be targeted by autophagy-inducing fusion constructs as described herein).

In certain embodiments of the above uses, the disease or disorder may be caused by, or associated with, any one or more of misfolded proteins, altered proteins, protein inclusions, protein aggregates, lipid droplets, bacteria, cytoplasmic DNA, or mitochondria. In certain embodiments, for example, the disease or disorder may be a neurodegenerative disease or atherosclerosis. In certain embodiments, the disease or disorder may be Alzheimer's, CTE, and/or Tauopathy-related disease; Parkinson's; Frontal Temporal Dementia; and/or Amyotrophic Lateral Sclerosis (ALS).

In certain embodiments, the exosomes may further comprise an autophagy-activating agent, a gene silencing nucleic acid, or both, as already described in detail herein.

In certain embodiments of the above uses, the subject in need of treatment may be an animal, such as a mammal or a human subject.

In certain embodiments, the fusion construct; the nucleic acid; the expression vector; the host cell; the exosome; the composition; the exosome; or any combination thereof; of the above described uses may be for administration in combination with at least one autophagy-activating agent, at least one gene silencing agent, or both. In certain embodiments, the autophagy-activating agent may comprise an mTOR inhibitor. In certain embodiments, the autophagy-activating agent may comrpise one or more of rapamycin, sirolimus, eversolimus, tacrolimus, INK128, pp242, starvation, or other mTORC1 and/or mTORC inhibitor. In certain embodiments, the autophagy-activating agent may comprise Trehalose and/or Beclin1 peptide. Examples of autophagy-activating agents have already been described in detail hereinabove. In certain embodiments, the gene silencing agent may comprise any suitable gene silencing agent (for example, an siRNA, shRNA, miRNA, antisense oligonucleotide (AON), pre-miRNA, or precursor thereof), which may target a therapeutically relevant target in the cell or subject to be treated, which may be the same as or different from the cellular target targeted by the biologically active agent of the fusion construct. In certain embodiments, the gene silencing agent may be provided in the exosome via constructs and/or approaches as described in WO2017/054085, entitled "Exosome Packaging of Nucleic Acid.

In still another embodiment, there is provided herein a kit comprising at least one of:
a fusion construct as described herein;
a nucleic acid as described herein;
an expression vector as described herein;
a host cell as described herein;
an exosome as described herein;
a composition as described herein;
an exosome as described herein;
instructions for performing a method as described herein;
one or more containers for securing one or more components of the kit;
or any combination thereof.

### EXAMPLE 1 - Atg5 Dissociates the V1V0-ATPase to Promote Exosome Production and Tumor Metastasis Independent of Canonical Macroautophagy

Autophagy and Autophagy-related genes (Atg) have been attributed prominent roles in tumorigenesis, tumor growth and metastasis. Extracellular vesicles called exosomes are also implicated in cancer metastasis. This Example demonstrates that exosome production is strongly reduced in cells lacking Atg5 and Atg16L1, but this is independent of Atg7 and canonical autophagy. Atg5 specifically decreases acidification of late endosomes where exosomes are produced, disrupting the acidifying V₁V₀-ATPase by removing a regulatory component, ATP6V1E1, into exosomes. The effect of Atg5 on exosome production promotes the migration and *in vivo* metastasis of orthotopic breast cancer cells. These findings uncover mechanisms controlling exosome release, and identify means by which autophagy-related genes may contribute to metastasis in autophagy-independent pathways.

Exosomes are 40-120 nm vesicles released by many cell types (Colombo et al., 2014). As endosomes mature their membranes bud inward to produce vesicles inside the endosomal lumen (Hanson and Cashikar, 2012). These Multivesicular Bodies (MVB) can subsequently fuse with the plasma membrane to release their intraluminal vesicles to the extracellular space where they are called exosomes (Colombo et al., 2014).

Mechanisms underlying exosome production are slowly being elucidated. ESCRT and ESCRT-associated proteins are required for budding of vesicles into endosomes (Colombo et al., 2013). Rab27a and b control fusion of MVB with the plasma membrane to release exosomes (Ostrowski et al., 2010). However, steps controlling MVB fate after generation of intraluminal vesicles and before release of exosomes have remained obscure.

A complex of Autophagy-related genes (Atg) including Atg5-Atg12 and Atg16L1 terminates an enzymatic cascade culminating in the covalent modification of LC3-I (Atg8) with the lipid phosphatidylethanolamine (PE) to form LC3-II (Bento et al., 2016). Generation of LC3-II is canonically involved in the expansion of the phagophore and its closure to form a double-membraned autophagosome. Autophagosomes then fuse with late endosomes and lysosomes to degrade their engulfed cytoplasmic contents (Bento et al., 2016). While examples of non-canonical autophagy have recently been described that are independent of Atg5 and Atg7, the majority of autophagy pathways described to date are dependent on these two canonical factors.

The Atg5-Atg12 and Atg16L1 complex, along with LC3 has also been observed to localize to endosomes and phagosomes to secure the acidification of these compartments and lysosomal degradation of pathogens or apoptotic cells they enclose (Florey et al., 2011; Martinez et al., 2015). Other studies have also shown that autophagy genes promotes lysosomal trafficking and degradation of endosomal contents (Peng et al., 2014; Zhou et al., 2013). The mechanism underlying the effects of Atg5 on acidification of phagosomes and endosomes are unclear (Ktistakis and Tooze, 2016).

If Atg5, Atg16L1 and LC3 localize to multiple types of endosomes it is plausible that they localize to subpopulations of MVB producing exosomes as well, and could impact exosome biogenesis or release. Several studies have begun to investigate the effects of Atg genes on exosome production but the results of this research have frequently been contradictory. A unique covalent Atg3-12 complex, that like the Atg5-Atg12 complex requires Atg7 for its formation, promotes exosome release (Murrow et al., 2015). While this agrees with a subsequent report that Atg7 is required for release of hepatitis C in exosome-like vesicles (Shrivastavaet al., 2016), it contradicts a previous report that Atg7 has no impact on exosome release (Sahu et al., 2011). Other studies have observed that Bafilomycin or other inhibitors of lysosomal acidification, that also can inhibit autophagy and autophagic degradation increase exosome release (Alvarez-Erviti et al., 2011). Effects of these inhibitors on exosome release have at times been attributed to a blockade in autophagy although the mechanism is unknown. These apparent contradictions may be due to the reliance of each of these studies on particular types of exosomes (e.g. hepatitis C virus containing) and western blot to quantify exosome release. Western blot cannot differentiate whether effects are due to larger contaminating vesicles, effects on a subpopulation of exosomes or specific exosome contents (Colombo et al., 2013; Lotvall et al., 2014; Tauro et al., 2013). To avoid these possibilities, recent guidelines recommend at least two methods to quantify exosome release including one that quantifies populations of exosomes using their characteristic size (Lotvall et al., 2014). In sum, the effect of genes required for autophagy like Atg5 and Atg16L1 on exosome production have not been studied previously, and published data on the effect of other Atg genes on exosome production are contradictory.

Here, using multiple orthogonal approaches, we demonstrate that Atg5 is key for exosome production but that this is independent of Atg7 and canonical autophagy. Atg5 detaches ATP6V1 E1 from the V₁V₀-ATPase that acidifies MVB. This disassembles the V₁V₀-ATPase complex, decreases the acidification of MVB and promotes release of exosomes. Atg5-dependent control of exosome production increases migration of breast cancer cells *in vitro* and promotes metastasis *in vivo* in an orthotopic model of breast cancer.

### Results

### Exosome levels are decreased in cells lacking Atg5 and Atg16L1, but not Atg7

Exosome preparations obtained by differential ultracentrifugation from the human breast cancer cell line MDA-MB-231 and mouse embryonic fibroblast (MEF) cells were predominantly composed of 40-120 nm particles (Nanoparticle tracking, dynamic light scattering, Fig.1A, Fig.7A), enriched in exosome markers (Tsg101, Flotillin2) but not markers of cellular debris derived from distinct mitochondrial compartments (Tomm20, Fig.1B), and exhibited the appearance of exosomes by electron microscopy (Fig.7A) (Thierry et al., 2006). In comparison to their wild-type counterparts, exosome preparations from equivalent amounts of Atg5^{-/-} MEF (Fig.7B) contained strikingly reduced levels of exosome markers (Flotillin2, Tsg101, Fig.1B) and exosome-sized particles by nanoparticle tracking and dynamic light scattering (Fig.1A,C, Fig.7A-C). Exosome levels were similarly reduced in ATG5^{-/-} human MDA-MB-231 cells (Fig.1A-C, Fig.7A-C). Decreased exosome production by Atg5^{-/-} cells was not due to differential cell numbers attained at the end of exosome production (Fig.7D). Transiently repressing Atg5 in MEFs using a tetracycline-dependent system (Fig.1D), also reduced numbers of exosome-sized particles and levels of exosome markers in exosome preparations (Fig.1E,F). These effects were rescued by re-induction of Atg5 (Fig.1D-F). HCT-116 cells lacking Atg16L1 also produced less exosomes (Fig.1G). In contrast, exosome production measured by western blot and particle concentration by nanoparticle tracking or dynamic light scattering was not decreased in Atg7^{-/-} MEF, Atg14^{-/-} MEF or when Atg7 was depleted with siRNA in MDA-MB-231 cells, despite LC3-II production being ablated (Fig.1H, Fig.7 E-I).

### Atg5 does not affect exosome uptake from media

Next, it was sought to systematically identify the step of exosome biogenesis or re-uptake affected by Atg5. Atg5^{-/-} MEFs exhibited no difference in uptake of DiO-labeled exosomes, or depletion of labeled exosomes from media, compared to wild-type cells (Fig.8A-C). This suggests that exosomes are not less abundant in cell culture supernatants of cells lacking functional Atg5 because of increased cellular uptake of exosomes.

### Atg5 does not affect budding of intraluminal vesicles into MVB

Production of a covalent Atg3-Atg12 complex that was recently demonstrated to be key for budding of intraluminal vesicles into MVB, was not decreased in Atg5^{-/-} MEF (Fig.9A). Atg5^{-/-} MDA-MB-231 and MEF also exhibited similar punctae of CD63, a hallmark exosome marker, inside endosomes labeled with Rab5, a marker frequently used to model budding into MVB involved in exosome production (Baietti et al., 2012; Gross et al., 2012; Trajkovic et al., 2008) (Fig.2A, Fig.9B). By electron microscopy, Atg5^{-/-} MEF exhibited a significantly increased number of intraluminal vesicles per MVB and increased MVB diameter (Fig.2B-C, Fig.9D-G). Despite this, MVB intraluminal vesicles in Atg5^{-/-} MEF were of similar size and morphology to MVB of wild-type cells (Fig.2B) and the percent of MVB exhibiting membrane invaginations, a measure of MVB budding and scission activity (Wemmer et al., 2011), was also unchanged (Fig.9C). Nor did MVB exhibit tubular or multicisternal protrusions in Atg5^{-/-} MEF as observed with some ESCRT mutants with impaired budding (Fig.2B) (Razi and Futter, 2006). Together, this suggests that Atg5 does not affect budding and scission of intraluminal vesicles into MVB (Futter et al., 2001; Hurley and Hanson, 2010).

### Loss of ATG5 increases MVB acidification independent of lysosomal fusion

By electron microscopy, it was observed that Atg5^{-/-} MEF exhibited a significantly increased number of intraluminal vesicles per MVB and increased MVB diameter (Fig.2B-C, Fig.9D-G), two signs of increased MVB acidification and maturation (Huotari and Helenius, 2011; Puchner et al., 2013). Markers of MVB that are also enriched in exosomes, namely NRhPE (N-lissamine rhodamine phosphatidylethanolamine) (Fig.9H) and mCherry-Alix (Vidal et al., 1997) exhibited unaltered co-localization with either of two lysosome markers (LAMP1, CathepsinB) in wild-type, Atg5-repressed, or Atg5-reinduced MEFs (Fig.9I-K). In contrast, co-localization of the exosome marker mCherry-Alix with Lysotracker that marks acidified compartments increased in Atg5^{-/-} cells measured using two independent measures of co-localization (Fig.2D,E, Fig.9L). Similarly, NRhPE co-localization with Lysotracker increased in ATG5^{-/-} MDA-MB-231 cells not at early stages of endocytosis, but at time points when it accumulates in MVB (Vidal et al., 1997) (Fig.2F-H). Similar results were observed in Atg5 MEF (Fig.9M-N). Finally, Alix colocalization with Lysotracker increased upon repression of Atg5 by tetracycline and returned to normal upon rescue of Atg5 levels (Fig.2I,J).

In direct contrast with the effect of Atg5 on MVB labeled with exosome markers Atg5 promoted acidification of dextran-containing endosomal compartments as previously published (Florey et al., 2011; Ktistakis and Tooze, 2016; Martinez et al., 2015; Peng et al., 2014; Zhou et al., 2013) (Fig.10A,B). Distinct from the effect of Atg5 on compartments containing Alix and NRhPE, repression of Atg5 reduced EGF trafficking to the lysosome (LAMP1, CathepsinB, Fig.10C-E) as previously reported. Strangely, this was not rescued when Atg5 was re-induced (Fig.10C-E). In sum, in Atg5^{-/-} cells, MVB exhibit increased size and number of intraluminal vesicles and compartments containing MVB and exosome markers (mCherry-Alix, NRhPE) exhibit increased acidification (Lysotracker staining), but do not exhibit increased colocalization with lysosome markers (LAMP1, CathepsinB). Together, this suggests that MVB acidification is accentuated in Atg5^{-/-} cells independent of lysosomal fusion and these effects contrast with other endosomal compartments (dextran, EGF).

### Atg5 causes disassociation of A TP6V1E1 from the V₁V₀-ATPase at MVB

To further understand the relationship between ATG5 and MVB acidification, we investigated the V₁V₀-ATPase, a multi-molecular proton pump mediating acidification of endosomes and lysosomes (Cotter et al., 2015). It was observed that ATP6V1E1 , an exposed peripheral stalk component of the cytoplasmic V1 complex co-localized with the MVB marker CD63 (Fig.3A). ATP6V1E1 was previously demonstrated in yeast and mammalian cells to detach from the V₁V₀-ATPase to decrease its activity (Sautin et al., 2005; Toei et al., 2010). SiRNA targeting ATP6V1E1 diminished Lysotracker staining of cells to a similar extent as siRNA targeting other critical components of the V₁V₀-ATPase such as VOC (Fig.3B), demonstrating that ATP6V1E1 is capable of regulating the V₁V₀-ATPase in mammalian cells. To quantify detachment of ATP6V1E1 from the V₁V₀-ATPase in Atg5^{-/-} cells, proximity ligation assays (PLA) for ATP6V1E1 and a core transmembrane component of the V₁V₀-ATPase, ATP6V0C, were performed. As expected, PLA dots indicating a close association of ATP6V1E1 with V₁V₀-ATPase were detected and dots were eliminated by siRNA targeting ATP6V1E1 validating the specificity of the assay (Fig.3C). Transient repression of Atg5 increased the amount of ATP6V1E1 associated with the V₁V₀-ATPase, and this returned to normal when Atg5 expression was restored (Fig.3D,E). Association of ATP6V1E1 with the V₁V₀-ATPase was also increased in cells genetically deleted of Atg5 (Fig.3F), but was unaltered in Atg7^{-/-} cells (Fig.3G). Loss of Atg5, but not Atg7 enhanced the association of ATP6V1E1 with the V₁V₀-ATPase at MVB marked by mCherry-Alix (Fig.3H). This mirrors the effect of Atg5, but not Atg7 on exosome production (Fig.1), suggesting that Atg5 detaches ATP6V1E1 from the V₁V₀-ATPase complex to regulate MVB acidification and exosome production independent of Atg7.

### The enrichment of LC3 in exosomes is diminished in cells lacking ATG5, notATG7

It was investigated how Atg5 might detach ATP6V1E1 from the V₁V₀-ATPase to affect MVB acidification. Atg5-mCherry localized to compartments co-labeled with both the exosome markers CD63 and Rab5 (Fig.4A). Interestingly, Atg5 and Atg16L1 were also abundant in exosomes produced by multiple cell types compared to their levels in similar amounts of cell lysate (Fig.4B). The Atg5 complex binds LC3 and lipidates it to form LC3-II. LC3 was also abundant in these exosome preparations (Fig.4B). Remarkably, exosomes were strongly enriched in lipid-modified LC3-II (vs. unmodified LC3-I) compared to the ratio of LC3-II to LC3-I in the corresponding cell lysates (Fig.4B,C). This suggests that Atg5 and LC3 are sorted into exosomes. One mechanism by which Atg5 could detach ATP6V1E1 from the V₁V₀-ATPase complex at MVB would be for Atg5 to remove ATP6V1E1 into the intraluminal vesicles budding into MVB that become exosomes. In agreement with this possibility ATP6V1E1 detected with a specific antibody was also detected in exosomes (Fig.4D).

It was first sought to validate that LC3 was found in *bona fide* exosomes. LC3 was enriched in exosome preparations from multiple mouse and human cells (MEF, MDA-MB-231, HCT-116, Neuro2A, 3T3 Fig.4B, Fig.11A). If LC3 is found in exosomes it should co-localize with endosomes and the intraluminal vesicles of MVB. Constitutively-active Rab5 (Rab5-Q79L) enlarges endosomes and faithfully documents the budding of intraluminal vesicles into MVB during exosome biogenesis (Baietti et al., 2012; Gross et al., 2012; Trajkovic et al., 2008).

Accordingly, punctae inside Rab5-Q79L endosomes were labeled by antibodies recognizing Alix, Tsg101 or CD63, established markers of intraluminal vesicles and exosomes (Fig.4E,F, Fig.11B-D). LC3 exhibited similar punctate staining within Rab5-Q79L+ endosomes (Fig.4E,F) that co-localized in part with exosome markers Alix, Tsg101 and CD63 in MEF and MDA-MB-231 cells (Fig.4E,F, Fig.11B-D). On sucrose density gradients of exosome preparations, LC3-II fractionated with the exosome markers Alix, CD63 and Flotillin-1 at densities characteristic of exosomes (Fig.4G) (Thery et al., 2006). Finally, LC3 antibody labeled vesicles of 30-100 nm in exosome preparations using electron microscopy (Fig.4H,I). Cumulatively this evidence demonstrates that LC3-II is enriched in *bona fide* exosomes from multiple cell types (Fig.4B-I).

LC3-II could associate with the surface, not the lumen of exosomes following the fusion of autophagosomes with endosomes as the internal membrane of the autophagosome starts to degrade. LC3-II was sensitive to Protease K-digestion only after treatment of exosome preparations with detergent (Fig.4J), which suggests that LC3-II is enclosed inside exosomes. Reinforcing this, whereas exosomes from wild-type cells are enriched in lipid-modified LC3-II, Atg7^{-/-} MEF that are incapable of canonical macroautophagy produce exosomes enriched in LC3-I (Fig.4K). This demonstrates that LC3 is recruited into exosomes by a process not requiring canonical macroautophagy or lipid modification of LC3.

Atg5 is abundant in exosomes (Fig.4B) and associates with LC3 suggesting Atg5 may recruit LC3 into exosomes. Accordingly, western blot of equal amounts of exosomes from wild-type and Atg5^{-/-} cells demonstrated that LC3 was nearly undetectable in exosomes from Atg5^{-/-} cells (Fig.4M). Similar results were found in exosomes from cells lacking Atg16L1 (Fig.4L). In Atg7^{-/-} cells LC3-I was recruited into exosomes at similar levels to LC3-II in wild-type cells (Fig.4K). This reinforces that exosome production and LC3 sorting into exosomes is independent of Atg7 and the formation of LC3-II. Cells genetically deleted of both Atg5 and Atg7 exhibited reduced exosome production (Fig.4N,O, Fig. 11E) and nearly eliminated the packaging of LC3-I into exosomes that was observed in Atg7^{-/-} cells (Fig.4P). Together, this suggests that Atg5 and Atg16L1 are required and/or key for recruitment of either LC3-I or LC3-II into exosomes, while Atg7 and canonical macroautophagy are not required.

### Atg5 Recruits ATP6V1E1 into exosomes to control exosome production

Atg5 detaches ATP6V1E1 from the V₁V₀-ATPase. It was tested whether LC3 could mediate this effect. Proximity ligation assays detected an association between ATP6V1E1 and LC3 that was lost when ATP6V1E1 was depleted with siRNA (Fig.5A). This suggests ATP6V1E1 is in the immediate vicinity of LC3 (Fig.5A). To test whether LC3 binds ATP6V1E1, ATP6V1E1 was immunoprecipitated (Fig.5B). LC3-I and LC3-II were pulled down in immunoprecipitates of ATP6V1E1 compared to immunoprecipitates of a non-specific isotype control antibody (Fig.5B). Specificity of the ATP6V1E1 band on western blots was confirmed with siRNA targeting ATP6V1E1 (Fig.4D). Finally, recombinant LC3-I coated on beads pulled down ATP6V1E1 from cell lysates, as well as its known binding partner p62, compared to control beads alone (Fig.5C,D). Together, this strongly suggests that LC3 and ATP6V1E1 associate.

LC3 binds many of its interactors using LIR (LC3-Interacting-Region) motifs. LIR motif prediction algorithms (Kalvari et al., 2014) did not detect a LIR motif in ATP6V1E1. Independently searching, we identified only one site that bore some resemblance to a canonical LIR motif (Fig.5E). Peptides of 4-18 amino acids including LIR motifs have been used in a wide variety of assays to bind LC3 and analyze LIR motifs (Johansen et al., 2017; Lee et al., 2017). Peptides including the canonical p62 LIR motif, the putative ATP6V1E1 LIR motif, or a mutated version of the ATP6V1E1 peptide were synthesized (Fig.5E). Peptides from the established p62 LIR motif (a positive control) pulled down recombinant LC3, whereas LC3 bound at background levels to control mutated peptide and the putative ATP6V1E1 LIR motif peptide (Fig.5F). Recombinant LC3 pulled down p62 and ATP6V1E1 from cell lysates (Fig.5C,D). Peptide containing the p62 LIR motif competed with endogenous p62 binding demonstrating that the assay measures binding of LC3 to LIR-motifs (Fig.5F). Nonetheless, p62 LIR motif peptide had no effect on pulldown of ATP6V1E1 (Fig.5F) suggesting that LC3 does not use its LIR-binding region to associate with ATP6V1E1. Compared to mutated control peptide, the peptide containing the putative ATP6V1E1 LIR motif did not affect binding of LC3 to either p62 or ATP6V1E1 (Fig.5F). Together, this suggests that ATP6V1E1 does not have a canonical LIR motif, and ATP6V1E1 associates with a site in LC3 that is independent of the LIR motif binding site.

In cells genetically deleted of Atg5^{-/-} no evident change in levels of ATP6V1E1 or other components of the V₁V₀-ATPase including V1A, VOC or VOD were apparent (Fig.5G-H, Fig.11F). However, in parallel with the decrease in LC3 levels in exosomes produced by Atg5^{-/-} cells, we observed a decrease in ATP6V1E1 levels in exosomes (Fig.5I, Fig.11F). Another component of the V₁ complex, ATP6V1A, could be detected at low levels in exosomes but its levels in exosomes were unchanged in exosomes produced by cells lacking Atg5 (Fig.5I, Fig. 11F). This suggests that ATP6V1E1 is selectively recruited into exosomes in an Atg5 dependent manner.

Atg5 detaches ATP6V1E1 from the V₁V₀-ATPase complex (Fig.3D-H), removes ATP6V1E1 into exosomes (Fig.5G-I, 11F), and causes decreased MVB acidification (Fig.2). If this is critical for the effect of Atg5 on exosome release then depleting ATP6V1E1 with siRNA should rescue exosome release in Atg5^{-/-} cells. Accordingly, while exosome release was decreased in Atg5^{-/-} cells, cells released more exosome size particles and exosome markers accumulated in exosome preparations when Atg5^{-/-} cells were treated with siRNA targeting ATP6V1E1 (Fig.5J,K). If V1V0 -ATPase complex inactivation is the critical effect of detaching ATP6V1E1 from the V₁V₀-ATPase complex then exosome release should also be restored in Atg5^{-/-} cells by inhibiting the V₁V₀-ATPase by other means. In agreement, treating cells with siRNA targeting an independent component of the V₁V₀-ATPase complex, ATP6V1A rescued production of exosome-sized particles in Atg5^{-/-} cells (Fig.5J). Similarly, chemically perturbing the V₁V₀-ATPase complex with Concanamycin rescued exosome production in Atg5^{-/-} cells (Fig.5L,M). This strongly suggests that disrupting V₁V₀-ATPase complex is key and/or required to restore exosome release in Atg5^{-/-} cells. V₁V₀-ATPase is postulated to also be involved in fusion of organelles with lysosomes or for exocytosis, independent of its established role in endolysosomal acidification (Wang and Hiesinger, 2013). Chloroquine and NH₄Cl both increase endolysosomal pH independently of the V₁V₀-ATPase, and both rescued exosome production in Atg5^{-/-} cells (Fig.5N,O). This suggests that the major effect of Atg5 on the V₁V₀-ATPase complex which controls exosome production is its increase of endolysosomal pH. Cumulatively, this suggests that Atg5 removes ATP6V1E1 into exosomes and away from the V₁V₀-ATPase complex thereby decreasing MVB acidification and allowing exosome release.

### Atg5 affects migration and metastasis of cancer cells by controlling exosome release

Atg5 and autophagy have been attributed many roles in cancer. One emerging role for Atg5 is in controlling cancer metastasis. Western blotting confirmed the presence of several proteins linked to cancer cell invasion in exosomes including Vimentin, Ras, and β-catenin at relative enrichment (vs. cell lysate) comparable to exosome markers like Tsg101 (Fig.6A). Consequently, it was hypothesized that Atg5 may affect migration and invasion of cancer cells by controlling exosome release. To test this in a tractable model, exosomes purified from 10⁵ cells of the highly invasive breast cancer cell line MDA-MB-231 were incubated with an equivalent number of MCF-7 cells, a minimally invasive breast cancer cell line. Exosomes from MDA-MB-231 cells promoted migration of MCF-7 cells in transwell and wound healing assays while exosomes from an equivalent number of ATG5^{-/-} MDA-MB-231 cells had no effect (Fig.6B,C, Fig.12A,B). Exosomes from MDA-MB-231 cells also promoted invasion of MCF-7 cells through a matrigel-coated transwell chamber while exosomes from similar numbers of ATG5 MDA-MB-231 cells had no effect (Fig.6D, Fig.12C). These effects were due to decreased numbers of exosomes released by ATG5^{-/-} MDA-MB-231 cells and not a change in their contents, as supplementing with equivalent amounts of exosomes from wild-type or ATG5^{-/-} MDA-MB-231 resulted in similar effects on migration and invasion of MCF-7 cells (Fig.6E).

To investigate the effect of Atg5 on exosome-mediated spread of breast cancer in an orthotopic model, 4T1 breast cancer cells were used. As with other cell lines tested, Atg5^{-/-} 4T1 cells (Fig.11G) released fewer exosomes than wild-type cells (Fig.6F,G). Interestingly, migration and invasion of Atg5^{-/-} 4T1 was increased, as measured by transwell assays, compared to its wild-type counterpart (Fig.6H,I). Replacing exosomes lacking in the media of Atg5^{-/-} cells with wild-type exosomes further increased migration of 4T1 cells (Fig.6H). Cumulatively, results in MDA-MB-231 and 4T1 breast cancer cell lines suggest that loss of exosome production in Atg5 knockout cells decreases migration of these cells.

To evaluate the *in vivo* consequences on metastasis of tumors the orthotopic 4T1 model of metastasis was adopted. People handling and injecting mice, measuring primary tumors and analyzing metastasis in tissues were blinded to treatment groups. 4T1 cells were washed in PBS and implanted in the mammary pad. Wild-type, and Atg5^{-/-} 4T1 cells were compared to Atg5^{-/-} cells whose lack of exosomes was supplemented by pre-treating with wild-type exosomes before implantation. Primary tumors formed after two weeks by wild-type and Atg5^{-/-} 4T1 cells were equivalent in volume and mass, whereas the volume and mass of tumors from Atg5^{-/-} cells supplemented with wild-type exosomes was significantly reduced (Fig.6K,L), despite the unchanged proliferation of these cells in vitro (Fig.6J). Mice were sacrificed 3 weeks later. In this version of the 4T1 model previous evidence demonstrated that no metastatic nodules are observed in lung or other organs when primary tumors are removed at 2 weeks as performed here (Tao et al., 2008). Accordingly, no metastatic nodules were observed in sectioned lower left lung lobes of mice implanted with wild-type or Atg5^{-/-} 4T1 cells. However, replacing the exosomes of Atg5^{-/-} cells caused the appearance of metastatic nodules, which were confirmed by a clinical pathologist in 4 of 10 mice (Fig.6M, N, p>0.01 Wald's modified confidence interval). This suggests Atg5 is required and/or key for effective exosome production that enhances migration of breast cancer cells from the site of the primary tumor to establish metastasis.

### Discussion

Existing literature has suggested contradicting effects of autophagy genes on exosome production. These papers frequently examined single genes, relied on western blots to quantify exosomes, and examined extracellular vesicles with unique cargoes like hepatitis C virus in single cell types. Here we systematically evaluated the effects of multiple Atg genes often in more than one cell type and with more rigorous quantification of exosome quantity by nanoparticle tracking, dynamic light scattering and western blot for canonical exosome markers. This evidence suggests that Atg5, independent of Atg7 and canonical macroautophagy, is critical and/or key for regulating exosome production. Effects of Atg6/Beclinl on exosome production that were previously observed (Shrivastava et al., 2016) may be independent of autophagy. Beclin1 forms a complex with class III phosphinositide 3-kinase (PI3K) and dictates the allocation of class III PI3K between Atg14L1 complexes involved in phagophore formation for autophagy and complexes involved in budding of vesicles into MVB (McKnight et al., 2014). Therefore, Beclinl is likely to affect exosome production by altering the amount of class III PI3K participating in budding of vesicles into MVB (Futter et al., 2001) independent of autophagy and the mechanisms described here. As well, the increased release of exosomes previously observed when cells are treated with Bafilomycin and other inhibitors of the V₁V₀-ATPase complex (Alvarez-Erviti et al., 2011; Parolini et al., 2009) are likely independent of canonical autophagy as these inhibitors also increased exosome production in Atg5^{-/-} cells (Fig.5). While results demonstrate that exosome production is independent of canonical Atg7-dependent autophagy, autophagosome-like structures and degradation have been observed in cells lacking Atg7 (Chang et al., 2013; Ktistakis and Tooze, 2016; Nishida et al., 2009; Ra et al., 2016). Therefore, it is not excluded that one of the complex emerging forms of Atg7-independent non-canonical autophagy may participate in exosome production. However, in terms of membrane topology there is no straightforward model for how a large double-membraned autophagosome participates in generating ~100 nm intraluminal vesicles within endosomes. In contrast, Atg5, Atg16L1 and LC3 have been observed to localize to endosomes, phagosomes and lysosome-like granules that are related to MVB independent of double-membrane autophagosomes (Munz, 2016). Without wishing to be bound by theory, knowledge of exosome biogenesis and the present data are consistent with a model in which Atg5, Atg16L1 and LC3 are found on the cytoplasmic surface of MVB and are packaged into intraluminal vesicles budding into MVB.

Atg5 is abundant in exosomes (Fig.4B) and is required and/or key for LC3 sorting into exosomes. LC3-I rather than LC3-II is sorted into exosomes in Atg7^{-/-} cells, demonstrating that LC3 sorting into exosomes occurs without Atg7 and canonical autophagy (Fig.4K,N-P) and LC3-II is not required for exosome production. This suggests that Atg5 physically brings LC3 into exosomes. LC3 associates with the V₁V₀-ATPase component ATP6V1E1 according to PLA assays (Fig.5A), immunoprecipitations (Fig.5B) and pulldowns with recombinant LC3 (Fig.5C). The present data suggests that as LC3 is packaged into exosomes it takes ATP6V1E1 with it, thereby inactivating the V₁V₀-ATPase at MVB and causing MVB pH to increase in an Atg5-dependent manner. Several lines of evidence suggest that functions of the V₁V₀-ATPase in acidification and fusion of organelles can be dissected (Wang and Hiesinger, 2013). Exosome production in Atg5^{-/-} cells was rescued by increasing endolysosomal pH for only 30 min with NH₄Cl to minimize secondary effects of neutralizing endolysosomal pH (Fig.5N-O). This suggests that the effect of the V₁V₀-ATPase on Atg5-dependent exosome production is due to effects on MVB acidification, but a direct role of the V₁V₀-ATPase in membrane fusion in this process is not formally excluded.

Atg5 decreased acidification of MVB (Fig.2). In contrast, Atg5 has been observed in other systems to increase acidification of LC3-associated compartments including autophagolysosomes, LC3-associated phagosomes and endosomes (Peng et al., 2014; Romao et al., 2013; Sanjuan et al., 2007; Zhou et al., 2013). Consistent with this literature it is found that Atg5 promoted acidification or lysosomal fusion of compartments destined for degradation (EGF, dextran, Fig.10A-E), while limiting acidification of MVB containing exosome markers (Alix, NRhPE, Fig.2D-J, Fig.9I-L). This suggests Atg5 has contrasting effects on discrete populations of endosomes and MVB with distinct fates (lysosomal fusion, Golgi-trafficking, plasma membrane fusion). One model consistent with the evidence is that on MVB, LC3 may remove ATP6V1E1 into intraluminal vesicles/exosomes and decrease MVB acidification whereas when LC3 remains on the surface of the endosome, phagosome or autophagosome it may recruit ATP6V1E1 and anchor or stabilize the V₁V₀-ATPase to promote acidification of these organelles.

Exosomes have been demonstrated to promote invasion of cancer cells (Kosaka et al., 2016). Recent studies have highlighted a role for exosomes in trafficking to future sites of metastasis and establishing a metastatic niche for cancer cells to spread into (Hoshino et al., 2015). The present Example demonstrates that exosomes also promote metastasis of cancer cells by acting directly on cancer cells to promote their migration from the site of the primary tumor. The present results demonstrate that one mechanism by which Atg5 controls cancer cell migration and metastasis (Kenific et al., 2016; Sharifi et al., 2016) is to control release of exosomes. This effect of Atg5 on exosome release may contribute to the effects of autophagy on cancer cell invasion previously observed (Kenific et al., 2016; Sharifi et al., 2016). An increasing number of changes in growth factor and metabolic cues are being discovered to directly impact the Atg5-Atg16L1 complex by direct phosphorylation for example (Russell et al., 2014). The present results thus suggest that control of exosome release by Atg5-Atg16L1 may contribute to the complex effects of autophagy genes, autophagy activators or inhibitors, lysosome-targeting drugs, and tumor microenvironment on cancer metastasis, for example.

### Materials and Methods

### Experimental Model and Subject Details

### Cell Lines

Mouse embryonic fibroblasts (MEF) lacking a copy of Atg5 (Kuma et al., 2004), and wild-type and ATG16L1^{-/-} HCT-116 were a gift of D. Philpott (University of Toronto, Canada). Atg7^{-/-} and Atgl4^{-/-} cells were a gift of Herbert Virgin (University of Washington). Doxycycline-regulated Atg5 MEF generated previously were also used (Hosokawa et al., 2007).

MDA-MB-231 (HTB-26, ATCC) cells were genetically deleted of ATG5 using Compozr Zinc Fingers technology (product number CKOZFND1217-1KT, Sigma-Aldrich). Mutation of ATG5 was confirmed using Surveyor Mutation Detection Assay (706020, Transgenomic), PCR (ZFN primer F sequence was CAAAATGGGCCAAATAGCTAAA (SEQ ID NO: 12) and ZFN primer R sequence was ACTTACCATTTTGCAATCCCA (SEQ ID NO: 13)) and western blot. All cells, including Neuro2A (CCL-131, ATCC) and 3T3 (CRL-1658, ATCC), were cultured in Dulbecco's Modified Eagle Medium (DMEM) containing 10% fetal bovine serum and penicillin-streptomycin. To generate 4T1 Atg5^{-/-} cells Crispr Cas9 guide RNAs (gRNA) for murine ATG5 were generated using the online CRISPR design tool targeting exon 1 (http://crispr.mit.edu/). Atg5 knock-out 4T1 cell line for injection into mice was generated using the following gRNA: Top: caccGAAGATGTGCTTCGAGATGTGTGG (SEQ ID NO: 14), bottom: aaacCCACACATCTCGAAGCACATCTTC (SEQ ID NO: 15). To generate Atg7 & Atg5 double knockout MEF cells, Atg7 was knocked out using the following gRNA: Top: caccGAAGTTGAACGAGTACCGCC (SEQ ID NO: 16), Bottom: aaacGGCGGTACTCGTTCAACTTC (SEQ ID NO: 17) on Atg5^{-/-} MEFs. The knockout of each protein was confirmed using western blot. Identity of cell lines was not authenticated by the authors.

### Mice

Eight week old female Balb/C mice (Charles River) were randomized into 3 groups (10 mice per group) to receive 3×10⁵ 4T1 cells in 50 µl PBS injected in the lower mammary pad. Mice were housed at 4 per cage and fed with standard food and water *ad libitum.* All experiments involving mice were performed in accordance with relevant institutional and national guidelines and regulations.

### METHOD DETAILS

### Exosome Enrichment

Exosomes were enriched by differential centrifugation as previously described (Thery et al., 2006). Briefly, cells were cultured in media containing 10% FBS previously depleted of exosomes by centrifugation at 100 000 g for 16 h (Thery et al., 2006). To purify exosomes, supernatant from cell cultures was centrifuged at 300 g (10 min), 2000 g (10 min) and 10 000 g (30 min, SW32 rotor). At each step the supernatant was recovered. After centrifugation at 100 000 g (1 h 10 min, SW32 rotor) the supernatant was removed and the pellet was resuspended. The pellet was washed in PBS by a final centrifugation at 100 000 g (20 min, TLA100.3 rotor). The exosome-enriched pellet was re-suspended in PBS for further analyses.

For density separation, exosomes were purified as described (Laulagnier, 2015). Briefly, Neuro2A cell culture media was collected, and a cocktail of protease inhibitors added (Roche). Media were cleared of debris by two successive centrifugation steps (2 000 g for 10 min, 20 000 g for 20 min) and filtration through a 0.22 µm filter (Millex GV PVDF, Millipore). The medium was centrifuged for 2 h at 100 000 g (2 h, SW32 rotor). The resulting pellet was resuspended in 0.211 M sucrose, 3 mM imidazole pH 7.4, and loaded onto a continuous sucrose gradient (0.3-1.4 M). Gradient was centrifuged at 200 000 g (18 h, SW41Ti rotor), and 10 fractions (1 ml) were collected). The sucrose density of each fraction was determined by refractometry. Fractions were washed in 3 mM imidazole, pH 7.4, and centrifuged at 200,000 g (2 h, SW41Ti rotor). Exosome pellets were resuspended in Laemmli buffer and used for Western blot analysis.

### Dynamic Light Scattering

Preparations of enriched exosomes were analyzed by dynamic light scattering on a Protein Solutions Dynapro Instrument using Dynamics V6 software. Data were acquired every 10 seconds at 4°C and 10% laser power for at least 200 seconds per sample. The intensity (Cnt/s) and size (nm) were generated automatically by the instrument.

### Nanoparticle Tracking Analysis

Preparations of enriched exosomes were analyzed using either a Nanosight (Fig.1A,C) or a ParticleMetrix Zetaview Nanoparticle tracking instrument (all other graphs). Post-acquisition parameters on the Nanosight were: were Min Bright: 15, Min size: 10 nm, Max size: 200nm.

The ZetaView Nanoparticle Tracking instrument (ParticleMetrix) is calibrated for experiments following every instrument start-up. Focusing and alignment are performed automatically using 102nm polystyrene beads (Microtrac 900383). Exosome samples were diluted in PBS into the acceptable range for measurement (typically 1:1000 - 1:100,000). One mL of sample was injected into the machine and allowed to slow down according to the built in particle drift sensor. If the sample falls within the acceptable reading concentration range, video acquisition and analysis was performed using the following parameters. Acquisition Parameters: Sensitivity (85), Shutter speed (40), Frame rate per second (30), Resolution (Highest), Positions measured (11). Post-acquisition parameters: Minimum brightness (15), Minimium size in pixels (10), Maximum size in pixels (500).

The analysis reports containing the particle size (nm) and concentration (particles/ml) were generated automatically. The particles between 45 nm and 105 nm were summed to quantify exosome-sized particles. In experiments in Fig.5N,O measuring exosome production after treating cells with NH₄Cl or Chloroquine for short time periods, background readings from exosome-depleted media alone were subtracted. Only in these experiments was the background reading high enough to impact the particle counts measured.

### Exosome Uptake Assay

Exosomes purified using the procedure described above were re-suspended in 20 µl of PBS. Vybrant DIO Cell-Labeling Solution (V22886, Invitrogen) was used and the manufacturer's protocol was followed. Briefly, 5 µl of the cell-labeling solution was added to the exosome preparation and incubated for 20 min at 37°C. Excess label was removed by centrifugation (100,000g, 20 min, TLA 100.3 rotor) and the pellet was re- suspended in 500 µl of DMEM. Equal amounts of DIO-labeled exosomes were added to cells in triplicate and DMEM alone was added to control wells. Cells were incubated with DIO-labeled exosomes for 1 h. Supernatant was retained to measure uptake of DIO-labeled exosomes by disappearance of fluorescence (Synergy Hi Hybrid Multi-Mode Microplate Reader, BioTek). To measure internalization of exosomes into cells, these were re-suspended in 500 µl of PBS for analysis on a flow cytometer (Beckman Coulter Cyan ADP 9).

### Endosomal Labeling and Trafficking Assays

To label MVB, cells were incubated with 3µM NRhPE (Avanti Polar Lipids) at 4°C for 15 min then transferred to 37°C, 5% CO₂ for the indicated incubation times, as previously described (Vidal et al., 1997). pHrodo Green Dextran (10 kDa, ThermoFisher) was incubated with cells for 10 minutes and cells were transferred to ice for immediate analysis by flow cytometry. After initial readings 20 mM NH₄Cl was added to cells to neutralize the pH-dependent increase in pHrodo fluorescence to measure dextran internalization and acidification of dextran-containing compartments (geometric mean fluorescence intensity before/after NH₄Cl). To image EGFR trafficking cells were incubated with EGF-Alexa488 (100 ng/mL, ThermoFisher) in serum-free DMEM and imaged at 30 min where its localization in late endosomes and lysosomes is maximal (Vanlandingham and Ceresa, 2009).

### Immunofluorescence Microscopy

Cells were prepared for microscopy as previously described (Gibbings, 2012). Cells cultured on coverslips (Deckglaser 18 mm Ø) were fixed in 2% paraformaldehyde in PBS (10 min) 48 h post transfection where cells were transfected. Cells were then rinsed twice in PBS and permeabilized with 0.2% Triton-X-100 in PBS containing 20 mM NH₄Cl (10 min). After washing with PBS, cells were blocked with 5% milk in PBS (1 hour), washed three times in PBS and incubated with 2-5 µg/ml primary antibody in PBS at 4°C overnight. Following primary incubation, cells were washed three times (5 min) in PBS and incubated for 1 hour at room temperature with 1/250 dilution of highly cross-adsorbed secondary antibodies (goat anti-mouse Alexa Fluor 488, 546 or 633, goat anti-rabbit Alexa Flour 488 or 546, goat anti-rat Alexa Fluor 546, donkey anti-goat Alexa Fluor 488 [ThermoFisher], or goat anti-rabbit DyLight 405 [Thermo Scientific]). After washing three times (5 min), cells were mounted with Vectashield Mounting Media. For live cell imaging, cells were plated on Cellview culture dishes with 2.2 cm² glass bottom.

Epifluorescence microscopy was performed using a Zeiss AxioObserver.D1 or Zeiss AxioObserver.Z1 microscope. Images were acquired with a 63x Plan-Apochromat 1.4 Oil DIC objective and AxioCam MRm CCD (monochrome), using Zeiss AxioVision Rel. 4.8 software. Confocal microscopy was performed using a Zeiss LSM 510 Meta/AxioVert 200 microscope. Images were acquired with a 63x Plan-Apochromat 1.4 Oil DIC objective, 2 Photo Multiplier Tubes and 1 Meta detector, using Zeiss ZEN software. Confocal images for preparing 3D models were acquired in 0.5-1 µm Z-stacks.

### Stimulated Emission-Depletion (STED) Microscopy

Subconfluent cells were cultured on glass coverslips (Deckglaser 18 mm Ø). Forty-eight h after transfection, cells were fixed with 4% paraformaldehyde in 10 mM PBS (10 mM phosphate buffer - pH 7.2, 154 mM NaCl). After cells were washed with PBS at room temperature, then incubated overnight at 4°C with primary antibody prepared in Ab Buffer (10 mM PBS, 3% BSA, 0.3% Triton X-100). After washing, the cells were incubated for 1 hour at room temperature with STED CW-compatible secondary as well as tertiary antibodies prepared in Ab buffer. Cells were mounted with Prolong Gold ^{™} Antifade (P36930, Life Technology) mounting medium and cured for 24 h at room temperature before imaging. Secondary antibodies used include: ChromeoTM 505 anti-mouse IgG (15050, Active Motif, Concentration 1:500), Biotin-anti-rabbit IgG (B8895, Sigma, Concentration 1:800), Streptavidin V500 (561419, BD Horizon, Concentration 1:200).

Continuous wave stimulated emission depletion (STED CW) images were acquired using a Leica SP5/STED CW microscope, and a 100x oil immersion objective (HCX PL APO CS 100x/1.40 OIL STED, Leica Germany) essentially as described (Zheng et al., 2011). Dual colour excitation of fluorophores was achieved using an argon-ion laser exciting at 458 nm and 514 nm respectively. STED depletion was achieved with a 592 nm CW laser line. Fluorescence outputs were captured in sequential scans using base parameters of 600 Hz frequency at a 2.54x zoom giving a resolution of 29.8 nm × 29.8 nm per pixel. The first scan at 514 nm excitation wavelength (ChromeoTM 505) was performed at 50% laser power, 750 gain, -0.3 offset, with frame averaging and accumulation of 3, and a detection window of 520 - 580 nm. The second scan at 458 nm excitation wavelength (streptavidin V500) was performed at 85% (LC3) or 55% (Rab5) laser power, 800 gain, -1.0 offset, with frame averaging and accumulation of 3, and a detection window of 465 - 485 nm. STED 592 depletion laser power was 100%. Images were processed using Leica STED deconvolution software (LAS AF v2.6.3.8173). First a point spread function (PSF) was generated using a Lorentz transformation of 70 nm. Then the image was deconvolved using the generated PSF and signal energy set to regularization parameter 0.05. Images were further processed using the baseline mean function, and background was reduced by 1500 (in the 16-bit image).

### Image Analysis

Images were analyzed using ImageJ software. Three-dimensional image renderings were made using ImageJ. Raw microscope image channels were thresholded and merged using the imbedded "Threshold > Triangle" and "Merge Channels" options, respectively. The composite image was rendered as a 3D image with the "Show Color Surfaces" plugin with the radius of smoothing set to 0.20 pixels.

To detect and quantify the colocalization of fluorescently labelled subcellular structures, an object-based colocalization method was employed named 'SQUASSH' (Segmentation and Quantification of Subcellular SHapes) (Rizk et al., 2014). The ImageJ plugin - MosaicSuite was downloaded and run on each of the images. Background fluorescence was eliminated with a rolling ball window size of 10 pixels. For segmentation, PSF model for the wide-field microscope used was estimated to be 0.84 (xy) and 0.79 (z). Regularization parameter and minimum object intensity of channel 1 and channel 2 were set at 0.15, 0.075 and 0.15 respectively. A Gaussian noise model was used. Cell masks were set at 0.25 and 0.2 for channel 1 and channel 2 respectively. Another ImageJ plugin Just Another Co-localization Plugin (JACoP) was used to obtain Li's intensity correlation co-efficient of co-localization (Li et al., 2004). In quantified experiments at least 3 slides were imaged per condition, with 5-7 images per slide and approximately 10 cells/image to provide data from about 250-350 cells.

### Electron Microscopy

Cultures of MEF cells were fixed *in situ* with 0.1 M cacodylate buffer containing 2% glutaraldehyde until processing for embedding. Cells were post-fixed in 1% osmium tetroxide (EMS, PA, USA) in cacodylate buffer at 4°C. After washing in buffer, MEF cells were dehydrated in graded ethanol, infiltrated and embedded in Epon 812 (MECALAB, Quebec, Canada), as described (Luft, 1961). Ultrathin sections were obtained using a Reichert Ultracut S ultramicrotome, and mounted on formvar-carbon coated nickel grids (MECALAB, Quebec, Canada). Sections were stained with uranyl acetate and examination was performed with a Philips CM 100 electron microscope. To analyse MVB, we excluded organelles containing a signature double-membrane of autophagosomes or organelles that were electron dense like lysosomes or contained heterogenous luminal contents that are characteristic of lysosomes and autophagolysosomes (Huotari and Helenius, 2011). MVB frequently contained dense surface patches associated with vesicle budding (Murk et al., 2003), and MVB exclusively containing vesicles of 30-120 nm were analysed (Huotari and Helenius, 2011).

LC3 was detected in exosome preparations by electron microscopy as previously described (Chivet et al., 2014). Briefly, Neuro2a cell culture media was collected, and a cocktail of protease inhibitors added (Roche). Media were cleared of debris by two successive centrifugation steps (2 000 g for 10 min, 20 000 g for 20 min) and filtration through a 0.22 µm filter (Millex GV PVDF, Millipore). The medium was centrifuged for 100 000 g (2 h, SW32 rotor). The resulting pellet was washed in PBS and resuspended in 40 µl 2 % paraformaldehyde in PBS. Four µL drops were spotted onto Formvar-carbon-coated grids for 20 min, treated with PBS-glycine (50 mM), blocked with PBS-BSA (10 mg/ml) then permeabilised with 0.05% saponin in PBS-BSA before consecutive incubation with rabbit anti-LC3A/B (Cell signalling, 1 h), followed by goat anti-rabbit 1.4 nm gold conjugated-F(ab') (Nanoprobes, 45 min). The grids were then fixed 1 min with 1 % glutaraldehyde in PBS and finally treated with the HQ silver intensification kit (Nanoprobes) for 6 min before negative staining. Grids were observed with a Jeol JEM 1200EX transmission electron microscope.

### Recombinant LC3 Pull-Down

In brief, 0.5 µg of His-tagged recombinant LC3 (His-tag, Cat# ADI-APR-100-0200, Enzo Life Sciences) was rotated in 50 µl with 5 µl of His-tagged Dynabeads (Cat# 10103D, Thermofisher) in a binding/wash buffer (50 mM Sodium Phosphate, pH 8.0, 150 mM NaCl, 0.01% Tween^{®}-20, 0.5% Triton X-100) at room temperature (RT) for 10 min. Then 150 µl of binding/wash buffer containing 5% BSA was added to the tubes and rotated for 5 minutes. Tubes were placed on a magnet and supernatant discarded. Cell lysate (100ul, 150ug) collected in Pull-down Buffer (3.25 mM Sodium phosphate pH 7.4, 70 mM NaCl, 0.01% Tween^{®}-20, 0.5% Triton X-100) was incubated on a roller for 10 min. Supernatant was discarded and beads were washed 4 times with 300 µl Binding/Wash Buffer. His-LC3 was eluted with 150 mM Imidazole, 50 mM Sodium phosphate pH 8.0, 300 mM NaCl, 0.01% Tween^{®}-20 with gentle shaking for 5 min. Supernatants were separated from beads on a magnet. The median intensities of bands for p62, ATP6V1E1 and background were quantified over 3 independent experiments using ImageQuantTL (GE Healthcare).

Experiments with competitive peptides were performed as above, except 50 µmoles of peptide was added during incubation with cell lysate. The following peptides were synthesized by Genscript with an N-terminal biotin: p62 LIR: SGGDDDWTHLSS (SEQ ID NO: 18), ATP6V1E1 putative LIR: RCRKQDFPLVKA (SEQ ID NO: 19), and ATP6V1E1 mutated putative LIR: RCRKAILPLQKA (SEQ ID NO: 20).

### LC3-Interacting Region Peptide Pulldown Assay

The following peptides were synthesized by Genscript with an N-terminal biotin: p62 LIR: SGGDDDWTHLSS (SEQ ID NO: 18), ATP6V1E1 putative LIR: RCRKQDFPLVKA (SEQ ID NO: 19), and ATP6V1E1 mutated putative LIR: RCRKAILPLQKA (SEQ ID NO: 20). Fifteen µl of Dynabeads^{®} MyOne ^{™} Streptavidin T1 (Cat#65601, ThermoFisher) were washed with PBS, then 10 nmoles of biotinylated peptides and 100 ng of recombinant LC3 were added. The mixture was rotated at 4 C over night. The beads were then washed with PBS 3 times and collected for further western analysis.

### Scratch wound assay

MCF-7 cells were grown to confluence in 6 well plates containing DMEM with FBS. Wells were marked with a reference line through their center. Once cells reached confluence they were scratch wounded using a 200 µl pipette tip (2 wounds/well perpendicular to the reference line), washed with PBS, and supplied with fresh media. Exosomes isolated from equivalent amounts of wild-type MDA-MB-231, Atg5^{-/-} MDA-MB-231, or MCF-7 cells in 6 well plates were added to the media of scratch-wounded MCF-7 cells. An image on either side of the reference line at each scratch wound was taken at time 0 and 20 h. In each image, the distance of the wound was measured at 6 points including the largest and smallest distances.

### Motility and invasion assay

Motility chambers (BD Bioscience, Cat#354578) and invasion chambers (BD Bioscience, Cat#354480) were rehydrated and equilibrated for 2 h with 500 µl of serum free DMEM media. After 2 h, the medium in the inserts was aspirated and inserts were placed into wells containing DMEM with 10% FBS. MCF-7 cells (50 × 10³) were added to each chamber in serum-free media. Exosomes isolated from equivalent amounts of wild-type MDA-MB-231, ATG5^{-/-} MDA-MB-231, or MCF-7 cells in 6 well plates were added to the media of MCF-7 cells. The chambers were incubated for 24 h with exosomes. The media was then removed and the upper surface of the membrane was scrubbed 20 times with a cotton swab. Cells on the lower surface of the scrubbed membranes were fixed and stained with Diff-Kwik kit (Thermo Fisher Cat# 9990700) according to the manufacturer's instruction.

### Proximity Ligation Assays

Proximity ligation assays (PLA) were performed according to the manufacturer's protocol using the following reagents Duolink^{®} In Situ Detection Reagents Green (Cat# DU092014-100RXN, Sigma); Duolink^{®} In Situ Detection Reagents Orange (Cat# DU092007-100RXN, Sigma); Duolink^{®} In Situ PLA^{®} Probe Anti-Rabbit PLUS (Cat# DU092002, Sigma); Duolink^{®} In Situ PLA^{®} Probe Anti-Mouse MINUS (Cat# DU092004, Sigma). In brief, cells were plated either on cover slips or glass slides at a concentration of 10⁵/ml. Cells were fixed using 4% formaldehyde in PBS, then permeablized using 20 mM NH₄Cl, 0.2% Triton in PBS for 20 minutes at room temperature. Hydrophobic pen was used to enclose a 1 cm² area, then 40 µl of reaction volume was added. All subsequent incubations were performed at 37°C in a humid chamber. Non-specific binding was minimized with the manufacturer's blocking buffer for 30 min. Primary ATP6V1E1 (Host: mouse Cat#H00000529-M02, Abnova) and ATP6VOC Antibody (Cat# PA5-23972, Thermofisher), or ATP6V1E1 (Hostmouse Cat# H00000529-M02, Abnova) and Anti-LC3 antibody (Host: Rabbit, Cat# L7543-200UL, Sigma) were incubated with coverslips for 1 h (1:50, manufacturer's antibody diluent). Coverslips were washed with provided buffer A twice for 5 min, incubated with provided PLA probes for 1 hr and washed with buffer A (twice, 5 min). Coverslips were incubated with ligation mix for 30 mins washed twice with buffer A and incubated with amplification mix. Coverslips were then washed with provided Buffer B twice (10 min), followed by 1 min washing with 0.01x Wash Buffer B. The samples were mounted with Duolink In Situ Mounting Medium with DAPI in dark for 15 min at room temperature. The images were taken using a Zeiss Axio0bserver.Z1 fluorescence microscope at 20X. In experiments including Alix-mCherry cells were fixed for PLA 30 hours post-transfection. Three biological replicates were performed for each condition. The Duolink^{®} ImageTool (Cat# DU090806-1EA, Sigma) was used to analyze images to obtain the number of PLA signals in the cytoplasm. Analyzed images in figures were generated automatically by the software. The number of signals per cell was calculated as Total number of Signals in Cytoplasm / Total number of Cells. The number of signals per cell was then normalized to the control.

### Orthotopic Model of 4T1 Breast Cancer

Equal amounts of 4T1 cells (wild-type and Atg5^{-/-}) were plated at a concentration of 10⁶ per 10 cm dish, and pretreated either with vehicle or 10 µg of exosomes collected from 4T1 wild type cells for 6 days. Media and exosomes were changed every two days. Cells were collected, washed in PBS, counted and re-suspended in PBS. Eight week old female Balb/C mice (Charles River) were randomized into 3 groups (10 mice per group) to receive 3×10⁵ cells in 50 µl PBS injected in the lower mammary pad. Two weeks post-injection, the primary tumors were surgically excised. Primary tumors were weighed and measured using calipers and its volume was calculated using the formula (W × W × L)/2. Lower left lung lobes were collected 23 days after the removal of primary tumors and sectioned (5 µm, 20 sections). Every 5^{th} section was stained using standard H&E staining. Stained sections were scanned using a Quorum Slide Scanner, and images were analyzed using Aperio imagescope (v12.3). All people handling or injecting cells into mice, removing and measuring tumors, staining and quantifying slides were blinded to the treatment groups. All experiments involving mice were performed in accordance with relevant institutional and national guidelines and regulations.

### Quantification and statistical analysis

Two-tailed t-tests or ANOVA were employed to evaluate statistical significance, p≤ 0.05 was considered as significant. SPSS V21.0 was used for statistical analysis. Error bars represent SEM.

### Resources Table:

| REAGENT or RESOURCE | SOURCE | \| IDENTIFIER |
|---|---|---|
| Antibodies | | |
| Alix (immunofluorescence. Host: mouse) | BD Transduction Laboratories | 49/AIP1 |
| Alix (Host: mouse) | Cell Signaling Technology | 2171 |
| Atg5 (Host: rabbit) | Cell Signaling Technology | 8540 |
| Atg7 (Host: rabbit) | Cell Signaling Technology | 8558 |
| Atg12 (Host: rabbit) | Cell Signaling Technology | 4180 |
| Atg3 (Host: rabbit) | Cell Signaling Technology | 3415 |
| Human CD63 (Host: mouse) | Santa Cruz | Sc-5275 |
| Mouse CD63 (Host: mouse) | MBL International | R5G2 |
| LC3A/B (Host: rabbit) | Cell Signaling Technology | 4108 |
| Flotillin2 (Host: rabbit) | Cell Signaling Technology | 3438 |
| Tsg101 (Host: mouse) | Genetex | 4A10 |
| Tomm20 (Host: rabbit) | Santa Cruz | Clone FL-145 |
| Rab5 (Host: rabbit) | Cell Signaling Technology | 3547 |
| ChromeoTM 505 anti-mouse IgG | Active Motif | 15050 |
| Biotin-anti-rabbit IgG | Sigma-Aldrich | B8895 |
| Streptavidin V500 | BD Horizon | 56149 |
| 1.4 nm NanoGold conjugated-F(ab') | Nanoprobes | 2003 |
| ATP6V1E1 (Host: rabbit) | ThermoFisher | PA5-29899 |
| ATP6V1E1 (PLA assay, Host: mouse) | Abnova | Clone 4E11 |
| ATP6V1A (Host: rabbit) | Abeam | Ab137574 |

| | | |
|---|---|---|
| ATP6V0C (Host: rabbit) | ThermoFisher | PA5-23972 |
| ATP6V0D1 (Host: mouse) | Abeam | Ab56441 |
| LC3B (Host: rabbit) | Sigma-Aldrich | L7543 |
| Alix (density gradient, Host: rabbit) | CovoJab | Pab0204 |
| Flotillin1 (density gradient, Host: mouse) | BD Transduction Laboratories | 610820 |
| GFP (Host: mouse) | Abeam | Ab3277 |
| Goat anti-mouse Alexa Fluor 488 | ThermoFisher | A11029 |
| Goat anti-mouse Alexa Fluor 546 | ThermoFisher | A11030 |
| Goat anti-mouse Alexa Fluor 633 | ThermoFisher | A21052 |
| Goat anti-rabbit Alexa Fluor 488 | ThermoFisher | A11034 |
| Goat anti-rabbit Alexa Fluor 546 | ThermoFisher | A11035 |
| Anti-goat Alexa Fluor 488 | ThermoFisher | A11015 |
| Anti-rat Alexa Fluor 546 | ThermoFisher | A11081 |
| Goat anti-rabbit DyLight 405 | ThermoFisher | 35550 |
| | | |

| Chemicals, Peptides, and Recombinant Proteins | | |
|---|---|---|
| p62 LIR: biotin-SGGDDDWTHLSS | Gen script | >75% purity |
| ATP6V1E1 putative LIR: biotin-RCRKQDFPLVKA | Genscript | >75% purity |

| | | |
|---|---|---|
| ATP6V1 E1 mutated putative LIR: biotin-RCRKAILPLQKA | Genscript | >75% purity |
| p62 LIR: SGGDDDWTHLSS | Genscript | >75% purity |
| ATP6V1E1 putative LIR: RCRKQDFPLVKA | Genscript | >75% purity |
| ATPBV1E1 mutated putative LIR: RCRKAILPLQKA | Genscript | >75% purity |
| His-tagged recombinant LC3 | Enzo Life Sciences | ADI-APR-100-0200 |
| 1% Osmium Tetroxide | Electron Microscopy Sciences | 19110 |
| Epon 812 | Mecalab | 3137N |
| Prolong Gold Antifade | ThermoFisher | P36930 |

| Critical Commercial Assays | | |
|---|---|---|
| ATG5 Compozr Zinc Fingers | Sigma-Aldrich | CKQZFND1217-1KT |
| Lysotracker Blue | ThermoFisher | L7526 |
| Lipofectamine 2000 | ThermoFisher | 11668-019 |
| Vybrant DIQ Cell-Labeling Solution | ThermoFisher | V22886 |
| phRodo Green Dextran (10 kDa) | ThermoFisher | P35368 |
| EGF-biotin-Alexa488 | ThermoFisher | E13345 |
| Lissamine Rhodamine B phosphatidylethanolamine 16:0 | Avanti Polar Lipids | 810158 |
| HQ Silver Intensification Kit | Nanoprobes | 2012 |
| Duolink^{®} In Situ Detection Reagents Green | Sigma-Aldrich | DUO92014-100RXN |
| Duolink^{®} In Situ Detection Reagents Orange | Sigma-Aldrich | DUO92007-100RXN |
| Duolink^{®} In Situ PLA^{®} Probe Anti-Rabbit PLUS | Sigma-Aldrich | DUO92002 |
| Duolink^{®} In Situ PLA^{®} Probe Anti-Mouse MINUS | Sigma-Aldrich | DUO92004 |
| | | |

| Experimental Models: Cell Lines | | |
|---|---|---|
| MDA-MB-231 (female, breast cancer cell line) | ATCC | HTB-26 |
| 4T1 (female, breast cancer cell line) | ATCC | CRL-2539 |
| Neuro2a (male, neuroblastoma) | ATCC | CCL-131 |
| 3T3 (male) | ATCC | CR L-1658 |
| Mouse embryonic fibroblasts (sex unavailable) | Dana Philpott (University of Toronto) | |
| Mouse embryonic fibroblasts (*Atg5*^{*-*/*-*}, sex unavailable) | Dana Philpott (University of Toronto) | |
| Wild-type and *ATG16L1*^{*-*/*-*} HCT-116 (male) | Dana Philpott (University of Toronto) | |
| Mouse embryonic fibroblasts (*Atg7*^{-/-}, sex unavailable) | Herbert Virgin (Washington University) | |
| Mouse embryonic fibroblasts (*Atg16L1*^{*-*/*-*} sex unavailable) | Herbert Virgin (Washington University) | (Cadwell et al., 2008) |
| Mouse embryonic fibroblasts (*Atg14*^{*-*/*-*} sex unavailable) | Herbert Virgin (Washington University) | |
| Mouse embryonic fibroblasts (*Atg5*^{*-*/-} with inducible *Atg5*, sex unavailable) | Noboru Mizushima (University of Tokyo), via XiaoHui Zha (University of Ottawa) | (Hosokawa et al., 2006) |
| | | |

| Experimental Models: Organisms/Strains | | |
|---|---|---|
| Balb/c mice (female, 8 weeks) | Charles River | Strain 028 |
| | | |

| Oligonucleotides | | |
|---|---|---|
| ATP6V1E1 Silencer Select SiRNA | ThermoFisher | ID: s1803 |
| Sense: CCCAAAGACUAAAGAUUAUtt | | |
| Anti-Sense: AUAAUCUUUAGUCUUUGGGtt | | |
| ATP6V1A Silencer Select SiRNA | ThermoFisher | ID: s1793 |
| Sense: CAUGGUCCAUUAUUCGUGAtt | | |
| Anti-Sense: UCACGAAUAAUGGACCAUGtg; | | |

| | | |
|---|---|---|
| ATP6V1 E1 Silencer Select SiRNA | ThermoFisher | ID: s62774 |
| Sense: GCAAUCCCUAUGUAUAAAtt | | |
| Anti-Sense: UUUUAUACAUAGGGAUUGCtt | | |
| ATG7 Silencer Select SiRNA | ThermoFisher | ID: s20650 |
| Sense:GGAACACUGUAUAACACCAtt | | |
| Anti-Sense:UGGUGUUAUACAGUGUUCCaa | | |
| Silencer Select Negative Control siRNA 1 | ThermoFisher | 4390843 |
| Atg5 Crispr Guide RNA | IDT | custom |
| Top: GaccGAAGATGTGCTTCGAGATGTGTGG, bottom: aaacCCACACATCTCGAAGCACATCTTC, | | |
| Atg7 Crispr Guide RNA | IDT | custom |
| Top: caccGAAGTTGAACGAGTACCGCC, Bottom: aaacGGCGGTACTCGTTCAACTTC | | |
| | | |

| Recombinant DNA | | |
|---|---|---|
| DsRed-Rab5-QL | Addgene | 29688 (Taelman et al., 2010) |
| DsRed-Rab5-WT | Addgene | 13050 (Sharma et al., 2003) |
| pmCherry-ATG5 | Addgene | 13.095 (Hamacher-Brady et al., 2007) |
| mCherry-Alix | Addgene | 21504 (Lee et al., 2006)611 |
| HcRed-hLC3 | Addgene | 24991 (Tanida et al., 2008) |
| HcRed-LC3deltaGly221 | Addgene | 24992 (Tanida et al., 2008) |

| | | |
|---|---|---|
| TagBFP-LAMP1 | Addgene | 55263 (Rizzo et al., 2009) |
| EBFP2-CathepsinB | Addgene | 55236 (Rizzo et al., 2009) |
| GFP-p62 | Peter Kim | (Pankiv et al., 2007) |
| GFP-p62deltaLIR | Peter Kim | (Pankiv et al., 2007) |
| | | |

| Other | | |
|---|---|---|
| Motility Chamber | BD Bioscience | 354578 |
| Invasion Chamber | BD Bioscience | 354480 |
| Diff-Kwik | ThermoFisher | 9990700 |
| Dynabeads MyOne Streptavidin T1 | ThermoFisher | 65601 |
| His-tagged DynaBeads | ThermoFisher | 1013D |
| | | |

### EXAMPLE 2 - Autophagy-Related Gene-5 (Atg5) Controls Exosome Production and Loading with a Network of Autophagy Substrates Independent of Macroautophagy

Exosomes are extracellular vesicles with emerging roles in tumor invasion and the intercellular spread of proteins including SOD1 and α-synuclein implicated in pathology of neurodegenerative diseases. This Example demonstrates that while exosome release is independent of macroautophagy, altered endosomal acidification abrogates exosome release in cells lacking a subset of genes, like Atg5, which are critical and/or key for macroautophagy. Lack of Atg5 impedes invasion of cancer cells promoted by exosomes. Moreover, Atg5 also controls an alternate fate for an extended network of proteins, including SOD1 and α-synuclein that are normally degraded by autophagy: extracellular release in exosomes. These results demonstrate a mechanism controlling exosome production and contents that may underpin the role of exosomes in cancer cell invasion and the spread of several neurodegenerative diseases.

In macroautophagy (autophagy hereafter) an isolation membrane expands to engulf cytoplasmic contents eventually enclosing them entirely within an autophagosome. Autophagosome contents are subsequently degraded upon fusion with lysosomes. Generation of the autophagosome involves many Autophagy-related genes (Atg)(Mizushima et al., 2011), including an enzymatic cascade culminating in the covalent modification of LC3-I (Atg8) with the lipid phosphatidylethanolamine (PE) to form LC3-II. Generation of LC3-II involves Atg7 and a complex containing AtgS-Atg12 (Mizushima et al., 2011) and Atg16L1(Fujita et al., 2008; Romanov et al., 2012). A group of autophagy receptors including p62 (SQSTM1), NDP52 (CALCOCO2) and OPTN bind specific substrates for degradation by autophagy. These autophagy receptors also harbor LC3-Interacting-Regions (LIR) that allow them to link substrates for autophagic degradation to LC3 and the forming autophagosome (Birgisdottir et al., 2013).

For example, the autophagy receptor p62 is recruited to aggregates of proteins like SOD1 (Gal et al., 2009), Huntingtin (Bjorkoy et al., 2005) and α-synuclein (Watanabe et al., 2012) promoting their degradation by autophagy. Failure of autophagy to degrade these aggregates is believed to contribute to neurodegenerative diseases such as Amyotrophic Lateral Sclerosis (ALS)(Menzies et al., 2015). As disease progresses, aggregates of these proteins accumulate in distant regions of the brain but how these proteins spread remains uncertain (Aguzzi and Lakkaraju, 2015; Brettschneider et al., 2015). Emerging evidence suggests that a major mechanism involved in transporting disease-linked proteins like SOD1, Tau and α-synuclein to new sites are extracellular vesicles called exosomes (13, 15, 16). Exosomes are attributed a growing list of physiological functions in addition to mediating the intercellular spread of disease-associated proteins throughout the brain in several neurodegenerative diseases (12, 15, 16). Exosomes also promote metastasis and invasion in cancer. The protein content of exosomes conspicuously diverges from that of the cells that produce them (Colombo et al., 2014). How proteins including those involved in neurodegenerative diseases such as SOD1, prion protein, Htt, Tau and α-synuclein are selectively incorporated into exosomes has been considered unresolved and may have fundamental importance in the progression of these diseases.

Exosomes are 40-120 nm vesicles released by many cell types (Colombo et al., 2014). As endosomes acidify and mature their membranes bud inward to produce vesicles inside their lumen (Hanson and Cashikar, 2012). These Multivesicular Bodies (MVB) can subsequently fuse with the plasma membrane to release their intraluminal vesicles to the extracellular space where they are called exosomes (Colombo et al., 2014). Mechanisms responsible for production of exosomes have not been understood in much detail. One major challenge in this endeavor is defining exosome-producing MVB among several MVB subpopulations. For example, lipid lysobisphosphatidic acid (LBPA) is frequently used as a specific marker of intraluminal vesicles of MVB. These MVB are prone to lysosomal fusion and degradation (White et al., 2006) rather than extracellular release and exosomes are not enriched in LBPA (Laulagnier et al., 2004; Wubbolts et al., 2003). Therefore, LBPA is likely a poor marker of MVB involved in exosome release. In contrast internalized EGFR label a subpopulation of MVB largely distinct from MVB that contain LBPA (White et al., 2006) and EGFR family receptors are abundant on exosomes (Higginbotham et al., 2011). This suggests EGFR+ MVB are likely involved in producing exosomes. However it is also clear that a subset of EGFR+ MVB fuse with lysosomes suggesting distinct subpopulations of EGFR containing MVB have two opposed destinies: lysosomal degradation or plasma membrane release and intercellular communication. Other studies have showed that the two destinies of EGFR+ MVB can be distinguished. For example, the fluorescently tagged lipid N-lissamine phosphatidylethanolamine (NRhPE) selectively accumulates in MVB and rather than undergoing lysosomal fusion, NRhPE is released on exosomes (Vidal et al., 1997). Therefore, study of exosome production involves careful selection of MVB markers and evidence pertaining to MVB types fated for lysosomal fusion cannot be extrapolated to MVB types involved in exosome production.

The intersections between autophagosomes and endosomes have been studied in some detail. Early studies on autophagy demonstrated that autophagosomes fuse with endosomes en route to degradation in the lysosome (Berg et al., 1998; Gordon and Seglen, 1988), potentially forming multilamellar, degradative structures associated with LBPA staining (White et al., 2006). Studies have demonstrated that Atg proteins particularly Atg5, Atg16L1 and LC3 localize to endosomes and phagosomes to secure the acidification of these compartments and lysosomal degradation of pathogens or apoptotic cells they enclose (Florey et al., 2011; Martinez et al., 2015). Other studies have also shown that autophagy promotes lysosomal trafficking and degradation of endosomal contents (Peng et al., 2014; Tooze et al., 2014; Zhou et al., 2013).

Throughout such studies it is unclear and unlikely that the specific subpopulation of MVB involved in producing exosomes were studied.

Several studies have begun to investigate the effects of Atg genes on exosome production but the results of this research have frequently been contradictory. A unique Atg3-12 complex, that is made in an Atg7-dependent process promotes exosome release (Murrow et al., 2015). While this agrees with a subsequent report that Atg7 is required for release of hepatitis C in exosome-like vesicles (Shrivastava et al., 2015) it contradicts a previous report that Atg7 has no impact on exosome release (Sahu et al., 2011). These apparent contradictions may be due to the reliance of each of these studies on particular types of exosomes (e.g. hepatitis C virus containing) and western blot to quantify exosome release which can mistake effects on a subpopulation of exosomes or specific exosome contents for all exosomes (Colombo et al., 2013; Lotvall et al., 2014; Mathivanan et al., 2010). In sum, the effect of canonical genes key for autophagy like Atg5 on exosome production have not been previously studied, and published data on the effect of other Atg genes on exosome production are contradictory, and in need of further investigation.

In this Example, using multiple approaches including exosome particle quantification and analysis of exosome proteomes, it is demonstrated that Atg5 and Atg16L1 may be required and/or key for exosome production in an autophagy-independent manner. Moreover, Atg5 shapes the protein content of exosomes by recruiting LC3 and a subset of autophagy receptors including p62 and its ligands into exosomes.

### Results

Exosome preparations obtained by differential ultracentrifugation from both human breast cancer cell line MDA-MB-231 and mouse embryonic fibroblast (MEF) cells were predominantly composed of 30-120 nm particles (Nanosight particle tracking, dynamic light scattering, Fig.13A, Fig.19A), enriched in exosome markers (Fig.13B), and exhibiting the appearance of exosomes (electron microscopy, Fig.19A) (Thery et al., 2006). Exosome preparations from Atg5^{-/-} MEF contained strikingly reduced levels of exosome markers (Flotillin2, Tsg101, Fig.13B) and particles the size of exosomes (Fig.13C, Fig.19B-D). Similar results were obtained with ATG5^{-/-} human MDA-MB-231 cells (Fig.13D-F, Fig.19E-H). Transiently repressing Atg5 in MEFs using a tetracycline-dependent system, also reduced numbers of exosome-sized particles and exosome markers (Fig.13G). These effects were reversed by re-induction of Atg5 (Fig.13G). MEF expressing mutant Atg16L1 (Atg16L1^{ΔCCD}) (Saitoh et al., 2008) tended to produce less exosomes, while this effect was significant in HCT-116 cells entirely lacking Atg16L1 (Fig.13H, Fig.20A-DI). Upstream signaling complexes like mTOR and ULK1/2 signal to the autophagy machinery to activate autophagy (Russell et al., 2014). Activating autophagy with mTOR inhibitor pp242 (Feldman et al., 2009) increased exosome production, while exosome production was decreased in ULK1/2^{-/-} MEF cells (Fig.20E-I). In contrast Atg7^{-/-} MEFs had unaltered levels of exosome production (Fig.13G, Fig.20J). This demonstrates that exosome production does not require macroautophagy, but is strongly affected by Atg5 and Atg16L1.

In part, these studies sought to systematically identify the step of exosome biogenesis or re-uptake affected by Atg5. Atg5^{-/-} MEFs exhibited no difference in uptake of DiO-labeled exosomes, or depletion of labeled exosomes from the media, compared to wild-type cells (Fig.21A-C). This strongly suggests that exosomes are not less abundant in cell culture supernatants of cells lacking functional Atg5' because of altered cellular uptake of exosomes.

Atg5 may affect exosome production by promoting formation of intraluminal vesicles in MVB destined to become exosomes. Production of a covalent Atg3-Atg12 complex that was recently demonstrated to be required for budding of intraluminal vesicles into MVB, was not decreased in Atg5^{-/-} MEF (Fig.22A). ATG5^{-/-} MDA-MB-231 and Atg5^{-/-} MEF also exhibited similar punctae of CD63, a hallmark exosome marker, inside Rab5+ organelles by confocal and super-resolution microscopy (Fig.14A, Fig.22B). By electron microscopy, MVB in Atg5^{-/-} MEF contained intraluminal vesicles of similar morphology to MVB of wild-type cells (Fig.14B). The percent of MVB profiles exhibiting membrane invaginations, a measure of MVB budding and scission activity (Wemmer et al., 2011), was also unchanged in Atg5^{-/-} MEF (Fig.22C). Nor did MVB exhibit tubular or multicisternal protrusions in Atg5^{-/-} MEF as observed with some ESCRT mutants with impaired budding (Razi and Futter, 2006). Together, this strongly suggests that Atg5 does not affect budding and scission of intraluminal vesicles into MVB (Futter et al., 2001; Hurley and Hanson, 2010; Johnson et al., 2006).

Atg5 could also affect exosome production by redirecting MVB from fusing with the plasma membrane to fuse with lysosomes. The co-localization of MVB and exosome-enriched markers (N-lissamine rhodamine phosphatidylethanolamine [NRhPE] (Fig.23A,B) and mCherry-Alix Fig.17E (Fang et al., 2007; Vidal et al., 1997)) with lysosome markers (LAMP1, CathepsinB) was unchanged in wild-type, AtgS-repressed, or Atg5-reinduced MEFs (Fig.22D,E). In contrast, colocalization of internalized EGF (30 min) with lysosome markers (LAMP1, CathepsinB) was decreased in Atg5-repressed cells (Fig.22F,G). This suggests that Atg5 promotes fusion of EGF-trafficking endosomes with lysosomes consistent with the ability of Atg5 to promote lysosomal trafficking of several other endosomal compartments (Florey et al., 2011; Martinez et al., 2015; Peng et al., 2014; Tooze et al., 2014; Zhou et al., 2013). In contrast, these results suggest that unlike other endosomal compartments fusion of exosome-producing MVBs, with lysosomes is unaffected by Atg5.

In Atg5^{-/-} MEF, the number of intraluminal vesicles per MVB and MVB diameter increased (Fig.14C, Fig.23C-G). Others have suggested that this is indicative of increased MVB acidification and maturation (Huotari and Helenius, 2011; Puchner et al., 2013). In agreement, co-localization of the exosome marker mCherry-Alix with a marker of acidified endolysosomal compartments (Lysotracker) increased in Atg5-/- cells (Fig.14D-G). Similarly, NRhPE colocalization with Lysotracker increased in ATG5^{-/-} MDA-MB-231 cells four to six h after internalization (Fig.14F-G). Similar results were observed in Atg5^{-/-} MEF (23H-K). In contrast with MVB labeled with exosome markers and in agreement with results on other endosomal compartments Atg5 promoted acidification of dextran-containing endosomal compartments (Florey et al., 2011; Martinez et al., 2015; Peng et al., 2014; Tooze et al., 2014; Zhou et al., 2013) (Fig.23N). In sum, compartments containing MVB and exosome markers (mCherry-Alix, NRhPE) exhibit increased size, number of intraluminal vesicles and acidification (Lysotracker staining), but do not exhibit increased colocalization with lysosome markers (LAMP1, CathepsinB). Together, this suggests that MVB maturation and acidification is accentuated in Atg5^{-/-} cells but this does not affect all endosomal compartments (dextran, EGF).

Together, confocal and electron microscopy results strongly suggest that acidification of exosome-producing MVB is increased in Atg5^{-/-} cells in parallel with a drop in exosome production. If increased MVB acidification in Atg5^{-/-} cells causes the drop in exosome release, blocking acidification of MVB may promote exosome release. Two independent inhibitors of the V0-ATPase complex (Bafilomycin A1, Concanamycin) abrogated staining of cells with Lysotracker (Fig. 1 SA), in agreement with their established ability to block endosomal acidification (van Deurs et al., 1996; van Weert et al., 1995). Treatment with either BAF (Fig.15B,C) or ConA (Fig.15D, E) restored exosome production of Atg5^{-/-} cells (Fig. 1 SA-G). This strongly suggests that enhanced acidification of NRhPE and Alix-mCherry+ MVB in Atg5^{-/-} MEF cells causes defective exosome production.

Exosome release is severely blunted in Atg5^{-/-} cells. To ascertain what effects Atg5-controlled exosome release might have, the proteome of exosomes from MDA-MB-231 cells was analyzed. The top category of proteins in exosomes was cellular movement, the second highest ranked disease was cancer, and among the top six canonical pathways were Integrin signaling, actin cytoskeleton signaling and RhoGDI signaling. A network of physically- interacting proteins involved in cancer cell invasion was also evident in exosomes (Fig.16A). Western blotting confirmed the presence of several of these proteins linked to cancer cell invasion in exosomes including Vimentin, Ras, EGFR, and β-catenin (Fig.16B). Consequently, we hypothesized that exosomes affect migration and invasion of cancer cells and that Atg5 affects spread of this invasive phenotype between heterogeneous (Burrell et al., 2013; Friedl and Alexander, 2011) cancer cells by controlling exosome release. To test this in a tractable model, exosomes purified from 500 × 10⁵ cells of the highly invasive breast cancer cell line MDA-MB-231 were incubated with an equivalent number of MCF-7 cells, a minimally invasive breast cancer cell line. Exosomes from MDA-MB-231 cells promoted migration of MCF-7 cells in transwell and wound healing assays while exosomes from an equivalent number of ATG5^{-/-} MDA-MB-231 cells had no effect (Fig.16C,D, Fig.24A,B). Exosomes from MDA-MB-231 cells also promoted invasion of MCF-7 cells through a matrigel-coated transwell chamber while exosomes from ATG5^{-/-} MDA-MB-231 cells had no effect (Fig.16E, Fig.24C).

Exosomes produced by ATG5^{-/-} cells lack the ability to promote migratory and invasive phenotypes in neighboring cells (Fig.16C-E). This may be because ATG5^{-/-} cells produce less exosomes. Alternatively, the contents of exosomes from ATG5^{-/-} cells may be altered and this may diminish their capacity to promote invasion. MCF-7 exhibited similar invasiveness when incubated with equal amounts of exosomes from either wild-type or ATG5^{-/-} MDA-MB-231 cells (Fig.16F). This suggests that ATG5 promotes invasion of breast cancer cells predominantly by increasing the amount of exosomes produced and not by alterations to the exosome proteome in ATGS^{-/-} cells.

It may be possible that Atg5 impacts the content of exosomes without having measurable effects on invasion of breast cancer cells. To query this, equal amounts of exosomes from wildtype and ATG5^{-/-} MDA-MB-231 cells were analyzed using mass-spectrometry. The proteome of wild-type and ATG5^{-/-} MDA-MB-231 exosomes (1885 proteins) demonstrated a global decrease in canonical exosome markers (CD81, CD82, CD9 (Colombo et al., 2013), PrP, Tsg101, Alix (Baietti et al., 2012), Flotillin2, Hsp70 (Sahu et al., 2011)) in exosome preparations from ATG5^{-/-} cells. This suggests that ATG5^{-/-} decreases production of all subsets of exosomes. Intriguingly, exosomes from wild-type cells were reproducibly highly enriched (2-10⁸-fold) in a subset of 157 proteins (for example, Lir proteins, Calcoco2 (ndp52), Sqstml (p62), Tax1bp1, Bnip3, Clathrin, Pp1a, see Western blots of Figure 18). Among these were LC3A and LC3B. Western blot confirmed that the autophagosome marker LC3 was enriched in exosome preparations (Fig.17A, Fig.25A-D). Remarkably, exosomes were strongly enriched in lipid-modified LC3-II compared to cell lysates (vs. unmodified LC3-I) (Fig.17A, Fig.25A). LC3-II was also enriched in exosome preparations from mouse neuronal cells (Neuro2A) and mouse fibroblasts (3T3, Fig.25B,C). Independent antibodies recognizing either LC3A or LC3B similarly detected enrichment of LC3-II in exosomes (Fig.25B). On sucrose density gradients of exosome preparations, LC3-II fractionated with the exosome markers Alix, CD63 and Flotillin-1 at densities characteristic of exosomes (Fig.17B) (Thery et al., 2006). Finally, LC3 antibody labeled vesicles of 30-100 nm in exosome preparations while no labeling was detected with a control antibody (Fig.17C,D). Cumulatively this evidence indicates that LC3-II is enriched in *bona fide* exosomes (Fig. 17).

If LC3 is found in exosomes it should co-localize with endosomes and the intraluminal vesicles of MVB. Constitutively-active Rab5 (Rab5-Q79L) enlarges endosomes and faithfully documents the budding of intraluminal vesicles into MVB during exosome biogenesis (Baietti et al., 2012; Gross et al., 2012; Trajkovic et al., 2008). Accordingly, punctae inside Rab5-Q79L endosomes were labeled by specific antibodies (Fig.25C) recognizing Alix, Tsg101 or CD63, established markers of intraluminal vesicles and exosomes (Fig.25D-F). LC3 labeled with a specific antibody (Fig.G,H), exhibited similar punctate staining within Rab5-Q79L+ endosomes (Fig.17E,F) that co-localized in part with exosome markers Alix, Tsg101 and CD63 in MEF and MDA-MB-231 cells (Fig.17E,F). Super-resolution Stimulated Emission-Depletion (STED) microscopy further resolved discrete punctae of CD63 and LC3 in Rab5+ endosomes (Fig.17G, Fig25I). Together, this suggests that a subset of the LC3 co-localized with endosomal markers is due to its presence in intraluminal vesicles of MVB destined to become exosomes.

LC3-II could associate with the surface of exosomes following the fusion of autophagosomes with endosomes as the internal membrane of the autophagosome starts to degrade. LC3-II was sensitive to Protease K-digestion only after treatment of exosome preparations with detergent (Fig.26A). This strongly suggests that LC3-II is enclosed inside exosomes independent of macroautophagy. Reinforcing this, whereas exosomes from wild-type cells are enriched in lipid-modified LC3-II, Atg16l1^{ΔCCD}) and Atg7^{-/-} MEF that are incapable of macroautophagy produce exosomes enriched in LC3-I (Fig.18A,B). This demonstrates that LC3 is recruited into exosomes by a process not requiring macroautophagy or lipid modification of LC3.

Proteomics data suggested that LC3 levels were starkly reduced in exosomes from Atg5-/- cells. In accord, Western blot of equal amounts of exosomes from wild-type and Atg5^{-/-} cells demonstrated that LC3 was nearly undetectable in exosomes from Atg5^{-/-} cells (Fig.18D). Similar results were found in exosomes from cells lacking Atg16L1 (Fig.18E). Together, this suggests that Atg5 and full-length Atg16L1 are required and/or key for LC3 recruitment into exosomes (Fig.18D,E).

LC3 binds to LIR motifs in autophagy receptors to recruit a range of substrates. Among the subset of 157 proteins reproducibly decreased (2-10⁸-fold) in exosomes from Atg5^{-/-} cells were LC3-interacting autophagy receptors NDP52, BNIP3, Optineurin (OPTN), and p62 and other proteins with experimentally-defined LIR motifs (Fig.18E, for example, Lir proteins, Calcoco2 (ndp52), Sqstml (p62), Tax1bp1, Bnip3, Clathrin, Ppla, see Western blots of Figure 18). In Western blots of equivalent amounts of exosomes from Atg5^{-/-} cells and wild-type cells, levels of NDP52 and p62 were sharply reduced in exosomes produced by Atg5^{-/-} cells (Fig.18F). Similarly, levels of several other proteins identified to bind p62 or LC3 in a recent definition of the autophagy protein interaction network (Behrends et al., 2010) (KEAP1, PPP1A, SOD1) were also distinctly decreased in exosomes from Atg5^{-/-} cells (Fig.18F).

Analysis of known physical interactions of proteins reduced in exosomes from Atg5^{-/-} cells revealed a highly interconnected network with nodes centered on LC3A, LC3B and autophagy receptors (Fig.18G, p62 [SQSTM1], NDP52 [CALCOCO2], BNIP3 and OPTN). To test if LC3 controls sorting of LC3-binding proteins into exosomes LC3 was over-expressed. Wild-type LC3 modestly increased p62 abundance in exosomes independent of effects on p62 levels in cells, or amount of exosomes produced (Fig.18H). In accord with the ability of LC3-I to be robustly sorted into exosomes (Fig.18A,B), over-expression of mutated LC3 incapable of forming LC3-II similarly increased p62 sorting into exosomes (Fig.18H). Mutating the LC3-binding motif in p62 (p62-ΔLIR) interrupted its sorting into exosomes, while there was no effect on levels of total exosomes captured (Fig.18I, Fig.27A,B). This demonstrates that LC3 controls sorting of p62 into exosomes and this involves and/or requires the LC3-binding LIR motif in p62.

Atg5 controls sorting of LC3 and a network of LC3-binding autophagy receptors into exosomes (Fig.18A-I). We tested whether the autophagy receptor p62 controls sorting of its cargoes into exosomes. p62 associates and co-localizes with several proteins implicated in neurodegenerative diseases that have also been detected in exosomes. These include SOD1 (Gal et al., 2009; Gomes et al., 2007; Kabuta et al., 2006), and α-synuclein (Emmanouilidou et al., 2010; Kuusisto et al., 2001; Webb et al., 2003). To test the ability of p62 to recruit these cargoes into exosomes Neuro2A cells were treated with siRNA targeting p62 or control siRNA. P62 siRNA caused levels of SOD1 and α-synuclein to increase slightly in cells consistent with roles for p62 in promoting their degradation (Fig. 18J). In contrast, p62 siRNA starkly diminished the amount of SOD1 and α-synuclein in exosomes without affecting levels of exosomal marker proteins (Tsg101, Flotillin2, Fig. 18K). When cells were transfected with plasmid expressing α-synuclein, the levels of α-synuclein in exosomes was also reduced when cells were co-transfected with p62 siRNA (Fig. 18L). In agreement with its well-established binding to ubiquitinated proteins and association with ubiquitinated aggregates in several neurodegenerative diseases, knockdown of p62 reduced levels of many but not all polyubiquitinated proteins in exosomes (Fig.18M). In addition to requiring p62 (Fig.18J), sorting of SOD1 into exosomes also required Atg5 (Fig.18N) that sorts LC3 into exosomes (Fig.18A). Therefore, p62 controls the packaging of a network of proteins implicated in neurodegenerative diseases into exosomes. Together this demonstrates that Atg5 recruits a cascade of proteins into autophagosomes and propels the extracellular release of LC3, autophagy receptors like p62 and their ligands like SOD1 and α-synuclein.

Autophagy receptors are present in exosomes derived from multiple types of mouse and human cell lines. To determine whether these proteins are also found in exosomes from healthy human subjects, these proteins were blotted in exosomes from human serum, plasma, urine and saliva. The autophagy receptors p62 and NDP52 as well as Atg5 were abundant in exosomes from human plasma, serum, and urine but below detection levels in saliva. (Fig. 18O). This suggests the mechanism described here for packaging of autophagy receptors and their cargoes in exosomes may be triggered *in vivo* in humans.

### Discussion

It is now evident that subpopulations of exosomes exist (Colombo et al., 2013; Mathivanan et al., 2010). Consequently, recent guidelines for studying extracellular vesicles strongly discourage relying on Western blot of exosome markers to quantify exosomes as it risks misinterpreting effects on a subpopulation of exosomes or specific proteins as effects on all exosomes (Lotvall et al., 2014). For this reason, we quantified number of exosome-sized vesicles using dynamic light scattering and Nanosight particle tracking and confirmed effects on exosome numbers using western blot for multiple markers of exosomes and the total exosome proteome. We find that Atg5 and Atg16L1 both strongly promote exosome production, but this is independent of Atg7 and canonical macroautophagy. An increasing number of signaling pathways that allow upstream activators of autophagy such as ULK1/2 and mTOR inhibitors to phosphorylate and activate core proteins required for autophagy are being studied(Russell et al., 2014). Such signaling pathways may enable ULK1 and mTOR inhibitors to influence Atg5 or Atg16L1 and modulate exosome production (Fig.20E-I).

In this Example, we found opposing effects of Atg5 on lysosomal fusion of compartments containing cargo destined for degradation (dextran, EGF) compared to endosomes containing markers of exosomes. Atg5 promoted acidification and lysosomal fusion of compartments destined for degradation (EGF, dextran, Fig. 22D-G, 23M-N), while limiting acidification of MVB containing exosome markers destined for extracellular release (Alix, NRhPE, Fig.14B-G, Fig. 23H-L). While each of these markers may traffick through late endosomes and MVB, it is clear that discrete populations of MVB with distinct fates (lysosomal fusion, Golgi-trafficking, plasma membrane fusion) exist. Therefore, the heterogeneity of endosomal compartments likely accounts for the disparate effects of Atg5 on distinct endosomal markers and renders evident to study each endosomal compartment individually.

In a similar vein, apparently conflicting effects of Atg5 on acidification and lysosomal fusion of other endolysosomal compartments have been described in the literature (Kim et al., 2012; Peng et al., 2014; Romao et al., 2013; Sanjuan et al., 2007; Zhou et al., 2013). For example, studies of several other types of endosomes and LC3-associated phagosomes destined for degradation have demonstrated that Atg5 increased acidification and lysosomal fusion (Kim et al., 2012; Peng et al., 2014; Sanjuan et al., 2007; Zhou et al., 2013). In contrast, in some circumstances acidification and lysosomal fusion of LC3-associated phagosomes is attenuated, delaying pathogen degradation, but promoting loading of pathogen-derived peptides on MHC class II for presentation at the plasma membrane (Romao et al., 2013). Cumulatively, this suggests a model wherein LC3 and Atg5 slow lysosomal trafficking of contents destined for extracellular release (exosomes) or recycling to the plasma membrane, while accelerating the acidification and lysosomal fusion of endocytic compartments geared for degradation of contents (high dose EGF Fig.22F,G; dextran Fig.23N) (Peng et al., 2014; Sanjuan et al., 2007; Zhou et al., 2013).

When MVB were over-acidified in Atg5^{-/-} cells, exosome release was strongly inhibited (Fig.13-14). Blocking acidification of MVB with either of two inhibitors rescued exosome production (Fig. 15). This is consistent with studies demonstrating that neutralizing endosomal pH or reducing extracellular pH toward that of late endosomes increases exosome release (Alvarez-Erviti et al., 2011; Parolini et al., 2009). Previous studies had speculated that the effects on exosome production of V₀ATPase inhibitor Bafilomycin which effects acidification of endosomes and lysosomes is due to a role for autophagy in exosome production. The present results demonstrate that while Bafilomycin does increase exosome production this is independent of autophagy, as it also occurs in Atg5-/- cells.

Exosomes have been demonstrated to promote invasion of cancer cells (Peinado et al., 2012). The present results demonstrate that Atg5 may control the ability of exosomes to promote cancer cell migration and invasiveness. This effect of Atg5 on exosome release may contribute to the effects of autophagy on cancer cell invasion previously observed (Kenific and Debnath, 2015). This adds to the complex effects of Atg genes and mTOR inhibitors in cancer. For example, the results suggest that mTOR inhibitors in addition to enhancing survival of cancer cells in limiting tumor environments may also promote metastasis of tumor cells (Mathew et al., 2009) by increasing exosome release (Fig.20H,I).

LC3 recruits autophagy receptors and substrates to the isolation membrane for enclosure in autophagosomes. The results demonstrate that Atg5 also controls packaging of LC3 into exosomes. LC3 then recruits autophagy receptors like p62 and their substrates like SOD1, and α-synuclein into exosomes (Fig.18H-K). This is likely to impact the emerging physiological functions of exosomes. For example, p62 over-expression can induce tumorigenesis (62, 65) and p62 binds proteins like KEAP1 and TWIST which control cancer cell signaling and metastasis. Inclusion of ligands such as these in exosomes may contribute to the effects of exosomes on tumorigenesis (Melo et al., 2014) and metastasis.

P62 also associates with protein aggregates containing proteins such as SOD1, Tau and α-synuclein involved in several neurodegenerative diseases (Gal et al., 2009; Kabuta et al., 2006; Kuusisto et al., 2001; Menzies et al., 2015; Webb et al., 2003). Evidence increasingly demonstrates that spread of these diseases between cells and throughout the brain is mediated by the inclusion of oligomeric or misfolded versions of these proteins into exosomes (Aguzzi and Lakkaraju, 2015; Asai et al., 2015; Grad et al., 2014). Our results demonstrate that p62 recruits SOD1 and α-synuclein into exosomes. Previous studies suggest that p62 preferentially binds mutant, oligomeric or misfolded forms of SOD1 and α-synuclein (Gal et al., 2009; Watanabe et al., 2012) and according to previous studies these disease-associated forms are preferentially recruited into exosomes (Danzer et al., 2012; Grad et al., 2014). The present results thus suggest that LC3 and p62 may redirect misfolded proteins it associates with such as SOD1, TDP-43, Tau, α-synuclein, Huntingtin, and PrP from degradation in autophagolysosomes to extracellular release in exosomes thereby enabling their spread throughout the brain.

### Materials and Methods

### Antibodies, Plasmids and Reagents

Antibodies recognizing the following proteins were used: Alix (49/AIP1, BD Transduction Laboratories), Alix (2171, Cell Signaling Technology), Atg5 (8540, Cell Signaling Technology), Atg6 (3495, Cell Signaling Technology), Atg7 (8558, Cell Signaling Technology), Atg12 (4180, Cell Signaling Technology), Atg3 (3415, Cell Signaling Technology), anti-human CD63 (sc5275, Santa cruz), anti-mouse CD63 mAb (R5G2, MBL International), LC3 A/B (4108, Cell Signaling Technology), TSG101 (clone M19, sc-6037, Santa Cruz), TSG101 (4A10, Genetex), TOM20 (clone FL-145, Santa Cruz), Rab5 (3547, Cell Signaling Technology). Antibodies used in Fig.13H (sucrose gradient) are Alix (pab0204, Covalab), Flotillin1 (610820, BD Transduction Laboratories) and anti-GFP (vs.GFP-CD63,ab3277, Abcam) in Neuro2A cells.

Plasmids expressing the following proteins were used: DsRED-Rab5-QL (29688, Addgene), DsRed-Rab5 WT (13050, Addgene), pmCherry-ATG5 (13095, Addgene), mCherry-Alix (21504, Addgene), Rab7-dsRed (12661, Addgene), Rab11 WT-dsRed (12679, Addgene), HcRed-hLC3 (24991, Addgene), HcRed-LC3AGly221 (24992, Addgene), TagBFP-LAMP1 (55263, Addgene), EBFP2-CathepsinB (55236, Addgene). Plasmids expressing GFP-p62 and GFP-p62ΔLIR were generated by T. Johansen (Pankiv et al., 2007) and a gift of P.Kim (University of Toronto). Other reagents used included: pp242 (P0037, Sigma-Aldrich), Lysotracker Blue (L7526, Life Technologies), Lissamine Rhodamine B phosphatidylethanolamine (16:0, 810144, Avanti Polar Lipids) and Lipofectamine 2000 (11668-019, Life Technologies).

### Cells

Mouse embryonic fibroblasts (MEF) lacking a copy of Atg5 (Kuma et al., 2004), ULK1/2 (Cheong et al., 2011), or expressing Atg16L1 lacking the coiled-coil domain (Saitoh et al., 2008), and wildtype and ATG16L1^{-/-} HCT-116 were a gift of D. Philpott (University of Toronto, Canada). Atg7^{-/-} cells were a gift of Herbert Virgin (University of Washington). Doxycycline-regulated Atg5 MEF generated previously were also used (Hosokawa et al., 2007). The knockout of Atg16l1 beta were confirmed using western blot and/or qPCR. Primers used for Atg1611 beta knockdown confirmation were: Primers used for ATG16L1 qPCR were Set1: F: GGC GTT CGA GGA GAT CAT TCT G (SEQ ID NO: 21), R:TAT CAT TCC ACG CAC CAT CAT G (SEQ ID NO: 22) and Set 2:F: CCA GGA ACT GGT CAC CAG ATG (SEQ ID NO: 23), R: CAA CAG GTA GAG GTT CCT TTG C (SEQ ID NO: 24). RT-qPCR was performed with the MiScript II Reverse Transcriptase system (Qiagen) and GoTaq^{®} qPCR Master Mix (Promega A6002).

MDA-MB-231 (HTB-26, ATCC) cells were genetically deleted of ATG5 using Compozr Zinc Fingers technology (product number CKOZFND1217-1KT, Sigma-Aldrich). Mutation of ATG5 was confirmed using Surveyor Mutation Detection Assay (706020, Transgenomic), PCR (ZFN primer F sequence was CAAAATGGGCCAAATAGCTAAA (SEQ ID NO: 25) and ZFN primer R sequence was ACTTACCATTTTGCAATCCCA (SEQ ID NO: 26)) and western blot. All cells, including Neuro2A (CCL-131, ATCC) and 3T3 (CRL-1658, ATCC), were cultured in Dulbecco's Modified Eagle Medium (DMEM) containing 10% fetal bovine serum and penicillin-streptomycin.

### Exosome Enrichment

Exosomes were enriched by differential centrifugation as previously described (Thery et al., 2006). Briefly, MEF or MDA-MB-231 cells were cultured in media containing FBS depleted of exosomes by centrifugation at 100 000 g for 16 h (Thery et al., 2006). To purify exosomes supernatant from cell cultures was centrifuged at 400 g (7 min), 2000 g (10 min) and 10 000 g (30 min, SW32 rotor). At each step the supernatant was recovered. After centrifugation at 100 000 g (1 h 10 min, SW32 rotor) the supernatant was removed and the pellet was resuspended. The pellet was washed in PBS by a final centrifugation at 100 000 g (20 min, TLA100.3 rotor). The exosome-enriched pellet was re-suspended in PBS for further analyses.

For density separation, exosomes were purified as described (Laulagnier, 2015). Briefly, Neuro2A cell culture media was collected, and a cocktail of protease inhibitors added (Roche). Media were cleared of debris by two successive centrifugation steps (2 000 g for 10 min, 20 000 g for 20 min) and filtration through a 0.22 µm filter (Millex GV PVDF, Millipore). The medium was centrifuged for 2 h at 100 000 g (2 h, SW32 rotor). The resulting pellet was resuspended in 0.211 M sucrose, 3 mM imidazole pH 7.4, and loaded onto a continuous sucrose gradient (0.3-1.4 M). Gradient was centrifuged at 200 000 g (18 h, SW41Ti rotor), and 10 fractions (1 ml) were collected). The sucrose density of each fraction was determined by refractometry. Fractions were washed in 3 mM imidazole, pH 7.4, and centrifuged at 200,000 g (2 h, SW41Ti rotor). Exosome pellets were resuspended in Laemmli buffer and used for Western blot analysis.

### Dynamic Light Scattering

Preparations of enriched exosomes were analyzed by dynamic light scattering on a Protein Solutions Dynapro Instrument using Dynamics V6 software. Data were acquired every 10 seconds at 4°C and 10% laser power for at least 200 seconds per sample. The intensity (Cntls) and size (nm) were generated automatically by the instrument.

### Nanosight Particle Tracking Analysis

Preparations of enriched exosomes were analyzed using a Nanosight LM10 instrument with Nanoparticle Tracking Analysis software Version 2.3. Measurement temperature was 22°C and time setting was 90 seconds. The analysis conditions were set as: Blur 3X3, Detection Threshold 3, Min Track Length 9, and Min Expected Size 30nm. The analysis reports containing the particle size (nm) and concentration (particles/m1) were generated automatically.

### Exosome Uptake Assay

Exosomes purified using the procedure described above were re-suspended in 20 µl of PBS. Vybrant DIO Cell-Labeling Solution (V22886, Invitrogen) was used and the manufacturer's protocol was followed. Briefly, 5 µl of the cell-labeling solution was added to the exosome preparation and incubated for 20 min at 37°C. Excess label was removed by centrifugation (100,000g, 20 min, TLA 100.3 rotor) and the pellet was re-suspended in 500 µl of DMEM.

Equal amounts of DIO-labeled exosomes were added to cell in triplicate and DMEM alone was added to control wells. Cells were incubated with DIO-labeled exosomes for 1 h. Supernatant was retained to measure uptake of DIO-labeled exosomes by disappearance of fluorescence (Synergy H1 Hybrid Multi-Mode Microplate Reader, BioTek). To measure internalization of exosomes into cells, these were re-suspended in 500 µl of PBS for analysis on a flow cytometer (Beckman Coulter Cyan ADP 9).

### Endosomal Labeling and Trafficking Assays

To label MVB cells were incubated with 3µM NRhPE (Avanti Polar Lipids) at 4°C for 15 min then transferred to 37°C, 5% CO₂ for the indicated incubation times, following the protocol described (Vidal et al., 1997). pHrodo Green Dextran (10 kDa, ThermoFisher) was incubated with cells for 10 minutes and cells were transferred to ice for immediate analysis by flow cytometry. After initial readings 20 mM NH₄Cl was added to cells to neutralize the pH-dependent increase in pHrodo fluorescence to measure dextran internalization and acidification of dextran containing compartments (geometric mean fluorescence intensity before/after NH₄Cl). To image EGFR trafficking cells at stages where its localization was in late endosomes and lysosomes cells in serum-free media were incubated with EGF-Alexa488 (100 ng/mL, ThermoFisher) for 30 min when its localization in intraluminal vesicles of MVB is maximal (Vanlandingham and Ceresa, 2009).

### Immunofluorescence Microscopy

Cells were prepared for microscopy as previously described (Gibbings, 2012). Cells cultured on coverslips (Deckglaser 18 mm Ø) were fixed in 2% paraformaldehyde in PBS (10 min) 48 h post transfection. Cells were then rinsed twice in PBS and permeabilized with 0.2% Triton-X-100 in PBS and 20 mM NH₄Cl (10 min). After washing with PBS, cells were blocked with 5% milk in PBS (1 hour), washed three times in PBS and incubated with 2-5µg/ml primary antibody in PBS at 4°C overnight. Following primary incubation, cells were washed three times (5 min) in PBS and incubated for 1 hour at room temperature with 1/250 dilution of highly cross-adsorbed secondary antibodies (goat anti-mouse Alexa Fluor 488, 546 or 633, goat anti-rabbit Alexa Flour 488 or 546, goat anti-rat Alexa Fluor 546, donkey anti-rat Alexa Fluor 488 or anti-goat Alexa Fluor 488 [Invitrogen Molecular Probes], or goat anti-rabbit DyLight 405 [Thermo Scientific]). After washing three times (5 min), cells were mounted with Vectashield Mounting Media. For live cell imaging, cells were plated on Cellview culture dishes with 2.2 cm2 glass bottom. Epifluorescence microscopy was performed using a Zeiss Axio0bserver.D1 or Zeiss Axio0bserver.Z1 microscope. Images were acquired with a 63x Plan-Apochromat 1.4 Oil DIC objective and AxioCam MRm CCD (monochrome), using Zeiss AxioVision Rel. 4.8 software. Confocal microscopy was performed using a Zeiss LSM 510 Meta/AxioVert 200 microscope. Images were acquired with a 63x Plan-Apochromat 1.4 Oil DIC objective, 2 Photo Multiplier Tubes and 1 Meta detector, using Zeiss ZEN software. Confocal images for preparing 3D models were acquired in 0.5-1 µm Z-stacks.

### Stimulated Emission-Depletion (STED) Microscopy

Subconfluent cells were cultured on glass coverslips (Deckglaser 18 mm Ø). Post 48 h of transfection, cells were fixed with 4% paraformaldehyde in 10 mM PBS (10 mM phosphate buffer - pH 7.2, 154 mM NaCl). After cells were washed with PBS at room temperature, then incubated overnight at 4°C with primary antibody prepared in Ab Buffer (10 mM PBS, 3% BSA, 0.3% Triton X-100). After washing, the cells were incubated for 1 hour at room temperature with STED CW-compatible secondary as well as tertiary antibodies prepared in Ab buffer. Cells were mounted with Prolong Gold ^{™} Antifade (P36930, Life Technology) mounting medium and cured for 24 h at room temperature before imaging. Secondary antibodies used include: ChromeoTM 505 anti-mouse IgG (15050, Active Motif, Optimized Final Concentration 1:500), Biotin-antirabbit IgG (B8895, Sigma, Optimized Final Concentration 1:800), Streptavidin V500 (561419, BD Horizon, Optimized Final Concentration 1:200).

Continuous wave stimulated emission depletion (STED CW) images were acquired using a Leica SP5/STED CW microscope, and a 100x oil immersion objective (HCX PL APO CS 100x/1.40 OIL STED, Leica Germany) essentially as described (Zheng et al., 2011). Dual colour excitation of fluorophores was achieved using an argon-ion laser exciting at 458 nm and 514 nm respectively. STED depletion was achieved with a 592 nm CW laser line. Fluorescence outputs were captured in sequential scans using base parameters of 600 Hz frequency at a 2.54x zoom giving a resolution of 29.8 nm × 29.8 nm per pixel. The first scan at 514 nm excitation wavelength (ChromeoTM 505) was performed at 50% laser power, 750 gain, -0.3 offset, with frame averaging and accumulation of 3, and a detection window of 520 - 580 nm. The second scan at 458 nm excitation wavelength (streptavidin V500) was performed at 85% (LC3) or 55% (Rab5) laser power, 800 gain, -1.0 offset, with frame averaging and accumulation of 3, and a detection window of 465 - 485 nm. STED 592 depletion laser power was 100%. Images were processed using Leica STED deconvolution software (LAS AF v2.6.3.8173). First a point spread function (PSF) was generated using a Lorentz transformation of 70 nm. Then the image was deconvolved using the generated PSF and signal energy set to regularization parameter 0.05. Images were further processed using the baseline mean function, and background was reduced by 1500 (in the 16-bit image).

### Image Analysis:

Images were analyzed using ImageJ software. Three-dimensional image renderings were made using ImageJ. Raw microscope image channels were thresholded and merged using the imbedded "Threshold > Triangle" and "Merge Channels" options, respectively. The composite image was rendered as a 3D image with the "Show Color Surfaces" plugin with the radius of smoothing set to 0.20 pixels.

To detect and quantify the colocalization of fluorescently labelled subcellular structures, we employed an object-based colocalization method named Squassh' (segmentation and quantification of subcellular shapes) (Rizk et al., 2014). The ImageJ plugin - MosaicSuite was downloaded and run on each of the images. Background fluorescence was eliminated with a rolling ball window size of 10 pixels. For segmentation, PSF model for the wide-field microscope used was estimated to be 0.84 (xy) and 0.79 (z). Regularization parameter and minimum object intensity of channel 1 and channel 2 were set at 0.15, 0.075 and 0.15 respectively. A Gaussian noise model was used. Cell masks were set at 0.25 and 0.2 for channel 1 and channel 2 respectively. Co-localization of segmented objects is demonstrated in Fig.22. Another ImageJ plugin Just Another Co-localization Plugin (JACoP) was used to obtain Li's intensity correlation co-efficient of co-localization (Li et al., 2004). In quantified experiments at least 3 slides were images per condition, with 5-7 images per slide and approximately 10 cells/image to provide data from about 250-350 cells.

### Electron Microscopy

Cultures of MEF cells were fixed *in situ* with 0.1 M cacodylate buffer containing 2% glutaraldehyde until processing for embedding. Cells were post-fixed in 1% osmium tetroxide (EMS, PA, USA) in cacodylate buffer at 4°C. After washing in buffer, MEF cells were dehydrated in graded ethanol, infiltrated and embedded in Epon 812 (MECALAB, Quebec, Canada), as described (Luft, 1961). Ultrathin sections were obtained using a Reichert Ultracut S ultramicrotome, and mounted on formvar-carbon coated nickel grids (MECALAB, Quebec,Canada). Sections were stained with uranyl acetate and examination was performed with a Philips CM 100 electron microscope. To analyse MVB, we excluded organelles containing a signature double-membrane of autophagosomes or organelles that were electron dense like lysosomes or contained heterogenous luminal contents that are characteristic of lysosomes and autophagolysosomes (Huotari and Helenius, 2011). MVB frequently containing dense surface patches associated with vesicle budding (Murk et al., 2003), and exclusively containing vesicles of 30-120 nm were analysed (Fig.16C) (Huotari and Helenius, 2011).

LC3 was detected in exosome preparation by electron microscopy as previously described (Chivet et al., 2014). Briefly, Neuro2a cell culture media was collected, and a cocktail of protease inhibitors added (Roche). Media were cleared of debris by two successive centrifugation steps (2 000 g for 10 min, 20 000 g for 20 min) and filtration through a 0.22 µm filter (Millex GV PVDF, Millipore). The medium was centrifuged for 100 000 g (2 h, SW32 rotor). The resulting pellet was washed in PBS and resuspended in 40 µl 2 % paraformaldehyde in PBS. Four µL drops were spotted onto Formvar-carbon-coated grids for 20 min, treated with PBS-glycine (50 mM), blocked with PBS-BSA (10 mg/ml) then permeabilised with 0.05 % saponin in PBS-BSA before consecutive incubation with rabbit anti-LC3A/B (Cell signalling, 1 h), followed by goat anti-rabbit 1.4 nm gold conjugated-F(ab') (Nanoprobes, 45 min). The grids were then fixed 1 min with 1 % glutaraldehyde in PBS and finally treated with the HQ silver intensification kit (Nanoprobes) for 6 min before negative staining. Grids were observed with a Joel JEM 1200EX transmission electron microscope.

### Scratch wound assay

MCF-7 cells were grown to confluence in 6 well plates containing DMEM with FBS. Wells were marked with a reference line through their center. Once cells reached confluence they were scratch wounded using a 200µ1 pipette tip (2 wounds/well perpendicular to the reference line), washed with PBS, and supplied with fresh media. Exosomes isolated from equivalent amounts of wild-type MDA-MB-231, Atg5^{-/-} MDA-MB-231, or MCF-7 cells in 6 well plates were added to the media of scratch-wounded MCF-7 cells. An image on either side of the reference line at each scratch wound was taken at time 0 and 20 h. In each image, the distance of the wound was measured at 6 points including the largest and smallest distances.

### Motility and invasion assay

Motility chamber (BD Bioscience, Cat#354578) and invasion chamber (BD Bioscience, Cat#354480) were rehydrated and equilibrated for 2hrs with 500u1 of serum free DMEM media. After 2 h, the medium in the inserts was aspirated and inserts were placed into wells containing DMEM with 10% FBS. MCF-7 cells (50 × 10³) were added to each chamber in serum-free media. Exosomes isolated from equivalent amounts of wild-type MDA-MB-231, ATG5^{-/-} MDA-MB-231, or MCF-7 cells in 6 well plates were added to the media of MCF-7 cells. The chambers were incubated for 24 h with exosomes. The media was then removed and the upper surface of the membrane was scrubbed 20 times with a cotton swab. Cells on the lower surface of the scrubbed membranes were fixed and stained with Diff-Kwik kit (Thermo Fisher Cat# 9990700) according to the manufacturer's instruction.

### Proteomics of Exosomes and Analysis

Equivalent amounts of exosomes from wild-type and ATG5^{-/-} MDA-MB-231 were trypsin digested and analyzed by mass spectrometry. Total proteins in exosomes were analyzed using Ingenuity Pathway Analysis (Qiagen). Proteins defined as being involved in cancer cell invasion and epithelial-mesenchymal transition, were selected and Pathway network analysis was performed using GeneMania. Proteins enriched greater than 2-fold in exosomes from wild-type cells (vs.exosomes from ATG5^{-/-} MDA-MB-231) in both independent samples were selected as being enriched in wild-type exosomes. Proteins with experimentally defined LC3-binding LIR motifs were derived from (Birgisdottir et al., 2013). LC3 and known LIR-motif proteins that were detected in exosomes were analyzed by GeneMania to model a network of putative Physical Interactions of LC3 binding proteins in exosomes.

### Analysis of GFP p62 sorting into exosomes

Anti-CD63 antibody (H5C6, Developmental Studies Hybridoma Bank) was adhered to latex beads using an established protocol (Ostrowski et al., 2010). Cells were transfected with plasmids 2 days before cell supernatants were centrifuged at 400g (5 min), 2000 g (10 min), filtered through a 0.22 µM filter and incubated with anti-CD63 labeled beads overnight as described (Ostrowski et al., 2010). Beads were incubated with anti-PrP-PE before analysis by flow cytometry. Total amount of GFP fluorescence on beads was normalized to fluorescence of PrP-PE antibody (total exosomes).

### Statistics

Two-tailed t-tests or ANOVA were employed to evaluate statistical significance, p≤0.05 was considered as significant. SPSS V21.0 was used for statistical analysis.

### EXAMPLE 3 - LC3 Constructs, Exosome Packaging, and Degradation of Neurodegenerative Disease-Related Cellular Targets by Autophagy

Exosomes as described herein were further investigated for delivering drugs and/or active agents, such as antibodies, from the blood into the brain, and effects thereof were tested. In this Example, LC3 constructs were developed, packaged into exosomes, and delivered to brain cells where degradation of neurodegenerative disease-related cellular targets via autophagy was assessed.

Extracellular vesicles of ~100 nm in size, called exosomes, may transfer drugs including, for example, RNA, peptides, and/or proteins, into the brain of pre-clinical animal models. Exosomes may be generated by inward budding of late endosomes to create vesicles inside late endosomes, which are then released to the extracellular space when these late endosomes (or multivesicular bodies) fuse with the plasma membrane. This may result in exosomes with cytoplasmic contents in their interior and plasma membrane receptors on their surface. The receptors on the exosome surface may enable targeting to specific tissues. Exosomes may fuse with cells to deliver their interior contents, which may include RNA and/or protein, into the cytoplasm. Exosomes may cross the blood-brain barrier. For example, certain exosomes from the brain have been found in blood of patients, and certain exosomes packaged with siRNA may reduce target expression in the mouse brain. Certain exosomes packaged with siRNA and administered by IV may accumulate in the brain parenchyma beyond CD31+ blood vessels and may reduce expression of siRNA targets there (see Figure 39, showing (Top left) exosomes labeled with DiR and injected IV accumulate in the brain and meninges as measured by IVIS preclinical imaging of harvested tissues; (Top Right) After IV injection exosomes labeled with PKH67 fluorescence dye and carrying GFP siRNA are detected in brain parenchyma beyond the CD31+ endothelium by confocal microscopy; (Bottom left) Detection of siRNA specific to exosomes by FISH in brain parenchyma (beyond CD31+ endothelium after IV injection of exosomes; Bottom right (Quantification of siRNA in brain parenchyma by FISH after IV injection; and (Bottom middle) Quantification of loss of GFP fluorescence and GFP mRNA in cortex after IV injection of exosomes packaged with GFp siRNA or control siRNA).

In the present Example, the inventors recognized that traditional antibodies may be somewhat poorly adapted to eliminate certain targets such as, for example, large aggregates of misfolded proteins. Antibodies which access misfolded proteins in the extracellular space may instigate phagocytosis and elimination of misfolded proteins by microglia. For the few traditional-type antibodies which actually enter into cells, how they can degrade misfolded proteins or large inclusions in cells not entirely clear. It is believed that traditional antibodies inside cells may degrade some ligands when the cytoplasmic protein TRIM21 binds the antibody Fc region. TRIM21 recognition of antibodies activates innate immune responses; however, this would be risky in neurodegenerative diseases such as ALS, Parkinson's, and Alzheimer's, where inflammation and innate immune response are increasingly thought to be a driving force behind pathology. Triggering TRIM21 signaling with antibodies may risk increasing immune responses which is generally undesirable.

In contrast, the present inventors hypothesized that autophagy may eliminate large aggregates in neurodegenerative diseases without inflammation. TRIM21 has been reported to instigate degradation of antibodies and their ligands predominantly via the proteasome. The proteasome degrades single proteins, but its capacity to eliminate oligomers, large aggregates, or inclusions containing additional types of molecules like lipid or RNA is less clear. In contrast, a mechanism to eliminate large, complex substrates from cells is autophagy. In autophagy, a new membrane is formed that grows within minutes to engulf and enclose cytoplasmic contents into the newly formed autophagosome. The autophagosome then fuses with the lysosome to degrade its contents. Autophagy is believed to involve a cascade of >35 Autophagy (Atg) proteins including Atg7, Atg5 and Atg16L121. LC3 is a major component of the forming autophagosome membrane. From this position, it binds autophagy receptors using LC3-Interacting Regions (LIR) to identify and recruit substrates from the cytoplasm into the forming autophagosome for degradation. LC3 is important for the degradation of large complex substrates like misfolded protein aggregates in neurodegenerative diseases or lipid droplets in metabolic diseases. Mice with disabled autophagy in neurons accumulate protein aggregates and die of severe neurodegeneration, while experimentally activating autophagy may eliminate protein aggregates in models of several neurodegenerative diseases. For this reason, autophagy-activating drugs are actively sought as therapeutics for neurodegeneration, with a goal of eliminating misfolded proteins without activating immune responses.

In this Example, the present inventors designed modified antibody constructs, packaged them into exosomes, and tested these compositions for degrading cellular targets involved in neurodegenerative diseases by autophagy.

A select group of proteins and RNAs from the cytoplasm are packaged into exosomes, while most other cytoplasmic components are found only at background levels. The present inventors discovered that the autophagy protein LC3 is highly enriched in exosomes, independent of autophagy (Atg7^{-/-}), as shown in Figure 28 A-B. Figure 28 A shows that LC3-II was enriched in exosomes, as indicated by Western blot of equal amounts of lysate from exosomes (Exo) or cells. Figure 28 B shows that LC3-I is packaged into exosomes made by autophagy-deficient Atg7^{-/-} cells, like LC3-II in exosomes made by WT cells. Data indicated that fusing diverse proteins like luciferase, dsRed to LC3 may induce ~400-fold more packaging of the proteins into exosomes versus simple over-expression approaches (see Figure 28 C-E). Figure 28 C shows luciferase light measured in exosomes compared to its level in cells (fusion to LC3 packages about 500x more cellular LC3 into exosomes). Figure 28 D and E show Western blots of exosomes transfected with Luciferase or Luciferase-LC3 fusion (D), or dsRed or dsRed-LC3 fusion (E). Fusion of LC3 to intrabodies, antibodies engineered for intracellular expression (scFv, nanobodies etc.), also resulted in packaging of intrabodies into exosomes. In Figure 29 A, anti-TDP-43 scFv antibodies were fused to LC3, and the Antibody-LC3 construct was packaged into exosomes efficiently. Figure 29 A shows expression of scFv-LC3 fusion constructs and packaging of the constructs into exosomes. The scFv in this experiment was anti-TDP-43 VH7 antibody scFv. Detection of the fusion was by Western blot with anti-LC3 antibody or antibody vs. HA tag incorporated into the linker region in the fusion. Figure 29 B shows that the Antibody-LC3 construct binds to TDP-43 antigen target. Figure 29 C shows that the antibody-LC3 construct was degraded by autophagy, while antibody alone was not (bafilomycin used in this test inhibits autophagy). As shown in Figure 29 C, IgG^{d}-LC3 fusion versus TDP-432, but not IgG^{d}, accumulates when autophagy is inhibited with Bafilomycin. In Figure 29 C, expression of scFv-LC3 construct in cells was achieved using lentivirus. These results demonstrate that if such constructs are expressed by virus (i.e. lentivirus, adeno-associated virus, or oncolytic virus), they may be degraded by autophagy, since they accumulate in cells treated with Bafilomycin which is an inhibitor of autophagy. In Figure 29 C, cells were expressing anti-TDP-43 antibody scFv clone Vh7. Figure 29 D shows that IgG^{d}-LC3 vs TDP-43 is packaged in exosomes. The inventors hypothesized that exosomes may deliver intrabody-LC3 chimeras with high efficiency to tissues like liver, small intestine and kidney (IV), and if injected (for example) into the spinal cord (as ASOs or AAV in patients in neurodegeneration), throughout the spinal cord and the brain. Once intrabody-LC3 enters target cells, it may act like an autophagy receptor: the intrabody may bind with high specificity to its target while LC3 may recruit the autophagy machinery to engulf and degrade the bound target (see Figure 29 C and Figure 30). Intrabody-LC3 fusions may be targeted for degradation by autophagy, while intrabody alone is not (see Figure 29 C), indicating the potential for intrabody-LC3 to degrade targets by autophagy. Such constructs may recognize misfolded proteins with remarkable specificity using antibodies or derivatives thereof, and may induce their degradation, without an inflammatory response, via autophagy.

Without wishing to be bound by theory, and solely for illustrative purposes intended for the person of skill in the art, Figure 30 shows a proposed model for targeted autophagy, in which an active agent, such as an antibody or derivate thereof (such as an antigen-binding fragment or scFV, for example), is fused with an LC3 protein sequence (or portion thereof), and packaged into exosomes by expression in stable cell lines producing exosomes. In the depicted model, the cells used for producing the packaged exosomes are lacking or depleted of protein such as Atg7, so as to be capable of packaging LC3 fusions into exosomes while being substantially incapable of autophagy so as to avoid degradation of the LC3 fusions during exosome production. As shown in the Figure, exosomes may be generated by inward budding of late endosomes to create vesicles inside late endosomes, which may then be released to the extracellular space when these late endosomes (or multivesicular bodies) fuse with the plasma membrane. This may result in exosomes with cytoplasmic contents in their interior and plasma membrane receptors on their surface. The receptors on the exosome surface may enable targeting to specific tissues. The so-produced exosomes are obtained/isolated/purified from the cells, and then administered to a cell or subject in need thereof. In the depicted model, the packaged exosomes are administered to a mouse by IV or IT administration as shown. The administered exosomes may fuse with cells in the mouse (for example, brain cells) to deliver their interior contents (i.e. the LC3-antibody fusion), which may further include RNA and/or protein, into the cytoplasm, where the antibody portion of the fusion construct binds the cellular target (such as TDP-43 inclusion in this model). Binding to the cellular target results in the LC3 portion of the fusion construct triggering degradation of the cellular target via autophagy through recruitment of LC3 to autophagosomes.

Figure 31 shows expression of scFv-LC3 fusion constructs in cells using lentivirus. These results demonstrate that if these constructs are expressed by virus (e.g. lentivirus, adeno-associated virus, or oncolytic virus), they are degraded by autophagy, since they accumulate in Atg7 knockout cells. Shown in this Figure are anti-Tau clone 4E4 antibody scFv and anti-TDP-43 clone VH3 antibody scFv constructs.

Next, exosome-based delivery of anti-TDP-43/LC3 fusion construct was tested for degradation of TDP-43+ ALS substrates by autophagy. Figure 32 shows experiments in which scFv-LC3 constructs targeting TDP-43 (clone VH7) were stably expressed in cells using lentiviral transduction. Exosomes were purified from the media of these cells and added onto primary mouse mixed motor neurons. Two or six hours later the levels of cytoplasmic TDP43 was measured by Western blot, or the number of stress granules remaining in cells was quantified. These results demonstrate that exosomes packaged with scFV-LC3 conjugates can deliver the constructs into target cells and decrease levels of target proteins and large substrates (i.e. about 200nm - about 10um stress granules in this example) which contain those targets. In (A), a Western blot of primary mixed motor neurons is shown, where scFv-anti-TDP-43-LC3 fusion delivered by exosomes reduced levels of TDP-43 in cells. In (B), quantification by microscopy of stress granules in primary mixed motor neurons treated with arsenite for 1 hour is shown. Cells were fixed 30 minutes after removal or arsenite. scFv-anti-TDP-43-LC3 fusion delivered by exosomes reduced G3BP1+ stress granules in cells (stress granules also contain TDP-43). Figure 33 A-B shows that the Antibody-LC3 fusion construct eliminated cellular aggregates linked to ALS much better than antibody alone. Stress granules are TDP-43+ precursors of aggregates/inclusions in ALS. As shown in Figure 33, the number of stress granules per cell was much lower following treatment with anti-TDP-43/LC3 fusion versus anti-TDP-43 and GFP control. In Figure 33, scFv-LC3 fusion targeted TDP-43 (clone VH7) was expressed in HeLa cells treated with arsenite for 30 min to induce stress granules and left to begin eliminating stress granules by removing arsenite for 30 min. Stress granules contain TDP-43 and are a cellular model or homologue of aggregates in the neurodegenerative disease Amyotrophic Lateral Sclerosis (ALS) which contain TDP-43 in about 95% of patients. Anti-TDP-43-LC3 fusion exhibited increased elimination of stress granules (G3BP1) in cells versus those transfected with GFP (control) or anti-TDP-43 scFv alone. These results demonstrate that the LC3 tag of the fusion increased the elimination of substrates like stress granules.

Results indicate that exosomal delivery of active agents (such as, but not limited to, antibodies or derivatives thereof) fused with LC3 may allow for autophagy-based degradation of undesirable disease-related targets such as, but not limited to, misfolded protein, protein inclusion, and/or protein aggregate associated with a neurodegenerative disease or disorder such as, but not limited to, Alzheimer's, Parkinson's, Frontal Temporal Dementia and/or Amyotrophic Lateral Sclerosis (ALS). These results indicate advantages versus traditional antibody approaches (for example, antibodies are typically ineffective at targeting intracellular targets, and do not achieve degradation by autophagy and may result in inflammatory responses), and yet still benefit from high-specificity (especially versus traditional small-molecule approaches) and reduced risk of certain off-target toxicities associated with small-molecule and/or siRNA approaches. Because targeted autophagy may be employed, large aggregates may be targeted for degradation, which are not easily addressed by traditional proteasome approaches, for example.

### Materials and Methods

Studies performed include those in which HcRed, Luciferase (an enzyme), and three different scFv antibody derivative-containing fusion constructs were prepared. One scFv recognizes Tau (clone 4E4), and two recognize TDP-43 (clone VH7 and 41D1).

In terms of Anti-scFv sequences, the following were used:
(a) Anti-TDP43 scFv clone VH7 was used, which was derived from the constructs described in WO2016/086320, entitled "TDP-43-Binding Polypeptides Useful for the Treatment of Neurodegenerative Diseases",
(b) Anti-TDP-43 scFv 41D1 was derived from the sequence of NI-205.41D1 as described in US 9,587,014**,**
(c) Anti-Tau scFv 4A3 was derived from sequence NI-105.4A3 as described in US 2012/0087861 - Human Anti-Tau Antibodies,
(d) Anti-Tau scFv 4E4 was derived from sequence NI-105.4A3 as described in US 2012/0087861A1 - Human Anti-Tau Antibodies,

- For scFv in (b-d), scFv were constructed from the heavy and light chains of antibodies described in the identified references. The heavy and light chains from these references were fused by a standard S(GGGGS)₃ linker. These sequences were then fused upstream of another linker (GSAGSAAGSGEF (SEQ ID NO: 11); Waldo et al., Nat Biotech 17:691-695), an HA tag, and the LC3a coding sequence, such that translation produces a fusion protein consisting of the heavy and light scFv chains, the HA tag, and LC3a.

Nucleic acids encoding the fusion constructs were stably transduced into cells using lentivirus to generate stable expression using the CMV promoter. Cells were cultured in Lonza Ultraculture medium for 24 h and exosomes were harvested by tangential flow filtration concentration and dialysis on a 750 kDa pore size. Exosomes were then incubated with target cells between 2-24 h. After this time point cells were lysed and levels of cytoplasmic TDP-43 were measured by western blot after elimination of nuclei. In other experiments stress granules were induced by treatment of cells with arsenite for 30 min and then arsenite was removed for 30 min and cells were fixed for immunofluorescence to measure the persistence of stress granules using antibodies targeting G3BP1, PABP or DDX3. The number of stress granules were compared to fusions like scFv VH7 with and without LC3. In some experiments cells were pre-treated for 2-24 h with mTOR inhibitors to induce autophagy.

Nucleic acid and amino acid sequences relating to certain fusion constructs used in studies performed include the following:

### HcRed-LC3b

Nucleotide sequence: 
atggtgagcggcctgctgaaggagagtatgcgcatcaagatgtacatggagggcaccgtgaacggccactacttcaagtgcgagggcga
gggcgacggcaacccccttcgccggcacccagagcatgagaatccacgtgaccgagggcgccccctgcccttcgccttcgacatcctgg
ccccctgctgcgagtacggcagcaggaccttcgtgcaccacaccgccgagatccccgacttcttcaagcagagcttccccgagggcttca
cctgggagagaaccaccacctacgaggacggcggcatcctgaccgcccaccaggacaccagcctggagggcaactgcctgatctacaa
ggtgaaggtgcacggcaccaacttccccgccgacggccccgtgatgaagaacaagagcggcggctgggagcccagcaccgaggtggt
gtaccccgagaacggcgtgctgtgcggccggaacgtgatggccctgaaggtgggcgaccggcacctgatctgccaccactacaccagct
accggagcaagaaggccgtgcgcgccctgaccatgcccggcttccacttcaccgacatccggctccagatgctgcggaagaagaagga
cgagtacttcgagctgtacgaggccagcgtggcccggtacagcgacctgcccgagaaggccaactccggactcagatctatgccgtcgg
agaagaccttcaagcagcgccgcaccttcgaacaaagagtagaagatgtccgacttattcgagagcagcatccaaccaaaatcccggtga
taatagaacgatacaagggtgagaagcagcttcctgttctggataaaacaaagttccttgtacctgaccatgtcaacatgagtgagctcatca
agataattagaaggcgcttacagctcaatgctaatcaggccttcttcctgttggtgaacggacacagcatggtcagcgtctccacaccaatct
cagaggtgtatgagagtgagaaagatgaagatggattcctgtacatggtctacgcctcccaggagacgttcgggatgaaattgtcagtgtaa

(SEQ ID NO: 37)
Amino acid sequence:

MVSGLLKESMRIKMYMEGTVNGHYFKCEGEGDGNPFAGTQSMRIHVTEGAPLPFAFDI
LAPCCEYGSRTFVHHTAEIPDFFKQSFPEGFTWERTTTYEDGGILTAHQDTSLEGNCLIY
KVKVHGTNFPADGPVMKNKSGGWEPSTEVVYPENGVLCGRNVMALKVGDRHLICHH
YTSYRSKKAVRALTMPGFHFTDIRLQMLRKKKDEYFELYEASVARYSDLPEKANSGLRS
MPSEKTFKQRRTFEQRVEDVRLIREQHPTKIPVIIERYKGEKQLPVLDKTKFLVPDHVNM
SELIKIIRRRLQLNANQAFFLLVNGHSMVSVSTPISEVYESEKDEDGFLYMVYASQETFG
MKLSV*

(SEQ ID NO: 38)

### scFv-LC3 fusion constructs

The scFv-LC3 fusion constructs all had the following pattern of sequence regions:
[scFv region] - [linker sequence¹ and HA tag] - [LC3 variant]
¹ - this linker is a validated sequence Nature Biotechnology volume 17, pages 691-695 (1999),
Linker sequence and HA tag protein sequence:
   GSAGSAAGSGEFYPYDVPDYA
   (SEQ ID NO: 39)
LC3 variant sequences:
   LC3a protein sequence: 
SMPSDRPFKQRRSFADRCKEVQQIRDQHPSKIPVIIERYKGEKQLPVLDKTKFLVPDHVN
MSELVKIIRRRLQLNPTQAFFLLVNQHSMVSVSTPIADIYEQEKDEDGFLYMVYASQETF
GF*
(SEQ ID NO: 40)
LC3b protein sequence: 
SMPSEKTFKQRRTFEQRVEDVRLIREQHPTKIPVIIERYKGEKQLPVLDKTKFLVPDHVN
MSELIKIIRRRLQLNANQAFFLLVNGHSMVSVSTPISEVYESEKDEDGFLYMVYASQETF
GMKLSV*
(SEQ ID NO: 41)
LC3c protein sequence: 
SMPPPQKIPSVRPFKQRKSLAIRQEEVAGIRAKFPNKIPVVVERYPRETFLPPLDKTKFLVP
QELTMTQFLSIIRSRMVLRATEAFYLLVNNKSLVSMSATMAEIYRDYKDEDGFVYMTY
ASQETFGCLESAAPRDGSSLEDRPCNPL*
(SEQ ID NO: 42)
LC3a nucleotide sequence: agcATGCCATCAGATAGGCCGTTCAAACAACGAAGATCTTTCGCGGATAGATGCAAG 
GAAGTACAACAAATTAGGGACCAGCACCCTTCCAAAATACCCGTCATCATTGAACG
CTACAAGGGGGAGAAGCAGCTCCCAGTGCTTGACAAGACGAAATTTCTCGTTCCCG
ACCACGTCAACATGTCTGAGTTGGTAAAGATCATCCGAAGGCGACTCCAGCTCAAC
CCTACACAAGCCTTTTTCTTGCTCGTCAACCAACACTCAATGGTGTCAGTATCCACG
CCCATCGCTGACATCTACGAACAAGAAAAAGACGAGGATGGATTCCTGTATATGGT
ATATGCCTCTCAGGAAACATTCGGCTTCtaa

(SEQ ID NO: 43)
LC3b nucleotide sequence: 
AGCATGCCGAGCGAAAAACTTTTAAGCAGAGGAGGACGTTTGAACAGAGAGTAGA
AGACGTGCGCCTTATCCGCGAACAACATCCCACTAAGATCCCGGTCATAATTGAACG
ATATAAGGGGGAAAAACAACTGCCAGTGCTGGACAAAACGAAATTTCTTGTCCCAG
ATCATGTCAACATGAGTGAGTTGATAAAAATCATTCGACGGCGACTGCAACTCAATG
CAAACCAGGCGTTCTTCCTTCTTGTTAATGGGCATAGCATGGTCTCTGTATCTACGCC
TATATCCGAAGTGTACGAGTCAGAGAAAGATGAAGATGGATTCTTGTATATGGTATA
CGCAAGTCAGGAAACGTTCGGAATGAAGTTGAGTGTGtaa

(SEQ ID NO: 44)
LC3c nucleotide sequence:

AGCATGCCACCTCCTCAAAAAATTCCGAGTGTGCGACCTTTCAAGCAAAGAAAGTCT
TTGGCTATTCGACAGGAGGAGGTTGCAGGTATCCGGGCTAAGTTCCCGAACAAAAT
CCCAGTTGTGGTGGAACGATACCCGAGGGAAACGTTCCTGCCCCCTTTGGACAAGA
CGAAATTCCTTGTGCCCCAAGAACTCACTATGACCCAGTTCCTGAGTATAATACGGT
CCCGCATGGTGCTCCGAGCAACGGAGGCCTTCTATCTCCTTGTTAACAACAAGTCAT
TGGTATCCATGTCTGCTACCATGGCAGAAATCTACCGGGATTACAAAGACGAAGAC
GGTTTTGTCTACATGACTTACGCATCCCAGGAAACTTTTGGTTGCCTGGAAAGCGCG
GCACCAAGGGATGGCAGTTCCCTTGAAGACAGGCCGTGCAATCCGCTCtaa

(SEQ ID NO: 45)

NOTE, LC3 nucleotide sequences were identical between constructs. The linker nucleotide sequences were not identical as some were modified to allow synthesis, nevertheless the amino acid sequence of all the linker-HA tag sequences was identical.

### VH7VK9-LC3a

Nucleotide sequence: 
atggccCTcCAGCAGTCTGGAGGAGGCTTGGTGCAACCTGGAGGcTCCATGAAACTCTC
CTGTGTTGCCTCTGGATTCACTTCCAGTAACTACTGGTTGAACTGGGTCCGCCAGTCT
CCAGAGAGGGGGCTTGAGTGGGTTGCTGAAATTAGATTGAAATCTAATAATTATGC
AACAAATTATGCGGAGTCTGTGAAAGGGAGGTTCACCATCTCAAGAGACGATTCCA
AAAGTAGTGTCTACCTGCAAGTGAACAACTTAAGAGCTGAAGACACTGGCATTTATT
ACTGTACCAGGTCAACAGCTCGGGCTACCCCATACTACTTTGACTACTGGGGCCAAG
GGACCACGGTCACCTccggaggcggcggctctggcggcggcggctccggaggcggcggctctGAGCTCACCC
AGTCTCCATCCTCCCTGGCTGTGTCAGCCGGAGAGAAGGTCACTATGAGCTGCAAAT
CCAGTCAGAGTCTGCTCAACAGTAGAGCCCGAAAGAACTTCTTGACTTGGTACCAGC
AGAAACCAGGGCAGTCTCCTAAATTGCTGATCTATTGGGCATCCACTAGGGAATCTG
GGGTCCCTGATCGCTTCACAGGCAGTGGATCTGGGACAGATTTCACTCTCACCATCA
GCAGTGTGCAGGCTGAAGACCTGGCAGTTTATTACTGCAAACAGTCTTATAATCTGT
ACACGTTCGGAGGGGGCACCAAGCTCGAGGGGTCCGCTGGCAGTGCCGCAGGGTCT
GGCGAATTTTACCCATACGATGTTCCAGATTACGCTagcATGCCATCAGATAGGCCGT
TCAAACAACGAAGATCTTTCGCGGATAGATGCAAGGAAGTACAACAAATTAGGGAC
CAGCACCCTTCCAAAATACCCGTCATCATTGAACGCTACAAGGGGGAGAAGCAGCT
CCCAGTGCTTGACAAGACGAAATTTCTCGTTCCCGACCACGTCAACATGTCTGAGTT
GGTAAAGATCATCCGAAGGCGACTCCAGCTCAACCCTACACAAGCCTTTTTCTTGCT
CGTCAACCAACACTCAATGGTGTCAGTATCCACGCCCATCGCTGACATCTACGAACA
AGAAAAAGACGAGGATGGATTCCTGTATATGGTATATGCCTCTCAGGAAACATTCG
GCTTCtaa

(SEQ ID NO: 46)
Protein sequence:

MALQQSGGGLVQPGGSMKLSCVASGFTSSNYWLNWVRQSPERGLEWVAEIRLKSNNY
ATNYAESVKGRFTISRDDSKSSVYLQVNNLRAEDTGIYYCTRSTARATPYYFDYWGQG
TTVTSGGGGSGGGGSGGGGSELTQSPSSLAVSAGEKVTMSCKSSQSLLNSRARKNFLTW
YQQKPGQSPKLLIYWASTRESGVPDRFTGSGSGTDFTLTISSVQAEDLAVYYCKQSYNL
YTFGGGTKLEGSAGSAAGSGEFYPYDVPDYASMPSDRPFKQRRSFADRCKEVQQIRDQ
HPSKIPVIIERYKGEKQLPVLDKTKFLVPDHVNMSELVKIIRRRLQLNPTQAFFLLVNQHS
MVSVSTPIADIYEQEKDEDGFLYMVYASQETFGF*

(SEQ ID NO: 47)

### VH7VK9-LC3b

Nucleotide sequence: 
atggccCTcCAGCAGTCTGGAGGAGGCTTGGTGCAACCTGGAGGcTCCATGAAACTCTC
CTGTGTTGCCTCTGGATTCACTTCCAGTAACTACTGGTTGAACTGGGTCCGCCAGTCT
CCAGAGAGGGGGCTTGAGTGGGTTGCTGAAATTAGATTGAAATCTAATAATTATGC
AACAAATTATGCGGAGTCTGTGAAAGGGAGGTTCACCATCTCAAGAGACGATTCCA
AAAGTAGTGTCTACCTGCAAGTGAACAACTTAAGAGCTGAAGACACTGGCATTTATT
ACTGTACCAGGTCAACAGCTCGGGCTACCCCATACTACTTTGACTACTGGGGCCAAG
GGACCACGGTCACCTccggaggcggctctggcggcggctccggaggcggctctGAGCTCACCC
AGTCTCCATCCTCCCTGGCTGTGTCAGCCGGAGAGAAGGTCACTATGAGCTGCAAAT
CCAGTCAGAGTCTGCTCAACAGTAGAGCCCGAAAGAACTTCTTGACTTGGTACCAGC
AGAAACCAGGGCAGTCTCCTAAATTGCTGATCTATTGGGCATCCACTAGGGAATCTG
GGGTCCCTGATCGCTTCACAGGCAGTGGATCTGGGACAGATTTCACTCTCACCATCA
GCAGTGTGCAGGCTGAAGACCTGGCAGTTTATTACTGCAAACAGTCTTATAATCTGT
ACACGTTCGGAGGGGGCACCAAGCTCGAGGGGTCCGCTGGCAGTGCCGCAGGGTCT
GGCGAATTTTACCCATACGATGTTCCAGATTACGCTAGCATGCCGAGCGAAAAACT
TTTAAGCAGAGGAGGACGTTTGAACAGAGAGTAGAAGACGTGCGCCTTATCCGCGA
ACAACATCCCACTAAGATCCCGGTCATAATTGAACGATATAAGGGGGAAAAACAAC
TGCCAGTGCTGGACAAAACGAAATTTCTTGTCCCAGATCATGTCAACATGAGTGAGT
TGATAAAAATCATTCGACGGCGACTGCAACTCAATGCAAACCAGGCGTTCTTCCTTC
 
TTGTTAATGGGCATAGCATGGTCTCTGTATCTACGCCTATATCCGAAGTGTACGAGT
CAGAGAAAGATGAAGATGGATTCTTGTATATGGTATACGCAAGTCAGGAAACGTTC
GGAATGAAGTTGAGTGTGtaa

(SEQ ID NO: 48)
Protein sequence:

MALQQSGGGLVQPGGSMKLSCVASGFTSSNYWLNWVRQSPERGLEWVAEIRLKSNNY
ATNYAESVKGRFTISRDDSKSSVYLQVNNLRAEDTGIYYCTRSTARATPYYFDYWGQG
TTVTSGGGGSGGGGSGGGGSELTQSPSSLAVSAGEKVTMSCKSSQSLLNSRARKNFLTW
YQQKPGQSPKLLIYWASTRESGVPDRFTGSGSGTDFTLTISSVQAEDLAVYYCKQSYNL
YTFGGGTKLEGSAGSAAGSGEFYPYDVPDYASMPSEKTFKQRRTFEQRVEDVRLIREQH
PTKIPVIIERYKGEKQLPVLDKTKFLVPDHVNMSELIKIIRRRLQLNANQAFFLLVNGHSM
VSVSTPISEVYESEKDEDGFLYMVYASQETFGMKLSV*

(SEQ ID NO: 49)

### VH7VK9-LC3c

Nucleotide sequence: 
atggccCTcCAGCAGTCTGGAGGAGGCTTGGTGCAACCTGGAGGcTCCATGAAACTCTC
CTGTGTTGCCTCTGGATTCACTTCCAGTAACTACTGGTTGAACTGGGTCCGCCAGTCT
CCAGAGAGGGGGCTTGAGTGGGTTGCTGAAATTAGATTGAAATCTAATAATTATGC
AACAAATTATGCGGAGTCTGTGAAAGGGAGGTTCACCATCTCAAGAGACGATTCCA
AAAGTAGTGTCTACCTGCAAGTGAACAACTTAAGAGCTGAAGACACTGGCATTTATT
ACTGTACCAGGTCAACAGCTCGGGCTACCCCATACTACTTTGACTACTGGGGCCAAG
GGACCACGGTCACCTccggaggcggcggctctggcggcggcggctccggaggcggcggctctGAGCTCACCC
AGTCTCCATCCTCCCTGGCTGTGTCAGCCGGAGAGAAGGTCACTATGAGCTGCAAAT
CCAGTCAGAGTCTGCTCAACAGTAGAGCCCGAAAGAACTTCTTGACTTGGTACCAGC
AGAAACCAGGGCAGTCTCCTAAATTGCTGATCTATTGGGCATCCACTAGGGAATCTG
GGGTCCCTGATCGCTTCACAGGCAGTGGATCTGGGACAGATTTCACTCTCACCATCA
 
GCAGTGTGCAGGCTGAAGACCTGGCAGTTTATTACTGCAAACAGTCTTATAATCTGT
ACACGTTCGGAGGGGGCACCAAGCTCGAGGGGTCCGCTGGCAGTGCCGCAGGGTCT
GGCGAATTTACCCATACGATGTTCCAGATTACGCTAGCATGCCACCTCCTCAAAAA
ATTCCGAGTGTGCGACCTTTCAAGCAAAGAAAGTCTTTGGCTATTCGACAGGAGGAG
GTTGCAGGTATCCGGGCTAAGTTCCCGAACAAAATCCCAGTTGTGGTGGAACGATAC
CCGAGGGAAACGTTCCTGCCCCCTTTGGACAAGACGAAATTCCTTGTGCCCCAAGAA
CTCACTATGACCCAGTTCCTGAGTATAATACGGTCCCGCATGGTGCTCCGAGCAACG
GAGGCCTTCTATCTCCTTGTTAACAACAAGTCATTGGTATCCATGTCTGCTACCATGG
CAGAAATCTACCGGGATTACAAAGACGAAGACGGTTTTGTCTACATGACTTACGCAT
CCCAGGAAACTTTTGGTTGCCTGGAAAGCGCGGCACCAAGGGATGGCAGTTCCCTTG
AAGACAGGCCGTGCAATCCGCTCtaa

(SEQ ID NO: 50)
Protein sequence:

MALQQSGGGLVQPGGSMKLSCVASGFTSSNYWLNWVRQSPERGLEWVAEIRLKSNNY
ATNYAESVKGRFTISRDDSKSSVYLQVNNLRAEDTGIYYCTRSTARATPYYFDYWGQG
TTVTSGGGGSGGGGSGGGGSELTQSPSSLAVSAGEKVTMSCKSSQSLLNSRARKNFLTW
YQQKPGQSPKLLIYWASTRESGVPDRFTGSGSGTDFTLTISSVQAEDLAVYYCKQSYNL
YTFGGGTKLEGSAGSAAGSGEFYPYDVPDYASMPPPQKIPSVRPFKQRKSLAIRQEEVA
GIRAKFPNKIPVVVERYPRETFLPPLDKTKFLVPQELTMTQFLSIIRSRMVLRATEAFYLL
VNNKSLVSMSATMAEIYRDYKDEDGFVYMTYASQETFGCLESAAPRDGSSLEDRPCNP
L*

(SEQ ID NO: 51)

### 41D1-LC3a

Nucleotide sequence: 
atggccGAAGTTCAGCTTGTTGAAAGTGGGGGCGGCCTGGTTCAACCCGGCGGTTCCCT
TCGGCTGTCATGCGCTGCATCTGGCTTTACATTCTCAACCTATTACATGTCTTGGGTT
 
CGCCAGGCTCCTGGCAAAGGACTTGAGTGGGTTGCAAATATCAAGCAAGATGGTTC
CGAGAAATACTACGTGGATTCTGTCAAAGGCAGATTCACTATTTCTCGGGACAACGC
ACGGAATAGTCTTTACTTGCAGATGCACTCCCTTAGGGCAGAAGACACAGCGGTCTA
CTACTGTGCATCTCCTCCAGGATGGTGGGACAAGGAACTCTTGTTACTGTTTCTTCT
TCCGGAGGAGGAGGAAGCGGAGGAGGAGGATCCGGAGGAGGCGGATCTGATATTG
TAATGACGCAAACTCCACTCAGTCTCTCAGTTACTCCTGGGCAGCCTGCGTCTATCT
CCTGCAAATCTAGCCAATCATTGCTGCACAGTGACGGCAAAACGTACTTGTATTGGT
ATCTcCAGAAGCCTGGTCAGCCACCACAGCTCTTGATTTATGAGGTTTCTAATAGGTT
TAGTGGCGTACCTGACAGGTTTTCTGGTTCAGGGTCAGGGACGGATTTCACCCTTAA
GATTTCAAGAGTAGAGGCGGAAGACGTAGGTGTGTACTACTGCATGCAGTCTATCC
AACTTCCCGTCACTTTTGGCGGTGGGACCAAAGTTGAGATAAAAGGGTCCGCTGGCA
GTGCCGCAGGGTCTGGCGAATTTTACCCATACGATGTTCCAGATTACGCTagcATGCC
ATCAGATAGGCCGTTCAAACAACGAAGATCTTTCGCGGATAGATGCAAGGAAGTAC
AACAAATTAGGGACCAGCACCCTTCCAAAATACCCGTCATCATTGAACGCTACAAG
GGGGAGAAGCAGCTCCCAGTGCTTGACAAGACGAAATTTCTCGTTCCCGACCACGT
CAACATGTCTGAGTTGGTAAAGATCATCCGAAGGCGACTCCAGCTCAACCCTACAC
AAGCCTTTTTCTTGCTCGTCAACCAACACTCAATGGTGTCAGTATCCACGCCCATCG
CTGACATCTACGAACAAGAAAAAGACGAGGATGGATTCCTGTATATGGTATATGCC
TCTCAGGAAACATTCGGCTTCtaa

(SEQ ID NO: 52)
Protein sequence:

MAEVQLVESGGGLVQPGGSLRLSCAASGFTFSTYYMSWVRQAPGKGLEWVANIKQDG
SEKYYVDSVKGRFTISRDNARNSLYLQMHSLRAEDTAVYYCASPPGWWGQGTLVTVSS
SGGGGSGGGGSGGGGSDIVMTQTPLSLSVTPGQPASISCKSSQSLLHSDGKTYLYWYLQ
KPGQPPQLLIYEVSNRFSGVPDRFSGSGSGTDFTLKISRVEAEDVGVYYCMQSIQLPVTF
GGGTKVEIKGSAGSAAGSGEFYPYDVPDYASMPSDRPFKQRRSFADRCKEVQQIRDQHP
SKIPVIIERYKGEKQLPVLDKTKFLVPDHVNMSELVKIIRRRLQLNPTQAFFLLVNQHSM
VSVSTPIADIYEQEKDEDGFLYMVYASQETFGF*

(SEQ ID NO: 53)

### 4E4-LC3a

Nucleotide sequence: 
atggccTCCTATGAGCTGACTCAGCCACCCTCGGTGTCAGTGTCCCCAGGACAGACGGC
CAGGATCTCCTGCTTTGGAGATACATTGCCAAAGCAATATACTTATTGGTATCAGCA
GAAGCCTGGCCAGGCCCCTGTGTTAGTGATTTATAAAGACACTGAGAGGCCCTCAG
GcATCCCCGAGCGATTCTCTGGCTCCAGCTCAGGGACAACAGTCACCTTGACCATCA
GTGGAGTCCAGGCAGAAGACGAGGCTGACTATTACTGTCTATCAGCTGACAATTCTG
CTACATGGGTTTTTGGAGGAGGAACAAAAGTTACAGTCCTCTCCGGAGGAGGTGGA
TCTGGCGGTGGCGGTTCCGGAGGTGGAGGATCTGAAGTTCAATTGGTTGAATCTGGA
GGAGGCTTGGTTCAACCTGGAGGATCTCTGAAACTCTCTTGTGCTGCTTCTGGATTC
AATTTCAACATCTCTGCTATACACTGGGTCCGCCAGGCTTCCGGGAAAGGGCTGGAG
TGGGTTGGCCGAATAAGAAGTAAATCTCACAATTACGCGACTTTATATGCTGCGTCC
CTGAAAGGCCGGTTCACCCTCTCCAGAGATGATTCAAGGAACACGGCGTATCTGCA
AATGAGCAGCCTGCAAACCGAGGATATGGCCGTCTATTACTGTACTGTTCTGAGTGC
GAATTACGACACCTTTGACTACTGGGGCCAGGGAACCCTGGTCACCGTCTCCTCGGG
GTCCGCTGGCAGTGCCGCAGGGTCTGGCGAATTTACCCATACGATGTTCCAGATTA
CGCTagcATGCCATCAGATAGGCCGTTCAAACAACGAAGATCTTTCGCGGATAGATGC
AAGGAAGTACAACAAATTAGGGACCAGCACCCTTCCAAAATACCCGTCATCATTGA
ACGCTACAAGGGGGAGAAGCAGCTCCCAGTGCTTGACAAGACGAAATTTCTCGTTC
CCGACCACGTCAACATGTCTGAGTTGGTAAAGATCATCCGAAGGCGACTCCAGCTC
AACCCTACACAAGCCTTTTTCTTGCTCGTCAACCAACACTCAATGGTGTCAGTATCC
ACGCCCATCGCTGACATCTACGAACAAGAAAAAGACGAGGATGGATTCCTGTATAT
GGTATATGCCTCTCAGGAAACATTCGGCTTCtaa

(SEQ ID NO: 54)
Protein sequence:

MASYELTQPPSVSVSPGQTARISCFGDTLPKQYTYWYQQKPGQAPVLVIYKDTERPSGIP

ERFSGSSSGTTVTLTISGVQAEDEADYYCLSADNSATWVFGGGTKVTVLSGGGGSGGG
GSGGGGSEVQLVESGGGLVQPGGSLKLSCAASGFNFNISAIHWVRQASGKGLEWVGRIR
SKSHNYATLYAASLKGRFTLSRDDSRNTAYLQMSSLQTEDMAVYYCTVLSANYDTFDY
WGQGTLVTVSSGSAGSAAGSGEFYPYDVPDYASMPSDRPFKQRRSFADRCKEVQQIRD
QHPSKIPVIIERYKGEKQLPVLDKTKFLVPDHVNMSELVKIIRRRLQLNPTQAFFLLVNQH
SMVSVSTPIADIYEQEKDEDGFLYMVYASQETFGF*

(SEQ ID NO: 55)

### HJ8.5-LC3a

Nucleotide sequence: 
atggccGACATTGTGCTGACACAGTCTCCTGCTTCCTTAGCTGTATCTCTGGGACAGAG
GGCCACCATCTCATGCAGGGCCAGCCAAAGTGTCAGTACATCTAGATATAGTTATAT
ACACTGGTACCAACAGAAACCAGGACAGCCACCCAAACTCCTCATCAAGTATGCAT
CCAACCTAGAATCTGGGGTCCCTGCCAGGTTCAGTGGCAGTGGGTCTGGGACAGACT
TCACCCTCAACATCCATCCTCTGGAGGAGGAGGATGCTGCAACATATTACTGTCATC
ATTCTTGGGAAATTCCTTTGACATTTGGAGCTGGAACAAAATTGGAATTGAAATCCG
GAGGAGGTGGATCTGGAGGTGGAGGATCCGGAGGTGGAGGATCTGAAGTTAAGGTT
GAGGAGTCTGGAGGAGGCTTGGTGCAACCTGGAGGcTCCATGAAACTCTCCTGTGTT
GTCTCTGGATTCACTTTCAGTAACTACTGGGTGAACTGGGTCCGCCAGTCTCCAGAG
AAGGGGCTTGAGTGGGTTGCTCAAATTAGATTGAAATCTGATAATTATGCAACACAT
TATGAGGAGTCTGTGAAAGGGAGGTTCACCATCTCAAGAGATGATTCCAAAAGTAG
TGTCTATCTGCAAATGAACAACCTAAGGGCTGAAGACAGTGGAATTTATTACTGCAC
TAACTGGGAAGACTACTGGGGCCAAGGCACCACTCTCACAGTCTCCTCAGGGTCCG
CTGGCAGTGCCGCAGGGTCTGGCGAATTTACCCATACGATGTTCCAGATTACGCTag
cATGCCATCAGATAGGCCGTTCAAACAACGAAGATCTTTCGCGGATAGATGCAAGGA
AGTACAACAAATTAGGGACCAGCACCCTTCCAAAATACCCGTCATCATTGAACGCT
ACAAGGGGGAGAAGCAGCTCCCAGTGCTTGACAAGACGAAATTTCTCGTTCCCGAC
CACGTCAACATGTCTGAGTTGGTAAAGATCATCCGAAGGCGACTCCAGCTCAACCCT
ACACAAGCCTTTTTCTTGCTCGTCAACCAACACTCAATGGTGTCAGTATCCACGCCC
 
ATCGCTGACATCTACGAACAAGAAAAAGACGAGGATGGATTCCTGTATATGGTATA
TGCCTCTCAGGAAACATTCGGCTTCtaa

(SEQ ID NO: 56)
Protein sequence:

MADIVLTQSPASLAVSLGQRATISCRASQSVSTSRYSYIHWYQQKPGQPPKLLIKYASNL
ESGVPARFSGSGSGTDFTLNIHPLEEEDAATYYCHHSWEIPLTFGAGTKLELKSGGGGSG
GGGSGGGGSEVKVEESGGGLVQPGGSMKLSCVVSGFTFSNYWVNWVRQSPEKGLEWV
AQIRLKSDNYATHYEESVKGRFTISRDDSKSSVYLQMNNLRAEDSGIYYCTNWEDYWG
QGTTLTVSSGSAGSAAGSGEFYPYDVPDYASMPSDRPFKQRRSFADRCKEVQQIRDQHP
SKIPVIIERYKGEKQLPVLDKTKFLVPDHVNMSELVKIIRRRLQLNPTQAFFLLVNQHSM
VSVSTPIADIYEQEKDEDGFLYMVYASQETFGF*

(SEQ ID NO: 57)

Accordingly, in another embodiment, there is provided herein a fusion construct, protein, peptide, nucleic acid, or expression vector, having at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity with any one of SEQ ID NOs: 37-57, or with any other protein, peptide, or nucleic acid described herein.

### EXAMPLE 4 - Comparison of LC3 Sequences and GABARAP Proteins Between Human and Mice

In Example 3 above, the majority of fusion constructs tested included a functional moiety comprising human LC3 protein, either LC3A or LC3B. It will be understood that LC3 has various homologues and/or isoforms and is found in various different mammals, and it is contemplated that these homologues and/or isoforms and/or species variants may also be used in fusion constructs as described herein. Furthermore, given the sequence variations found among closely related LC3 homologues, it is contemplated that LC3 derivatives and/or mimics having sequence variation from natural LC3 sequence may also be used. In this Example, LC3 homologue sequences were analyzed to determine sequence variations found among human LC3A, human LC3B, human LC3C, mouse LC3A, mouse LC3B, human GABARAPL1, human GABARAPL2, mouse GABARAPL1, and mouse GABARAPL2. It is contemplated that all may be modified similarly from a -I to a -II form in appropriate cells, and that C-terminus cleavage may not be a significant factor in this consideration.

Amino acid sequences of these homologues are shown below: SEQ ID NO: 1
>NP_115903.1 microtubule-associated proteins 1A/1B light chain 3A isoform a [Homo sapiens] (human LC3A) 
MPSDRPFKQRRSFADRCKEVQQIRDQHPSKIPVIIERYKGEKQLPVLDKTKFLVPDHVNM
SELVKIIRRRLQLNPTQAFFLLVNQHSMVSVSTPIADIYEQEKDEDGFLYMVYASQETFG
F
 SEQ ID NO: 2
>NP_073729.1 microtubule-associated proteins 1A/1B light chain 3B [Homo sapiens] (human LC3B) 
MPSEKTFKQRRTFEQRVEDVRLIREQHPTKIPVIIERYKGEKQLPVLDKTKFLVPDHVNM
SELIKIIRRRLQLNANQAFFLLVNGHSMVSVSTPISEVYESEKDEDGFLYMVYASQETFG
MKLSV
 SEQ ID NO: 3
>NP_001004343.1 microtubule-associated proteins 1A/1B light chain 3C [Homo sapiens] (human LC3C) 
MPPPQKIPSVRPFKQRKSLAIRQEEVAGIRAKFPNKIPVVVERYPRETFLPPLDKTKFLVP
QELTMTQFLSIIRSRMVLRATEAFYLLVNNKSLVSMSATMAEIYRDYKDEDGFVYMTY
ASQETFGCLESAAPRDGSSLEDRPCNPL
 SEQ ID NO: 4
>NP_080011.1 microtubule-associated proteins 1A/1B light chain 3A [Mus musculus] (mouse LC3A) 
MPSDRPFKQRRSFADRCKEVQQIRDQHPSKIPVIIERYKGEKQLPVLDKTKFLVPDHVNM
SELVKIIRRRLQLNPTQAFFLLVNQHSMVSVSTPIADIYEQEKDEDGFLYMVYASQETFG
F
 SEQ ID NO: 5
>NP_080436.1 microtubule-associated proteins 1A/1B light chain 3B isoform 1 [Mus musculus] (mouse LC3B) 
MPSEKTFKQRRSFEQRVEDVRLIREQHPTKIPVIIERYKGEKQLPVLDKTKFLVPDHVNM
SELIKIIRRRLQLNANQAFFLLVNGHSMVSVSTPISEVYESERDEDGFLYMVYASQETFGT
AMAV
 SEQ ID NO: 6
>NP_001350527.1 gamma-aminobutyric acid receptor-associated protein-like 1 isoform 1 [Homo sapiens]
   (human GABARAPL1) 
MKFQYKEDHPFEYRKKEGEKIRKKYPDRVPVIVEKAPKARVPDLDKRKYLVPSDLTVG
QFYFLIRKRIHLRPEDALFFFVNNTIPPTSATMGQLYEVMVLVAQYWMPSSAVWHPLAL
VLDALITHLRSGAEGVIYPDPLTYGSVRL
 SEQ ID NO: 7
>NP_009216.1 gamma-aminobutyric acid receptor-associated protein-like 2 [Homo sapiens] (human GABARAPL2) 
MKWMFKEDHSLEHRCVESAKIRAKYPDRVPVIVEKVSGSQIVDIDKRKYLVPSDITVAQ
FMWIIRKRIQLPSEKAIFLFVDKTVPQSSLTMGQLYEKEKDEDGFLYVAYSGENTFGF
 SEQ ID NO: 8
>NP_065615.1 gamma-aminobutyric acid receptor-associated protein-like 1 [Mus musculus] (mouse GABARAPL1) 
MKFQYKEDHPFEYRKKEGEKIRKKYPDRVPVIVEKAPKARVPDLDKRKYLVPSDLTVG
QFYFLIRKRIHLRPEDALFFFVNNTIPPTSATMGQLYEDNHEEDYFLYVAYSDESVYGK
 SEQ ID NO: 9
>NP_080969.2 gamma-aminobutyric acid receptor-associated protein-like 2 [Mus musculus] (mouse GABARAPL2)

MKWMFKEDHSLEHRCVESAKIRAKYPDRVPVIVEKVSGSQIVDIDKRKYLVPSDITVAQ
FMWIIRKRIQLPSEKAIFLFVDKTVPQSSLTMGQLYEKEKDEDGFLYVAYSGENTFGF

An alignment of some LC3 homologue sequences of human and mouse is set out below. The present inventors have identified that human LC3A, LC3B, LC3C, mouse LC3A, and mouse LC3B are all found in exosomes. From alignment and sequence analysis, it appears that amino acid sequence conservation of about 50% or more from any of the LC3 homologues may be sufficient for packaging into exosomes. Considering GABARAP proteins, which are LC3 homologues, the sequence variability increases even further.

### Alignment and identity matrix:

*MAP1LC3A (LC3A) Homo sapiens*
*MAP1LC3B (LC3B) Homo sapiens*
*MAP1LC3C (LC3C) Homo sapiens*
*MAP1LC3A (LC3A) Mus musculus*
*MAP1LC3B (LC3B) Mus musculus*

Percent Identity Matrix - created by Clustal2.1 # #

| | | | | | | |
|---|---|---|---|---|---|---|
| 1: | NP_001004343.1 | 100.00 | 58.68 | 58.68 | 53.60 | 52.80 |
| 2: | NP_115903.1 | 58.68 | 100.00 | 100.00 | 81.82 | 81.82 |
| 3: | NP_080011.1 | 58.68 | 100.00 | 100.00 | 81.82 | 81.82 |
| 4: | NP_073729.1 | 53.60 | 81.82 | 81.82 | 100.00 | 95.20 |
| 5: | NP_080436.1 | 52.80 | 81.82 | 81.82 | 95.20 | 100.00 |

| | | |
|---|---|---|
| NP_001004343.1 | MPPPQKIPSVRPFKQRKSLAIRQEEVAGIRAKFPNKIPWVERYPRETFLPPLDKTKFLV | 60 |
| NP_115903.1 | ------MPSDRPFKQRRSFADRCKEVQQIRDQHPSIUPVIIERYKGEKOLPVLDKTKFLV | 54 |
| NP_080011.1 | ------MPSDRPFKQRRSFADRCKEVQQIRDQHPSKIPVIIERYKGEKQLPVLDKTKFLV | 54 |
| NP_073729.1 | ------MPSEKTFKQRRTFEQRVEDVRLIREQHPTKIPVIlERYKGEKQLPVLDKTKFLV | 54 |
| NP_080436.1 | ------MPSEKTFKQRRS FEQRVE DVRLIREQHPTKI PVI lERYKGEKQLPVLDKTKFLV | 54 |
| | :** : ****::: * ::* ** :.*.****::*** *. ** ******** | |

| | | |
|---|---|---|
| NP_001004343.1 | PQELTMTQFLSIIRSRMVLRATEAFYLLVNNKSLVSMSATMAEIYRDYKDEDGFVYMTYA | 120 |
| NP_115903.1 | PDHVNMSELVKIIRRRLQLNPTQAFFLLVNQHSMVSVSTPIADIYEQEKDEDGFLYMVYA | 114 |
| NP_080011.1 | PDHVNMSELVKIIRRRLQLNPTQAFFLLVNQHSMVSVSTPIADIYEQEKDEDGFLYMVYA | 114 |
| NP_073729.1 | PDHVMMSELIKIIRRRLQLNANQAFFLLVMGHSMVSVSTPISEVYESEKDEDGFLYMVYA | 114 |
| NP_080436.1 | PDHVNMSELIKIIRRRLQLNANQAFFLLVNGHSMVSVSTPISEVYESERDEDGFLYMVYA | 114 |
| | *:.:.*::::.*** *: *. .:**:**** :*:**:*: ::::*.. :*****:**.** | |

| | | |
|---|---|---|
| NP_001004343.1 | SQETFGCLESAAPRDGSSLEDRPCNPL | 147 |
| NP_115903.1 | SQETFGF-------------------- | 121 |
| NP_080011.1 | SQETFGF-------------------- | 121 |
| NP_073729.1 | SQETFGMKLSV---------------- | 125 |
| NP_080436.1 | SQETFGTAMAV---------------- | 125 |
| | ****** | |

As shown, from alignment and sequence analysis, it appears that amino acid sequence conservation of about 50% or more from any of the LC3 homologues may be sufficient for packaging into exosomes.

Considering GABARAP proteins, which are LC3 homologues, the sequence variability increases even further. Alignments and identity matrix is shown below.

### Alignment and identity matrix:

*MAP1LC3A (LC3A) Homo sapiens*
*MAP1LC3B (LC3B) Homo sapiens*
*MAP1LC3C (LC3C) Homo sapiens*
*MAP1LC3A (LC3A) Mus musculus*
*MAP1LC3B (LC3B) Mus musculus*
*GABARAPL1 Homo sapiens*
*GABARAPL2 Homo sapiens*
*GABARAPL1 Mus musculus*
*GABARAPL2 Mus musculus*

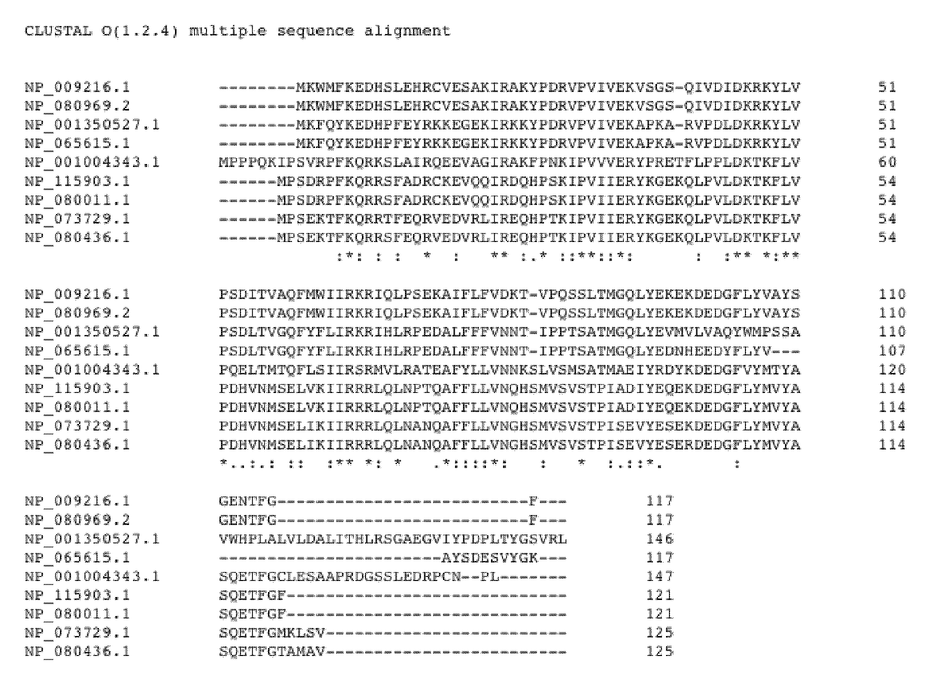

Percent Identity Matrix - created by Clustal2.1

| | | | | | | | | | |
|---|---|---|---|---|---|---|---|---|---|
| 1: KP_009216.1 | 100.00 | 100.00 | 51.28 | 61.11 | 41.38 | 40.52 | 40.52 | 38.79 | 38.79 |
| 2: NP_080969.2 | 100.00 | 100.00 | 51.28 | 61.11 | 41.38 | 40.52 | 40.52 | 38.79 | 38.79 |
| 3: NP_001350527.1 | 51.28 | 51.28 | 100.00 | 85.47 | 31.39 | 28.21 | 28.21 | 26.45 | 25.62 |
| β: NP_065615.1 | 61.11 | 61.11 | 85.47 | 100.00 | 38.74 | 34.58 | 34.58 | 32.71 | 32.71 |
| 5: KP_001004343.1 | 41.38 | 41.38 | 31.39 | 38.74 | 100.00 | 58.68 | 58.68 | 53.60 | 52.80 |
| 6: NP_115903.1 | 40.52 | 40.52 | 28.21 | 34.58 | 58.68 | 100.00 | 100.00 | 81.82 | 81.82 |
| 7: NP_080011.1 | 40.52 | 40.52 | 28.21 | 34.58 | 58.68 | 100.00 | 100.00 | 81.82 | 81.82 |
| 8: NP_073729.1 | 38.79 | 38.79 | 26.45 | 32.71 | 53.60 | 81.82 | 81.82 | 100.00 | 95.20 |
| 9: NP_080436.1 | 38.79 | 38.79 | 25.62 | 32.71 | 52.80 | 81.82 | 81.82 | 95.20 | 100.00 |

These results indicate that sequence variation from the amino acid sequence of a given natural LC3 homologue may still allow for exosome packaging, for example.

### EXAMPLE 5 - LC3A, LC3B, and LC3C Fusion Construct Expression, and Packaging of Various scFv-LC3 Fusion Constructs/Conjugates into Exosomes

Experiments were performed to assess LC3A, LC3B, and LC3C fusion construct/conjugate expression in cells, and to assess packaging of various scFv-LC3 fusion constructs/conjugates into exosomes.

Figure 34 shows results indicating that scFv may be conjugated to any of LC3A, LC3B, or LC3C and be expressed in cells. In this Figure, anti-TDP-43 close VH7 is shown. In Figure 35, scFv conjugated to LC3 are expressed at similar levels in cells as scFv which are unconjugated. The data are for 3 scFvs fused to LC3 derived from anti-TDP-43 VH7, anti-TDP-43, 41D1, and anti-Tau 4E4.

Figure 36 shows results indicating that fusion constructs of scFv conjugated to LC3 were expressed at similar levels in cells as scFv that were unconjugated. The data are for 3 scFv fused to LC3 derived from anti-TDP-43 VH7, anti-TDP-43 41D1, and anti-Tau 4E4. Figure 37 shows that various fusion constructs of scFv conjugated to LC3 were packaged into exosomes produced by multiple cell types. Cells were transduced with scFv-LC3 conjugates, and exosomes were purified from these and analyzed by Western blot. The data are for 3 scFv fused to LC3 derived from anti-TDP-43 41D1, anti-Tau HJ, and anti-Tau 4E4. Flotillin2 is a marker of exosomes.

### EXAMPLE 6 - Fusion Constructs Used in Combination with an Autophagy-Activating Agent Increased Targeted Autophagy, and Increased Elimination of Anti-TDP-43 VH7 scFv Targets (Stress Granules)

Experiments were performed to assess whether elimination of anti-TDP-43 VH7 scFv targets by autophagy, triggered by scFv-LC3 fusion constructs, could be further enhanced by treatment with an autophagy-activating agent. In this example, mTOR inhibitor INK128 was used as the autophagy-activating agent.

Cells were transfected with scFv versus TDP-43 fused to LC3, and treated or not treated with the mTOR inhibitor INK128 to assess autophagy activation. Stress granules were induced by arsenite treatment for 1 h and then removed to allow cells to recover for 30 min before fixation and staining for stress granules (G3BP1+). Results are shown in Figure 38 A. As indicated, the % of stress granules remaining in the scFv-LC3 fusion construct (VH7 clone) treatment condition was beneficially improved by treatment with INK128 inhibitor. INK128 inhibitor alone did not have a measureable effect in the % stress granules remaining, suggesting that the effect observed with the scFv-LC3 fusion construct + INK128 condition was more than an additive effect. Representative images are shown in Figure 38 B.

These results indicate that autophagy-activating agents (i.e. mTOR inhibitor INK128 in this example), such as mTOR inhibitors or other autophagy-activating drugs, may be used to increase elimination of fusion construct targets, such as binding targets of scFv-LC3 fusion constructs.

One or more illustrative embodiments have been described by way of example. It will be understood to persons skilled in the art that a number of variations and modifications can be made without departing from the scope of the invention as defined in the claims.
Alvarez-Erviti, L., Seow, Y., Schapira, A.H., Gardiner, C., Sargent, I.L., Wood, M.J.A., and Cooper, J.M. (2011). Lysosomal dysfunction increases exosome-mediated alpha-synuclein release and transmission. Neurobiol. Dis. 42, 360-367.
Baietti, M.F., Zhang, Z., Mortier, E., Melchior, A., Degeest, G., Geeraerts, A., Ivarsson, Y., Depoortere, F., Coomans, C., Vermeiren, E., et al. (2012). Syndecan-syntenin-ALIX regulates the biogenesis of exosomes. Nat. Cell Biol. 14, 677-685.
Bento, C.F., Renna, M., Ghislat, G., Puri, C., Ashkenazi, A., Vicinanza, M., Menzies, F.M., and Rubinsztein, D.C. (2016). Mammalian autophagy: how does it work? Annu. Rev. Biochem. 85, 685-713.
Cadwell, K., Liu, J.Y., Brown, S.L., Miyoshi, H., Loh, J., Lennerz, J.K., Kishi, C., Kc, W., Carrero, J.A., Hunt, S., et al. (2008). A key role for autophagy and the autophagy gene Atg1611 in mouse and human intestinal Paneth cells. Nature 456, 259-263.
Chang, T.-K., Shravage, B. V, Hayes, S.D., Powers, C.M., Simin, R.T., Wade Harper, J., and Baehrecke, E.H. (2013). Uba1 functions in Atg7- and Atg3-independent autophagy. Nat. Cell Biol. 15, 1067-1078.
Colombo, M., Moita, C., van Niel, G., Kowal, J., Vigneron, J., Benaroch, P., Manel, N., Moita, L.F., Thery, C., Raposo, G., et al. (2013). Analysis of ESCRT functions in exosome biogenesis, composition and secretion highlights the heterogeneity of extracellular vesicles. J. Cell Sci. 126, 5553-5565.
Colombo, M., Raposo, G., and Thery, C. (2014). Biogenesis, Secretion, and Intercellular Interactions of Exosomes and Other Extracellular Vesicles. Annu. Rev. Cell Dev. Biol 30, 255-289.
Cotter, K., Stransky, L., McGuire, C., and Forgac, M. (2015). Recent Insights into the Structure, Regulation, and Function of the V-ATPases. Trends Biochem. Sci. 40, 611-622.
Florey, 0., Kim, S.E., Sandoval, C.P., Haynes, C.M., and Overholtzer, M. (2011). Autophagy machinery mediates macroendocytic processing and entotic cell death by targeting single membranes. Nat. Cell Biol. 13, 1335-1343.
Futter, C.E., Collinson, L.M., Backer, J.M., and Hopkins, C.R. (2001). Human VPS34 is required for internal vesicle formation within multivesicular endosomes. J. Cell Biol. 155, 1251-1263.
Gross, J.C., Chaudhary, V., Bartscherer, K., and Boutros, M. (2012). Active Wnt proteins are secreted on exosomes. Nat. Cell Biol. 14, 1036-1045.
Hamacher-Brady, A., Brady, N.R., Logue, S.E., Sayen, M.R., Jinno, M., Kirshenbaum, L.A., Gottlieb, R.A., and Gustafsson, A.B. (2007). Response to myocardial ischemia/reperfusion injury involves Bnip3 and autophagy. Cell Death Differ. 14, 146-157.
Hanson, P.I., and Cashikar, A. (2012). Multivesicular Body Morphogenesis. Annu. Rev. Cell Dev. Biol. 28, 337-362.
Hoshino, A., Costa-Silva, B., Shen, T.-L., Rodrigues, G., Hashimoto, A., Tesic Mark, M., Molina, H., Kohsaka, S., Di Giannatale, A., Ceder, S., et al. (2015). Tumour exosome integrins determine organotropic metastasis. Nature 527, 329-335.
Hosokawa, N., Hara, Y., and Mizushima, N. (2006). Generation of cell lines with tetracycline-regulated autophagy and a role for autophagy in controlling cell size. FEBS Lett. 580, 2623-2629.
Huotari, J., and Helenius, A. (2011). Endosome maturation. EMBO J. 30, 3481-3500.
Hurley, J.H., and Hanson, P.I. (2010). Membrane budding and scission by the ESCRT machinery: it's all in the neck. Nat. Rev. Mol. Cell Biol. 11, 556-566.
Johansen, T., Birgisdottir, B., Huber, J., Kniss, A., D??tsch, V., Kirkin, V., and Rogov, V. V. (2017). Methods for Studying Interactions Between Atg8/LC3/GABARAP and LIR-Containing Proteins. In Methods in Enzymology, pp. 143-169.
Kalvari, I., Tsompanis, S., Mulakkal, N.C., Osgood, R., Johansen, T., Nezis, I.P., and Promponas, V.J. (2014). iLIR: A web resource for prediction of Atg8-family interacting proteins. Autophagy 10, 913-925.
Kenific, C.M., Wittmann, T., and Debnath, J. (2016). Autophagy in adhesion and migration. J. Cell Sci. jcs.188490.
Kosaka, N., Yoshioka, Y., Fujita, Y., and Ochiya, T. (2016). Versatile roles of extracellular vesicles in cancer. J. Clin. Invest. 126, 1163-1172.
Ktistakis, N.T., and Tooze, S.A. (2016). Digesting the Expanding Mechanisms of Autophagy. Trends Cell Biol. 26, 624-635.
Lee, H.H., Elia, N., Ghirlando, R., Lippincott-Schwartz, J., and Hurley, J.H. (2008). Midbody Targeting of the ESCRT Machinery by a Noncanonical Coiled Coil in CEP55. Science (80-. ). 322, 576-580.
Lee, Y., Jun, Y., Choi, H., Huh, Y.H., Kaang, B., Jang, D., and Lee, J. (2017). Development of LC3/GABARAP sensors containing a LIR and a hydrophobic domain to monitor autophagy. EMBO J. e201696315.
Lotvall, J., Hill, A.F., Hochberg, F., Buzas, E.I., Vizio, D. Di, Gardiner, C., Gho, Y.S., Kurochkin, I. V., Mathivanan, S., Quesenberry, P., et al. (2014). Minimal experimental requirements for definition of extracellular vesicles and their functions: A position statement from the International Society for Extracellular Vesicles. J. Extracell. Vesicles 3.
Martinez, J., Malireddi, R.K.S., Lu, Q., Cunha, L.D., Pelletier, S., Gingras, S., Orchard, R., Guan, J.-L., Tan, H., Peng, J., et al. (2015). Molecular characterization of LC3-associated phagocytosis reveals distinct roles for Rubicon, NOX2 and autophagy proteins. Nat. Cell Biol. 17, 893-906.
McKnight, N.C., Zhong, Y., Wold, M.S., Gong, S., Phillips, G.R., Dou, Z., Zhao, Y., Heintz, N., Zong, W.X., and Yue, Z. (2014). Beclin 1 Is Required for Neuron Viability and Regulates Endosome Pathways via the UVRAG-VPS34 Complex. PLoS Genet. 10.
MCinz, C. (2016). The macroautophagy machinery in endo- and exocytosis. J. Mol. Biol.
Murrow, L., Malhotra, R., and Debnath, J. (2015). ATG12-ATG3 interacts with Alix to promote basal autophagic flux and late endosome function. Nat. Cell Biol. 17, 300-310.
Nishida, Y., Arakawa, S., Fujitani, K., Yamaguchi, H., Mizuta, T., Kanaseki, T., Komatsu, M., Otsu, K., Tsujimoto, Y., and Shimizu, S. (2009). Discovery of Atg5/Atg7-independent alternative macroautophagy. Nature 461, 654-658.
Ostrowski, M., Carmo, N.B., Krumeich, S., Fanget, I., Raposo, G., Savina, A., Moita, C.F., Schauer, K., Hume, A.N., Freitas, R.P., et al. (2010). Rab27a and Rab27b control different steps of the exosome secretion pathway. Nat. Cell Biol. 12, 19-30-13.
Pankiv, S., Clausen, T.H., Lamark, T., Brech, A., Bruun, J.A., Outzen, H., Overvatn, A., Bjorkoy, G., and Johansen, T. (2007). p62/SQSTM1 binds directly to Atg8/LC3 to facilitate degradation of ubiquitinated protein aggregates by autophagy* [S]. J. Biol. Chem. 282, 24131-24145.
Parolini, I., Federici, C., Raggi, C., Lugini, L., Palleschi, S., De Milito, A., Coscia, C., lessi, E., Logozzi, M., Molinari, A., et al. (2009). Microenvironmental pH is a key factor for exosome traffic in tumor cells. J. Biol. Chem. 284, 34211-34222.
Peng, J.Y., Zhang, R., Cui, Y.T., Liu, H.D., Zhao, X.X., Huang, L., Hu, M.X., Yuan, X.X., Ma, B.Y., Ma, X.W., et al. (2014). Atg5 regulates late endosome and lysosome biogenesis. Sci. China Life Sci. 57,59-68.
Puchner, E.M., Walter, J.M., Kasper, R., Huang, B., and Lim, W.A. (2013). Counting molecules in single organelles with superresolution microscopy allows tracking of the endosome maturation trajectory. Proc. Natl. Acad. Sci. 110, 16015-16020.
Ra, E.A., Lee, T.A., Won Kim, S., Park, A., Choi, H. jin, Jang, I., Kang, S., Hee Cheon, J., Cho, J.W., Eun Lee, J., et al. (2016). TRIM31 promotes Atg5/Atg7-independent autophagy in intestinal cells. Nat. Commun. 7, 11726.
Razi, M.M.R., and Futter, C.E. (2006). Distinct Roles for Tsg101 and Hrs in Multivesicular Body Formation and Inward Vesiculation. Mol. Biol. Cell 125, 351-369.
Rizzo, M.A., Davidson, M.W., and Piston, D.W. (2009). Fluorescent protein tracking and detection: Fluorescent protein structure and color variants. Cold Spring Harb. Protoc. 4.
Romao, S., Gasser, N., Becker, A.C., Guhl, B., Bajagic, M., Vanoaica, D., Ziegler, U., Roesler, J., Dengjel, J., Reichenbach, J., et al. (2013). Autophagy proteins stabilize pathogen-containing phagosomes for prolonged MHC II antigen processing. J. Cell Biol. 203, 757-766.
Russell, R.C., Yuan, H.-X., and Guan, K.-L. (2014). Autophagy regulation by nutrient signaling. Cell Res. 24, 42-57.
Sahu, R., Kaushik, S., Clement, C.C., Cannizzo, E.S., Scharf, B., Follenzi, A., Potolicchio, I., Nieves, E., Cuervo, A.M., and Santambrogio, L. (2011). Microautophagy of Cytosolic Proteins by Late Endosomes. Dev. Cell 20, 131-139.
Sanjuan, M.A., Dillon, C.P., Tait, S.W.G., Moshiach, S., Dorsey, F., Connell, S., Komatsu, M., Tanaka, K., Cleveland, J.L., Withoff, S., et al. (2007). Toll-like receptor signalling in macrophages links the autophagy pathway to phagocytosis. Nature 450, 1253-1257.
Sautin, Y.Y., Lu, M., Gaugler, A., Zhang, L., and Gluck, S.L. (2005). Phosphatidylinositol 3-kinase-mediated effects of glucose on vacuolar H+-ATPase assembly, translocation, and acidification of intracellular compartments in renal epithelial cells. Mol. Cell. Biol. 25, 575-589.
Sharifi, M.N., Mowers, E.E., Drake, L.E., Collier, C., Chen, H., Zamora, M., Mui, S., and Macleod, K.F. (2016). Autophagy Promotes Focal Adhesion Disassembly and Cell Motility of Metastatic Tumor Cells through the Direct Interaction of Paxillin with LC3. Cell Rep. 15, 1660-1672.
Sharma, D.K., Choudhury, A., Singh, R.D., Wheatley, C.L., Marks, D.L., and Pagano, R.E. (2003). Glycosphingolipids internalized via caveolar-related endocytosis rapidly merge with the clathrin pathway in early endosomes and form microdomains for recycling. J. Biol. Chem. 278, 7564-7572.
Shrivastava, S., Devhare, P., Sujijantarat, N., Steele, R., Kwon, Y.-C., Ray, R., and Ray, R.B. (2016). Knockdown of Autophagy Inhibits Infectious Hepatitis C Virus Release by the Exosomal Pathway. J. Virol. 90, 1387-1396.
Taelman, V.F., Dobrowolski, R., Plouhinec, J.L., Fuentealba, L.C., Vorwald, P.P., Gumper, I., Sabatini, D.D., and De Robertis, E.M. (2010). Wnt signaling requires sequestration of Glycogen Synthase Kinase 3 inside multivesicular endosomes. Cell 143, 1136-1148.
Tanida, I., Yamaji, T., Ueno, T., Ishiura, S., Kominami, E., and Hanada, K. (2008). Consideration about negative controls for LC3 and expression vectors for four colored fluorescent protein-LC3 negative controls. Autophagy 4,131-134.
Tao, K., Fang, M., Alroy, J., and Sahagian, G.G. (2008). Imagable 4T1 model for the study of late stage breast cancer. BMC Cancer 8, 228.
Tauro, B.J., Greening, D.W., Mathias, R.A., Mathivanan, S., Ji, H., and Simpson, R.J. (2013). Two Distinct Populations of Exosomes Are Released from LIM1863 Colon Carcinoma Cell-derived Organoids. Mol. Cell. Proteomics 12, 587-598.
Thierry, Thery, C., Amigorena, S., Raposo, G., and Clayton, A. (2006). Isolation and Characterization of Exosomes from Cell Culture Supernatants. Curr. Protoc. Cell Biol. Chapter 3,1-29.
Toei, M., Saum, R., and Forgac, M. (2010). Regulation and isoform function of the V-ATPases. Biochemistry 49, 4715-4723.
Trajkovic, K., Hsu, C., Chiantia, S., Rajendran, L., Wenzel, D., Wieland, F., Schwille, P., Brugger, B., and Simons, M. (2008). Ceramide Triggers Budding of Exosome Vesicles into Multivesicular Endosomes. Science (80-. ). 319, 1244-1247.
Vidal, M., Mangeat, P., and Hoekstra, D. (1997). Aggregation reroutes molecules from a recycling to a vesicle- mediated secretion pathway during reticulocyte maturation. J. Cell Sci. 110 ( Pt 1, 1867-1877.
Wang, D., and Hiesinger, P.R. (2013). The vesicular ATPase: A missing link between acidification and exocytosis. J. Cell Biol. 203, 171-173.
Wemmer, M., Azmi, I., West, M., Davies, B., Katzmann, D., and Odorizzi, G. (2011). Bro1 binding to Snf7 regulates ESCRT-III membrane scission activity in yeast. J. Cell Biol. 192, 295-306.
Zhou, J., Tan, S.-H., Nicolas, V., Bauvy, C., Yang, N.-D., Zhang, J., Xue, Y., Codogno, P., and Shen, H.-M. (2013). Activation of lysosomal function in the course of autophagy via mTORC1 suppression and autophagosome-lysosome fusion. Cell Res. 23, 508-523.
Aguzzi, A., and Lakkaraju, A.K. (2015). Cell Biology of Prions and Prionoids: A Status Report. Trends Cell Biol.
Alvarez-Erviti, L., Seow, Y., Schapira, A.H., Gardiner, C., Sargent, I.L., Wood, M.J., and Cooper, J.M. (2011). Lysosomal dysfunction increases exosome-mediated alpha-synuclein release and transmission. Neurobiol Dis 42, 360-367.
Asai, H., Ikezu, S., Tsunoda, S., Medalla, M., Luebke, J., Haydar, T., Wolozin, B., Butovsky, 0., Kugler, S., and Ikezu, T. (2015). Depletion of microglia and inhibition of exosome synthesis halt tau propagation. Nat Neurosci 18, 1584-1593.
Baietti, M.F., Zhang, Z., Mortier, E., Melchior, A., Degeest, G., Geeraerts, A., Ivarsson, Y., Depoortere, F., Coomans, C., Vermeiren, E., et al. (2012). Syndecan-syntenin-ALIX regulates the biogenesis of exosomes. Nat Cell Biol 14, 677-685.
Behrends, C., Sowa, M.E., Gygi, S.P., and Harper, J.W. (2010). Network organization of the human autophagy system. Nature 466, 68-76.
Berg, T.O., Fengsrud, M., Stromhaug, P.E., Berg, T., and Seglen, P.O. (1998). Isolation and characterization of rat liver amphisomes. Evidence for fusion of autophagosomes with both early and late endosomes. J Biol Chem 273, 21883-21892.
Birgisdottir, A.B., Lamark, T., and Johansen, T. (2013). The LIR motif - crucial for selective autophagy. J Cell Sci 126, 3237-3247.
Bjorkoy, G., Lamark, T., Brech, A., Outzen, H., Perander, M., Overvatn, A., Stenmark, H., and Johansen, T. (2005). p62/SQSTM1 forms protein aggregates degraded by autophagy and has a protective effect on huntingtin-induced cell death. J Cell Biol 1 7/, 603-614.
Brettschneider, J., Del Tredici, K., Lee, V.M., and Trojanowski, J.Q. (2015). Spreading of pathology in neurodegenerative diseases: a focus on human studies. Nat Rev Neurosci 16, 109-120.
Burrell, R.A., McGranahan, N., Bartek, J., and Swanton, C. (2013). The causes and consequences of genetic heterogeneity in cancer evolution. Nature 501, 338-345.
Colombo, M., Moita, C., van Niel, G., Kowal, J., Vigneron, J., Benaroch, P., Manel, N., Moita, L.F., Thery, C., and Raposo, G. (2013). Analysis of ESCRT functions in exosome biogenesis, composition and secretion highlights the heterogeneity of extracellular vesicles. J Cell Sci 126, 5553-5565.
Colombo, M., Raposo, G., and Thery, C. (2014). Biogenesis, secretion, and intercellular interactions of exosomes and other extracellular vesicles. Annu Rev Cell Dev Biol 30, 255-289.
Danzer, K.M., Kranich, L.R., Ruf, W.P., Cagsal-Getkin, 0., Winslow, A.R., Zhu, L., Vanderburg, C.R., and McLean, P.J. (2012). Exosomal cell-to-cell transmission of alpha synuclein oligomers. Mol Neurodegener 7, 42.
Emmanouilidou, E., Melachroinou, K., Roumeliotis, T., Garbis, S.D., Ntzouni, M., Margaritis, L.H., Stefanis, L., and Vekrellis, K. (2010). Cell-produced alpha-synuclein is secreted in a calcium-dependent manner by exosomes and impacts neuronal survival. J Neurosci 30, 6838-6851.
Fang, Y., Wu, N., Gan, X., Yan, W., Morrell, J.C., and Gould, S.J. (2007). Higher-order oligomerization targets plasma membrane proteins and HIV gag to exosomes. PLoS Biol 5, e158.
Feldman, M.E., Apsel, B., Uotila, A., Loewith, R., Knight, Z.A., Ruggero, D., and Shokat, K.M. (2009). Active-site inhibitors of nnTOR target rapamycin-resistant outputs of mTORC1 and mTORC2. PLoS Biol 7, e38.
Florey, 0., Kim, S.E., Sandoval, C.P., Haynes, C.M., and Overholtzer, M. (2011). Autophagy machinery mediates macroendocytic processing and entotic cell death by targeting single membranes. Nat Cell Biol 13, 1335-1343.
Friedl, P., and Alexander, S. (2011). Cancer invasion and the microenvironment: plasticity and reciprocity. Cell 147, 992-1009.
Fujita, N., Itoh, T., Omori, H., Fukuda, M., Noda, T., and Yoshimori, T. (2008). The Atg16L complex specifies the site of LC3 lipidation for membrane biogenesis in autophagy. Mol Biol Cell 19, 2092-2100.
Futter, C.E., Collinson, L.M., Backer, J.M., and Hopkins, C.R. (2001). Human VPS34 is required for internal vesicle formation within multivesicular endosomes. J Cell Biol 155, 1251-1264.
Gal, J., Strom, A.L., Kwinter, D.M., Kilty, R., Zhang, J., Shi, P., Fu, W., Wooten, M.W., and Zhu, H. (2009). Sequestosome 1/p62 links familial ALS mutant SOD1 to LC3 via an ubiquitin-independent mechanism. J Neurochem 111,1062-1073.
Gomes, C., Keller, S., Altevogt, P., and Costa, J. (2007). Evidence for secretion of Cu,Zn superoxide dismutase via exosomes from a cell model of amyotrophic lateral sclerosis. Neurosci Lett 428, 43-46.
Gordon, P.B., and Seglen, P.O. (1988). Prelysosomal convergence of autophagic and endocytic pathways. Biochem Biophys Res Commun 151, 40-47.
Grad, L.I., Yerbury, J.J., Turner, B.J., Guest, W.C., Pokrishevsky, E., O'Neill, M.A., Yanai, A., Silverman, J.M., Zeineddine, R., Corcoran, L., et al. (2014). Intercellular propagated misfolding of wild-type Cu/Zn superoxide dismutase occurs via exosome-dependent and -independent mechanisms. Proc Natl Acad Sci U S A 111, 3620-3625.
Gross, J.C., Chaudhary, V., Bartscherer, K., and Boutros, M. (2012). Active Wnt proteins are secreted on exosomes. Nat Cell Biol 14, 1036-1045.
Hanson, P.I., and Cashikar, A. (2012). Multivesicular body morphogenesis. Annu Rev Cell Dev Biol 28, 337-362.
Higginbotham, J.N., Demory Beckler, M., Gephart, J.D., Franklin, J.L., Bogatcheva, G., Kremers, G.J., Piston, D.W., Ayers, G.D., McConnell, R.E., Tyska, M.J., et al. (2011). Amphiregulin exosomes increase cancer cell invasion. Curr Biol 21, 779-786.
Huotari, J., and Helenius, A. (2011). Endosome maturation. EMBO J 30, 3481-3500.
Hurley, J.H., and Hanson, P.I. (2010). Membrane budding and scission by the ESCRT machinery: it's all in the neck. Nat Rev Mol Cell Biol 11, 556-566.
Johnson, E.E., Overmeyer, J.H., Gunning, W.T., and Maltese, W.A. (2006). Gene silencing reveals a specific function of hVps34 phosphatidylinositol 3-kinase in late versus early endosomes. J Cell Sci 119, 1219-1232.
Kabuta, T., Suzuki, Y., and Wada, K. (2006). Degradation of amyotrophic lateral sclerosis-linked mutant Cu,Zn-superoxide dismutase proteins by macroautophagy and the proteasome. J Biol Chem 281, 30524-30533.
Kenific, C.M., and Debnath, J. (2015). Cellular and metabolic functions for autophagy in cancer cells. Trends Cell Biol 25, 37-45.
Kim, H.J., Zhong, Q., Sheng, Z.H., Yoshimori, T., Liang, C., and Jung, J.U. (2012). Beclin-1-interacting autophagy protein Atg14L targets the SNARE-associated protein Snapin to coordinate endocytic trafficking. J Cell Sci 125, 4740-4750.
Kuusisto, E., Salminen, A., and Alafuzoff, I. (2001). Ubiquitin-binding protein p62 is present in neuronal and glial inclusions in human tauopathies and synucleinopathies. Neuroreport 12, 2085-2090.
Laulagnier, K., Motta, C., Hamdi, S., Roy, S., Fauvelle, F., Pageaux, J.F., Kobayashi, T., Salles, J.P., Perret, B., Bonnerot, C., et al. (2004). Mast cell- and dendritic cell-derived exosomes display a specific lipid composition and an unusual membrane organization. Biochem J 380,161-171.
Lotvall, J., Hill, A.F., Hochberg, F., Buzas, E.I., Di Vizio, D., Gardiner, C., Gho, Y.S., Kurochkin, I.V., Mathivanan, S., Quesenberry, P., et al. (2014). Minimal experimental requirements for definition of extracellular vesicles and their functions: a position statement from the International Society for Extracellular Vesicles. Journal of extracellular vesicles 3, 26913.
Martinez, J., Malireddi, R.K., Lu, Q., Cunha, L.D., Pelletier, S., Gingras, S., Orchard, R., Guan, J.L., Tan, H., Peng, J., et al. (2015). Molecular characterization of LC3-associated phagocytosis reveals distinct roles for Rubicon, NOX2 and autophagy proteins. Nat Cell Biol 17, 893-906.
Mathew, R., Karp, C.M., Beaudoin, B., Vuong, N., Chen, G., Chen, H.Y., Bray, K., Reddy, A., Bhanot, G., Gelinas, C., et al. (2009). Autophagy suppresses tumorigenesis through elimination of p62. Cell 137, 1062-1075.
Mathivanan, S., Lim, J.W., Tauro, B.J., Ji, H., Moritz, R.L., and Simpson, R.J. (2010). Proteomics analysis of A33 immunoaffinity-purified exosomes released from the human colon tumor cell line LIM1215 reveals a tissue-specific protein signature. Mol Cell Proteomics 9, 197-208.
Melo, S.A., Sugimoto, H., O'Connell, J.T., Kato, N., Villanueva, A., Vidal, A., Qiu, L., Vitkin, E., Perelman, L.T., Melo, C.A., et al. (2014). Cancer Exosomes Perform Cell-Independent MicroRNA Biogenesis and Promote Tumorigenesis. Cancer Cell 26, 707-721.
Menzies, F.M., Fleming, A., and Rubinsztein, D.C. (2015). Compromised autophagy and neurodegenerative diseases. Nat Rev Neurosci 16, 345-357.
Mizushima, N., Yoshimori, T., and Ohsumi, Y. (2011). The role of Atg proteins in autophagosome formation. Annu Rev Cell Dev Biol 27, 107-132.
Murrow, L., Malhotra, R., and Debnath, J. (2015). ATG12-ATG3 interacts with Alix to promote basal autophagic flux and late endosome function. Nat Cell Biol 1 7, 300-310.
Parolini, I., Federici, C., Raggi, C., Lugini, L., Palleschi, S., De Milito, A., Coscia, C., lessi, E., Logozzi, M., Molinari, A., et al. (2009). Microenvironmental pH is a key factor for exosome traffic in tumor cells. J Biol Chem 284, 34211-34222.
Peinado, H., Aleckovic, M., Lavotshkin, S., Matei, I., Costa-Silva, B., Moreno-Bueno, G., Hergueta-Redondo, M., Williams, C., Garcia-Santos, G., Ghajar, C., et al. (2012). Melanoma exosomes educate bone marrow progenitor cells toward a pro-metastatic phenotype through MET. Nat Med 18, 883-891.
Peng, J., Zhang, R., Cui, Y., Liu, H., Zhao, X., Huang, L., Hu, M., Yuan, X., Ma, B., Ma, X., et al. (2014). Atg5 regulates late endosome and lysosome biogenesis. Science China Life sciences 57, 59-68.
Puchner, E.M., Walter, J.M., Kasper, R., Huang, B., and Lim, W.A. (2013). Counting molecules in single organelles with superresolution microscopy allows tracking of the endosome maturation trajectory. Proc Natl Acad Sci U S A 110, 16015-16020.
Razi, M., and Futter, C.E. (2006). Distinct roles for Tsg101 and Hrs in multivesicular body formation and inward vesiculation. Mol Biol Cell 17, 3469-3483.
Romanov, J., Walczak, M., Ibiricu, I., Schuchner, S., Ogris, E., Kraft, C., and Martens, S. (2012). Mechanism and functions of membrane binding by the Atg5-Atg12/Atg16 complex during autophagosome formation. EMBO J 31, 4304-4317.
Romao, S., Gasser, N., Becker, A.C., Guhl, B., Bajagic, M., Vanoaica, D., Ziegler, U., Roesler, J., Dengjel, J., Reichenbach, J., et al. (2013). Autophagy proteins stabilize pathogen-containing phagosomes for prolonged MHC II antigen processing. J Cell Biol 203, 757-766.
Russell, R.C., Yuan, H.X., and Guan, K.L. (2014). Autophagy regulation by nutrient signaling. Cell Res 24, 42-57.
Sahu, R., Kaushik, S., Clement, C.C., Cannizzo, E.S., Scharf, B., Follenzi, A., Potolicchio, I., Nieves, E., Cuervo, A.M., and Santambrogio, L. (2011). Microautophagy of cytosolic proteins by late endosomes. Dev Cell 20, 131-139.
Saitoh, T., Fujita, N., Jang, M.H., Uematsu, S., Yang, B.G., Satoh, T., Omori, H., Noda, T., Yamamoto, N., Komatsu, M., et al. (2008). Loss of the autophagy protein Atg16L1 enhances endotoxin-induced IL-lbeta production. Nature 456, 264-268.
Sanjuan, M.A., Dillon, C.P., Tait, S.W., Moshiach, S., Dorsey, F., Connell, S., Komatsu, M., Tanaka, K., Cleveland, J.L., Withoff, S., et al. (2007). Toll-like receptor signalling in macrophages links the autophagy pathway to phagocytosis. Nature 450, 1253-1257.
Shrivastava, S., Devhare, P., Sujijantarat, N., Steele, R., Kwon, Y.C., Ray, R., and Ray, R.B. (2015). Knockdown of autophagy inhibits infectious hepatitis C virus release by exosomal pathway. J Virol.
Thery, C., Amigorena, S., Raposo, G., and Clayton, A. (2006). Isolation and characterization of exosomes from cell culture supernatants and biological fluids. Curr Protoc Cell Biol Chapter 3, Unit 3 22.
Tooze, S.A., Abada, A., and Elazar, Z. (2014). Endocytosis and autophagy: exploitation or cooperation? Cold Spring Harb Perspect Biol 6, a018358.
Trajkovic, K., Hsu, C., Chiantia, S., Rajendran, L., Wenzel, D., Wieland, F., Schwille, P., Brugger, B., and Simons, M. (2008). Ceramide triggers budding of exosome vesicles into multivesicular endosomes. Science 319, 1244-1247.
van Deurs, B., Holm, P.K., and Sandvig, K. (1996). Inhibition of the vacuolar H(+)-ATPase with bafilomycin reduces delivery of internalized molecules from mature multivesicular endosomes to lysosomes in HEp-2 cells. EurJ Cell Biol 69, 343-350.
van Weert, A.W., Dunn, K.W., Geuze, H.J., Maxfield, F.R., and Stoorvogel, W. (1995). Transport from late endosomes to lysosomes, but not sorting of integral membrane proteins in endosomes, depends on the vacuolar proton pump. J Cell Biol 130, 821-834.
Vidal, M., Mangeat, P., and Hoekstra, D. (1997). Aggregation reroutes molecules from a recycling to a vesicle-mediated secretion pathway during reticulocyte maturation. J Cell Sci 110 (Pt 16), 1867-1877.
Watanabe, Y., Tatebe, H., Taguchi, K., Endo, Y., Tokuda, T., Mizuno, T., Nakagawa, M., and Tanaka, M. (2012). p62/SQSTM1-dependent autophagy of Lewy body-like alpha-synuclein inclusions. PLoS One 7, e52868.
Webb, J.L., Ravikumar, B., Atkins, J., Skepper, J.N., and Rubinsztein, D.C. (2003). Alpha-Synuclein is degraded by both autophagy and the proteasome. J Biol Chem 278, 25009-25013.
Wemmer, M., Azmi, I., West, M., Davies, B., Katzmann, D., and Odorizzi, G. (2011). Bro1 binding to Snf7 regulates ESCRT-III membrane scission activity in yeast. J Cell Biol 192, 295-306.
White, I.J., Bailey, L.M., Aghakhani, M.R., Moss, S.E., and Futter, C.E. (2006). EGF stimulates annexin 1-dependent inward vesiculation in a multivesicular endosome subpopulation. EMBO J 25, 1-12.
Wubbolts, R., Leckie, R.S., Veenhuizen, P.T., Schwarzmann, G., Mobius, W., Hoernschemeyer, J., Slot, J.W., Geuze, H.J., and Stoorvogel, W. (2003). Proteomic and biochemical analyses of human B cellderived exosomes. Potential implications for their function and multivesicular body formation. J Biol Chem 278, 10963-10972.
Zhou, J., Tan, S.H., Nicolas, V., Bauvy, C., Yang, N.D., Zhang, J., Xue, Y., Codogno, P., and Shen, H.M. (2013). Activation of lysosomal function in the course of autophagy via mTORC1 suppression and autophagosome-lysosome fusion. Cell Res 23, 508-523.
Birgisdottir, A.B., Lamark, T., and Johansen, T. (2013). The LIR motif - crucial for selective autophagy. J Cell Sci 126, 3237-3247.
Cheong, H., Lindsten, T., Wu, J., Lu, C., and Thompson, C.B. (2011). Ammonia-induced autophagy is independent of ULK1/ULK2 kinases. Proc Natl Acad Sci USA 108,11121-11126.
Chivet, M., Javalet, C., Laulagnier, K., Blot, B., Hemming, F.J., and Sadoul, R. (2014). Exosomes secreted by cortical neurons upon glutamatergic synapse activation specifically interact with neurons. Journal of extracellular vesicles 3, 24722.
Gibbings, D., Mostowy, S., Jay, F., Schwab, Y., Cossart, P., Voinnet, 0. (2012). Selective autophagy degrades DICER and AGO2 and regulates miRNA activity. Nature Cell Biology in press.
Hosokawa, N., Hara, Y., and Mizushima, N. (2007). Generation of cell lines with tetracycline-regulated autophagy and a role for autophagy in controlling cell size. FEBS Lett 581, 2623-2629.
Huotari, J., and Helenius, A. (2011). Endosome maturation. EMBO J 30, 3481-3500.
Kuma, A., Hatano, M., Matsui, M., Yamamoto, A., Nakaya, H., Yoshinori, T., Ohsumi, Y., Tokuhisa, T., and Mizushima, N. (2004). The role of autophagy during the early neonatal starvation period. Nature 432, 1032-1036.
Laulagnier, K., Javalet C., Hemming, F.J., Sadoul, R. (2015). Purification and analysis of exosomes released by mature cortical neurons following synaptic activation. Methods in Molecular Biology.
Li, Q., Lau, A., Morris, T.J., Guo, L., Fordyce, C.B., and Stanley, E.F. (2004). A syntaxin 1, Galpha(o), and Ntype calcium channel complex at a presynaptic nerve terminal: analysis by quantitative immunocolocalization. J Neurosci 24, 4070-4081.
Luft, J.H. (1961). Improvements in epoxy resin embedding methods. The Journal of biophysical and biochemical cytology 9, 409-414.
Murk, J.L., Humbel, B.M., Ziese, U., Griffith, J.M., Posthuma, G., Slot, J.W., Koster, A.J., Verkleij, A.J., Geuze, H.J., and Kleijmeer, M.J. (2003). Endosomal compartmentalization in three dimensions: implications for membrane fusion. Proc Natl Acad Sci USA 100,13332-13337.
Ostrowski, M., Carmo, N.B., Krumeich, S., Fanget, I., Raposo, G., Savina, A., Moita, C.F., Schauer, K., Hume, A.N., Freitas, R.P., et al. (2010). Rab27a and Rab27b control different steps of the exosome secretion pathway. Nat Cell Biol 12, 19-30; sup pp 11-13.
Pankiv, S., Clausen, T.H., Lanark, T., Brech, A., Bruun, J.A., Outzen, H., Overvatn, A., Bjorkoy, G., and Johansen, T. (2007). p62/SQSTM1 binds directly to Atg8/LC3 to facilitate degradation of ubiquitinated protein aggregates by autophagy. The Journal of biological chemistry 282, 24131-24145.
Rizk, A., Paul, G., lncardona, P., Bugarski, M., Mansouri, M., Niemann, A., Ziegler, U., Berger, P., and Sbalzarini, I.F. (2014). Segmentation and quantification of subcellular structures in fluorescence microscopy images using Squassh. Nat Protoc 9, 586-596.
Saitoh, T., Fujita, N., Jang, M.H., Uematsu, S., Yang, B.G., Satoh, T., Omori, H., Noda, T., Yamamoto, N., Komatsu, M., et al. (2008). Loss of the autophagy protein Atg16L1 enhances endotoxin-induced IL-lbeta production. Nature 456, 264-268.
Thery, C., Amigorena, S., Raposo, G., and Clayton, A. (2006). Isolation and characterization of exosomes from cell culture supernatants and biological fluids. Curr Protoc Cell Biol Chapter 3, Unit 3 22.
Vanlandingham, P.A., and Ceresa, B.P. (2009). Rab7 regulates late endocytic trafficking downstream of multivesicular body biogenesis and cargo sequestration. J Biol Chem 284, 12110-12124.
Vidal, M., Mangeat, P., and Hoekstra, D. (1997). Aggregation reroutes molecules from a recycling to a vesicle-mediated secretion pathway during reticulocyte maturation. J Cell Sci 110 (Pt 16), 1867-1877.
Zheng, Y.Z., Boscher, C., Inder, K.L., Fairbank, M., Loo, D., Hill, M.M., Nabi, I.R., and Foster, L.J. (2011). Differential impact of caveolae and caveolin-1 scaffolds on the membrane raft proteome. Mol Cell Proteomics 10, M110 007146.

## Claims

1. A fusion construct comprising a biologically active agent functionally linked with a functional moiety comprising an LC3 or GABARAP protein, wherein the biologically active agent comprises an antibody, an antigen-binding fragment thereof, a single-chain variable fragment (scFv), or a nanobody which specifically binds a cellular target comprising misfolded proteins, protein inclusions, protein aggregates, lipid droplets, or mitochondria.

2. The fusion construct of claim 1, wherein the biologically active agent comprises an scFv or a nanobody from a camel, llama, shark, or other animal.

3. The fusion construct of claim 1 or 2, wherein the cellular target comprises TDP-43 inclusions found in amyotrophic lateral sclerosis (ALS) patients; Tau fibrils found in Alzheimer's, chronic traumatic encephalophathy (CTE), corticobasal degeneration, progressive supranuclear palsy, and/or other Tauopathies patients; synuclein, alpha-synuclein, Lewy bodies, and/or damaged mitochondria found in Parkinson's patients; Htt repeats in Huntington's disease; dipeptide repeats produced by C9ORF72 intronic repeats; misfolded SOD1; and/or hyperphosphorylated or fibrillary Tau.

4. The fusion construct of any one of claims 1-3, wherein the biologically active agent is functionally linked with the functional moiety directly, or indirectly via one or more linkers and/or intervening groups; preferably wherein the biologically active agent is functionally linked to an N-terminal portion or end of the functional moiety, or wherein the biologically active agent is functionally linked to a C-terminal portion or end of the functional moiety.

5. The fusion construct of any one of claims 1-4, wherein the LC3 or GABARAP protein comprises an LC3A, LC3B, LC3C, GABARAP, GABARAPL1, GABARAPL2, or GABARAPL3 homologue; more preferably wherein the LC3 or GABARAP protein comprises LC3A, or wherein the LC3 or GABARAP protein comprises LC3B.

6. The fusion construct of any one of claims 1-5, wherein the biologically active agent comprises anti-TDP-43 scFv clone VH7; anti-TDP-43 scFv 41Dl; anti-Tau scFv 4A3; anti-Tau scFv 4E4; adipophilin; perilipin2; PINK1; or Parkin.

7. A nucleic acid encoding the fusion construct of any one of claims 1-6, or an expression vector for expressing the fusion construct of any one of claims 1-6, or a host cell comprising the nucleic acid or the expression vector and expressing the fusion construct of any one of claims 1-6; preferably wherein the host cell produces exosomes comprising the fusion construct of any one of claims 1-6; more preferably wherein the host cell has knockdown of Atg7; still more preferably wherein the host cell is Atg7^{-/-}; even more preferably wherein the host cell is a 293 cell, a human neonatal fibroblast, an NSC-34 cell, an SH5Y cell, or a BV2 cell.

8. An exosome comprising the fusion construct of any one of claims 1-6.

9. A composition comprising one or more of the fusion construct of any one of claims 1-6; the nucleic acid, expression vector, or host cell of claim 7; and/or the exosome of claim 8; and further comprising an autophagy-activating agent, an siRNA or other gene silencing agent, or both; preferably wherein the autophagy-activating agent comprises an mTOR inhibitor; more preferably wherein the autophagy-activating agent comprises one or more of rapamycin, sirolimus, eversolimus, tacrolimus, INK128, pp242, starvation, or other mTORC1 and/or mTORC inhibitor; even more preferably wherein the autophagy-activating agent comprises Trehalose and/or Beclin1 peptide.

10. A method for packaging the fusion construct of any one of claims 1-6 into an exosome, said method comprising:
expressing the fusion construct in an exosome-producing cell or otherwise introducing the fusion construct into the exosome-producing cell; and
culturing the exosome-producing cell in a cell media under conditions in which the exosome-producing cell generates and secretes exosomes comprising the fusion construct into the cell media;
preferably wherein the exosome-producing cell has knockdown of Atg7; more preferably wherein the exosome-producing cell is Atg7^{-/-}; even more preferably wherein the exosome-producing cell is a 293 cell, a human neonatal fibroblast, an NSC-34 cell, an SH5Y cell, or a BV2 cell.

11. An in vitro method for delivering a biologically active agent into a cell, said method comprising:
expressing or introducing the fusion construct of any one of claims 1-6 into an exosome-producing cell;
culturing the exosome-producing cell in a cell media under conditions in which the exosome-producing cell generates and secretes exosomes comprising the fusion construct into the cell media;
obtaining, isolating, or purifying the secreted exosomes comprising the fusion construct from the cell media; and
contacting the cell with the secreted exosomes;
preferably, wherein the exosome-producing cell is a 293 cell, a human neonatal fibroblast, an NSC-34 cell, an SH5Y cell, or a BV2 cell;
more preferably wherein the exosome-producing cell is a 293 cell, and the cell is a liver cell, a fibroblast cell, or a motor neuron; or
wherein the exosome-producing cell is a human neonatal fibroblast, and the cell is a liver cell, a brain cell, a spinal cord cell, or a kidney cell; or
wherein the exosome-producing cell is an NSC-34 cell, and the cell is a liver cell, a small intestine cell, a brain cell, or a spinal cord cell; or
wherein the exosome-producing cell is an SH5Y cell, and the cell is a liver cell, a kidney cell, or a brain cell.

12. The construct of any one of claims 1-6; the nucleic acid, expression vector, or host cell of claim 7; the exosome of claim 8; the composition of claim 9; or any combination thereof; for use in treating or preventing a disease or disorder in a subject in need thereof; preferably wherein the disease or disorder is caused by, or associated with, any one or more of misfolded proteins, protein inclusions, protein aggregates, lipid droplets, or mitochondria; more preferably wherein the disease or disorder is a neurodegenerative disease or atherosclerosis; even more preferably wherein the disease or disorder is Alzheimer's, CTE, and/or Tauopathy-related disease; Parkinson's; frontal temporal dementia; and/or ALS.

13. The construct for use of claim 12, wherein the fusion construct, the nucleic acid, the expression vector, the host cell, the exosome, the composition, or any combination thereof, is for administration in combination with an autophagy-activating agent, a gene silencing agent, or both; preferably wherein the autophagy-activating agent comprises an mTOR inhibitor; more preferably wherein the autophagy-activating agent comprises one or more of rapamycin, sirolimus, eversolimus, tacrolimus, INK128, pp242, starvation, or other mTORC1 and/or mTORC inhibitor; even more preferably wherein the autophagy-activating agent comprises Trehalose and/or Beclin1 peptide.

## Patentansprüche

1. Fusionskonstrukt, umfassend ein biologisch aktives Agens, das funktionsmäßig mit einer funktionellen Gruppierung, die ein LC3- oder GABARAP-Protein umfasst, verknüpft ist, wobei das biologisch aktive Agens einen Antikörper, ein antigenbindendes Fragment davon, ein einkettiges variables Fragment (scFv= single-chain variable fragment) oder einen Nanokörper umfasst, der/das spezifisch an ein zelluläres Ziel, das falsch gefaltete Proteine, Proteineinschlüsse, Proteinaggregate, Lipidtröpfchen oder Mitochondrien umfasst, bindet.

2. Fusionskonstrukt nach Anspruch 1, wobei das biologisch aktive Agens ein scFv oder einen Nanokörper von einem Kamel, Lama, Hai oder anderen Tier umfasst.

3. Fusionskonstrukt nach Anspruch 1 oder 2, wobei das zelluläre Ziel TDP-43-Einschlüsse, die bei Patienten mit amyotropher Lateralsklerose (ALS) gefunden werden, Tau-Fibrillen, die bei Patienten mit Morbus Alzheimer, chronischer traumatischer Encephalopathie (CTE), kortikobasaler Degeneration, progressiver supranukleärer Blickparese und/oder anderen Tauopathien gefunden werden, Synuclein, Alpha-Synuclein, Lewy-Körperchen und/oder geschädigte Mitochondrien, die bei Patienten mit Morbus Parkinson gefunden werden, repetitive Htt-Sequenzen bei Chorea Huntington, repetitive Dipeptidsequenzen, die durch repetitive C9ORF72-Intronsequenzen entstehen, falsch gefaltetes SOD1 und/oder hyperphosphoryliertes oder fibrilläres Tau umfasst.

4. Fusionskonstrukt nach einem der Ansprüche 1-3, wobei das biologisch aktive Agens funktionsmäßig mit der funktionellen Gruppierung direkt oder indirekt über einen oder mehrere Linker und/oder intervenierende Gruppen verknüpft ist; wobei vorzugsweise das biologisch aktive Agens funktionsmäßig mit einem N-terminalen Teil oder Ende der funktionellen Gruppierung verknüpft ist oder wobei das biologisch aktive Agens funktionsmäßig mit einem C-terminalen Teil oder Ende der funktionellen Gruppierung verknüpft ist.

5. Fusionskonstrukt nach einem der Ansprüche 1-4, wobei das LC3- oder GABARAP-Protein die Homologe LC3A, LC3B, LC3C, GABARAP, GABARAPL1, GABARAPL2 bzw. GABARAPL3 umfasst; wobei bevorzugter das LC3- oder GABARAP-Protein LC3A umfasst oder wobei das LC3- oder GABARAP-Protein LC3B umfasst.

6. Fusionskonstrukt nach einem der Ansprüche 1-5, wobei das biologisch aktive Agens Anti-TDP-43-scFv-Klon VH7, Anti-TDP-43-scFv 41D1, Anti-Tau-scFv 4A3, Anti-Tau-scFv 4E4, Adipophilin, Perilipin2, PINK1 oder Parkin umfasst.

7. Nukleinsäure, codierend das Fusionskonstrukt nach einem der Ansprüche 1-6, oder Expressionsvektor zum Exprimieren des Fusionskonstrukts nach einem der Ansprüche 1-6 oder Wirtszelle, umfassend die Nukleinsäure bzw. den Expressionsvektor und exprimierend das Fusionskonstrukt nach einem der Ansprüche 1-6; wobei bevorzugt die Wirtszelle Exosomen produziert, die das Fusionskonstrukt nach einem der Ansprüche 1-6 umfassen; wobei bevorzugter die Wirtszelle ein Atg7-Knockdown aufweist; wobei noch bevorzugter die Wirtszelle Atg7^{-/-} ist; wobei noch mehr bevorzugt es sich bei der Wirtszelle um eine 293-Zelle, einen menschlichen neonatalen Fibroblasten, eine NSC-34-Zelle, eine SH5Y-Zelle oder eine BV2-Zelle handelt.

8. Exosom, umfassend das Fusionskonstrukt nach einem der Ansprüche 1-6.

9. Zusammensetzung, umfassend ein oder mehrere von dem Fusionskonstrukt nach einem der Ansprüche 1-6, der Nukleinsäure, dem Expressionsvektor bzw. der Wirtszelle nach Anspruch 7 und/oder dem Exosom nach Anspruch 8 und weiter umfassend ein Autophagie aktivierendes Agens, eine siRNA oder anderes Gen-silencing-Agens oder beide; wobei bevorzugt das Autophagie aktivierende Agens einen mTOR-Hemmer umfasst; wobei bevorzugter das Autophagie aktivierende Agens ein oder mehrere der Folgenden umfasst: Rapamycin, Sirolimus, Eversolimus, Tacrolimus, INK128, pp242, Verhungern oder andere mTORC1- und/oder mTORC-Hemmer; wobei noch bevorzugter das Autophagie aktivierende Agens Trehalose und/oder Beclin1-Peptid umfasst.

10. Verfahren zum Verpacken des Fusionskonstrukts nach einem der Ansprüche 1-6 in ein Exosom, wobei das Verfahren Folgendes umfasst:
Exprimieren des Fusionskonstrukts in einer Exosomen produzierenden Zelle oder anderweitiges Einführen des Fusionskonstrukts in die Exosomen produzierende Zelle; und
Kultivieren der Exosomen produzierenden Zelle in einem Zellmedium unter Bedingungen, unter welchen die Exosomen produzierende Zelle Exosomen, die das Fusionskonstrukt umfassen, erzeugt und in das Zellmedium sezerniert;
wobei bevorzugt die Exosomen produzierende Zelle ein Atg7-Knockdown aufweist; wobei bevorzugter die Exosomen produzierende Zelle Atg7^{-/-} ist; wobei noch bevorzugter es sich bei der Exosomen produzierenden Zelle um eine 293-Zelle, einen menschlichen neonatalen Fibroblasten, eine NSC-34-Zelle, eine SH5Y-Zelle oder eine BV2-Zelle handelt.

11. In-vitro-Verfahren zum Zuführen eines biologisch aktiven Agens in eine Zelle, wobei das Verfahren Folgendes umfasst:
Exprimieren oder Einführen des Fusionskonstrukts nach einem der Ansprüche 1-6 in eine Exosomen produzierende Zelle;
Kultivieren der Exosomen produzierenden Zelle in einem Zellmedium unter Bedingungen, unter welchen die Exosomen produzierende Zelle Exosomen, die das Fusionskonstrukt umfassen, erzeugt und in das Zellmedium sezerniert;
Gewinnen, Isolieren oder Aufreinigen der sezernierten Exosomen, die das Fusionskonstrukt umfassen, aus dem Zellmedium; und
In-Kontakt-Bringen der Zelle mit den sezernierten Exosomen;
wobei bevorzugt es sich bei der Exosomen produzierenden Zelle um eine 293-Zelle, einen menschlichen neonatalen Fibroblasten, eine NSC-34-Zelle, eine SH5Y-Zelle oder eine BV2-Zelle handelt;
wobei bevorzugter es sich bei der Exosomen produzierenden Zelle um eine 293-Zelle handelt und die Zelle eine Leberzelle, ein Fibroblast oder eine motorische Nervenzelle ist; oder
wobei es sich bei der Exosomen produzierenden Zelle um einen menschlichen neonatalen Fibroblasten handelt und die Zelle eine Leberzelle, eine Hirnzelle, eine Rückenmarkzelle oder eine Nierenzelle ist; oder
wobei es sich bei der Exosomen produzierenden Zelle um eine NSC-34-Zelle handelt und die Zelle eine Leberzelle, eine Zelle des Dünndarms, eine Hirnzelle oder eine Rückenmarkzelle ist; oder
wobei es sich bei der Exosomen produzierenden Zelle um eine SH5Y-Zelle handelt und die Zelle eine Leberzelle, eine Nierenzelle oder eine Hirnzelle ist.

12. Konstrukt nach einem der Ansprüche 1-6; Nukleinsäure, Expressionsvektor oder Wirtszelle nach Anspruch 7; Exosom nach Anspruch 8; Zusammensetzung nach Anspruch 9 oder Kombination davon zur Verwendung beim Behandeln oder Vorbeugen einer Krankheit oder Störung bei einem Individuum, das daran Bedarf hat; wobei bevorzugt die Krankheit bzw. Störung von einem oder mehreren der Folgenden verursacht wird oder damit assoziiert ist: falsch gefaltete Proteine, Proteineinschlüsse, Proteinaggregate, Lipidtröpfchen oder Mitochondrien; wobei bevorzugter es sich bei der Krankheit bzw. Störung um eine neurodegenerative Krankheit oder Atherosklerose handelt; wobei noch bevorzugter es sich bei der Krankheit bzw. Störung um Morbus Alzheimer, CTE und/oder eine mit Tauopathie verwandte Krankheit, Morbus Parkinson, frontotemporale Demenz und/oder ALS handelt.

13. Konstrukt zur Verwendung nach Anspruch 12, wobei das Fusionskonstrukt, die Nukleinsäure, der Expressionsvektor, die Wirtszelle, das Exosom, die Zusammensetzung oder eine Kombination davon zur Verabreichung in Kombination mit einem Autophagie aktivierenden Agens, einem Gen-silencing-Agens oder beidem vorgesehen ist; wobei bevorzugt das Autophagie aktivierende Agens einen mTOR-Hemmer umfasst; wobei bevorzugter das Autophagie aktivierende Agens eines oder mehrere der Folgenden umfasst: Rapamycin, Sirolimus, Eversolimus, Tacrolimus, INK128, pp242, Verhungern oder andere mTORC1- und/oder mTORC-Hemmer; wobei noch bevorzugter das Autophagie aktivierende Agens Trehalose und/oder Beclin1-Peptid umfasst.

## Revendications

1. Construction de fusion comprenant un agent biologiquement actif fonctionnellement lié à un groupement fonctionnel comprenant une protéine LC3 ou GABARAP, dans laquelle l'agent biologiquement actif comprend un anticorps, un fragment de liaison à un antigène de celui-ci, un fragment variable monocaténaire (scFv) ou un nanocorps qui se lie spécifiquement à une cible cellulaire comprenant des protéines mal repliées, des inclusions de protéines, des agrégats de protéines, des gouttelettes de lipides ou des mitochondries.

2. Construction de fusion selon la revendication 1, dans laquelle l'agent biologiquement actif comprend un scFv ou un nanocorps provenant d'un chameau, d'un lama, d'un requin ou d'un autre animal.

3. Construction de fusion selon la revendication 1 ou 2, dans laquelle la cible cellulaire comprend des inclusions TDP-43 trouvées chez les patients atteints de sclérose latérale amyotrophique (SLA) ; des fibrilles Tau trouvées chez les patients atteints d'Alzheimer, d'encéphalopathie traumatique chronique (ETC), de dégénérescence corticobasale, de paralysie supranucléaire progressive et/ou d'autres tauopathies ; de la synucléine, de l'alpha-synucléine, des corps de Lewy et/ou des mitochondries endommagées trouvées chez les patients atteints de la maladie de Parkinson ; des répétitions Htt dans la maladie de Huntington ; des répétitions de dipeptides produites par des répétitions introniques C9ORF72 ; de la SOD1 mal repliée ; et/ou de la Tau hyperphosphorylée ou fibrillaire.

4. Construction de fusion selon l'une quelconque des revendications 1 à 3, dans laquelle l'agent biologiquement actif est fonctionnellement lié au groupement fonctionnel directement, ou indirectement via un ou plusieurs lieurs et/ou groupes intermédiaires ; de préférence dans laquelle l'agent biologiquement actif est fonctionnellement lié à une partie N-terminale ou une extrémité du groupement fonctionnel, ou dans laquelle l'agent biologiquement actif est fonctionnellement lié à une partie C-terminale ou une extrémité du groupement fonctionnel.

5. Construction de fusion selon l'une quelconque des revendications 1 à 4, dans laquelle la protéine LC3 ou GABARAP comprend un homologue de LC3A, LC3B, LC3C, GABARAP, GABARAPL1, GABARAPL2 ou GABARAPL3 ; plus préférablement dans laquelle la protéine LC3 ou GABARAP comprend LC3A, ou dans laquelle la protéine LC3 ou GABARAP comprend LC3B.

6. Construction de fusion selon l'une quelconque des revendications 1 à 5, dans laquelle l'agent biologiquement actif comprend le clone VH7 du scFv anti-TDP-43 ; le scFv anti-TDP-43 41Dl ; le scFv anti-Tau 4A3 ; le scFv anti-Tau 4E4 ; l'adipophiline ; la périlipine 2 ; PINK1 ; ou Parkine.

7. Acide nucléique codant pour la construction de fusion selon l'une quelconque des revendications 1 à 6, ou vecteur d'expression pour exprimer la construction de fusion selon l'une quelconque des revendications 1 à 6, ou cellule hôte comprenant l'acide nucléique ou le vecteur d'expression et exprimant la construction de fusion selon l'une quelconque des revendications 1 à 6 ; de préférence, la cellule hôte produisant des exosomes comprenant la construction de fusion selon l'une quelconque des revendications 1 à 6 ; plus préférablement, la cellule hôte ayant un knockdown d'Atg7 ; encore plus préférablement la cellule hôte étant Atg7^{-/-} ; encore plus préférablement, la cellule hôte étant une cellule 293, un fibroblaste néonatal humain, une cellule NSC-34, une cellule SH5Y ou une cellule BV2.

8. Exosome comprenant la construction de fusion selon l'une quelconque des revendications 1 à 6.

9. Composition comprenant une ou plusieurs des constructions de fusion selon l'une quelconque des revendications 1-6 ; acide nucléique, vecteur d'expression ou cellule hôte selon la revendication 7 ; et/ou exosome selon la revendication 8 ; et comprenant en outre un agent activateur d'autophagie, un ARNsi ou un autre agent de silençage de gène, ou les deux ; de préférence l'agent activateur d'autophagie comprenant un inhibiteur de mTOR ; plus préférablement l'agent activateur d'autophagie comprenant un ou plusieurs parmi rapamycine, sirolimus, eversolimus, tacrolimus, INK128, pp242, famine ou un autre inhibiteur de mTORC1 et/ou mTORC ; encore plus préférablement, l'agent activateur d'autophagie comprenant le tréhalose et/ou le peptide Beclin1.

10. Procédé pour encapsuler la construction de fusion selon l'une quelconque des revendications 1-6 dans un exosome, ledit procédé comprenant :
l'expression de la construction de fusion dans une cellule productrice d'exosomes ou l'introduction autrement de la construction de fusion dans la cellule productrice d'exosomes ; et
la culture de la cellule productrice d'exosomes dans un milieu cellulaire dans des conditions où la cellule productrice d'exosomes génère et sécrète des exosomes comprenant la construction de fusion dans le milieu cellulaire ;
de préférence, la cellule productrice d'exosomes présente un knockdown d'Atg7 ; plus préférablement, la cellule productrice d'exosomes est Atg7^{-/-} ; encore plus préférablement, la cellule productrice d'exosomes est une cellule 293, un fibroblaste néonatal humain, une cellule NSC-34, une cellule SH5Y ou une cellule BV2.

11. Procédé *in vitro* pour délivrer un agent biologiquement actif dans une cellule, ledit procédé comprenant :
l'expression ou l'introduction de la construction de fusion selon l'une quelconque des revendications 1-6 dans une cellule productrice d'exosomes ;
la culture de la cellule productrice d'exosomes dans un milieu cellulaire dans des conditions où la cellule productrice d'exosomes génère et sécrète des exosomes comprenant la construction de fusion dans le milieu cellulaire ;
l'obtention, l'isolement ou la purification des exosomes sécrétés comprenant la construction de fusion à partir du milieu cellulaire ; et
la mise en contact de la cellule avec les exosomes sécrétés ;
de préférence, dans lequel la cellule productrice d'exosomes est une cellule 293, un fibroblaste néonatal humain, une cellule NSC-34, une cellule SH5Y ou une cellule BV2 ;
plus préférablement, dans lequel la cellule productrice d'exosomes est une cellule 293 et la cellule est une cellule hépatique, une cellule fibroblastique ou un motoneurone ; ou
dans lequel la cellule productrice d'exosomes est un fibroblaste néonatal humain et la cellule est une cellule hépatique, une cellule cérébrale, une cellule de la moelle épinière ou une cellule rénale ; ou
dans lequel la cellule productrice d'exosomes est une cellule NSC-34 et la cellule est une cellule hépatique, une cellule de l'intestin grêle, une cellule cérébrale ou une cellule de la moelle épinière ; ou
dans lequel la cellule productrice d'exosomes est une cellule SH5Y, et la cellule est une cellule hépatique, une cellule rénale ou une cellule cérébrale.

12. Construction selon l'une quelconque des revendications 1 à 6 ; acide nucléique, vecteur d'expression ou cellule hôte selon la revendication 7 ; exosome selon la revendication 8 ; composition selon la revendication 9 ; ou toute combinaison de ceux-ci ; pour une utilisation dans le traitement ou la prévention d'une maladie ou d'un trouble chez un sujet en ayant besoin ; de préférence dans laquelle la maladie ou le trouble est provoqué par, ou associé à, une ou plusieurs protéines mal repliées, inclusions protéiques, agrégats protéiques, gouttelettes de lipides ou mitochondries ; plus préférablement, dans laquelle la maladie ou le trouble est une maladie neurodégénérative ou l'athérosclérose ; encore plus préférablement, dans laquelle la maladie ou le trouble est la maladie d'Alzheimer, de l'ETC et/ou une maladie liée à la Tauopathie ; la maladie de Parkinson ; la démence frontale temporale ; et/ou la SLA.

13. Construction pour une utilisation selon la revendication 12, dans laquelle la construction de fusion, l'acide nucléique, le vecteur d'expression, la cellule hôte, l'exosome, la composition ou toute combinaison de ceux-ci est destiné à être administré en combinaison avec un agent activateur d'autophagie, un agent de silençage de gène ou les deux ; de préférence dans laquelle l'agent activateur d'autophagie comprend un inhibiteur de mTOR ; plus préférablement dans laquelle l'agent activateur d'autophagie comprend un ou plusieurs éléments parmi la rapamycine, le sirolimus, l'eversolimus, le tacrolimus, l'INK128, le pp242, la famine ou un autre inhibiteur de mTORC1 et/ou de mTORC ; encore plus préférablement dans laquelle l'agent activateur d'autophagie comprend le tréhalose et/ou le peptide Beclin1.
